(19) Europäisches Patentamt
European Patent Office
Office européen des brevets

(11) EP 2 255 787 A1

(12) EUROPEAN PATENT APPLICATION
published in accordance with Art. 153(4) EPC

(43) Date of publication:
**01.12.2010 Bulletin 2010/48**

(21) Application number: **09713262.5**

(22) Date of filing: **18.02.2009**

(51) Int Cl.:
***A61K 9/127*** (2006.01) ***A61K 31/22*** (2006.01)
***A61K 31/551*** (2006.01) ***A61K 31/573*** (2006.01)
***A61K 31/663*** (2006.01) ***A61K 31/722*** (2006.01)
***A61K 31/727*** (2006.01) ***A61K 33/18*** (2006.01)
***A61K 33/24*** (2006.01) ***A61K 38/00*** (2006.01)
***A61K 38/46*** (2006.01) ***A61K 47/24*** (2006.01)
***A61K 47/26*** (2006.01) ***A61K 47/42*** (2006.01)
***A61K 48/00*** (2006.01)

(86) International application number:
**PCT/JP2009/052814**

(87) International publication number:
**WO 2009/104649 (27.08.2009 Gazette 2009/35)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **22.02.2008 JP 2008042262**

(71) Applicants:
- **Katayama Chemical Industries Co., Ltd.**
  **Osaka-shi**
  **Osaka 541-0045 (JP)**
- **Gifu University**
  **Gifu-shi**
  **Gifu 501-1193 (JP)**

(72) Inventors:
- **KISO, Makoto**
  **Gifu-shi**
  **Gifu 501-1193 (JP)**
- **ISHIDA, Hideharu**
  **Gifu-shi**
  **Gifu 501-1193 (JP)**
- **YAMASHITA, Taiji**
  **Gifu-shi**
  **Gifu 501-1193 (JP)**
- **IGARASHI, Koichi**
  **Mino-shi**
  **Osaka 562-0015 (JP)**
- **HIRAI, Masahiko**
  **Mino-shi**
  **Osaka 562-0015 (JP)**

(74) Representative: **UEXKÜLL & STOLBERG**
**Patentanwälte**
**Beselerstrasse 4**
**22607 Hamburg (DE)**

# (54) SYNTHETIC GLYCOLIPID-CONTAINING LIPOSOMES

(57) The present invention provides a glycolipid-containing liposome. In such a glycolipid-containing liposome, the glycolipid includes a plant ceramide portion and a sugar chain portion. The present invention also provides a method of producing a glycolipid-containing liposome. This method includes the following steps of: A) providing a glycolipid in which the glycolipid includes a plant ceramide portion and a sugar chain portion; and B) mixing the provided glycolipid with a liposome raw material and subjecting the mixture to conditions in which a liposome is formed.

Fig.4

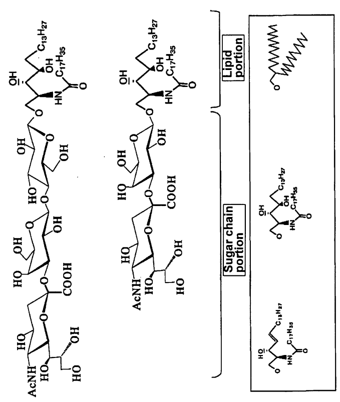


EP 2 255 787 A1

## Description

TECHNICAL FIELD

The present invention relates to a liposome using a synthesized glycolipid and a method of producing the same. The present invention particularly relates to a liposome using synthetic GM3 and synthetic GM4 and a method of synthesizing the same.

The present invention also relates to a liposome using a glycopilid including a plant ceramide portion and a sugar chain portion and a method of producing the same.

BACKGROUND ART

Currently, a liposome with a liposomal membrane surface bound to a probe (for example, sugar chain, antibody or the like) recognizing a target site has been developed as a drug delivery system (DDS) (Patent Documents 1-4 and Non-Patent Literature 1). As a raw material for such liposomes, naturally-derived glycolipids extracted from the brain of pigs or the like are generally used. However, naturally-derived glycolipid extract is not composed of a single glycolipid, but is contaminated by various glycolipids and unknown/unidentified substances derived from the brain. Thus, the percentage, purity and the like of the glycolipid component varies greatly among lots.

For example, in producing a liposome bound with a sugar chain, the sugar chain is bound to a glycolipid embedded on a liposomal membrane surface via a bridging spacer. Even if the same amount of sugar chain is used as a raw material, depending on variation in glycolipid component due to difference in the glycolipid among lots, the density of sugar chain bound on a membrane surface of the prepared sugar chain-bound liposome varies, which results in a difference in accumulation property. Thus, when utilizing a liposome using a naturally-derived glycolipid (liposome containing a naturally-derived glycolipid) to prepare a sugar chain-bound liposome, one had to adjust the amount of sugar chain for each lot of glycolipid and adjust the sugar chain density on the liposomal membrane surface to be within an optimal range for exhibiting the maximum accumulation property.

Furthermore, naturally-derived glycolipids may have been contaminated by unknown/unidenti fied substances derived from the brain, and thus are not suitable for use in human.

Patent Document 1: International Publication WO 2007/091661 pamphlet

Patent Document 2: Japanese Patent No. 3924606

Patent Document 3: Japanese Patent No. 3882034

Patent Document 4: International Publication WO 2007/018272 pamphlet

Non-patent Literature 1: Hirai, M., Minematsu, H., Kondo, N., Oie, K., Igarashi, K., Yamazaki, N., 2007. Accumulation of liposome with sialyl Lewis X to inflammation and tumor region: Application to in vivo bio-imaging. Biochem. Biophys. Res. Commum. 353, 553-558.

DISCLOSURE OF THE INVENTION

PROBLEMS TO BE SOLVED BY THE INVENTION

It is an object of the present invention to provide a novel liposome which can be utilized in biotechnology, particularly in DDS and molecular imaging.

It is another object of the present invention to provide a liposome useful for uniformly binding a target site-recognizing probe to a liposome surface and to provide a method of solving variation in accumulation property.

MEANS FOR SOLVING THE PROBLEMS

In order to solve the aforementioned problems, the present inventors performed wholehearted studies. As a result, the present inventors employed a synthesized glycolipid (for example, GM3 or GM4) as glycolipid and thus found a method which allows uniform binding of a target site-recognizing probe to a liposome surface to solve variation in accumulation property due to difference among naturally-derived glycolipid lots, and have completed the present invention.

Furthermore, the present inventors unexpectedly found that a glycolipid-containing liposome exhibits at least

the same, or rather improved physical properties in comparison with a conventional liposome containing a naturally-derived glycolipid, and that such can be used as a substitution for the liposome containing a naturally-derived glycolipid, and have completed the present invention.

Conventionally, glycolipids extracted from bovine brain or the like were used. Glycolipids contained therein are not homogeneous, and are a mixture of, for example, gangliosides of GM series having one sialic acid residue, GD series having two sialic acid residues, GT series having three sialic acid residues, and GQ series having sialic acid four residues, or the like. Furthermore, in naturally-derived glycolipids, mixing ratio also varies among lots. In the present invention, the inventors chemically synthesized GM4 and GM3 with comparatively simple structure and found that a liposome can be prepared by using a homogeneous glycolipid, and have completed the present invention.

Furthermore, in the present invention, the inventors found that use of a glycolipid including a plant ceramide portion increases targeting property of the liposome, and have completed the present invention.

In order to achieve the aforementioned objects, the present invention provides the following means as examples.

(Item 1)

A glycolipid-containing liposome, wherein the glycolipid contains a synthetic glycolipid and does not contain a component accompanying a naturally-derived glycolipid.

(Item 2)

The liposome according to the preceding item, wherein the glycolipid is selected from the group consisting of GM3 and GM4.

(Item 3)

The liposome according to the preceding items, wherein the liposome has absorbance of 0.5 to 3.0 at 680nm.

(Item 4)

The liposome according to the preceding items, wherein the liposome has a lipid amount of 0.5 to 5 mg/mL.

(Item 5)

The liposome according to the preceding items, wherein the liposome contains human serum albumin (HSA), an amount of which is 0.1 to 1 mg/mL.

(Item 6)

The liposome according to the preceding items, wherein the liposome has a mean particle size of 50 to 300 nm.

(Item 7)

The liposome according to the preceding items, wherein the liposome has a Z potential of -30mV to -120mV.

(Item 8a)

The liposome according to the item 1, wherein the liposome encapsulates a desired substance.

(Item 8b)

The liposome according to any of the preceding items, wherein the desired substance is selected from the group consisting of cy5.5, cy5, cy7, cy3B, cy3.5, Alexa Fluor350, Alexa Fluor488, Alexa Fluor532, Alexa Fluor546, Alexa Fluor555, Alexa Fluor568, Alexa Fluor594, Alexa Fluor633, Alexa Fluor647, Alexa Fluor680, Alexa Fluor700, Alexa Fluor750, fluorescein-4-isothiocyanate (FITC), europium-containing label, GFP, CFP, YFP, luciferases, antibody, tPA, β-galactosidase, albumin, botulinus toxin, diphtherotoxin, methylprednisolone, prednisolone phosphate, peptide, gold colloid, Gd complex, Fe complex, cisplatin, pravastatin, heparin,fasudilhydrochloride, clodronic acid, water-soluble iodine, chitin, chitosan, plasmid DNA and RNAi.

(Item 9)

The liposome according to the preceding items, wherein the liposome includes a target-recognizingprobe on a surface thereof.

(Item 10)

The liposome according to the preceding items, wherein the target-recognizing probe is selected from the group consisting of sugar chain, antibody, antigen, peptide, nucleic acid and hyaluronic acid.

(Item 11)

The liposome according to the preceding items, in which an amount of the sugar chain added is such that a bond density of sugar chain is 0.5 to 500 μg/mL.

(Item 12)

The liposome according to the preceding items, in which the amount of the sugar chain added is 0.1 to 50μg/mL.

(Item 13)

A method of producing a glycolipid-containing liposome, including the following steps of:

A) providing a synthetic glycolipid; and
B) mixing the provided synthetic glycolipid with a liposome raw material and subjecting the mixture to conditions in which a liposome is formed.

(Item 14)

The method according to the preceding item, wherein the step A) includes the following steps of:

(a) reacting a protected sugar with a protected lipid amide under conditions in which the protected sugar binds with the protected lipid amide, so as to produce a sugar-lipid amide acceptor precursor;
(b) allowing the sugar-lipid amide acceptor precursor to react under conditions in which an intramolecular condensation reaction in the sugar-lipid amide acceptor precursor proceeds, so as to produce a sugar-lipid amide acceptor;
(c) reacting the sugar-lipid amide acceptor with a protected sugar chain donor under conditions in which the sugar-lipid amide acceptor binds with the protected sugar chain donor, so as to produce a protected glycolipid; and
(d) performing deprotection reaction of the protected glycolipid under conditions in which the protected sugar chain donor is deprotected, so as to produce a glycolipid.

(Item 15)

The method according to the preceding items, wherein the protected sugar is a protected sugar selected from the group consisting of:

[Chem. 6]

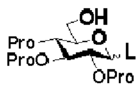
glucose

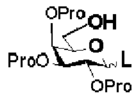
galactose

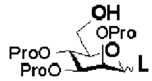
mannose

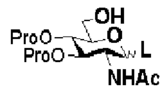
N-acetylglucosamine

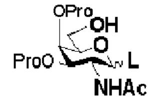
N-acetylgalactosamine

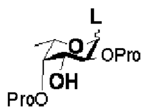
fucose

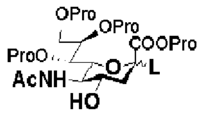
sialic acid

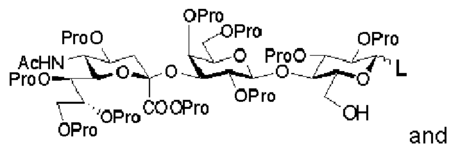
and

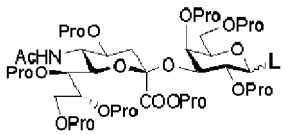
,

wherein:

PRO is independently a protecting group selected from the group consisting of benzoyl (Bz), pivaloyl (Piv), MPM (p-methoxybenzyl), methoxyphenyl (MP), acetyl, benzyl and methyl;
L is independently a leaving group selected from the group consisting of -SPh, $-SCH_3$, $-SCH_2CH_3$, -F, $-OPO(OPh)_2$ wherein Ph is a phenyl, $-OPO(N(CH_3)_2)_2$, and trichloroacetimidate.

(Item 16)

The method according to the preceding items, wherein the protected lipid amide is the following formula:
[Chem. 7]

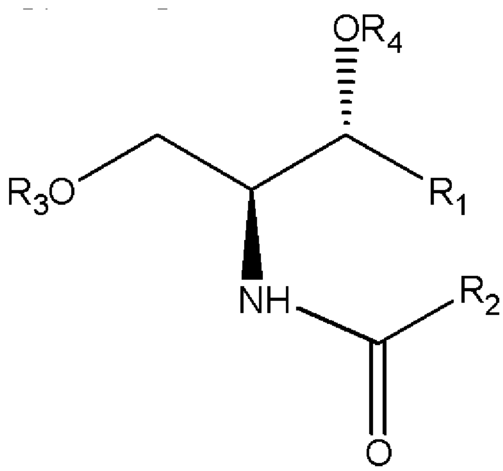
,

wherein:

$R_1$ and $R_2$ are independently selected from an alkyl group or an alkenyl group;
$R_3$ is selected from the group consisting of tert-butyldiphenylsilyl(TBDPS), tert-butyldimethylsilyl (TBDMS), triisopropylsilyl (TIPS), trityl (Tr), isopropylidene ketal and methoxybenzylidene acetal;
$R_4$ is a protecting group selected from the group consisting of succinyl, malonyl, phthaloyl, oxalyl, carbonyl, benzoyl,

acetyl and pivaloyl.

(Item 17)

The method according to any of the preceding items, wherein:

the protected lipid amide is selected from the group consisting of

[Chem. 8]

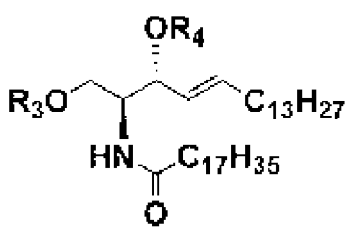

sphingosine-type ceramide and

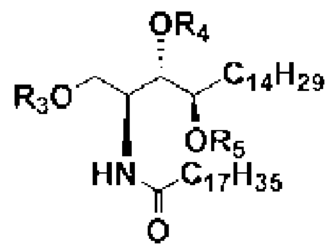

phytosphingosine-type ceramide

,

the sugar-lipid amide acceptor precursor is

[Chem. 9]

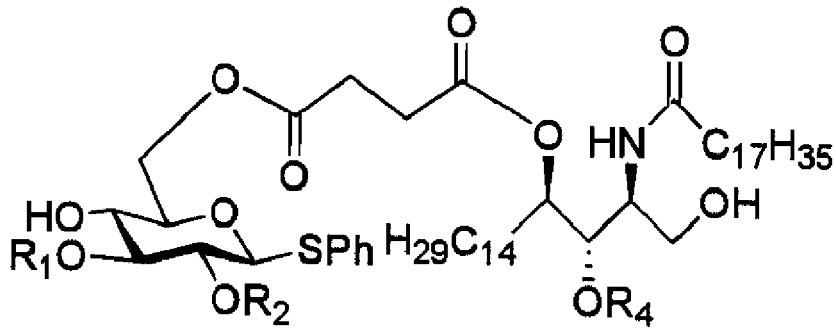

or

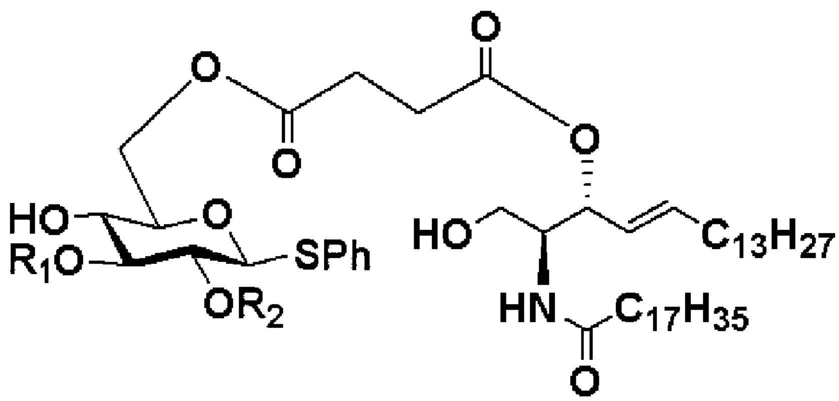

;

the sugar-lipid amide acceptor is

[Chem. 10]

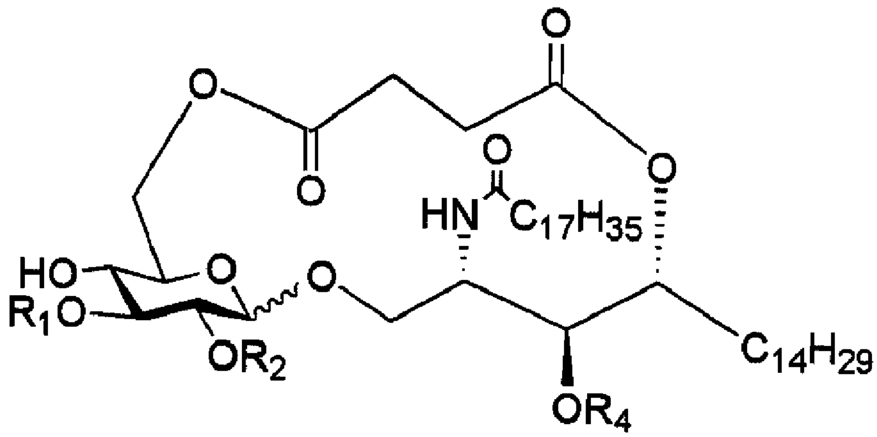

or

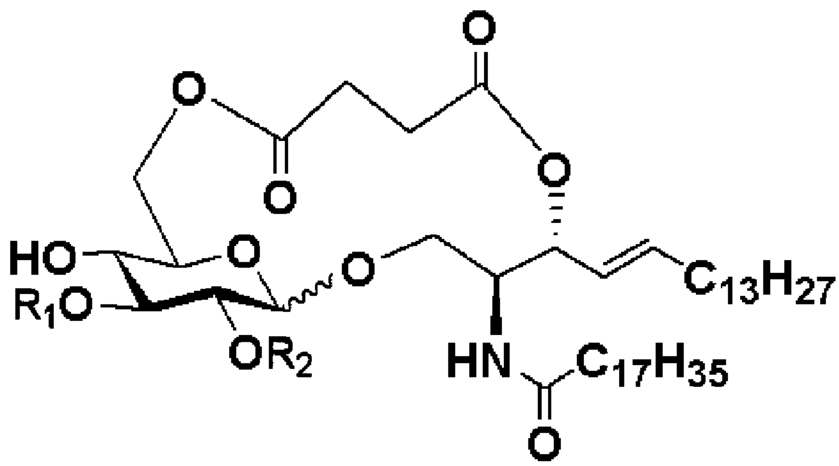

,

wherein:

$R_1$ is p-methoxybenzyl (MPM), methoxyphenyl (MP) or allyl;
$R_2$ is MPM, Bz, MP or allyl;
$R_3$ is selected from the group consisting of TBDPS, TBDMS, TIPS, Tr, isopropylidene ketal and methoxybenzylidene acetal;
$R_4$ and $R_5$ are independently a protecting group selected from the group consisting of succinyl, malonyl, phthaloyl, oxalyl, carbonyl, benzoyl, acetyl and pivaloyl; and
Ph is a phenyl.

(Item 18)

The method according to the preceding items, wherein the conditions in which the protected sugar binds with a protected lipid amide are conditions in which an alcohol binds with a carboxylic acid.

(Item 19)

The method according to the preceding items, wherein the step (a) includes mixing and reacting the protected sugar chain with the lipid amide in a solvent in the presence of a reagent at a predetermined reaction temperature for a predetermined reaction time, wherein:

the reaction temperature is equal to or higher than room temperature;
the solvent is selected from the group consisting of tetrahydrofuran (THF), $CH_2Cl_2$, benzene, toluene, N,N-dimethylformamide (DMF) and combinations thereof;
the reagent is selected from the group consisting of triphenylphosphine ($PPh_3$), diethyl azodicarboxylate (DEAD), 1-methyl-3-(3-dimethylaminopropyl)-carbodiimide hydrochloride (WSC), 2,4,6-trichlorobenzoyl chloride, triethylamine ($Et_3N$) and4-dimethylaminopyridine (DMAP), and combinations thereof; and
the reaction time is 2 to 4 hours.

(Item 20)

The method according to the preceding items, wherein the reagent is $PPh_3$ and DEAD, the solvent is THF, and the reaction temperature is 90 degrees Celsius or higher.

(Item 21)

The method according to the preceding items, wherein the reagent is WSC or 2, 4, 6-trichlorobenzoyl chloride, $Et_3N$ and DMAP, the solvent is $CH_2Cl_2$, and the reaction temperature is 30 to 60 degrees Celsius.

(Item 22)

The method according to the preceding items, wherein the reagent is WSC, the solvent is $CH_2Cl_2$, and the reaction temperature is room temperature.

(Item 23)

The method according to the preceding items, wherein the reaction time is 3 hours.

(Item 24)

The method according to the preceding items, wherein:

the solvent is tetrahydrofuran (THF);
the reagent is triphenylphosphine ($PPh_3$: 3.0 equivalents) and DEAD (3.0 equivalents), wherein the equivalent is an equivalent with respect to the protected sugar chain;
the reaction temperature is 90 degrees Celsius, and the reaction is performed at reflux.

(Item 25)

The method according to the preceding items, wherein:

the solvent is $CH_2Cl_2$;
the reagent is 1-methyl-3-(3-dimethylaminopropyl)-carbodiimide hydrochloride (WSC: 3.0 equivalents), wherein the equivalent is an equivalent with respect to the protected sugar chain; and
the temperature is room temperature.

(Item 26)

The method according to the preceding items, wherein:

the solvent is $CH_2Cl_2$;
the reagent is 2,4,6-trichlorobenzoyl chloride (1.1 equivalents), triethylamine ($Et_3N$: 1.5 equivalents) and 4-dimethylaminopyridine (DMAP: 3.0 equivalents), wherein the equivalent is an equivalent with respect to the protected sugar chain; and
the temperature is room temperature.

(Item 27)

The method according to the preceding items, wherein the step (a) includes further adding a spacer precursor

to the sugar and the protected lipid amide, so that the protected lipid amide binds with the sugar via a spacer.

(Item 28)

The method according to the preceding items, wherein the spacer is previously bound with the sugar or the protected lipid amide.

(Item 29)

The method according to the preceding items, further including allowing the protected lipid amide under conditions in which a hydroxyl group at the 1-position of the protected lipid amide is deprotected, so as to deprotect the hydroxyl group at the 1-position.

(Item 30)

The method according to the preceding items, wherein, when the spacer precursor is succinic acid and the succinic acid binds to $R_3$ of the protected lipid amide:

$R_3$ is isopropylidene ketal or methoxybenzylidene acetal;
$R_4$ is

[Chem. 11]

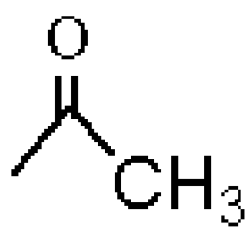

(Ac) or
[Chem. 12]

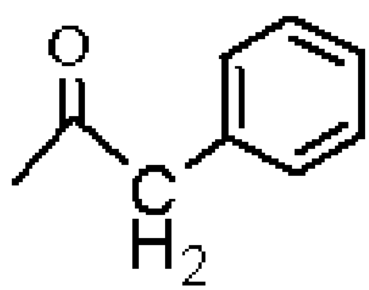

(Bx); and
$R_5$ is Ac or Bx.

(Item 31)

The method according to the preceding items, wherein, when the spacer precursor is succinic acid and the succinic acid binds to $R_4$ of the protected lipid amide:

$R_3$ is selected from the group consisting of trityl (Tr), TBDPS and TBDMS;
$R_4$ is selected from the group consisting of succinyl, malonyl, oxalyl, carbonyl, glutaryl and phthaloyl; and $R_5$ is acetyl or benzoyl.

(Item 32)

The method according to the preceding items, wherein, when the spacer precursor is succinic acid and the succinic acid binds to $R_5$ of the protected lipid amide:

$R_3$ is selected from the group consisting of Tr, TBDPS and TBDMS;
$R_4$ is selected from the group consisting of succinyl, malonyl, oxalyl, carbonyl, glutaryl and phthaloyl; and $R_5$ is acetyl or benzoyl.

(Item 33)

The method according to the preceding items, in which a sugar residue at a reducing terminal side of the oligosaccharide binds with the protected lipid amide via a spacer.

(Item 34)

The method according to the preceding items, wherein the step (b) is performed in the presence of activating agent for activating the intramolecular condensation reaction, and the activating agent is selected from the group consisting of N-iodosuccinimide (NIS), trifluoromethanesulfonic acid (TfOH), trimethylsilyl trifluoromethanesulfonate (TMSOTf), dimethyl(methylthio)sulfonium triflate (DMTST), N-bromosuccinimide(NBS) and combinations thereof.

(Item 35)

The method according to any of the preceding items, wherein the step (b) is performed:

at a reaction temperature of -80 degrees Celsius to room temperature;
in a solvent selected from the group consisting of $CH_2Cl_2$, diethylether, acetonitrile, diethylether, acetonitrile, propionitrile, toluene, nitromethane and combinations thereof;
in the presence of reagent selected from the group consisting of N-iodosuccinimide (NIS), trifluoromethanesulfonic acid (TfOH), dimethyl(methylthio)sulfonium triflate (DMTST), molecular sieves 4 angstroms (MS4Å), molecular sieves 3 angstroms (MS3Å) and combinations thereof; and
for a reaction time of 1 to 48 hours.

(Item 36)

The method according to the preceding items, wherein the reaction temperature is -20 to 0 degree Celsius to zero degrees Celsius.

(Item 37)

The method according to the preceding items, wherein the solvent is dichloromethane.

(Item 38)

The method according to the preceding items, wherein the reagent is N-iodosuccinimide (NIS) and trifluoromethanesulfonic acid (TfOH).

(Item 39)

The method according to the preceding items, wherein the reaction time is 1 to 5 hours.

(Item 40)

The method according to the preceding items, wherein the step (b) includes reacting using $CH_2Cl_2$ as a solvent and using N-iodosuccinimide (NIS), trifluoromethanesulfonic acid (TfOH), TMSOTf and molecular sieves 4 angstroms (MS4Å) as reagents for a reaction time of 1.5 hours at a reaction temperature of 0 degree Celsius.

(Item 41)

The method according to the preceding items, wherein the step (b) includes reacting using $CH_2Cl_2$ as a solvent and using N-iodosuccinimide (NIS), trifluoromethanesulfonic acid (TfOH) and molecular sieves 4 angstroms (MS4Å) as reagents for a reaction time of 5 hours at -40 degrees Celsius.

(Item 42)

The method according to the preceding items, wherein the step (b) includes reacting using $CH_2Cl_2$ as a solvent and using N-iodosuccinimide (NIS), trifluoromethanesulfonic acid (TfOH) and molecular sieves 4 angstroms (MS4Å) as reagents for a reaction time of 36 hours, initially at a reaction temperature of -80 degrees Celsius, subsequently at a reaction time of -60 degrees Celsius, subsequently at a reaction time of -40 degrees Celsius, and subsequently at a reaction time of 0 degree Celsius.

(Item 43)

The method according to the preceding items, wherein the step (b) includes reacting using acetonitrile (MeCN) as a solvent and using N-iodosuccinimide (NIS), trifluoromethanesulfonic acid (TfOH) and molecular sieves 3 angstroms (MS3Å) as reagents for a reaction time of 48 hours, initially at a reaction temperature of -40 degrees Celsius and subsequently at a reaction temperature of 0 degree Celsius.

(Item 44)

The method according to the preceding items, wherein the step (b) includes reacting using acetonitrile (MeCN) as a solvent and using N-iodosuccinimide (NIS), trifluoromethanesulfonic acid (TfOH) and molecular sieves 3 angstroms (MS3Å) as reagents for a reaction time of 1.5 hour at a reaction temperature of -0 degree Celsius.

(Item 45)

The method according to the preceding items, wherein the step (b) includes reacting using acetonitrile (MeCN) as a solvent and using N-iodosuccinimide (NIS), trifluoromethanesulfonic acid (TfOH) and molecular sieves 3 angstroms (MS3Å) as reagents for a reaction time of three hours at a reaction temperature of -20 degrees Celsius.

(Item 46)

The method according to the preceding items, wherein the step (b) includes reacting using diethylether as a solvent and using N-iodosuccinimide (NIS), trifluoromethanesulfonic acid (TfOH) and molecular sieves 3 angstroms (MS3Å) as reagents for a reaction time of 25 hours, initially at a reaction temperature of 0 degree Celsius and subsequently at a reaction temperature of room temperature.

(Item 47)

The method according to the preceding items, wherein the step (b) includes reacting using acetonitrile (MeCN) as a solvent and using dimethyl(methylthio)sulfonium triflate (DMTST) and molecular sieves 3 angstroms (MS3Å) as reagents for a reaction time of 1 hour at a reaction temperature of 0 degree Celsius.

(Item 48)

The method according to the preceding items, wherein the step (b) includes reacting using $CH_2Cl_2$ as a solvent and using N-iodosuccinimide (NIS), trifluoromethanesulfonic acid (TfOH) and molecular sieves 4 angstroms (MS4Å) as reagents for a reaction time of 5 hours at a reaction temperature of 0 degree Celsius.

(Item 49)

The method according to the preceding items, wherein the step (b) includes reacting using $CH_2Cl_2$ as a solvent and using N-iodosuccinimide (NIS), trifluoromethanesulfonic acid (TfOH) and molecular sieves 4 angstroms (MS4Å) as reagents for a reaction time of 1.5 hour at a reaction temperature of -20 degrees Celsius.

(Item 50)

The method according to the preceding items, wherein the step (b) includes reacting using $CH_2Cl_2$ as a solvent and using dimethyl(methylthio)sulfonium triflate (DMTST) and molecular sieves 4 angstroms (MS4Å) as reagents for a reaction time of 2 hours at 0 degree Celsius.

(Item 51)

The method according to the preceding items, wherein the intramolecular condensation reaction is glycosylation.

(Item 52)

The method according to the preceding items, wherein the step (c) includes reacting: using a donor at more than 2.5 equivalents with respect to an acceptor; at a reaction temperature of -40 to 0 degree Celsius; in a solvent of $CH_2Cl_2$; and in the presence of trimethylsilyl trifluoromethanesulfonate (TMSOTf) reagent for a reaction time of one to 48 hours.

(Item 53)

The method according to the preceding items, wherein:

an equivalent of a donor with respect to the acceptor is 2.5 equivalents;
the reaction temperature is 0 degrees Celsius;
the solvent is $CH_2Cl_2$;
the reagent is TMSOTf; and
the reaction time is 7 hours.

(Item 54)

The method according to the preceding items, wherein the protected sugar chain donor is selected from the group consisting of
[Chem. 13]

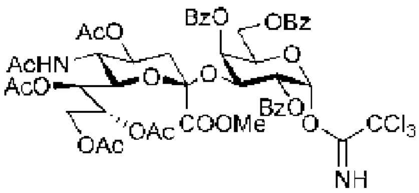

,

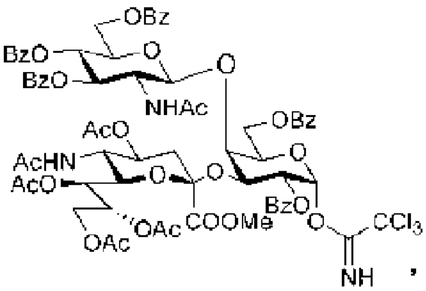

,

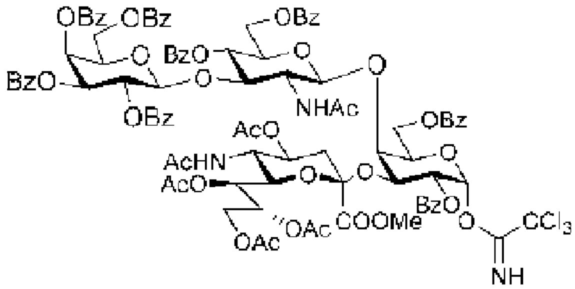

,

-SPh, $-SCH_3$, $-SCH_2CH_3$, -F, $-OPO(OPh)_2$ wherein Ph is phenyl, and $-OPO(N(CH_3)_2)_2$.

(Item 55)

The method according to the preceding items, wherein the step (d) is performed:

in a solvent of $CH_2Cl_2$;
in the presence of a reagent of trifluoroacetic acid;
at a reaction temperature of room temperature; and for a reaction time of 2 to 12 hours.

(Item 56)

The method according to the preceding items, wherein the reaction time is 2 hours.

(Item 57)

The method according to the preceding items, further including the step of (e) allowing a product of the step (d) to react under conditions in which an acyl protecting group is deprotected, so as to cause deprotection.

(Item 58)

The method according to the preceding items, wherein the step (e) is performed:

in a solvent of methanol ($CH_3OH$) or water ($H_2O$);
in the presence of a reagent of sodium methoxide ($NaOCH_3$) or KOH;
at a reaction temperature of room temperature to 100 degrees Celsius; and
for a reaction time of 1 hour to 1 week.

(Item 59)

The method according to the preceding items, wherein:

the solvent is methanol ($CH_3OH$);
the reagent is sodium methoxide ($NaOCH_3$);
the reaction temperature is room temperature; and
the reaction time is 12 hours.

(Item 60)

A method of producing

[Chem. 14]

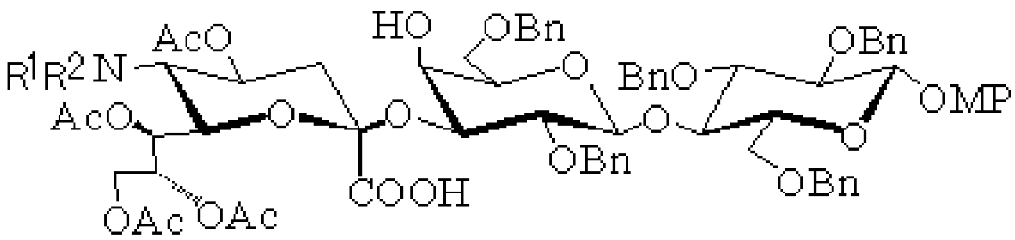

,

the method including the step (A) of reacting an acceptor compound

[Chem. 15]

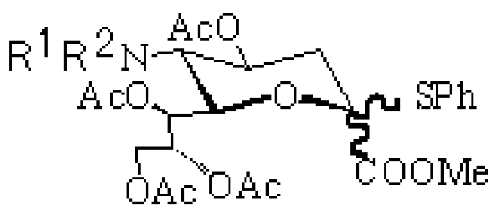

with a donor compound

[Chem. 16]

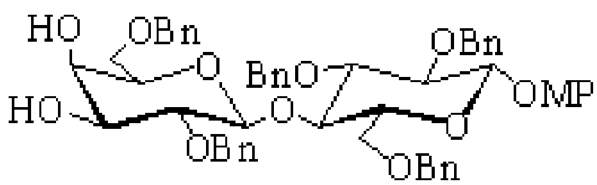

under conditions in which the acceptor compound binds with the donor compound, wherein:

$R^1$ is Ac or H;
$R^2$ is Ac or 2,2,2-trichloroethoxycarbonyl (Troc)

[Chem. 17]

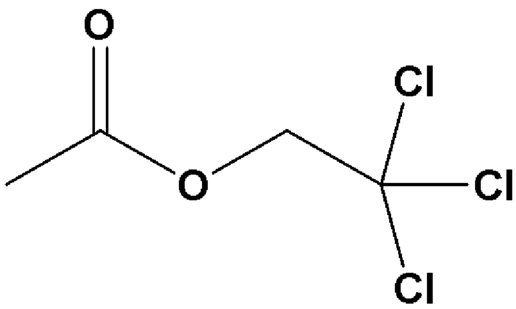

;

SPh is

[Chem. 18]

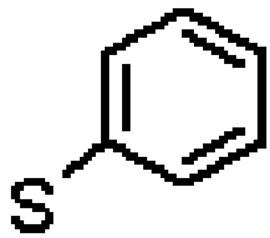

;

MP is

[Chem. 19]

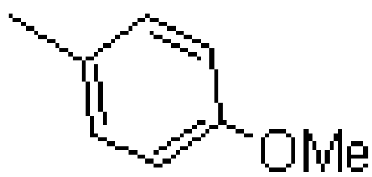

;

SE is

[Chem. 20]

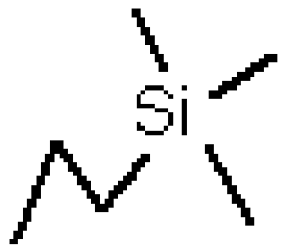

;

Ac is

[Chem. 21]

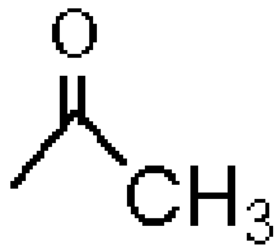

;

Bz is

[Chem. 22]

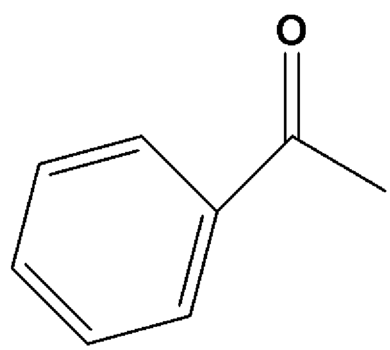

;

Bn is

[Chem. 23]

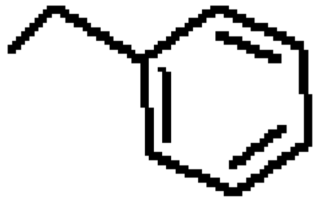

;

and

Me is methyl.

(Item 61)

The method according to the preceding items, wherein the step (A) is performed:

at a reaction temperature of -40 degrees Celsius to room temperature;
in a solvent of $CH_3CN$, $CH_2Cl_2$, diethylether, acetonitrile, propionitrile, toluene, nitromethane, or a combination thereof;
in the presence of a catalyst of N-iodosuccinimide (NIS), trifluoromethanesulfonic acid (TfOH), trimethylsilyl trifluoromethanesulfonate (TMSOTf) or a combination thereof; and
for a reaction time of 1 hour to 3 days.

(Item 62)

The method according to the preceding items, wherein the step (A) is performed:

at a reaction temperature of -50 degrees Celsius to room temperature;
in a solvent of $CH_3CN$, $CH_2Cl_2$, diethylether, acetonitrile, propionitrile, toluene, nitromethane or a combination thereof;
in the presence of a catalyst of N-iodosuccinimide (NIS), trifluoromethanesulfonic acid (TfOH), trimethylsilyl trifluoromethanesulfonate (TMSOTf) or a combination thereof; and
for a reaction time of 1 hour to 3 days.

(Item 63)

The method according to the preceding items, wherein the catalyst is selected from the group consisting of N-iodosuccinimide (NIS), trifluoromethanesulfonic acid (TfOH) and a combination thereof.

(Item 64)

The method according to the preceding items, wherein the solvent is $CH_3CN$, $CH_2Cl_2$, or a mixture solution of $CH_3CN$ and $CH_2Cl_2$.

(Item 65)

The method according to the preceding items, wherein the reaction temperature is of -30 to 0 degree Celsius.

(Item 66)

The method according to the preceding items, wherein the reaction time is 1 hour to 1 day.

(Item 67)

The method according to the preceding items, wherein the step (A) includes reacting using initially $CH_3CN$ and subsequently a mixture solution of $CH_3CN$ and $CH_2Cl_2$ as a solvent and using N-iodosuccinimide (NIS) and trifluoromethanesulfonic acid (TfOH) as catalysts for a reaction time of 2 days, initially at a reaction temperature of -30 degrees

Celsius and subsequently at a reaction temperature of room temperature.

(Item 68)

The method according to the preceding items, wherein the step (A) includes reacting using a mixture solution of $CH_3CN$ and $CH_2Cl_2$ as a solvent and using N-iodosuccinimide (NIS), trifluoromethanesulfonic acid (TfOH) and TMSOTf as catalysts for a reaction time of 3 days, initially at a reaction temperature of -30 degrees Celsius and subsequently at a reaction temperature of room temperature.

(Item 69)

The method according to the preceding items, wherein the step (A) includes reacting using a mixture solution of $CH_3CN$ and $CH_2Cl_2$ as a solvent and using N-iodosuccinimide (NIS) and trifluoromethanesulfonic acid (TfOH) as catalysts for a reaction time of 2 days, initially at a reaction temperature of -30 degrees Celsius and subsequently at a reaction temperature of 0 degree Celsius.

(Item 70)

The method according to the preceding items, wherein the step (A) includes reacting using a mixture solution of $CH_3CN$ and $CH_2Cl_2$ as a solvent and using N-iodosuccinimide (NIS) and trifluoromethanesulfonic acid (TfOH) as catalysts for a reaction time of 3 days at a reaction temperature of -30 degrees Celsius.

(Item 71)

The method according to the preceding items, wherein the step (A) includes reacting using $CH_2Cl_2$ as a solvent and using N-iodosuccinimide (NIS) and trifluoromethanesulfonic acid (TfOH) as catalysts for a reaction time of 1 day at a reaction temperature of -30 degrees Celsius.

(Item 72)

The method according to the preceding items, wherein the step (A) includes reacting using a mixture solution of propionitrile and $CH_2Cl_2$ as a solvent and using N-iodosuccinimide (NIS) and trifluoromethanesulfonic acid (TfOH) as catalysts for a reaction time of 6 hours at a reaction temperature of -50 degrees Celsius.

(Item 73)

A compound represented by the following formula:
[Chem. 24]

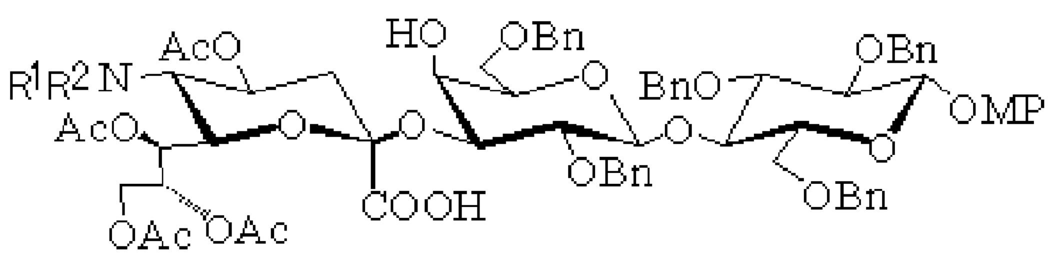

or
**[0125]**

[Chem. 25]

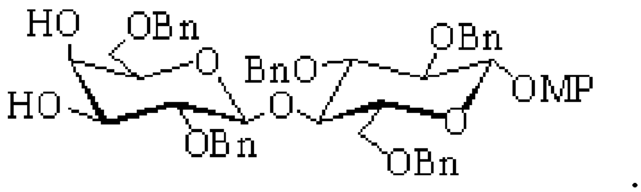

(Item 74)

A method for synthesizing an oligosaccharide, including the step of reacting an amino sugar having an amino group protected by trichloroethoxycarbonyl (Troc) with a sugar protected by methoxyphenyl (MP) under conditions in which the amino sugar protected by Troc binds with the sugar protected by MP.

(Item 75)

The method according to the preceding items, wherein the amino sugar has a leaving group L which leaves when the amino sugar binds with the sugar protected by MP.

(Item 76)

The method according to the preceding items, wherein the leaving group L is selected from the group consisting of -SPh, $-SCH_3$, $-SCH_2CH_3$, -F, $-OPO(OPh)_2$ wherein Ph is phenyl, and $-OPO(N(CH_3)_2)_2$.

(Item 77)

The method according to the preceding items, wherein the amino sugar protected by Troc is
[Chem. 26]

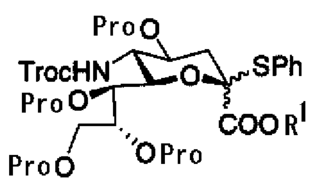

,

wherein:

Pro is independently a protecting group selected from the group consisting of acetyl (Ac), benzyl (Bn), benzoyl (Bz), pivaloyl (Piv), MPM (p-methoxybenzyl) and methoxyphenyl (MP); and
$R^1$ is an alkyl.

(Item 78)

The method according to the preceding items, wherein the sugar protected by MP is selected from the group consisting of
[Chem. 27]

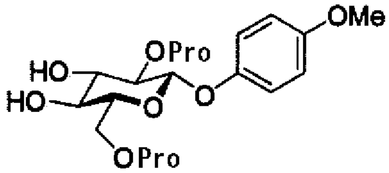

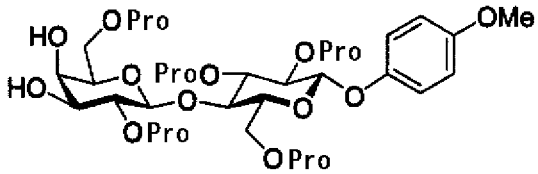

,

wherein Pro is independently a protecting group selected from the group consisting of acetyl(Ac), benzyl (Bn), benzoyl(Bz), pivaloyl (Piv), MPM (p-methoxybenzyl) and methoxyphenyl (MP).

(Item 79)

The method according to any of the preceding items, including the step of deprotecting the Troc group in the presence of Zn (Cu).

(Item 80)

A compound having a structure selected from the group consisting of the following structures:
[Chem. 28]

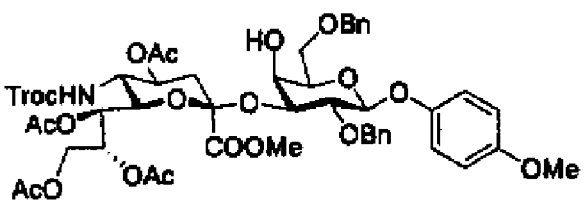

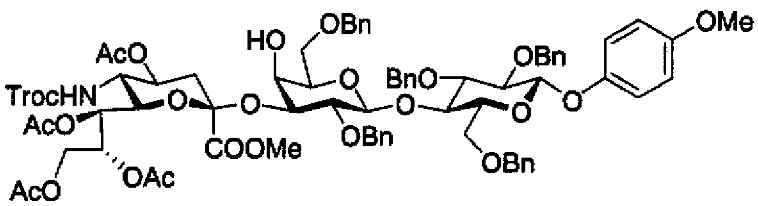

.

(Item 81)

A compound represented by the formula:
[Chem. 29]

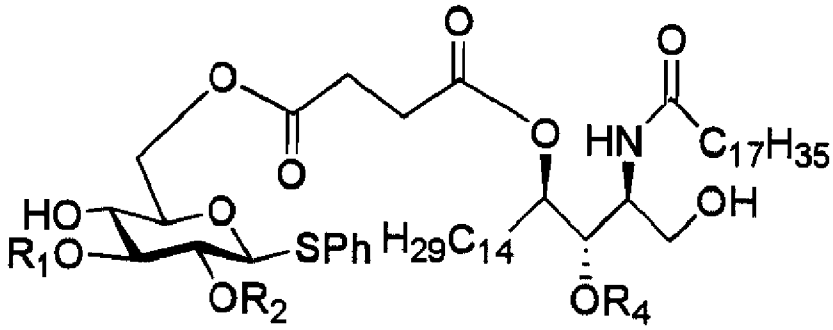

,

wherein:

$R_1$ and $R_2$ are independently selected from an alkyl group and an alkenyl group; and
$R_4$ is a protecting group selected from the group consisting of benzoyl, acetyl and pivaloyl.

(Item 82)

A compound represented by the formula:
[Chem. 30]

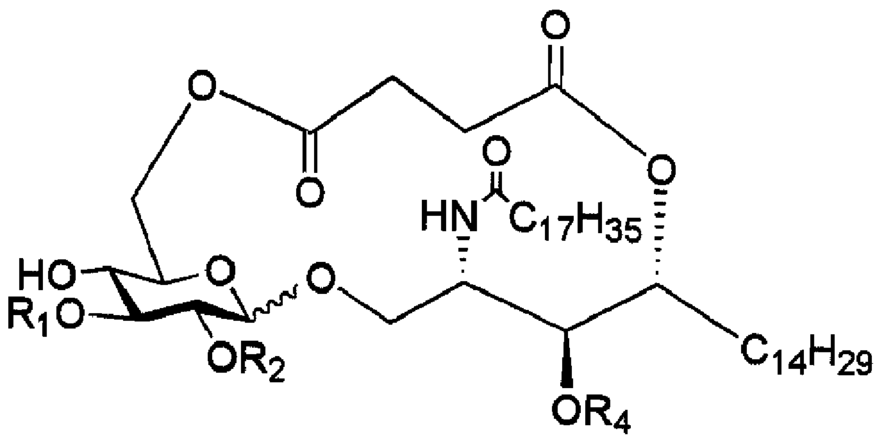

,

wherein:

$R_1$ and $R_2$ are independently selected from an alkyl group and an alkenyl group; and
$R_4$ is a protecting group selected from the group consisting of benzoyl, acetyl and pivaloyl.

(Item 83)

A glycolipid-containing liposome, wherein:

the glycolipid includes a plant ceramide portion and a sugar chain portion.

(Item 84)

The liposome according to the preceding item, wherein the plant ceramide portion is
[Chem. 31]

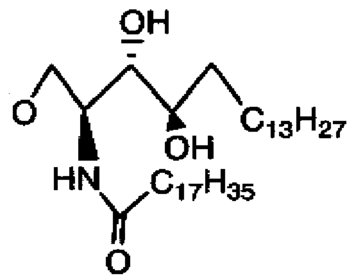

.

(Item 85)

The liposome according to the preceding items, wherein the sugar chain portion is
[Chem. 32]

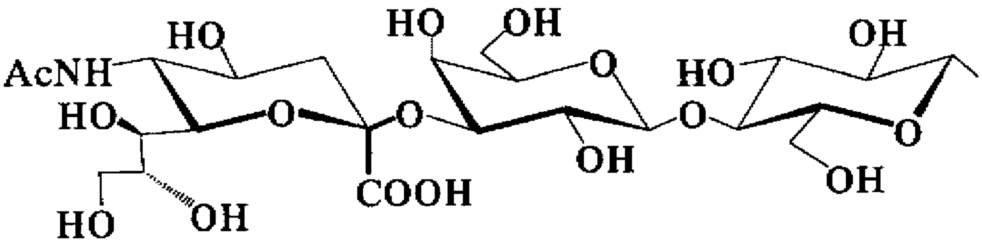

.

(Item 86)

The glycolipid-containing liposome according to the preceding items, wherein the glycolipid is
[Chem. 33]

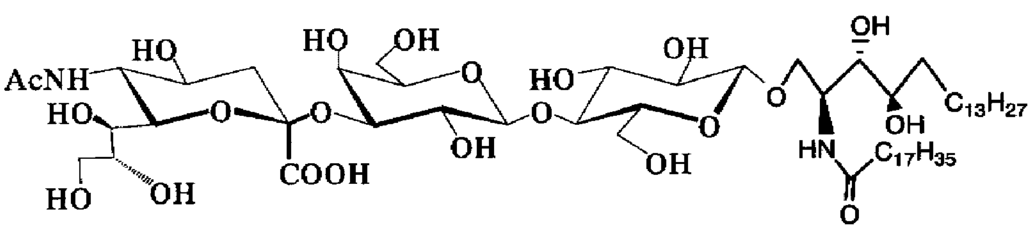

.

(Item 87)

The liposome according to the preceding items, wherein:

the glycolipid has

[Chem. 34]

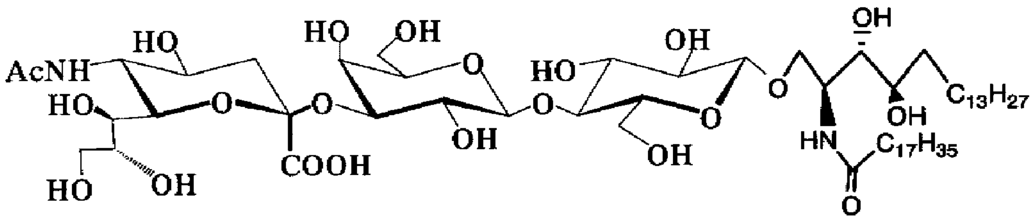

;

the glycolipid-containing liposome contains, as lipids composing the liposome, dipalmitoylphosphatidylcholine (DPPC), cholesterol, dicetylphosphate (DCP), the glycolipid and dipalmitoylphosphatidylethanolamine (DPPE) at a molar ratio of 35:40:5:15:5.

(Item 88)

The liposome according to the preceding items, wherein the liposome has absorbance of 0.5 to 3.0 at 680nm.

(Item 89)

The liposome according to the preceding items, wherein the liposome has a lipid amount of 0.5 to 5 mg/mL.

(Item 90)

The liposome according to the preceding items, wherein the liposome contains human serum albumin (HSA), an amount of which is 0.1 to 1 mg/mL.

(Item 91)

The liposome according to the preceding items, wherein the liposome has a mean particle size of 50 to 300 nm.

(Item 92)

The liposome according to the preceding items, wherein the liposome has a Z potential of -30mV to -120mV.

(Item 93)

The liposome according to the preceding items, wherein the liposome encapsulates a desired substance.

(Item 94)

The liposome according to the preceding items, wherein the desired substance is selected from the group consisting of: cy5.5, cy5, cy7, cy3B, cy3.5, Alexa Fluor350, Alexa Fluor488, Alexa Fluor532, Alexa Fluor546, Alexa Fluor555, Alexa Fluor568, Alexa Fluor594, Alexa Fluor633, Alexa Fluor647, Alexa Fluor680, Alexa Fluor700, Alexa Fluor750, fluorescein-4-isothiocyanate (FITC), europium-containing label, GFP, CFP, YFP, luciferases, antibody, tPA, β-galactosidase, albumin, botulinus toxin, diphtherotoxin, methylprednisolone, prednisolone phosphate, peptide, gold colloid, Gd complex, Fe complex, cisplatin, pravastatin, heparin, fasudil hydrochloride, clodronic acid, water-soluble iodine, chitin, chitosan, plasmid DNA and RNAi.

(Item 95)

The liposome according to the preceding items, wherein the liposome includes a target-recognizingprobe on a surface thereof.

(Item 96)

The liposome according to the preceding items, wherein the target-recognizing probe is selected from the group consisting of sugar chain, antibody, antigen, peptide, nucleic acid and hyaluronic acid.

(Item 97)

The liposome according to the preceding items, in which an amount of the sugar chain added is an amount such that a bond density of sugar chain is 0.5 to 500 μg/mL.

(Item 98)

The liposome according to the preceding items, in which an amount of the sugar chain added is 0.1 to 50μg/mL.

(Item 99)

The liposome according to the preceding items, wherein the glycolipid does not contain a component accompanying a naturally-derived glycolipid.

(Item 100)

A method of producing a glycolipid-containing liposome, the method including the steps of:

A) providing a glycolipid, wherein the glycolipid includes a plant ceramide portion and a sugar chain portion; and
B) mixing the provided glycolipid with a liposome raw material and subjecting the mixture to conditions in which a liposome is formed.

(Item 101)

The method according to the preceding item, wherein the step A) includes the following steps of:

(a) reacting a protected sugar with a protected lipid amide under conditions in which the protected sugar binds with the protected lipid amide, so as to produce a sugar-lipid amide acceptor precursor;
(b) allowing the sugar-lipid amide acceptor precursor to react under conditions in which an intramolecular condensation reaction in the sugar-lipid amide acceptor precursor proceeds, so as to produce a sugar-lipid amide acceptor;
(c) reacting the sugar-lipid amide acceptor with a protected sugar chain donor under conditions in which the sugar-lipid amide acceptor binds with the protected sugar chain donor, so as to produce a protected glycolipid; and
(d) performing a deprotection reaction of the protected glycolipid under conditions in which the protected sugar chain donor is deprotected, so as to produce a glycolipid,

wherein the protected lipid amide is
[Chem. 35]

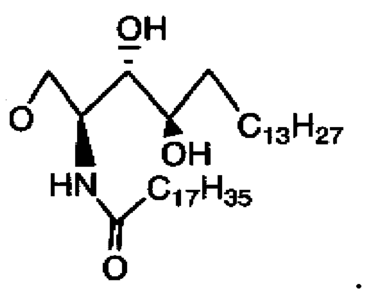

Therefore, these and other advantages of the present invention will be clear from the detailed description below.

## EFFECTS OF THE INVENTION

The present invention provides a liposome useful for uniformly binding a target site-recognizing probe to the liposome surface, so that the target site-recognizing probe can be uniformly bound to the liposome surface and variation in the accumulation property due to the difference among naturally-derived glycolipid lots can be solved.

Naturally-derived glycolipids may have been contaminated by unknown/unidentified substances derived from the brain, and thus are not suitable for use in a human. However, by using a synthesized glycolipid, the liposome may be suitable for use in a human.

By providing a liposome with improved target-directivity, a composition of the present invention achieves, a significantly improved accumulation property in a desired site (particularly tumors), and can provide a more accurate diagnosis, prophylaxis and therapy.

It should be understood that the effects attained by the present invention are not limited to the aforementioned effect but include all effects that can be understood by those skilled in the art in view of the matters described herein.

## BRIEF DESCRIPTION OF THE DRAWINGS

Fig. 1 illustrates one example of a calibration curve for measuring the amount of protein in a liposome. The X-axis corresponds to the protein standard concentration (μg/50μl), and the Y-axis corresponds to the absorbance at 540nm. y = 0.1594x + 0.0004, r = 0.9996.

Fig. 2 illustrates one example of a calibration curve for measuring the amount of lipids in a liposome. The X-axis corresponds to the cholesterol standard concentration (μg/20μl), and the Y-axis corresponds to the absorbance at 550nm. y = 0.0616x + 0.0067, r = 0.9992.

Fig. 3 illustrates an example of the particle size distribution of a synthetic GM3 sugar chain-modified liposome. The solid line represents the particle size distribution of a liposome prepared using a natural ganglioside derived from porcine brain. The broken line represents the particle size distribution of a synthetic GM3 (plantceramide) liposome. The X-axis corresponds to the particle size (diameter, nm), and the Y-axis corresponds to intensity (%).

Fig. 4 illustrates the structural formulas of the synthetic gangliosides GM3 and GM4, and the structures of natural ceramide, plant ceramide, and pseudo-ceramide. The upper structure illustrates the structural formula of GM3. The structure of GM3 includes, from the left to the right side, N-acetylneuraminic acid (sialic acid: NeuAc), galactose (Gal), glucose (Glu) and ceramide. The central structure illustrates the structural formula of GM4. The structure of GM4 includes, from the left to the right side, N-acetylneuraminic acid (sialic acid: NeuAc), galactose (Gal) and ceramide. The lower structures illustrate the three types of ceramide. From the left to the right side are illustrated: a natural ceramide, a plant ceramide and a pseudo-ceramide .

Fig. 5a illustrates one example of the particle size distribution of a synthetic GM3 (natural ceramide) liposome. The X-axis corresponds to the particle size (diameter, nm), and the Y-axis corresponds to intensity (%).

Fig. 5b illustrates one example of the particle size distribution of a synthetic GM3 (pseudo-ceramide) liposome. The X-axis corresponds to the particle size (diameter, nm), and the Y-axis corresponds to intensity (%).

Fig. 5c illustrates one example of the particle size distribution of a synthetic GM4 (plant ceramide) liposome. The X-axis corresponds to the particle size (diameter, nm), and the Y-axis corresponds to intensity (%).

Fig. 5d illustrates one example of the particle size distribution of a synthetic GM4 (pseudo-ceramide) liposome. The X-axis corresponds to the particle size (diameter, nm), and the Y-axis corresponds to intensity (%).

Fig. 6a illustrates an amount of FITC binding to a surface of a liposome prepared using a synthetic GM3 plant ceramide. The X-axis corresponds to the FITC concentration (μM) at the time of reaction, and the Y-axis corresponds to the amount of binding FITC (nM).

Fig. 6b illustrates an amount of FITC that binding to a surface of a liposome prepared using a synthetic GM3 pseudo-ceramide or synthetic GM3 natural ceramide. The X-axis corresponds to the FITC concentration (μM) at the time of reaction, and the Y-axis corresponds to the amount of binding FITC (nM).

Fig. 6c illustrates an amount of anti-E-selectin antibody binding to a surface of a liposome prepared using a synthetic GM4 plant ceramide or a synthetic GM4 pseudo-ceramide. The X-axis corresponds to the reaction concentration (μM), and the Y-axis corresponds to the amount of binding antibody (μg/mg Lipid).

Fig. 7A is a diagram of accumulation properties to born tumor of a sialyl Lewis X liposome and an anti-selectin antibody liposome confirmed using a tumor-bearing model mice. Fig. 7A illustrates the accumulation properties of the liposomes using a synthetic GM3 plant ceramide on the tumor site. The accumulation properties of a liposome without a sugar chain and the SLX liposome were confirmed. It was found that, in comparison with the liposome without a sugar chain, the SLX liposome accumulates on the tumor site. It was also found that the synthetic GM3 plant ceramide can be utilized as a raw material of a liposome. Fig. 7A shows, from the above, just after the administration, 24 hours after administration, 48 hours after administration, and 72 hours after administration. In the imaging diagram, the whiter color in the higher portion of the scale indicates the fluorescence signal with higher intensity, and the color in the lower portion of the scale indicates the fluorescence signal with lower intensity. The unit is represented as photon count (photon/second (ph/s); fluorescence signal photon count per second).

Fig. 7B is a diagram of accumulation properties to born tumor of a sialyl Lewis X liposome and an anti-E-selectin antibody liposome confirmed using the tumor-bearing model mice using the . Fig. 7B illustrates the accumulation properties of the liposomes using a synthetic GM4 plant ceramide on the tumor site. The accumulation properties of a liposome without antibody and an anti-E-selectin antibody liposome were confirmed. It was found that, in comparison with the liposome without antibody, the anti-E-selectin liposome accumulates on the tumor site. It was also found that the synthetic GM4 plant ceramide can be used as a raw material of liposome. Fig. 7B shows, from the above, just after administration, 24 hours after administration and 48 hours after administration. In the imaging diagram, the whiter color in the higher portion of the scale indicates the fluorescence signal with higher intensity, and the color in the lower portion of the scale indicates the fluorescence signal with lower intensity. The unit is represented as photon count (photon/second (ph/s); fluorescence signal photon count per second).

Fig. 7C is a diagram of tumor-bearing accumulation properties of a sialyl Lewis X liposome and an anti-E-selectin antibody liposome confirmed using the tumor-bearing model mice using the. Fig. 7B illustrates the accumulation properties of the liposomes using a synthetic GM4 pseudo-ceramide on the tumor site. The accumulation properties of a liposome without antibody and an anti-E-selectin antibody liposome were confirmed. Between the liposome without antibody and the anti-E-selectin liposome, no difference was observed in accumulation property on a tumor site. The scale shown in the rightmost portion shows intensity of a fluorescence signal obtained by imaging. Fig. 7C shows, from the above, just after administration, 24 hours after administration and 48 hours after administration. In the imaging diagram, the whiter color in the higher portion of the scale indicates the fluorescence signal with higher intensity, and the color in the lower portion of the scale indicates the fluorescence signal with lower intensity. The unit is represented as photon count (photon/second (ph/s) ; fluorescence signal photon count per second).

Fig. 8A is the image data of 24 hours after administration, illustrating the accumulation property of a liposome prepared using a natural ganglioside (Lot. No. TGANG14) on the tumor site. The upper figures show the group administered with 100μl of the liposome (0 μg/mL), and the lower figures show the group administered with 100μl of the liposome (50 μg/mL). The figures respectively show data of different individuals. The scale shown in the rightmost portion shows intensity of a fluorescence signal obtained by imaging. In the imaging diagram, the whiter color in the higher portion of the scale indicates the fluorescence signal with higher intensity, and the color in the lower portion of the scale indicates the fluorescence signal with lower intensity. The unit is represented as photon count (photon/second (ph/s) ; fluorescence signal photon count per second).

Fig. 8B is the image data of 48 hours after administration, illustrating the accumulation property of a liposome prepared using a natural ganglioside (Lot. No. TGANG14) on the tumor site. The upper figures show the group administered with 100μl of the liposome (0 μg/mL), and the lower figures show the group administered with 100μl of the liposome (50 μg/mL). The figures respectively show data of different individuals. The scale shown in the rightmost portion shows intensity of a fluorescence signal obtained by imaging. In the imaging diagram, the whiter color in the higher portion of the scale indicates the fluorescence signal with higher intensity, and the color in the lower portion of the scale indicates the fluorescence signal with lower intensity. The unit is represented as photon count (photon/second (ph/s) ; fluorescence signal photon count per second).

Fig. 8C is the image data of 24 hours after administration, illustrating the accumulation property of a liposome prepared using a natural ganglioside (Lot. No. TGANG13) on the tumor site. The upper figures show the group administered with 100μl of the liposome (0 μg/mL), and the lower figures show the group administered with 100μl

of the liposome (50 μg/mL). The figures respectively show data of different individuals. The scale shown in the rightmost portion shows intensity of a fluorescence signal obtained by imaging. In the imaging diagram, the whiter color in the higher portion of the scale indicates the fluorescence signal with higher intensity, and the color in the lower portion of the scale indicates the fluorescence signal with lower intensity. The unit is represented as photon count (photon/second (ph/s) ; fluorescence signal photon count per second).

Fig. 8D is the image data of 48 hours after administration, illustrating the accumulation property of a liposome prepared using a natural ganglioside (Lot. No. TGANG13) on the tumor site. The upper figures show the group administered with 100μl of the liposome (0 μg/mL), and the lower figures show the group administered with 100μl of the liposome (50 μg/mL). The figures respectively show data of different individuals. The scale shown in the rightmost portion shows intensity of a fluorescence signal obtained by imaging. In the imaging diagram, the whiter color in the higher portion of the scale indicates the fluorescence signal with higher intensity, and the color in the lower portion of the scale indicates the fluorescence signal with lower intensity. The unit is represented as photon count (photon/second (ph/s) ; fluorescence signal photon count per second).

Fig. 9 illustrates the relation between the density of sugar chain and the accumulation property of a liposome using a natural ganglioside (TGANG13). It was found that the density of sugar chain greatly influences the accumulation property on the inflammatory site. Fig. 9 shows, from the above, the groups in which the density of sugar chain at the time of binding reaction is 0 μg/mL, 15 μg/mL, 50 μg/mL, 100 μg/mL, 200 μg/mL, and 500 μg/mL. The scale shown in the rightmost portion shows intensity of a fluorescence signal obtained by imaging. In the imaging diagram, the whiter color in the higher portion of the scale indicates the fluorescence signal with higher intensity, and the color in the lower portion of the scale indicates the fluorescence signal with lower intensity. The unit is represented as photon count (photon/second (ph/s) ; fluorescence signal photon count per second).

Fig. 10 illustrates the relation between accumulation on the tumor site and the density of the sugar chain of a liposome using a natural ganglioside (Lot.TGANG14) . A corresponds to the image data of 24 hours after administration, and B corresponds to the image data of 48 hours after administration. Fig. 10 shows, from the above, imaging diagrams as the result of confirming the accumulation property of liposomes prepared so as to have a density of sugar chain of 0 μg/mL, 10 μg/mL, 20 μg/mL, 50 μg/mL and 500 μg/mL. As a result, the natural ganglioside (Lot.TGANG13) liposome exhibited the highest accumulation property at 50 μg/mL. On the other hand, the natural ganglioside (Lot.TGANG14) liposome could be confirmed to exhibit the highest accumulation property on the tumor site at 10 and 20 μg/mL. Thus, it could be confirmed that the bond density of the sugar chain to the liposome varied due to the difference between ganglioside lots. The scale shown in the rightmost portion shows intensity of a fluorescence signal obtained by imaging. In the imaging diagram, the whiter color in the higher portion of the scale indicates the fluorescence signal with higher intensity, and the color in the lower portion of the scale indicates the fluorescence signal with lower intensity. The unit is represented as photon count (photon/second (ph/s); fluorescence signal photon count per second).

Fig. 11 illustrates the relation between accumulation on the tumor site and the density of the sugar chain of a liposome using a natural ganglioside (Lot.TGANG16). A corresponds to the image data of 24 hours after administration, and B corresponds to the image data of 48 hours after administration. Fig. 10 shows, from the above, imaging diagrams as the result of confirming the accumulation property of liposomes prepared so as to have a density of sugar chain of 0μg/mL, 10μg/mL, 20μg/mL, 50μg/mL and 500μg/mL. As a result, the natural ganglioside (Lot.TGANG13) liposome exhibited the highest accumulation property at 50μg/mL. On the other hand, the natural ganglioside (Lot.TGANG14) liposome could be confirmed to exhibit the highest accumulation property on the tumor site at 10 and 20μg/mL. The scale shown in the rightmost portion shows intensity of a fluorescence signal obtained by imaging. In the imaging diagram, the whiter color in the higher portion of the scale indicates the fluorescence signal with higher intensity, and the color in the lower portion of the scale indicates the fluorescence signal with lower intensity. The unit is represented as photon count (photon/second (ph/s); fluorescence signal photon count per second).

Fig. 12A corresponds to the image data of 24 hours after administration, illustrating the accumulation property on the tumor site of a synthetic GM3 (plant ceramide) liposome. The upper figures show the group administered with 100μl of the liposome (0 μg/mL), and the lower figures show the group administered with 100μl of the liposome (15μg/mL). It could be confirmed that, at a bond density of sugar chain of 15μg/mL, in comparison with a liposome without a sugar chain, the liposome significantly accumulated on the tumor site after 24 hours from the administration. The scale shown in the rightmost portion shows intensity of a fluorescence signal obtained by imaging. In the imaging diagram, the whiter color in the higher portion of the scale indicates the fluorescence signal with higher intensity,

and the color in the lower portion of the scale indicates the fluorescence signal with lower intensity. The unit is represented as photon count (photon/second (ph/s); fluorescence signal photon count per second).

Fig. 12B corresponds to the image data of 48 hours after administration, illustrating the accumulation property on the tumor site of a synthetic GM3 (plant ceramide) liposome. The upper figures show the group administered with 100μl of the liposome (0 μg/mL), and the lower figures show the group administered with 100μl of the liposome (15μg/mL). It could be confirmed that, at a bond density of sugar chain of 15μg/mL, in comparison with a liposome without a sugar chain, the liposome significantly accumulated on the tumor site after 48 hours from the administration. The scale shown in the rightmost portion shows intensity of a fluorescence signal obtained by imaging. In the imaging diagram, the whiter color in the higher portion of the scale indicates the fluorescence signal with higher intensity, and the color in the lower portion of the scale indicates the fluorescence signal with lower intensity. The unit is represented as photon count (photon/second (ph/s); fluorescence signal photon count per second).

Fig. 12C illustrates the accumulation property on the tumor site of a synthetic GM3 (plant ceramide) liposome. The upper figures show the group administered with a liposome without a sugar chain, and the lower figures show the group administered with the sialyl Lewis X liposome. Both upper figures and lower figures show, from the above, the results of imaging just after the administration, 24, 48 and 72 hours after administration. It could be confirmed that, at a bond density of sugar chain of 15 μg/mL, in comparison with the liposome without a sugar chain, the liposome significantly accumulated on the tumor site for any of the periods corresponding to 24 hours, 48 hours and 72 hours after administration. The scale shown in the rightmost portion shows intensity of a fluorescence signal obtained by imaging. In the imaging diagram, the whiter color in the higher portion of the scale indicates the fluorescence signal with higher intensity, and the color in the lower portion of the scale indicates the fluorescence signal with lower intensity. The unit is represented as photon count (photon/second (ph/s); fluorescence signal photon count per second).

Fig. 13A is a schematic diagram of the addition of a sugar chain and FITC to a liposome. $BS^3$: bis(sulfosuccinimidyl) suberate, Tris: tris(hydroxymethyl)aminomethane, HSA: human serum albumin, DTSSP: 3,3-dithiobis(sulfosuccinimidylpropionate), FITC: fluorescein-4-isothiocyanate. A summary of each step is described below. At the same time as for liposome formation, the Cy5.5-labeled HSA is encapsulated (step A). On the surface of the liposome, Tris is bound via a crosslinking agent $BS^3$ (step B). The ganglioside embedded in the liposomal membrane forms a raft on the surface of the liposome, as shown by the white arrow. HSA is coupled on the raft. When the size and properties (ganglioside properties) of the raft change, the coupling amount of HSA also varies (step C). To the HSA on the surface of the liposome, the sugar chain and Tris are bound via a crosslinking agent. When the amount of HAS varies, the amounts of sugar chain and FITC bound to the surface also vary (step D).

Fig. 13B illustrates the relation between the amount of FITC (nM) and fluorescence intensity (calibration curve) . The X-axis corresponds to the amount of FITC (nM), and the Y-axis corresponds to fluorescence intensity. $y = 3E - 06x^2 + 0.0101x + 0.1749$, $R^2 = 0.9963$.

Fig. 14 is a graph showing the evaluation of the amount of sugar chain binding to a surface of a synthetic GM3 (plant ceramide) liposome based on the amount of binding FITC. Fig. 14 shows an amount of FITC binding to the surface of the liposome (FITC concentration at the time of reaction: 0 to 4000μM). FITC concentration-dependent behavior at the time of binding reaction was observed. At concentrations of equal to or higher than 1000μM, increases of the binding amount were not observed. It was found that, on the surface of the synthetic GM3 (plant ceramide) liposome and the natural ganglioside (TGANG14) liposome, thesamedegreeofFITCbinds. The X-axis corresponds to the FITC concentration (μM) at the time of reaction, and the Y-axis corresponds to the amount of binding FITC (nM). White rhomboids show the synthetic GM3 liposome, and black rhomboids show the liposome prepared using a naturally-derived ganglioside.

Fig. 15 is a detailed graph of the portion of 0 to 300μM in Fig. 14 illustrating the amount of FITC binding on the surface of the liposome. It was found that, on the surface of the synthetic GM3 (plant ceramide) liposome and the surface of the natural ganglioside (TGANG14) liposome, the same degrees of FITC bind. The X-axis corresponds to the FITC concentration (μM) at the time of reaction, and the Y-axis corresponds to the amount of binding FITC (nM). White rhomboids show the synthetic GM3 liposome, and black rhomboids show the liposome prepared using a naturally-derived ganglioside.

Fig. 16 is a correlation diagram illustrating the difference between a conventional liposome (natural ganglioside (TGANG14)) and a synthetic GM3 (natural ceramide) liposome in the amount of binding FITC. The X-axis corresponds to the amount of FITC in the conventional liposome (natural ganglioside (TGANG14)), and the Y-axis corresponds to the amount of FITC in the synthetic GM3 (natural ceramide) liposome. y = 0.8575x - 0.0019,$R^2$ = 0.9957.

Fig. 17A shows the imaging data obtained by using explore Optix. Fig. 17A shows, from the above, the imaging data of a rat administered with a plant GM3 liposome, a rat administered with a porcine-derived total ganglioside liposome, a rat administered with a pseudo-GM3 liposome, and a rat administered with a natural GM3 liposome. The scale shown in the rightmost portion shows intensity of a fluorescence signal obtained by imaging. In the imaging diagram, the whiter color in the higher portion of the scale indicates the fluorescence signal with higher intensity, and the color in the lower portion of the scale indicates the fluorescence signal with lower intensity. The unit is represented as photon count (photon/second(ph/s); fluorescence signal photon count per second).

Fig. 17B is a graph numerically expressing the imaging images shown in Fig. 17A based on photon count. A: rat administered with the plant GM3 liposome; B: rat administered with the porcine-derived total ganglioside liposome; C: rat administered with the pseudo-GM3 liposome; and D: rat administered with the natural GM3 liposome. The Y-axis corresponds to photon count (photon/second (ph/s)).

BEST MODE FOR CARRYING OUT THE INVENTION

Hereinafter, preferred embodiments of the present invention will be described. It should be recognized that those skilled in the art can appropriately carry out the embodiments and the like in view of the description of the invention and well-known and commonly used techniques in the art and readily understand actions and effects attained by the invention.

Hereinafter, embodiments of each invention are described in detail.

Hereinafter, the present invention will be described. Throughout herein, an article in the singular form (for example, "a", "an" or "the" in the English language) should be understood to include a concept of its plural form, unless specified otherwise. Furthermore, it should be understood that the terms used herein are used in a meaning normally used in the art, unless specified otherwise. If there is a contradiction, the present specification (including definitions) precedes.

(List of abbreviation)

The following abbreviated representations are used herein depending on necessity.

Ac: acetyl $Ac_2O$: acetic anhydride
BDA: benzaldehyde dimethyl acetal
$BF_3 \cdot OEt_2$: trifluoroborane diethyletherate
Bn: benzyl
Bz: benzoyl
$Bz_2O$: benzonic anhydride
CSA: (±)-camphorsulfonic acid
DBTO: dibutyl tin oxide
DBU: 1,8-diazabicyclo[5.4.0]undec-7-ene
DEAD: azodicarboxylic acid diethyl ester
DMAP: 4-dimethylaminopyridine
DMF: N,N-dimethylformamide
DMTST: dimethyl(methylthio)sulfonium triflate
Et: ethyl
Me: methyl
MP: p-methoxyphenyl
MPM: p-methoxyphenylmethyl MS3A: molecular sieves 3Å
MS4A: molecular sieves 4Å
$PPh_3$: triphenylphosphine
TBAB: tetrabutylammonium bromide
TEA: triethylamine
TFA: trifluoroacetic acid
THF: tetrahydrofuran
p-TsOH: p-toluenesulfonic acid

WSC: 1-ethyl-3-(3-dimethylaminopropyl)-carbodiimide hydrogenchloride

(General technique)

In carrying out organic-chemical techniques in the present invention, one can refer to experimental manuals *such as* JIKKENKAGAKUKOZA (Lecture of Experimental Chemistry), The Chemical Society of Japan ed. Maruzen, 4th Edition, 1992), YUKIKAGAKU JIKKEN NO TEBIKI (Guidance to Organic Chemical Experiments) 1-5, KAGAKU DOJIN, TORIATSUKAI CHUI SHIYAKU LABO GUIDE (Laboratory Guide for Reagents Which Requires Careful Handling), TOKYO CHEMICAL INDUSTRY CO., LTD. ed., Kodansha Scientific Ltd., SEIRIKASSEI TOSA KENKYUHO SEIBUTSUKAGAKU JIKKENHO (Study of Physiologically Active Sugar Chain, Biochemical Experimental Method) 42, Japan Scientific Societies Press, SEIMITSU YUKIGOSEI JIKKENMANUAL (Precise Organic Synthesis) Nankodo Co., Ltd. and the like, the entire contents of which are incorporated herein as reference.

Regarding synthesis of glycolipids, one can refer to literatures such as Kameyama, A.; Ishida, H.; Kiso, M.; Hasegawa, A. Cabohydr. Res. 1990, 200, 269-285; Hasegawa, A.; Nagahama, T.; Ohki, H.; Kiso, M. J. Carbohydr. Chem. 1992, 11, 699-714; Ando, H.; Ishida, H.; Kiso, M.; Hasegawa, A. Carbohydr. Res. 1997, 300, 207-217; Ishida, H.-K.; Ishida, H.; Kiso, M.; Hasegawa, A. Carbohydr. Res. 1994, 260, C1-C6 and the like, the entire contents of which are incorporated herein as reference.

(Definition of terms)

Hereinafter, definition of the terms used particularly herein will be described.

As used herein, the term "sugar chain" refers to a compound having one or more sugar units (monosaccharides and/or derivatives thereof) bound to each other. When two or more sugar units are bound to each other, the unit sugars are bound to each other via a glycoside bond formed by dehydration condensation. Examples of such sugar chains include, but are not limited to, a wide variety of sugar chains, such as polysaccharides contained in a living body (glucose, galactose, mannose, fucose, xylose, N-acetylglucosamine, N-acetylgalactosamine, sialic acid and complexes and derivatives thereof), as well as sugar chains degraded or induced from degraded polysaccharides and composite biological molecules such as glycoproteins, proteoglycans, glycosaminoglycans, glycolipids and the like. Thus, the term "sugar chain" is used herein interchangeably as "polysaccharides," "sugars," and "carbohydrates." Furthermore, as used herein, the term "sugar chain" may mean both a sugar chain and a sugar chain-containing substance, unless specified otherwise. Typically, sugar chains are linear substances in which about 20 kinds of monosaccharides (glucose, galactose, mannose, fucose, xylose, N-acetylglucosamine, N-acetyl galactosamine, sialic acid, and complexes and derivatives thereof) are bound to each other and are attached to various intracellular and extracellular proteins and lipids. Sugar chains have different functions according to the monosaccharide sequence and are normally branched in a complicated manner. It is expected that there are several hundreds or more kinds of sugar chains with various structures in the human body, and it is believed that there are several tens of thousands or more kinds of structures useful in the human body. Sugar chains are supposedly involved in the higher functions of proteins and lipids in a living body, such as intercellular molecule/cell recognition function, but many parts of the mechanisms of such functions have not yet been elucidated. Sugar chains are currently attracting attention in the field of life science as the third life-related chain, following nucleic acids and proteins. In particular, there are high expectations of a function of sugar chains as a ligand (information molecule) in recognition of cell, and application of sugar chains to development of high-functional materials has been studied.

As used herein, the term "sugar chain group" is a name given when a sugar chain binds with another group. Depending on the case, monovalent or bivalent sugar chain groups are referred to. For example, sugar chain groups include sialyl Lewis X group, N-acetyllactosamine group and $\alpha$1,6-mannobiose group.

As used herein, the term "sugar" or "monosaccharide" refers to apolyhydroxyaldehyde or a polyhydroxyketone having at least one hydroxyl group and at least one aldehyde group or ketone group, that constitutes the basic unit of a sugar chain. The sugar, as used herein, is also referred to as carbohydrate, and these terms are used interchangeably. The term "sugar chain," as used herein, refers to a chain or a sequence of one or more sugars bound to each other, while the term "sugar" or "monosaccharide" refers to a unit constituting the sugar chain, when particularly specified. Herein, sugars having the repetition numbers n of 2, 3, 4, 5, 6, 7, 8, 9 and 10 are respectively referred to as diose, triose, tetrose, pentose, hexose, heptose, octose, nonose and decose. Sugars generally correspond to an aldehyde or a ketone of a chain polyhydric alcohol. The former is referred to as aldose, and the latter is referred to as ketose. In the present invention, sugars in any form may be used.

Sugar chains which may be used in the present invention may be synthesized by general glycolipid synthesis methods. Such methods include the following: (1) a method by chemical synthesis; (2) a fermentation technique using a genetically recombinant cell or microorganism; (3) a method by synthesizing using a sugar-hydrolyzing enzyme (glycosidase); and (4) a method by synthesizing using a sugar-transferring enzyme (glycosiltransferase) (see WO 2002/081723, Japanese Laid-Open Publication No. 9-31095, Japanese Laid-Open Publication No. 11-42096, Japanese

Laid-Open Publication No. 2004-180676, Kenichi Hatanaka, Shinichiro Nishimura, Tatsuro Ouchi and Kazukiyo Kobayashi, (1997) TOSHITSU NO KAGAKU TO KOGYO (Science and Industry of Saccharides), Kodansha Ltd., Tokyo, and the like).

Nomenclature system and abbreviations used for describing sugars in the present invention follow normal nomenclature system. For example, β-D-galactose

[Chem. 36]

β-D-Galactose

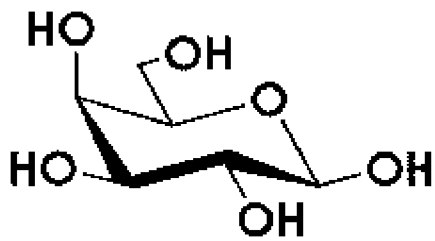

is expressed by Gal;
N-acetyl-α-D-galactosamine

[Chem. 37]

N-Acetyl-α-D-galactosamine

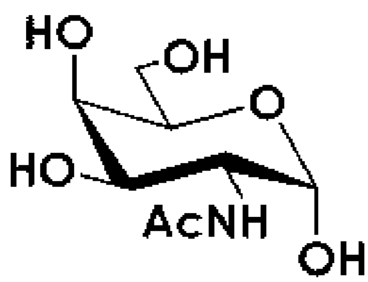

is expressed by GalNAc;
α-D-mannose

[Chem. 38]

α-D-Mannose

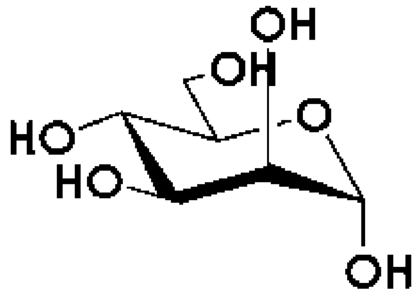

is expressed by Man;
β-D-glucose
[Chem. 39]

β-D-Glucose

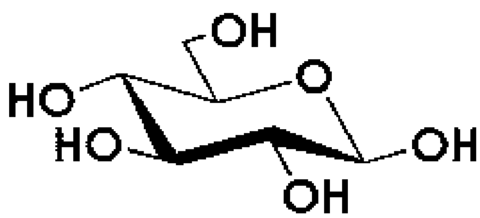

is expressed by Glc;
N-acetyl-β-D-glucosamine
[Chem. 40]

N-Acetyl-β-D-glucosamine

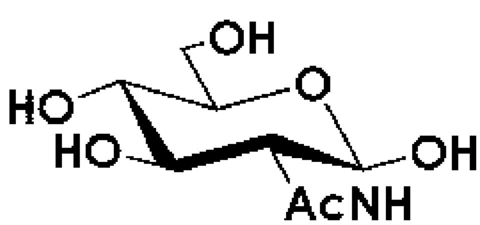

is expressed by GlcNAc;

α-L-fucose
[Chem. 41]

α-L-Fucose

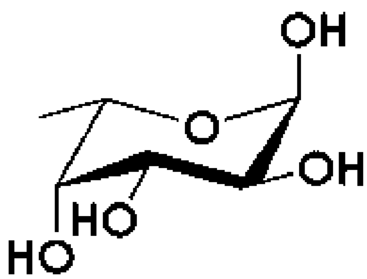

is expressed by Fuc;
α-N-acetylneuraminic acid
[Chem. 42]

α-N-Acetyl neuraminic acid

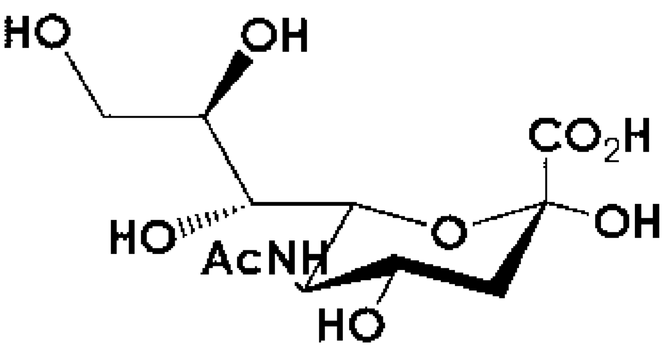

is expressed by Neu5Ac; and
ceramide is expressed by Cer.

Two cyclic anomers are expressed by α and β anomers. For representation reasons, they may also be expressed by "a" and "b" anomers. Thus, herein, "α" and "a" and "β" and "b" are used interchangeably in the expression of anomers.

As used herein, the term "galactose" refers to any isomer, typically β-D-galactose. Unless specified otherwise, the term "galactose" is used to refer to β-D-galactose.

As used herein, the term "acetyl galactosamine" refers to any isomer, typically N-acetyl-α-D-galactosamine. Unless specified otherwise, the term "acetyl galactosamine" is used to refer to N-acetyl-α-D-galactosamine.

As used herein, the term "mannose" refers to any isomer, typically α-D-mannose. Unless specified otherwise, the term "mannose" is used to refer to α-D-mannose.

As used herein, the term "glucose" refers to any isomer, typically β-D-glucose. Unless specified otherwise, the

term "glucose" is used to refer to β-D-glucose.

As used herein, the term "acetylglucosamine" refers to any isomer, typically N-acetyl-β-D-glucosamine. Unless specified otherwise, the term "acetylglucosamine" is used to refer to N-acetyl-β-D-glucosamine.

As used herein, the term "fucose" refers to any isomer, typically α-L-fucose. Unless specified otherwise, the term "fucose" is used to refer to α-L-fucose.

As used herein, the term "sialic acid" generically refers to neuraminic acid derivatives. N-acyl (N-acetyl or N-glycolyl)neuraminic acid and N-acyl-O-acetylneuraminic acid are naturally present. "Sialic acid" is a generic term for amino sugars having a carbon number of 9 or more, and is expressed as N- or 0-acyl derivatives of neuraminic acid.

As used herein, the term "acetylneuraminic acid" refers to any isomer, typically α-N-acetylneuraminic acid. Unlessspecifiedotherwise,theterm"acetylneuraminic acid" is used to refer to α-N-acetylneuraminic acid.

It shouldbe noted that, as used herein, the notations, names, and abbreviated names (such as Glc) and the like of sugars are different in a case in which they express a monosaccharide and a case in which they are used in a sugar chain. In a sugar chain, a sugar unit is bound to another saccharide unit, with a hydrogen atom or a hydroxyl group removed from the other saccharide unit by dehydration condensation. Accordingly, it is understood that, when the abbreviated symbols of sugars are used to represent a monosaccharide, all hydroxyl groups are present, but when they are used in a sugar chain, such refer to the state where a hydroxyl group has been removed as a result of dehydration condensation with a hydroxyl group of the other sugar bound to the sugar and only oxygen remains.

When a sugar is covalently bound with albumin, a reducing terminal of the sugar is aminated, and the sugar can bind to another component such as albumin via the amine group. It should be noted that in such a case "sugar" refers to a sugar with a hydroxyl group of the reducing terminal thereof substituted with an amine group.

Generally, monosaccharides bind to each other to form a disaccharide or a polysaccharide through glycoside bonds. The direction of the bond with respect to the ring plane is expressed by α and β. Particular carbon atoms forming a bond between two carbons are also described.

In the present specification, if necessary, a sugar chain is expressed by, but is not limited to:

[Chem. 44]

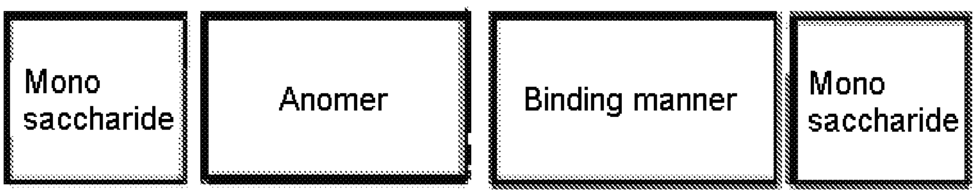


When this manner is used, for example, a β glycoside bond between galactose C-1 and glucose C-4 is expressed by β, 4. Thus, for example, sialyl Lewis X (SLX) is represented by Neu5Acα2,3Galβ1,4(Fucα1,3)GlcNAc. N-acetyllactosamine (G4GN) is represented by Galβ,4GlcNAc. α-1,6-Mannobiose (A6) is represented by Manα1,6Man. In the present specification, if necessary, other notation may also be used.

A branched sugar chain is expressed using a parenthesis, as it is arranged immediately left of the monosaccharide to be bound. For example, the following is expressed:

[Chem. 45]

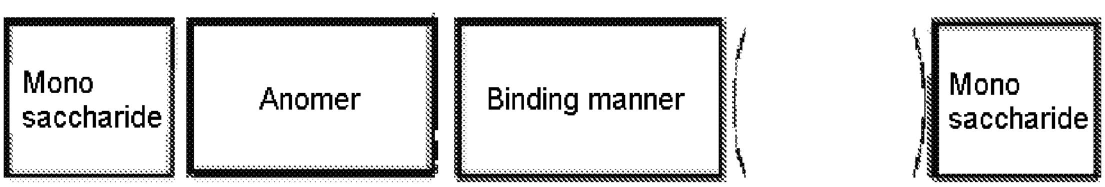


wherein the branched sugar chain in the parenthesis is expressed by:

[Chem. 46]

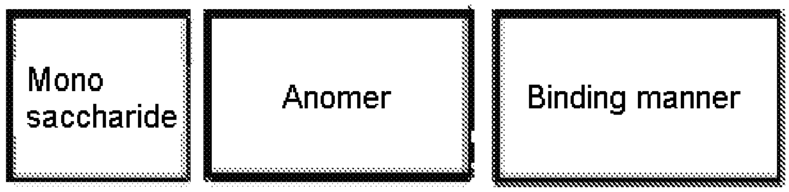


Thus, for example, when galactose C-1 and glucose C-4 are bound to each other via β-glycoside bond, and further glucose C-3 and fucose C-1 are bound to each other via α-glycoside bond, it is represented by Galβ1,4(Fucα1,3) Glc.

A monosaccharide is expressed basically by putting a number as small as possible to a (latent) carbonyl atom group. Under general standard of organic chemical nomenclature, even when an atom group superior to the (latent) carbonyl atom group has been introduced into a molecule, the numbering described above is used.

[Chem. 47]

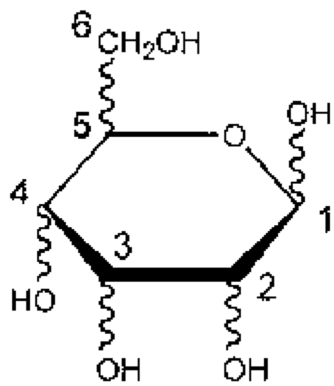

[Chem. 48]

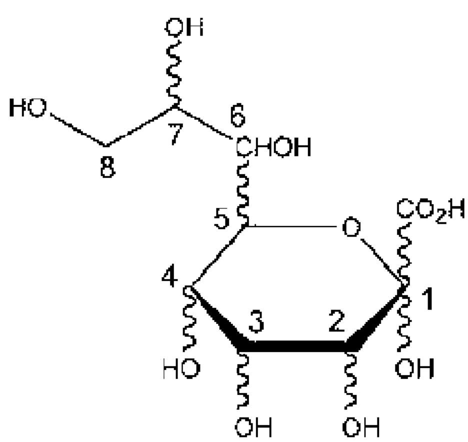

Sugar chains used herein include, but are not limited to, for example, sialyl Lewis X, N-acetyllactosamine, α1,6-mannobiose and combinations of two or more of them. The reason why combinations of two or more of them can

be used is, without being bound to a theory, that each of the sugar chains described above has specificity to lectin localized in a tissue or cell of a desired site of delivery and it is believed that such specificity is exhibited even if multiple sugar chains are present.

As used herein, the term "ganglioside" has the same meaning as used in the field of the art, and refers to glycosphingolipid including sialic acid. Examples of typical ganglioside include GM1, GM2, GM3, GM4, GD3, GD2, GD1a, GD1b, GT3, GT2, GT1a, GT1b, GT1c, GQ1b, GQ1c, GP1c and the like. The structures thereof are the following.

GM1:Gal$\beta$1-3GalNAc$\beta$1-4[NeuAc$\alpha$2-3]Gal$\beta$1-4Glc$\beta$1-1Cer
GM2:GalNAc$\beta$1-4[NeuAc$\alpha$2-3]Gal$\beta$1-4Glc$\beta$1-1Cer
GM3:NeuAc$\alpha$2-3Gal$\beta$1-4Glc$\beta$1-1Cer
GM4:NeuAc$\alpha$2-3Gal$\beta$1-1Cer
GD3:NeuAc$\alpha$2-8NeuAc$\alpha$2-3Gal$\beta$1-4Glc$\beta$1-1Cer
GD2:GalNAc$\beta$1-4[NeuAc$\alpha$2-8NeuAc$\alpha$2-3]Gal$\beta$1-4Glc$\beta$1-1Cer
GD1a:NeuAc$\alpha$2-3Gal$\beta$1-3GalNAc$\alpha$1-4[NeuAc$\alpha$2-3]Gal$\beta$1-4Glc$\beta$1-1Cer
GDlb:Galpl-3GalNAcpl-4[NeuAca2-8NeuAca2-3]Galpl-4Glcpl-1Cer
GT3:NeuAc$\alpha$2-8NeuAc$\alpha$2-8NeuAc$\alpha$2-3Gal$\beta$1-4Glc$\beta$1-1Cer
GT2:GalNAc$\alpha$1-4[NeuAc$\alpha$2-8NeuAc$\alpha$2-8NeuAc$\alpha$2-3]Gal$\beta$1-4Glc$\beta$1 -1Cer
GT1a:NeuAc$\alpha$2-8NeuAc$\alpha$2-3Gal$\beta$1-3GalNAc$\beta$1-4[NeuAc$\alpha$2-3]Gal$\beta$ 1-4Glc$\beta$1-1Cer
GT1b:NeuAc$\alpha$2-3Gal$\beta$1-3GalNAc$\beta$1-4[NeuAc$\alpha$2-8NeuAc$\alpha$2-3]Gal$\beta$ 1-4Glc$\beta$1-1Cer
GTlc:Gal$\beta$1-3GalNAc$\beta$1-4[NeuAc$\alpha$2-8NeuAc$\alpha$2-8NeuAc$\alpha$2-3]Gal$\beta$ 1-4Glc$\beta$1-1Cer
GQ1b:NeuAc$\alpha$2-8NeuAc$\alpha$2-3Gal$\beta$1-3GalNAc$\beta$1-4[NeuAc$\alpha$2-8NeuAc $\alpha$2-3]Gal$\beta$1-4Glc$\beta$1-1Cer
GQ1c:NeuAc$\alpha$2-3Gal$\beta$1-3GalNAc$\beta$1-4[NeuAc$\alpha$2-8NeuAc$\alpha$2-8NeuAc $\alpha$2-3]Gal$\beta$1-4Glc$\beta$1-1Cer
GPlc:NeuAc$\alpha$2-8NeuAc$\alpha$2-3Gal$\beta$1-3GalNAc$\beta$1-4[NeuAc$\alpha$2-8NeuAc $\alpha$2-8NeuAc$\alpha$2-3]Gal$\beta$1-4Glc$\beta$1-1Cer

As used herein, the term "glycolipid-containing liposome" refers to any liposome containing a glycolipid. A glycolipid-containing liposome of the present invention is **characterized in that** it mainly contains a synthesized glycolipid (synthetic glycolipid). A glycolipid is preferably incorporated in a liposomal membrane.

As used herein, the term "ganglioside-containing liposome" refers to any liposome containing ganglioside.

As used herein, "a component accompanying a naturally-derived ganglioside" refers to a component which accompanies ganglioside obtained by extraction from animals, plants or the like and which cannot be removed by normal operation. Ganglioside is extracted using liposolubility as an indicator, and thus is readily contaminated by liposolubleproteins. Furthermore, even after purification into lipids alone, since ganglioside is a trace component in the total lipids, it is difficult to remove neutral glycolipids which are present in an overwhelmingly larger amount. Moreover, in a final acidic glycolipid fraction, abundant acidic glycolipids having a sulfuric acid group, such as sulfatide, are present, and separation from such acidic glycolipids is difficult. Furthermore, since a large amount of salts are used in extraction and production processes, contamination by salts is inevitable, even if a desalting operation is performed. Without wishing to be bound to a theory, it has been clarified that the absence of such components allows the present invention to attain the significant effects described herein. Naturally-derived ganglioside includes GM1, GM2, GM3, GM4, GD3, GD2, GD1a, GD1b, GT3, GT2, GT1a, GT1b, GT1c, GQ1b, GQ1c, GP1c and the like. This is because it is difficult to change the composition of such components contained within ganglioside.

As used herein, "absorbance of liposome at 680nm" refers to the absorbance of encapsulated Cy5.5. A numerical value of absorbance can be used as an indicator of an amount of Cy5.5 encapsulated. If a liposome has absorbance of 0.5 to 3.0 at 680nm, it means that a fluorescence signal can be detected from outside of the body by observation of a living body by *in vivo* imaging.

As used herein, the term "antibody" refers to an immunoglobulin molecule having a specific amino acid sequence induced by an antigen which is an immunogen. Antibodies are produced by B cells and are present in the blood and body fluids. Characteristically, an antibody specifically reacts with an antigen. Antibodies may be present naturally, not being generated as a result of stimulus caused by antigen presentation. Fundamentally, an antibody has a molecular structure composed of two light chains and two heavy chains, and it may also be present as a dimer, trimer or pentamer. Examples thereof include, but are not limited to, for example, IgA, IgE, IgM, IgG and the like. Antibodies used in an antibody-modified liposome of the present invention may include antibodies to an antigen which is specifically expressed in organs, diseases, immune cells and the like. Antibodies used in the antibody-modified liposome of the present invention may preferably be, but are not limited to, for example, antibodies to E-selectin or p-selectin which are expressed in an inflamed vascular endothelium. In one embodiment, an antibody which may be used in the antibody liposome of the present invention may be, for example, an anti-EGFR antibody or IgG. As used herein, "antibody" includes polyclonal antibodies, monoclonal antibodies, multispecific antibodies, chimeric antibodies and anti idiotype antibodies, and fragments thereof (forexample, F(ab')2 and Fab fragments) and other conjugates produced by recombination. Furthermore,

such antibodies may be covalently bound to or recombinantly fused with an enzyme, for example, alkaline phosphatase, horseradish peroxidases, α-galactosidase or the like.

As used herein, "an amount of antibody added" refers to a final concentration of an antibody added. For example, in production of an antibody-modified liposome, "an amount of antibody added" refers to a final concentration of antibody at the time of reacting the liposome with an antibody solution. In a case of an antibody-modified liposome via a bridging spacer, such an amount of antibody added refers to an antibody concentration at the time of reacting a liposome bound to a bridging spacer with an antibody solution. Herein, the "amount of antibody added" may also be referred to as "antibody amount."

As used herein, an amount of protein "calculated on the basis of an amount of antibody added" refers to an amount of protein calculated on the basis of a final concentration of an antibody solution at the time of producing an antibody-modified liposome.

As used herein, the term "lectin" refers to a substance capable of binding to a sugar chain of a cell membrane composite sugar (glycoproteins and glycolipids), which is capable of giving an effect such as cell agglutination, induction of division, activation of functions and cytotoxicity. If a sugar chain is considered as an information molecule transmitted by a cell, lectin can be considered as a receiving molecule. Cells or tissues having a certain property have corresponding lectin patterns. Lectins are involved in infection, biophylaxis, immunity, fertilization, targeting to a target cell, cell differentiation, intercellular adhesion, quality control of neogenetic glycoproteins, intracellular selective transportation, and the like. Lectins have various sugar chain-binding properties, and are under strict regulation due to their inherent physical chemical properties of rapid binding and dissociation. They are also called sugar chain-recognizingproteins. Plantlectinshavebeenstudied for many years, and there are as many as about 300 kinds of known lectins. Recently, animal lectins have also been under intensive study, and new lectins are constantly being discovered. Various sugar chain-recognizing functions based on the lectin group (about 100 kinds) in the main lectin families present on animal cell membranes have been studied. In particular, the function as a receptor (information-receiving protein or target molecule) for receiving the structural information of sugar chain ligands having various structures, has been attracting attention.

As used herein, the term "complementary nucleic acid" is defined to have the broadest sense in the art, and refers to a nucleic acid that can form a base pair with its corresponding nucleic acid by the base-pairing rules of nucleic acids. Examples thereof include, but are not limited to, DNA, RNA and the like.

As used herein, the term "receptor" is also referred to as "acceptor," and refers to what is present in a cell and has a structure for recognizing a stimulus from outside the cell and to transmit such into the cell. Examples thereof include, but are not limited to, cell surface acceptors, intracellular acceptors and the like.

As used herein, the term "ligand" refers to a molecule that *per* se is adsorbed very tightly to a substance. Examples thereof include, but are not limited to, proteins, nucleic acids, chemical substances and the like.

As used herein, the term "aptamer" refers to a nucleic acid having a relatively small molecular weight, that is capable of recognizing structures of various substances (proteins, chemical substances and the like) and binds thereto. Examples thereof include, but are not limited to, RNA aptamers, DNA aptamers and the like.

As used herein, the term "antigen" refers to a substance that promotes production of an antibody. Examples thereof include, but are not limited to, macromolecular sugars, proteins, complexes thereof, viruses, cells and the like.

(Liposome)

As used herein, the term "liposome" normally refers to a closed vesicle composed of a lipid layer formed by lipids gathering in a membrane shape and an internal aqueous layer. It is also possible to incorporate cholesterol, glycolipid or the like, as well as the phospholipids typically used. Since a liposome is a closed vesicle internally containing water, it is also possible to retain a water-soluble agent or the like in such a vesicle. Accordingly, such a liposome is used for delivering a pharmaceutical or gene that cannot penetrate a cell membrane into a cell. Furthermore, such a liposome also has good biocompatibility and is highly anticipated as a nanoparticle-carrying material for DDS. In the present invention, a liposome may have structural units having a functional group imparting ester bond (for example, glycolipid, ganglioside, phosphatidylglycerol or the like) or structural units having a functional group imparting peptide bond (for example, phosphatidylethanolamine) for attaching a modifying group.

(Preparation of liposome)

A liposome can be prepared by any method known in the art. For example, a liposome may be prepared by an ultrasonication method, an ethanol injection method, a French press method, an ether injection method, a cholate method, a freeze drying method, or a reverse-phase evaporation method (see, for example, "LIPOSOME OYO NO SHINTENKAI - JINKO SAIBO NOKAIHATSUNIMUKETE (New Development in Liposome Application -For Development of an Artificial Cell), Kazunari Akiyoshi/Kaoru Tsujii Ed., NTS pp. 33-45 (2005)" and "Liposomes," Shoshichi Nojima, pp. 21-40, Nankodo (1988)).

Among them, for example, the cholate dialysis method is exemplified. In the cholate dialysis method, production is carried out by a) preparation of a mixed micelle of lipids and a surfactant, and b) dialysis of the mixedmicelle. Next, in a preferred embodiment of a sugar-chain liposome according to the present invention, a protein is used preferably as a linker. A glycoprotein in which a sugar chain is bound to a protein can be coupled with the liposome in the following two-phase reaction: a) periodate oxidation of a glycolipid (for example, ganglioside) portion on the liposomal membrane; and b) coupling of a glycoprotein to the oxidized liposome in a reductive amination reaction. In this way, it is possible to bind a glycoprotein having a desirable sugar chain to the liposome and to obtain various kinds of glycoprotein-liposome conjugates having a desired sugar chain. It is very important to study the particle size distribution for examination of purity and stability of the liposome. As methods of studying the particle size distribution, gel filtration chromatography (GPC), scanning electron microscopy (SEM), dynamic light scattering (DLS) and the like can be used. For example, it is possible to prepare a liposome containing dipalmitoylphosphatidylcholine, cholesterol, ganglioside, dicetylphosphate, dipalmitoylphosphatidylethanolamine and sodium cholate at a ratio of 35:40:15:5:5:167. Such liposomes are stable even upon preservation at 4 degrees Celsius for several months. *In vivo* stability of a liposome can be investigated using a mouse. A liposome is intravenously injected to a mouse. The blood of the mouse is sampled three hours after the injection, and serum is prepared. Using a membrane with a pore size of 0.03μm, ultrafiltration is performed to purify and collect the liposome. As a result of SEM observation of the liposome, it can be confirmed that the form of the liposome is not changed from before to after the *in vivo* treatment for 3 hours and collection of the liposome.

Liposomes can be also prepared using a freeze-drying method. The freeze-drying method was reported, for example, in H. Kikuchi, N. Suzuki et al., Biopharm. Drug Dispos., 17, 589-605 (1999) and the like. For example, a liposome can be prepared by the following method. A liposome solution is frozen at -40 to -50 degrees Celsius, and then is freeze-dried. A solution of a substance to be encapsulated is added to the freeze-dried powder, and rehydration is performed. The substance which has not been encapsulated is removed by ultrafiltration or dialysis. Sugars and the like are optionally added to the first liposome solution. In addition, the particle size is adjusted by a French press method or a membrane filtration method, in accordance with the desired purpose.

As used herein, the term "synthetic ganglioside-containing liposome" refers to a liposome prepared using a synthesized ganglioside. Herein, the terms "synthetic ganglioside-containing liposome" and "synthetic ganglioside liposome" may be used interchangeably. Synthesized ganglioside includes, but is not limited to, for example, synthesized GM3, GM4, GD3, GD2, GD1a, GD1b, GT3, GT2, GT1a, GT1b, GT1c, GQ1b, GQ1c and GP1c. This is because they have N-acetylneuraminic acid (Neu-5AC) in their terminus and thus have binding ability with a bridging spacer via coupling reaction.

As used herein, the term "synthetic GM3 liposome" refers to a liposome prepared using a synthesized GM3. Examples of synthetic GM3 liposome include, but are not limited to, synthetic GM3 liposomes containing a natural ceramide, synthetic GM3 liposomes containing a plant ceramide, and synthetic GM3 liposomes containing a pseudo-ceramide.

As used herein, the term "synthetic GM3 sugar chain-modified liposome" refers to a synthetic GM3 liposome containing a sugar chain. Asusedherein, the term "synthetic GM3 antibody-modified liposome" refers to a synthetic GM3 liposome containing an antibody.

As used herein, the term "synthetic GM4 liposome" refers to a liposome prepared using a synthesized GM4. Examples of synthetic GM4 liposome include, but are not limited to, synthetic GM4 liposomes containing a natural ceramide, synthetic GM4 liposomes containing a plant ceramide, and synthetic GM4 liposomes containing a pseudo-ceramide.

As used herein, the term "synthetic GM4 sugar chain-modified liposome" refers to a synthetic GM4 liposome containing a sugar chain. Asusedherein, the term "synthetic GM4 antibody-modified liposome" refers to a synthetic GM4 liposome containing an antibody.

As used herein, the term "natural ceramide" mainly refers to ceramide contained in ganglioside derived from an animal.

As used herein, the term "plant ceramide" refers to ceramide derived from a plant, which contains one hydroxyl group instead of a double bond contained in a natural ceramide. The present ceramide can be obtained inexpensively and in a large amount by culturing an enzyme, and is suitable for mass synthesis of ganglioside.

As used herein, the term "plant ceramide portion" refers to a portion of a glycolipid composed of plant ceramide.

As used herein, the term "sugar chain portion" refers to a portion of a glycolipid composed of sugar chain.

As used herein, the term "pseudo-ceramide" refers to a ceramide-like substance which does not contain a double bond or a functional group such as a hydroxyl group, and which has a branched alkyl structure.

As used herein, the term "liposome raw material" refers to a lipid capable of forming a liposome. "Lipid capable of forming a liposome" may be the same as "lipid forming a glycolipid-containing liposome" as described below.

As used herein, conditions "in which a liposome is formed" refer to conditions for liposome formation. Conditions in which a liposome is formed may be, for example, subjecting a mixture of a glycolipid (for example, synthetic glycolipid) and a liposome raw material to ultrafiltration, leaving the mixture to stand overnight, and the like. More preferably,

conditions in which a liposome is formed may be subjecting such a mixture to ultrafiltration at a molecular weight cutoff of 500 to 300,000 (preferably, 10,000).

As used herein, the term "lipid" refers to a long-chain aliphatic hydrocarbon or a derivative thereof. "Lipid" is a generic term indicating compounds composed of fatty acids, alcohols, amines, amino alcohols, aldehydes and the like, for example.

Examples of lipids composing a glycolipid-containing liposome of the present invention include, but are not limited to, for example, phosphatidylcholines, phosphatidylethanolamines, phosphatidic acids, long-chain alkylphosphate salts, glycolipids (gangliosides and the like), phosphatidylglycerols, sphingomyelins, cholesterols and the like.

Examples of the phosphatidylcholines include, but are not limited to, dimyristoylphosphatidylcholine, dipalmitoylphosphatidylcholine, distearoylphosphatidylcholine and the like.

Examples of the phosphatidylethanolamines include, but are not limited to, dimyristoylphosphatidylethanolamine, dipalmitoylphosphatidylethanolamine, distearoylphosphatidylethanolamine and the like.

Examples of the phosphatidic acids include dimyristoylphosphatidic acid, dipalmitoylphosphatidic acid, and distearoylphosphatidic acid. Examples of the long-chain alkylphosphates include, but are not limited to, dicetylphosphate and the like.

Examples of the glycolipids include galactosyl ceramides, glucosyl ceramides, lactosyl ceramides, phosphatides, globosides, gangliosides and the like. Examples of the gangliosides include, but are not limited to, ganglioside GM1 (Galβ1,3GalNAcβ1,4(NeuAα2,3)Galβ1,4Glcβ1,1'Cer), ganglioside GDla, ganglioside GTlb, and the like.

Preferred examples of the phosphatidylglycerols include dimyristoylphosphatidylglycerol, dipalmitoylphosphatidylglycerol, distearoylphosphatidylglycerol and the like.

Among them, phosphatidic acids, long-chain alkylphosphates, glycolipids and cholesterols have an effect of enhancing the liposome stability and thus are preferably contained as constituent lipids. For example, examples of a lipid composing a liposome used in the present invention include, but are not limited to, a lipid containing one or more lipids (molar ratio: 0 to 30%) selected from the group consisting of phosphatidylcholines (molar ratio: 0 to 70%), phosphatidylethanolamines (molar ratio: 0 to 30%), phosphatidic acids, and long-chain alkylphosphates; one or more lipids (molar ratio: 0 to 40%), selected from the group consisting of glycolipids, phosphatidylglycerols and sphingomyelins; and cholesterols (molar ratio: 0 to 70%). The lipid preferably contains a ganglioside, a glycolipid or a phosphatidylglycerol, since these lipids facilitate binding of a linker such as albumin.

Examples of lipids composing a glycolipid-containing liposome of the present invention include, but are not limited to, dipalmitoylphosphatidylcholine, cholesterol, ganglioside, dicetylphosphate, dipalmitoylphosphatidylethanolamine, sodium cholate, dicetylphosphatidylethanolamine-polyglycerin 8G, dimyristoylphosphatidylcholine, distearoylphosphatidylcholine, dioleoylphosphatidylcholine, dimyristoylphosphatidylserine, dipalmitoylphosphatidylserine, distearoylphosphatidylserine, dioleoylphosphatidylserine, dimyristoylphosphatidylinositol, dipalmitoylphosphatidylinositol, distearoylphosphatidylinositol, dioleoylphosphatidylinositol, dimyristoylphosphatidylethanolamine, distearoylphosphatidylethanolamine, dioleoylphosphatidylethanolamine, dimyristoylphosphatidic acid, dipalmitoylphosphatidic acid, distearoylphosphatidic acid, dioleoylphosphatidic acid, galactosylceramide, glucosylceramide, lactosylceramide, phosphatide, globoside, GM1 (Galβ1,3GalNAcβ1,4(NeuAα2,3)Galβ1,4Glcβ1,1'Cer), ganglioside GD1a, ganglioside GD1b, dimyristoylphosphatidylglycerol, dipalmitoylphosphatidylglycerol, distearoylphosphatidylglycerol, dioleoylphosphatidylglycerol, and the like. Preferably, the lipid may be dipalmitoylphosphatidylcholine, cholesterol, ganglioside, dicetylphosphate, dipalmitoylphosphatidylethanolamine, sodium cholate, dicetylphosphatidylethanolamine-polyglycerin 8G, dipalmitoylphosphatidylcholine, or dipalmitoylphosphatidylglycerol.

In a preferred embodiment, ganglioside, glycolipid or phosphatidylglycerol can be included in a liposome, and a linker such as peptide can be bound to it to bind a sugar chain.

By blending ganglioside, glycolipid or phosphatidylglycerol when producing a liposome, it is possible to produce a glycolipid-containing liposome modified with a sugar chain, which contains a sugar chain included in such a glycolipid as a constituent component.

In another preferred embodiment, a liposome in the present invention preferably contains phosphatidylethanolamine, because inclusion of phosphatidylethanolamine facilitates binding with a hydrophilicity-imparting group (such as tris(hydroxyalkyl)aminoalkane).

(Liposome modified with a target site-recognizing probe)

As used herein, the term "target site-recognizing probe" refers to a substance which serves as an indicator for detecting/observing a desired target site or substance. Examples of target site-recognizing probe include, but are not limited to, for example, sugar chain, antibody, antigen, functional peptide, nucleic acid (aptamer), hyaluronic acid and the like, because any substance can be used as long as it can be added to a liposome and allow detection/observation of a desired target site or substance. Preferably, a target site-recognizing probe can be chemically bound to a liposome via a bridging spacer.

As used herein, the term "liposome modified with a target site-recognizing probe" refers to a substance containing a target site-recognizing probe and a liposome, and preferably refers to a liposome modified by direct or indirect binding of a target site-recognizing probe (for example, sugar chain, antibody or the like).

(Sugar chain-modified liposome)

As used herein, the term "sugar chain-modified liposome" refers to a substance containing a sugar chain and a liposome, and preferably refers to a liposome modified by direct or indirect binding of a sugar chain. Herein, the terms "sugar chain-modified liposome," "sugar chain liposome" and "sugar chain-bound liposome" may be used interchangeably.

As used herein, the term "SLX-modified liposome" refers to a liposome modified with SLX. A form in which a sugar chain has bound to a liposome is specifically represented as:

structure I, X-$R^1$-$R^2$-$R^3$, wherein:

X is a group resulting from removal of a functional group a from a constitutional unit including the functional group a capable of forming a $CH_2$-NH bond with the linker protein contained in the liposome;
$R^1$ is a linker protein group;
$R^2$ is a linker protein-crosslinking group;
$R^3$ is a sugar chain; and
X and $R^1$ are bound via a $CH_2$-NH bond, $R^1$ and $R^2$ are bound via a peptide bond, and $R^2$ and $R^3$ are bond via a peptide bond. Thus, in more detail, the structure I can be represented

by the following structural formula:

$$X\text{-}CH_2\text{-}NH\text{-}R^1\text{-}NH\text{-}C(=O)\text{-}R^2\text{-}C(=O)\text{-}NH\text{-}R^3.$$

In the present invention, a sugar chain-modified liposome is hydrophilized by the following structure:

structure II, Y-$R^4$-$R^5$, wherein:

Y is a group resulting from removal of a functional group b from a constitutional unit including the functional group b capable of forming a peptide bond with a hydrophilic compound-crosslinking group contained in the liposome;
$R^4$ is a hydrophilic compound-crosslinking group;
$R^5$ is a hydrophilic compound group; and
Y and $R^4$ are bound via a peptide bond and $R^4$ and $R^5$ are bound via a peptide bond.

Thus, in more detail, the structure II can be represented by the following structural formula:

$$Y\text{-}NH\text{-}C(=O)\text{-}R^4\text{-}C(=O)\text{-}NH\text{-}R^5.$$

A sugar chain-modified liposome may have the aforementioned structures I and II, and may be optionally imparted with paramagnetism or fluorescence.

In the present invention, paramagnetism is imparted because at least one of the components of the glycolipid-containing liposome has paramagnetism, or because the glycolipid further has an additional component having paramagnetism.

As a component having paramagnetism, for example, paramagnetic metals (for example, gadolinium, iron and the like), contrast media (for example, iron oxide particles, gadolinium chelating agent, barium sulfate, water-soluble iodine and the like) may impart paramagnetism. But such a component is not limited to the above examples, because any substance may be used as long as it is capable of rendering a composition detectable in magnetic resonance imaging.

In the present invention, fluorescence is imparted because at least one of the components of the glycolipid-containing liposome has fluorescence, or because the glycolipid-containing liposome further has an additional component having fluorescence.

Examples of a component having fluorescence include, but are not limited to, for example, fluorochrome, fluorescent proteins (for example, GFP, CFP, YFP and the like), luminescent enzymes (for example, luciferases and the like). As a fluorochrome, for example, the following fluorochromes may impart fluorescence: cy5.5 (for example,

[Chem. 49]

<Cy5. 5>

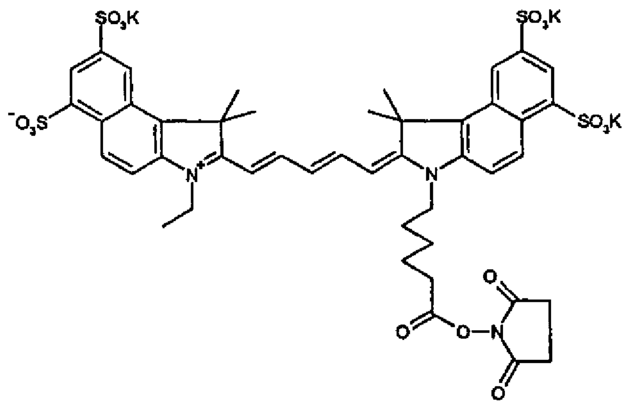

,

[Chem. 50]

<cy3>

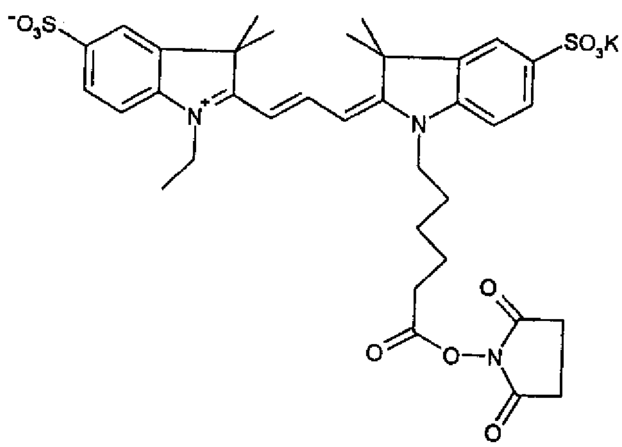

,

cy5, cy7, cy3B, cy3.5, Alexa Fluor350, Alexa Fluor488, Alexa Fluor532, Alexa Fluor546, Alexa Fluor555, Alexa Fluor568, Alexa Fluor594, Alexa Fluor633, Alexa Fluor647, Alexa Fluor680, Alexa Fluor700, Alexa Fluor750, fluorescein-4-isothiocyanate (FITC), europium-containing label, and combinations thereof.

A glycolipid-containing liposome modified with a target site-recognizing probe of the present invention (for example, sugar chain-modified liposome, antibody liposome and the like) may contain a target-recognizing probe (for example, sugar chain, antibody and the like) at a density appropriate for delivery to a desired site of delivery.

As used herein, the term "modification bond density" is an amount of sugar chain used in producing a sugar chain-modified liposome, and is expressed as a density of sugar chain binding to 1mg of lipid of a liposome (mg sugar chain/mg lipid). The terms "modification bond density" and "sugar chain density" may be used interchangeably. A modification bond density of a sugar chain-modified liposome is empirically almost proportional to a density of sugar chain binding to a liposome. Thus, those skilled in the art understand that modification bond density can be expressed by an amount of sugar chain used in preparation. Accordingly, herein, unless specified otherwise, modification bond

density is determined depending on an amount used in preparation. *In vitro,* for example, modification bond density can be indirectly determined using E-selectin. In a glycolipid-containing liposome of the present invention (for example, synthetic GM3 sugar chain-modified liposome), by selecting the type of sugar chain to be bound to a liposome andmodificationbonddensity, targetingpropertytoadesired site of delivery can be controlled. Hereinafter, Table 1 shows liposome numbers, sugar chain structure and modification bond density.

[Table 1]

**(Table 1)**

| Abbreviated name of sugar chain -modified liposome | Sugar chain level | Abbreviated name of sugar chain | Name (English) | Bond density (mg sugar chain / mg lipid) |
|---|---|---|---|---|
| **--** | **--** | **SLX** | **Sialyl Lewis X** | **-** |
| **K1-0*** | **0** | **-** | **-** | **-** |
| **K1-1** | **1** | **SLX** | **Sialyl Lewis X** | **0.0025** |
| **K1-2** | **2** | | | **0.0075** |
| **K1-3** | **3** | | | **0.025** |
| **K1-4** | **4** | | | **0.05** |
| **K1-5** | **5** | | | **0.1** |
| **K1-6** | **6** | | | **0.25** |
| **K1-7** | **7** | | | **0.5** |
| **--** | **--** | **G4GN** | **N-Acetyllactosamine** | **-** |
| **K2-0*** | **0** | **-** | **-** | **-** |
| **K2-1** | **1** | **G4GN** | **N-Acetyllactosamine** | **0.0025** |
| **K2-2** | **2** | | | **0.0075** |
| **K2-3** | **3** | | | **0.025** |
| **K2-4** | **4** | | | **0.05** |
| **K2-5** | **5** | | | **0.1** |
| **K2-6** | **6** | | | **0.25** |
| **K2-7** | **7** | | | **0.5** |
| **--** | **--** | **A6** | **$\alpha$ 1-6 Mannobiose** | **-** |
| **K3-0*** | **0** | **-** | **-** | **-** |
| **K3-1** | **1** | **A6** | **$\alpha$ 1-6 Mannobiose** | **0.0025** |
| **K3-2** | **2** | | | **0.0075** |
| **K3-3** | **3** | | | **0.025** |
| **K3-4** | **4** | | | **0.05** |
| **K3-5** | **5** | | | **0.1** |
| **K3-6** | **6** | | | **0.25** |
| **K3-7** | **7** | | | **0.5** |
| *: K1-0, K2-0 and K3-0 indicate liposomes not bound to a sugar chain. | | | | |

A sugar chain-modified liposome shown in the above Table may be produced by a method described below. Specifically, the method includes the steps of: (a) suspending and stirring a lipid in a methanol/chloroform solution, vaporizing the stirred solution and vacuum-drying the precipitate, thereby obtaining a lipid membrane; (b) suspending the lipid membrane in a suspending buffer solution and ultrasonicating the lipid membrane to provide a liposome; (c) hydrophilizing the liposome with tris(hydroxyalkyl)aminoalkane; (d) binding the hydrophilized liposome with a linker protein to produce a linker protein-bound liposome; and (e) binding a sugar chain described in the above table to the liposome to produce a sugar chain-modified liposome. By mixing the ultrasonically treated solution and a fluorescent label solution in the step (b), it is possible to impart fluorescence to the liposome.

Preferably, this fluorescent label solution may contain 1,1'-bis(ε-carboxypentyl)-3,3,3',3'-tetramethylindocarbocyanine-5,5'-disulfonate potassium salt, di-N-hydroxysuccinimide ester (cy5.5) or 1-(ε-carboxypentyl)-1'-ethyl-3,3,3',3'-tetramethylindocarboc yanine-5,5'-disulfonate potassium salt-N-hydroxysuccinimide ester (cy3).

In a preferred embodiment, in the production of a glycolipid-containing liposome of the present invention (for example, synthetic GM3 sugar chain-modified liposome), the linker protein in step (d) is human serum albumin. In step (e), the sugar chain is SLX, and the SLX and the liposome are bound under conditions appropriate for a desired treatment or diagnosis to produce an SLX-modified liposome.

The liposome and linker, and the linker and the sugar chain are preferably bound using a bifunctional crosslinking group (for example, 3,3'-dithiobis(sulfosuccinimidylpropionate)(DTSSP)) or the like.

As used herein, the term "linker" refers to a molecule which mediates binding of sugar chain and a surface of a liposome. In a glycolipid-containing liposome of the present invention (for example, synthetic GM3 sugar chain-modified liposome), a sugar chain may be bound to a surface of a liposome via a linker. A linker can be appropriately selected by those skilled in the art, but is preferably biocompatible, and more preferably, pharmaceutically acceptable. As used herein, the term "linker protein" refers to a polymer of protein, peptide and amino acid, among linker molecules. A linker protein used herein may be, for example, an organism-derived protein, preferably human-derived protein, more preferably, human-derived serum protein, and further more preferably, serum albumin. It has been confirmed by experimentation in mice that, particularly when human serum albumin is used, a high incorporation in each tissue is observed.

As used herein, "linker (protein) group" is a name given to a linker (protein) bound with another group. The term "linker (protein) group" refers to a monovalent or bivalent linker (protein) group depending on the case. Examples of linker (protein) group include, for example, mammal-derived protein groups, human-derived protein groups, human serum protein groups, and serum albumin groups. A linker (protein) group is preferably derived from a human, because it is believed to be highly compatible with administration to a human. Furthermore, a non-immunogenic protein is preferred.

As used herein, the term "crosslinking group" refers to a group forming a chemical bond between molecules of a chain macromolecule like bridging. Typically, the term "crosslinking group" refers to a group which acts between macromolecules such as a lipid, protein, peptide, sugar chain or the like and other molecules (for example, lipid, protein, peptide or sugar chain) and which forms a covalent bond binding a portion in or between molecules where there was a covalent bond. Herein, a crosslinking group varies depending on a desiredtargettobecrosslinked, andexamplesthereofinclude, but are not limited to, for example, aldehydes (for example, glutaraldehyde), carbodiimides, imide esters and the like. In the case of crosslinking an amino group-containing substance, an aldehyde-containing group, for example, glutaraldehyde can be used.

As used herein, the term "linker protein-crosslinking group" refers to a group forming a peptide bond between a liposome and a sugar chain. A linker protein-crosslinking group varies depending on the desired target to be crosslinked. For example, a bivalent reagent, such as bissulfosuccinimidyl suberate, disuccinimidyl glutarate, dithiobis succinimidyl propionate, disuccinimidyl suberate, 3,3'-dithiobis(sulfosuccinimidylpropionate), ethylene glycol bissuccinimidyl succinate, ethylene glycol bissulfosuccinimidyl succinate and the like can be used. Examples of a linker protein-crosslinking group which may be used herein include, for example, 3,3'-dithiobis(sulfosuccinimidylpropionate) group, bissulfosuccinimidylsuberate group, disuccinimidylglutarate group, dithiobissuccinimidylpropionate group, disuccinimidylsuberate group, ethyleneglycolbissuccinimidylsuccinate group, ethyleneglycolbissulfosuccinimidylsuccinate group and the like, but are not limited thereto, because any crosslinking agent having a reactive group which reacts with an amino group at both ends can form a peptide bond between a liposome and a sugar chain. Thus, other alternative examples include bis(sulfosuccinimidyl)glutarate-do group, bis(sulfosuccinimidyl)2,2,4,4-glutarate-d4 group, bis(sulfosuccinimidyl)suberate group, bis(sulfosuccinimidyl)suberate-do group, bis(sulfosuccinimidyl)2,2,7,7-suberate-$d_4$ group, bis(2-[succinimidoxycarbonyloxy]ethyl)sulfone group, disuccinimidyl glutarate, dithiobis(succinimidylpropionate) group, disuccinimidyltartrate group, ethyleneglycolbis(succinimidylsuccinate) group, sulfodisuccinimidyltartrate group, ethyleneglycolbis (sulfo-succinimidylsuccinate) group, tris-(succinimidylaminotristearate) group and the like.

As used herein, the term "biocompatibility" refers to the property of being compatible with a tissue or organ of an organism without causing toxicity, immune response, damage or the like. Examples of biocompatible buffer solutions include, but are not limited to, for example, phosphate buffered physiological saline(PBS), physiological saline, Tris buffer, carbonate buffer solution (CBS), tris(hydroxymethyl)methylaminopropanesulfonic acid buffer solution (TAPS), 2-[4-(2-hydroxylethyl)-1-piperazinyl]ethanesulfonic acid (HEPES), other Good's Buffer Solution (for example, 2-morpholinoethanesulfonic acid, monohydrate (MES), bis(2-hydroxyethyl)iminotris(hydroxymethyl)methane (Bis-tris), N-(2-acetamide)iminodiacetic acid (ADA), 1,3-bis[tris (hydroxymethyl)methylamino]propane(Bis-trispropane), piperazine-1,4-bis(2-ethanesulfonic acid) (PIPES), N-(2-acetamide)-2-aminoethanesulfonic acid (ACES), cholamine chloride, N,N-bis (2-hydroxyethyl)-2-aminoethanesulfonic acid (BES), 3-morpholinopropanesulfonic acid (MOPS), N-tris (hydroxymethyl) methyl-2-aminoethanesulfonic acid (TES), N-(2-hydroxyethyl)piperazine-N'-3-propanesulfonic acid (HEPPS), N-[tris(hydroxymethyl)methyl]glycin (Tricine), aminoacetamide(glycinamide), N,N-bis(2-hydroxyethyl)glycin (Bicine), N-cyclohexyl-2-aminoethanesulfonic acid (CHES), N-cyclohexyl-3-aminopropanesulfonic acid (CAPS)) and the like.

As used herein, the terms "protein," "polypeptide," "oligopeptide" and "peptide" have the same meaning in the present specification and refer to an amino acid polymer having any length. This polymer may be straight, branched or cyclic. An amino acid may be naturally-occurring or not naturally-occurring, and may be a modified amino acid. These terms may also encompass those assembled with a complex of a plurality of polypeptide chains. These terms further encompass a naturally-occurring or artificially modified amino acidpolymer. Examples of such a modification include, for example, formation of a disulfide bond, glycosylation, lipidation, acetylation, phosphorylation, or any other manipulation or modification (for example, conjugation with a label component). The definition also encompasses, for example, a polypeptide including one or two or more analog (s) of amino acids (including, for example, a non-naturally occurring amino acid and the like), peptide-like compounds (for example, peptoid) and other modifications known in the art.

It should be understood that, as used herein, when specified otherwise, the term "protein" refers to an amino acid polymer or a variant thereof having a relatively large molecular weight, and the term "peptide" may refer to an amino acid polymer or a variant thereof having a relatively small molecular weight. Examples of such molecular weight include, but are not limited to, for example, about 30kDa, preferably about 20kDa, and more preferably about 10kDa and the like.

As used herein, the term "organism-derived protein" refers to a protein derived from an organism, and refers to a protein derived from any organism (for example, any type of multicellular organism (for example, an animal (for example, vertebrate or invertebrate), a plant (for example, monocotyledon, dicotyledon or the like) or the like)). Preferably, a protein derived from a vertebrate (for example, Hyperotreta, Hyperotia, Chondrichthyes, Osteichthyes, Amphibia, Reptilia, Aves, mammals or the like), more preferably, a protein derived from a mammal (for example, Monotremata, Marsupialia, Edentata, Dermoptera, Chiroptera, Carnivora, Insectivora, Proboscidea, Perissodactyla, Artiodactyla, Tubulidentata, Squamata, Sirenia, Cetacea, Primates, Rodentia, Lagomorpha or the like) is used. More preferably, a protein derived from Primates (for example, chimpanzee, Japanese monkey or human) is used. Most preferably, a protein derived from a desired organism to be administered is used. Herein, in indicating an organism-derived protein in a state bound with another substance, the term "organism-derivedprotein group" is used.

As used herein, the term "human-derived serum protein" refers to a protein contained in a liquid portion of human blood, which remains when the human blood is naturally coagulated. Herein, in indicating a human-derived protein in a state bound with another substance, the term "human-derived protein group" is used.

As used herein, the term "serum albumin" refers to albumin contained in serum. Herein, in indicating serum albumin in a state bound with another substance, the term "serum albumin group" is used.

In a glycolipid-containing liposome of the present invention (for example, synthetic GM3 sugar chain-modified liposome), at least one of a liposomal membrane and a linker may be hydrophilized by binding a hydrophilic compound, preferably tris(hydroxyalkyl)aminoalkane.

As used herein, the term "hydrophilization" refers to binding a hydrophilic compound to a surface of a liposome. Examples of compounds used for hydrophilization include low-molecular weight hydrophilic compounds, preferably low-molecular weight hydrophilic compounds having at least one OH group, and more preferably low-molecular weight hydrophilic compounds having at least two OH groups. In addition, low-molecular weight hydrophilic compounds further having at least one amino group, i.e., hydrophilic compounds having at least one OH group and at least one amino group in the molecule are also included. Since hydrophilic compounds have a low molecular weight, they are unlikely to cause steric hindrance for a sugar chain, and do not prevent progress of sugar chain molecule recognition reaction by lectin on a target cellular membrane. Furthermore, hydrophilic compounds do not contain a sugar chain which may be bound by lectin and which is used in a sugar chain-modifying liposome of the present invention for directing to a specific target such as lectin. Examples of such hydrophilic compounds include, for example, amino alcohols such as tris(hydroxyalkyl)aminoalkanes, including tris(hydroxymethyl)aminomethane and the like. More specifically, examples of such hydrophilic compounds include tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxymethyl)aminopropane, tris(hydroxyethyl)aminopropane, tris(hydroxypropyl)aminopropane and the like.

A hydrophilized liposome and a non-hydrophilized liposome have different stability in a solution. A non-hydrophilized liposome has low stability, and may cause precipitation and aggregation of liposomes. On the other hand, a liposome, upon hydrophilization, has an improved stability, and does not cause precipitation or aggregation of liposomes.

As used herein, the term "alkyl" refers to a monovalent group formed as a result of removal of a hydrogen atom

from an aliphatic hydrocarbon (herein referred to as "alkane") such as methane, ethane or propane, and an alkyl is normally represented by the formula $C_nH_{2n+1}$- (wherein n is a positive integer). The alkyl may be a straight chain or a branched chain. As used herein, the term "substituted alkyl" refers to an alkyl group with H atom substituted with a substituent specified below. A specific example thereof may be C1 to C2 alkyl, C1 to C3 alkyl, C1 to C4 alkyl, C1 to C5 alkyl, C1 to C6 alkyl, C1 to C7 alkyl, C1 to C8 alkyl, C1 to C9 alkyl, C1 to C10 alkyl, C1 to C11 alkyl or C1 to C12 alkyl, C1 to C2 substituted alkyl, C1 to C3 substituted alkyl, C1 to C4 substituted alkyl, C1 to C5 substituted alkyl, C1 to C6 substituted alkyl, C1 to C7 substituted alkyl, C1 to C8 substituted alkyl, C1 to C9 substituted alkyl, C1 to C10 substituted alkyl, C1 to C11 substituted alkyl or C1 to C12 substituted alkyl. Regarding alkane, a specific example of alkane may be C1 to C2 alkane, C1 to C3 alkane, C1 to C4 alkane, C1 to C5 alkane, C1 to C6 alkane, C1 to C7 alkane, C1 to C8 alkane, C1 to C9 alkane, C1 to C10 alkane, C1 to C11 alkane, C1 to C12 alkane, C1 to C2 substituted alkane, C1 to C3 substituted alkane, C1 to C4 substituted alkane, C1 to C5 substituted alkane, C1 to C6 substituted alkane, C1 to C7 substituted alkane, C1 to C8 substituted alkane, C1 to C9 substituted alkane, C1 to C10 substituted alkane, C1 to C11 substituted alkane or C1 to C12 substituted alkane. Here, for example, C1 to C10 alkyl means a straight-chain or branched alkyl having 1 to 10 carbon atoms, and examples thereof include methyl ($CH_3$-), ethyl ($C_2H_5$-), n-propyl ($CH_3CH_2CH_2$-), isopropyl ($(CH_3)_2CH$-), n-butyl ($CH_3CH_2CH_2CH_2$-), n-pentyl ($CH_3CH_2CH_2CH_2CH_2$-), n-hexyl ($CH_3CH_2CH_2CH_2CH_2CH_2$-), n-heptyl ($CH_3CH_2CH_2CH_2CH_2CH_2CH_2$-), n-octyl ($CH_3CH_2CH_2CH_2CH_2CH_2CH_2CH_2$-), n-nonyl ($CH_3CH_2CH_2CH_2CH_2CH_2CH_2CH_2CH_2$-), n-decyl ($CH_3CH_2CH_2CH_2CH_2CH_2CH_2CH_2CH_2CH_2$-), $-C(CH_3)_2CH_2CH_2CH(CH_3)_2$, $-CH_2CH(CH_3)_2$, and the like. In addition, the C1 to C10 substituted alkyl is, for example, a C1 to C10 alkyl of which one or a plurality of hydrogen atoms are substituted with substituent. R preferably represents a C1 to C6 alkyl group, particularly preferably a C1 to C6 alkyl group.

If the substance according to the present invention or the functional group defined above is substituted with a substituent R, there may be single or multiple substituents R, and the groups Rmaybe respectively independently selected from the group consisting of hydrogen, alkyl, cycloalkyl, alkenyl, cycloalkenyl, alkynyl, alkoxy, carbocyclic group, heterocyclic group, halogen, hydroxy, thiol, cyano, nitro, amino, carboxy, acyl, thiocarboxy, amide, substituted amide, substituted carbonyl, substituted thiocarbonyl, substituted sulfonyl and substituted sulfinyl.

Furthermore, compounds obtained by introducing an amino group into low-molecular weight compounds having OH group(s) can also be used as hydrophilic compounds of the present invention. Such compounds include, but are not limited to, for example, compounds obtained by introducing an amino group into a sugar chain to which lectin does not bind, such as cellobiose. For example, on a lipid of a liposomal membrane, phosphatidylethanolamine, a bivalent reagent for crosslinking and tris(hydroxymethyl)aminomethane are used to hydrophilize the surface of the liposome. The general formula for hydrophilic compounds is represented by the following formula (1), formula (2), formula (3) and the like.

$Z\text{-}R^1(R^2OH)_n$: formula (1)

$H_2N\text{-}R^3\text{-}(R^4OH)_n$: formula (2)

$H_2N\text{-}R^5(OH)_n$: formula (3)

Here, $R^1$, $R^3$ and $R^5$ represent $C_1$ to $C_{40}$, preferably $C_1$ to $C_{20}$, and more preferably $C_1$ to $C_{10}$, straight or branched hydrocarbon chain. $R^2$ and $R^4$ are not present, or represent $C_1$ to $C_{40}$, preferably $C_1$ to $C_{20}$, and more preferably $C_1$ to $C_{10}$ straight or branched hydrocarbon chain. Z represents a reactive functional group directly binding to a liposome or binding to a bivalent reagent for crosslinking, and examples thereof include, for example, COOH, NH, $NH_2$, CHO, SH, NHS-ester, maleimide, imide ester, active halogen, EDC, pyridyl disulfide, azidophenyl, hydrazide and the like. n represents a natural number. A surface of a liposome hydrophilized using such a hydrophilic compound is thinly covered with the hydrophilic compound. However, since the thickness of the cover of such a hydrophilic compound is thin, even in the case of binding a sugar chain to the liposome, reactivity of the sugar chain or the like is not inhibited.

Asusedherein, the term "hydrophilic compound group" refers to the aforementioned hydrophilic compound upon binding with another group. A hydrophilic compound group may be monovalent or bivalent depending on the case.

As used herein, the term "hydrophilic compound-crosslinking group" refers to a group of which one end binds with a linker protein via a peptide bond and the other end binds with a sugar chain via a peptide bond, and which forms a peptide bond between a hydrophilic compound group and a liposome or a linker protein. Examples of hydrophilic compound-crosslinking group can include, for example, bis(sulfosuccinimidyl)suberate group, disuccinimidylglutarate group, dithiobissuccinimidylpropionate group, disuccinimidylsuberate group, 3,3'-dithiobis(sulfosuccinimidylpropionate) group, ethyleneglycolbissuccinimidylsuccinate group and ethyleneglycolbissulfosuccinimidylsuccinate group and the like. Preferably, the hydrophilic compound-crosslinking group is a bis(sulfosuccinimidyl)suberate group. The hydrophilic compound-crosslinking group may be substituted by a group other than the aforementioned groups, as long as it is a

crosslinking agent which has a reactive group reacting with an amino group at each of both ends and which is not cleaved even under oxidization/reduction conditions. Examples thereof include, but are not limited to, bis(sulfosuccinimidyl) glutarate-do group, bis(sulfosuccinimidyl)2,2,4,4-glutarate-d group, bis(sulfosuccinimidyl)suberate-do group, bis(sulfosuccinimidyl)2,2,7,7-suberate-$d_4$ group, bis(2-[succinimidoxycarbonyloxy]ethyl)sulfone group, disuccinimidyltartrate group, ethyleneglycolbis(succinimidylsuccinate) group, ethyleneglycolbis(sulfo-succinimidylsuccinate) group, tris-(succinimidylaminotristearate) group and the like.

Hydrophilization of a liposome can be also performed by employing a conventionally known method, for example, a method such as a method of producing a liposome using a phospholipid bound with polyethylene glycol, polyvinyl alcohol, maleic anhydride copolymer or the like via a covalent bond (Japanese Laid-Open Publication No. 2000-302685 (for example, it describes as follows: using a CNDAC-containing liposome preparation dilauroylphosphatidylcholine, dimyristoylphosphatidylcholine, dipalmitoylphosphatidylcholine, distearoylphosphatidylcholine; dipalmitoylphosphatidylglycerol, distearoylphosphatidylglycerol; sphingomyelin; cholesterol; N-monomethoxypolyethylene glycol succinyl-distearoylphosphatidylethanolamine in which the molecular weight of the polyethylene glycol portion is about 2000 (hereinafter, referred to as PEG2000-DSPE); CNDAC hydrochloride, glucose aqueous solution and trehalose aqueous solution and using the method of Bangham et al. (see J.Mol.Bio1.8,660-668(1964)), a crude dispersion liquid of a multilayer liposome was obtained.)). Among such methods, it is particularly preferred to hydrophilize the surface of a liposome using tris(hydroxymethyl)aminomethane. The method using tris(hydroxymethyl)aminomethane according to the present invention is preferable in comparison with conventional hydrophilizing methods using polyethylene glycol or the like in some viewpoints. For example, in the case of binding a sugar chain onto a liposome to utilize a molecule-recognizing function of the sugar chain for targeting property as in the present invention, tris(hydroxymethyl)aminome thane is particularly preferable, since it is a low-molecular weight substance and thus is less likely to cause steric hindrance for a sugar chain and does not prevent the progress of sugar chain molecule recognition reaction by lectin (sugar chain-recognizing protein) on the surface of a target cellular membrane, in comparison with a conventional method using a high-molecular weight substance such as polyethylene glycol.

Furthermore, a liposome according to the present invention has a good particle size distribution, composition and dispersion properties even after such a hydrophilization treatment, and also has an excellent long-term preservation and *in vivo* stability. Thus, the liposome of the present invention is preferable for use as a liposome preparation. For hydrophilizing the surface of a liposome using tris(hydroxymethyl)aminomethane, for example, a liposome solution obtained by a normal method using a lipid such as dimyristoylphosphatidylethanolamine, dipalmitoylphosphatidylethanolamine, distearoylphosphatidylethanolamine or the like is blended and reacted with a bivalent reagent such as bis sulfosuccinimidyl suberate, disuccinimidyl glutarate, dithiobis succinimidyl propionate, disuccinimidyl suberate, 3,3'-dithiobis(sulfosuccinimidyl propionate), ethylene glycol bissuccinimidyl succinate, ethylene glycol bissulfosuccinimidyl succinate or the like so as to bind the bivalent reagent to the lipid on the liposomal membrane, such as dipalmitoylphosphatidylethanolamine, and tris(hydroxymethyl)aminomethane is subsequently reacted with one of the binding arms of the bivalent reagent so as to bind a tris(hydroxymethyl)aminomethane on the surface of the liposome. For hydrophilizing the surface of a liposome using tris (hydroxymethyl) aminomethane, any substance other than the aforementioned substances may be used as long as it is incorporated into the surface of the liposomal membrane and has an amino group as a hydrophilic group.

(Antibody-modified liposome)

As used herein, the term "antibody-modified liposome" refers to a substance including an antibody and a liposome, and preferably refers to a liposome modified by directly or indirectly binding an antibody. Herein, the terms "antibody-modified liposome" and "antibody liposome" may be used interchangeably.

As used herein, the term "bridging spacer" refers to a substance capable of crosslinking an antibody with a liposome, for example, serum albumin, protein A, other proteins, bivalent crosslinking agents or the like.

A broad variety of bridging spacers suitable for conjugating an antibody with a serum albumin preferably have an atom of $-(CH_2)_1-$ to $-(CH_2)_n-$ (n is about 20) in a skeleton thereof. Such a diradical spacer may be optionally substituted, and may contain a double bond, disulfide bond, triple bond, aryl group, or a hetero atom in its chain. In one embodiment, this bridging spacer group may optionally have one or more carbons, each of which may be substituted orunsubstituted. Abond in the chain is optionally saturated orunsaturated, and may be branched or straight. In a bridging spacer compound, a carbon atom in its chain may be substituted with N, O or S. Preferably, a bridging spacer compound is a $C_1$-$C_{20}$ chain. Examples of substituents which may be preferably substituted can include aryl, carbonyl, amino, carboxy, hydroxy and the like. As a bridging spacer, a very broad variety of substances can be selected, and the bridging spacer varies depending on the desired target of crosslinking. Based on the description herein, those skilled in the art can appropriately select a bridging spacer in carrying out the present invention.

Herein, unless specified otherwise, the term "substitution" refers to replacing one or more hydrogen atoms in an organic compound or a substituent with another atom or atomic group, or forming a double bond or triple bond instead.

It is also possible to remove and substitute one hydrogen atom with a monovalent substituent or form a double bond together with a single bond. It is also possible to remove and substitute two hydrogen atoms with a bivalent substituent or form a triple bond together with a single bond.

Examples of substituent in the present invention include, but are not limited to, alkyl, cycloalkyl, alkenyl, cycloalkenyl, alkynyl, alkoxy, carbocyclic group, heterocyclic group, halogen, hydroxy, thiol, cyano, nitro, amino, carboxy, carbamoyl, acyl, acylamino, thiocarboxy, amide, substituted carbonyl, substituted thiocarbonyl, substituted sulfonyl and substituted sulfinyl. Such substituents can be appropriately utilized in the present invention in designing an amino acid.

Preferably, if a plurality of substituents are present, the plurality of substituents may be respectively and independently a hydrogen atom of alkyl, but it is unlikely that all of the plurality of substituents are hydrogen atoms. More preferably, if a plurality of substituents are present, the plurality of substituents may be respectively and independently selected from the group consisting of hydrogen and C1 to C6 alkyl. All of the substituents may be substituents other than hydrogen, but preferably at least one, more preferably 2 to n (wherein n is the number of substituents), of the substituents may be hydrogen. It may be preferable that the number of hydrogens is larger among substituents, because a large substituent or a polar substituent may hinder the effects of the present invention (particularly interaction with an aldehyde group). Thus, a substituent other than hydrogen may be preferably C1 to C6 alkyl, C1 to C5 alkyl, C1 to C4 alkyl, C1 to C3 alkyl, C1 to C2 alkyl, methyl or the like. However, it may also be preferable to have a large substituent because it may enhance the effects of the present invention.

Herein, C1, C2...Cn represents a carbon number. Thus, C1 is used for expressing a substituent having a carbon number of 1.

(Determination of the amounts of protein and lipid)

Regarding the amount of protein in a glycolipid-containingliposomeofthe present invention (for example, synthetic GM3 liposome, synthetic GM3 sugar chain-modified liposome or the like), for example, the amount of HSA encapsulated in a liposome and the total amount of protein coupled on a surface of the liposome can be measured by the BCA method.

[Chem. 51]

<Principle of BCA method>

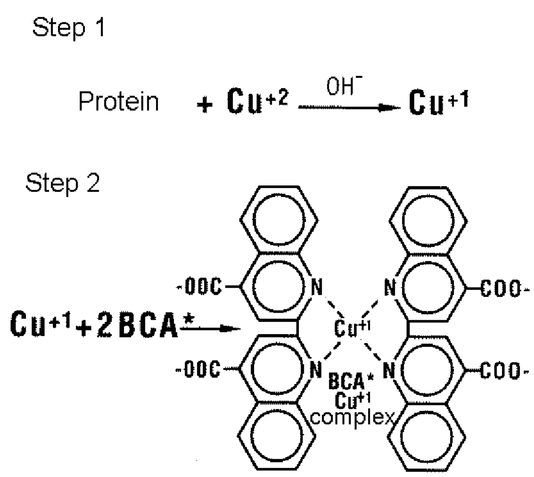


In measuring an amount of protein, for example, the Micro BCA™ Protein Assay Reagent Kit (Catalog No. 23235BN)(PIERCE Co.LTD) and the like can be used. As the standard substance, 2mg/ml albumin (BSA) may be used. (1) As a standard solution, the standard substance (2mg/ml: albumin) is diluted in PBS buffer solution to prepare 0, 0.25, 0.5, 1, 2, 3, 4 and 5μg/50μl solutions. (2) Cy5.5-encapsulating sugar chain-modified liposome is 20-times diluted with a PBS buffer solution to prepare the sample solution. (3) The standard solution and the sample solution are respectively dispensed in a test tube in an amount of 50μl. (4) To each test tube, 3% sodium lauryl sulfate solution (SDS solution) is added in an amount of 100μl. (5) Reagents A, B and C attached to the kit are mixed so that the ratio of reagent A: reagent B: reagent C = 48:2:50, and the mixture is added to each test tube in an amount of 150μl. (6) The test tube is

left to stand at 60 degrees Celsius for 1 hour. (7) After the temperature decreases to room temperature, absorbance at 540nm is measured, a calibration curve is made based on the standard solution, and the amount of protein in a liposome is measured. One example of a calibration curve is shown in Fig. 1.

The amount of protein in a glycolipid-containing liposome of the present invention may be, for example, in the range of 0.1 to 1mg/ml. The amount of protein in a Cy3-labeled sugar chain-modified liposome may be, for example, 0.24mg/ml or more. The amount of protein in a Cy5.5-labeled sugar chain-modified liposome may be, for example, 0.45mg/ml or more. The amount of protein in a Cy7-labeled sugar chain-modified liposome may be, for example, 0.20mg/ml or more.

In the present invention, the amount of lipid composing a glycolipid-containing liposome can be calculated by, for example, determining the amount of cholesterol.

<Principle for determination of lipid>

[Chem. 52]

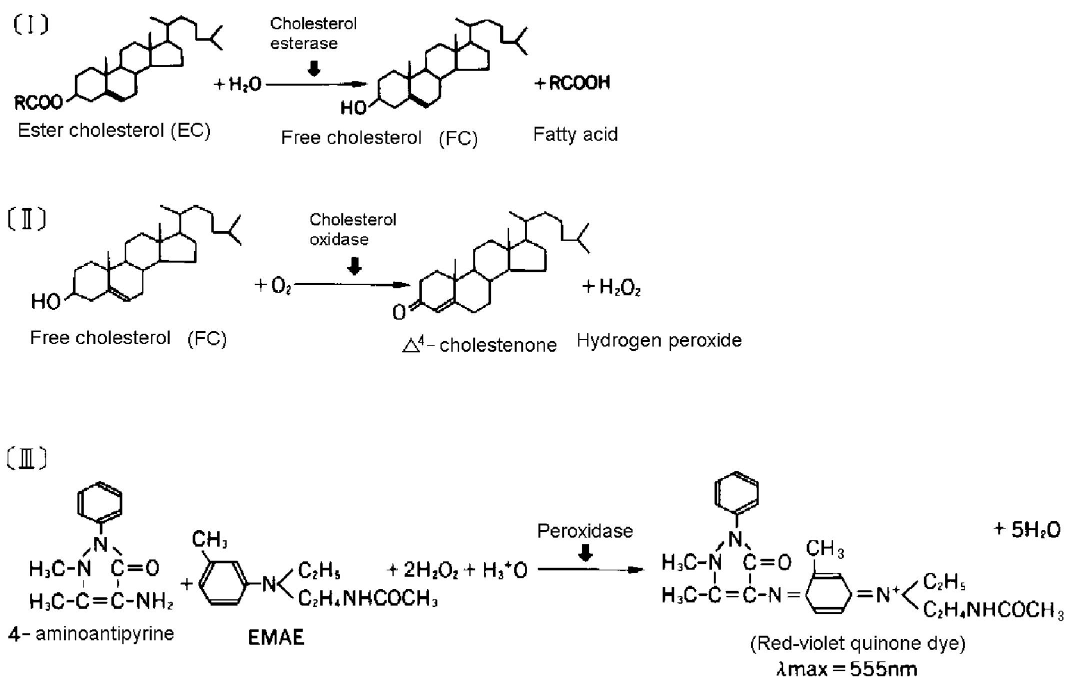


For determination of lipid, for example, the Determiner TC555 Kit (Catalog No. UCC/EAN128) (KYOWA Co. LTD) can be used. As the standard substance, 50mg/ml of cholesterol attached to the kit is used. (1) As a standard solution, the standard substance (50mg/ml: cholesterol) is diluted with a PBS buffer solution to prepare 0, 0.1, 0.25, 0.5, 0.75, 1, 5 and 10μg/20μl solutions. (2) A glycolipid-containing liposome is 5-times diluted with a PBS buffer solution to prepare the sample solution. (3) The standard solution and the sample solution are respectively dispensed in a test tube in an amount of 20μl. (4) To each test tube, TritonX-100 (10% solution) is added in an amount of 17μl, stirring is performed, and the solution is subsequently left to stand at 37 degrees Celsius for 40 minutes. (5) The enzyme reagent of the Determiner TC555 Kit is added in an amount of 300μl, stirring is performed, and the solution is subsequently left to stand at 37 degrees Celsius for 20 minutes. (6) Absorbance at 540nm is measured, a calibration curve (one example of a calibration curve is shown in Fig. 2) is made based on the standard solution, the amount of cholesterol in the liposome is measured, and the amount of lipid is determined.

A formula for calculating the amount of lipid from the amount of cholesterol is expressed as follows, for example:

Amount of lipid (μg/50μl) = amount of cholesterol (μg/50μl) × 4.51 (conversion factor). The ratio of protein in a liposome with respect to lipid in the liposome can be obtained, for example, based on results of the determination of protein and the determination of lipid described above. In the glycolipid-containing liposome of the present invention, preferably, the ratio of protein with respect to lipid is about 0.1 to about 0.5.

The amount of lipid in a glycolipid-containing liposome of the present invention may be, for example, in the range of 0.5 to 5mg/ml. The amount of lipid in a Cy3-labeled sugar chain-modified liposome may be, for example, 1.2mg/ml or more. The amount of lipid in a Cy5.5-labeled synthetic GM3 sugar chain-modified liposome may be, for example, 1.4mg/ml or more. The amount of lipid in a Cy7-labeled sugar chain-modified liposome may be, for example, 2.1mg/ml or more.

The particle size distribution and particle size of a glycolipid-containing liposome of the present invention (for example, synthetic GM3 liposome and synthetic GM3 sugar chain-modified liposome) can be measured, for example, using the Zetasizer Nano (Nan-ZS:MALVERN Co.LTD) and by diluting 50-times the liposome particles in purified water. One example of particle size distribution is shown in Fig. 3.

A glycolipid-containing liposome of the present invention (for example, synthetic GM3 liposome, synthetic GM3 sugar chain-modified liposome) preferably has a particle size of about 50nm to 200nm in the maximum area of particle size distribution, because a particle size of about 50nm to 200nm can avoid recognition by immune system cells such as macrophage and can avoid incorporation by the reticuloendothelial system (RES) of the liver or spleen to some extent. A sugar chain-modified liposome having a particle size of about 50nm to 200nm is suitable for encapsulating a drug, and for delivery of the drug to a target organ or diseased portion.

A glycolipid-containing liposome of the present invention (for example, synthetic GM3 liposome and synthetic GM3 sugar chain-modified liposome) has a mean particle size of about 50nm to about 300nm, preferably about 65nm to about 165nm, and more preferably about 100nm, because if the particle size of the liposome is too small, it nonspecifically enters the reticuloendothelial system of the liver/spleen and if the particle size is too large it is easily phagocytized by the immune system cells such as the macrophage. Furthermore, preferably, the glycolipid-containing liposome of the present invention is negatively charged. By being negatively charged, the interaction with negatively-charged cells in an organism can be prevented. The zetapotential of the surface of the glycolipid-containing liposome of the present invention is, in physiological saline at 37 degrees Celsius, -50 to 10mV, preferably -40 to 0mV, and more preferably -30 to -10mV. The zeta potential of the surface of the liposome may be, but is not limited to, -120mV to -30mV at 25 degrees Celsius. Preferably, the zeta potential of the surface of the liposome is less than -30mV at 25 degrees Celsius. The zeta potential of the surface of the liposome may be either less than -120mV (25 degrees Celsius) or -30mV or more, because any zeta potential is possible as long as aggregation of liposomes does not occur under such zeta potential.

(Simple evaluation of the added amount of sugar chain bound onto the liposomal membrane)

(FITC binding assay)

The crosslinking reagent DTSSP(PIERCE) is added to the liposome solution (liposome intermediate HSA), the solution is stirred, and free DTSSP is removed by ultrafiltration.

Ammonium hydrogen carbonate is added to FITC dissolved in purified water and the solution is stirred, thereby obtaining an aminated FITC solution. The aminated FITC is added to and reacted with the above liposome solution supplemented with DTSSP. Subsequently, the Tris solution is added and stirring is performed. Free FITC and Tris are removed by ultrafiltration, thereby obtaining FITC-bound liposome.

The amount of FITC bound to the liposome can be obtained by measuring the fluorescence (excitation wavelength: 495nm; fluorescence wavelength: 520nm).

(Evaluation of the amount of bound antibody)

The crosslinking reagent DTSSP(PIERCE) is added to the liposome solution (liposome intermediate HSA), the solution is stirred, and free DTSSP is removed by ultrafiltration.

An antibody (for example, anti-E-selectin antibody) is added to and reacted with the liposome solution. Subsequently, the Tris solution is added and stirring is performed. Free antibody and Tris are removed by ultrafiltration, thereby obtaining an antibody-bound liposome.

The amount of antibody bound onto a surface of the liposome can be measured by the Enzymed immuno assay (EIA) technique. PBS solution of a protein to be an antigen (for example, E-selectin (R&D Systems)) is added and immobilized to a 96-well microplate in an amount of 50μl per well. The antigen solution in the plate is discarded, 300μl per well of 2% BSA/PBS are added, and the plate is left to stand. The BSA solution is discarded and the plate is washed three times with PBS. Subsequently, 100μl per well of the antibody standard solution and the antibody-bound liposome sample are added. After leaving the plate to stand for one hour, the solution in the well is discarded and the plate is washed with PBS. HRP-labeled anti-mouse IgG antibody is diluted with 10% Tween20/9% EDTA/PBS. 100μl per well of the diluted solution is added, and the plate is left to stand for one hour. After discarding the solution in the

wells and washing the plate with PBS, the 1-Step™ TMB-Blotting (PIERCE) is added and allowed to react, and 100μl per well of a reaction stopper (2M sulfuric acid) are added. Absorbance at 450nm is measured, and thus the amount of antibody bound onto the surface of the liposome from the antibody standard solution can be obtained.

(Composition)

A composition of the present invention may optionally contain an appropriate formulation material or a pharmaceutically acceptable carrier. Examples of appropriate formulation material, or pharmaceutically acceptable carrier include, but are not limited to, an antioxidant agent, a preservative, a colorant, a fluorochrome, a flavoring agent, a diluent, an emulsifying agent, a suspending agent, a solvent, a filler, an extending agent, a buffer, a delivery vehicle and/or a pharmaceutical adjuvant. Typically, a composition of the present invention is administered in the form of a composition containing sialyl Lewis X (SLX) and optionally containing other active ingredients together with at least one physiologically acceptable carrier, excipient or diluents. For example, an appropriate vehicle may be a micelle, an injection solution, a physiological solution or an artificial cerebrospinal fluid, and may be supplemented with other substance generally used in a composition for parenteral delivery.
An acceptable carrier, excipient or stabilizing agent used herein is preferably nontoxic to a recipient, and preferably inactive at the dose and concentration used. Preferably, examples of such acceptable carrier, excipient or stabilizing agent used herein include, for example, phosphates, citrates, or other organic acid; ascorbic acid, α-tocopherol; low-molecular weight polypeptide; proteins (for example, serum albumin, gelatin or immunoglobulin); hydrophilic polymers (for example, polyvinyl pyrrolidone; amino acids (for example, glycin, glutamine, asparagines, arginine or lysine); monosaccharide, disaccharide and other carbohydrates (glucose, mannose, or dextrin); chelating agent (for example, EDTA); sugar alcohol (for example, mannitol or sorbitol); salt-forming pair ion (for example, sodium); nonionic surfactant (for example, Tween, Pluronic or polyethylene glycol (PEG)) and/or the like.
Examples of an appropriate carrier include neutral buffered physiological saline or physiological saline mixed with serum albumin. Preferably, the product is formulated as a lyophilized preparation using an appropriate excipient (for example, sucrose). Other standard carrier, diluent and excipient may be optionally contained. Other exemplary compositions contain a Tris buffer at a pH of about 7.0 to 8.5 or acetic acid buffer at a pH of about 4.0 to 5.5, and may further contain sorbitol or other appropriate substitute thereof.
Hereinafter, general method of preparing a composition of the present invention is described. It should be noted that animal drug compositions, quasi drugs, fisheries drug compositions, food compositions, cosmetic compositions and the like can also be produced by a known preparation method.
A composition or the like of the present invention is optionally blended with a pharmaceutically acceptable carrier, and for example, it can be parenterally administered as a liquid preparation such as an injection, a suspension, a solution, a spray or the like. Examples of pharmaceutically acceptable carrier include an excipient, a lubricating agent, a binding agent, a disintegrating agent, a disintegration inhibitor, an absorption enhancer, an absorbent, a wetting agent, a solvent, a solubilizing agent, a suspending agent, an isotonizing agent, a buffer, a soothing agent and the like. Furthermore, a preparation additive such as an antiseptic agent, an antioxidant, a coloring agent, a sweetening agent or the like can be optionally used. Moreover, it is also possible to blend a substance other than sialyl Lewis X and lipid to the composition of the present invention. Examples of parenteral administration route include, but are not limited to, intravenous administration, intramuscular administration, subcutaneous administration, intracutaneous administration, mucosal administration, intrarectal administration, intravaginal administration, topical administration, dermal administration and the like. In systemic administration, a pharmaceutical used in the present invention may be in the form of pharmaceutically acceptable aqueous solution which does not contain a pyrogenic substance. It is within the scope of techniques of those skilled in the art to consider pH, isotonicity, stability or the like with regard to the preparation of such pharmaceutically acceptable composition.
Preferred examples of solvent in the liquid preparation include an injection solution, alcohol, propylene glycol, macrogol, sesame oil, corn oil and the like.
Preferred examples of solubilizing agent in the liquid preparation include, but are not limited to, polyethylene glycol, propylene glycol, D-mannitol, benzyl benzoate, ethanol, tris aminomethane, cholesterol, triethanolamine, sodium carbonate, sodium citrate and the like.
Preferredexamples of a suspending agent in the liquid preparation include, for example, a surfactant such as stearyl triethanolamine, sodium lauryl sulfate, lauryl aminopropionic acid, lecithin, benzalkonium chloride, benzethonium chloride and glyceryl monostearate, for example, hydrophilic macromolecules such as polyvinyl alcohol, polyvinyl pyrrolidone, carboxymethylcellulose sodium, methylcellulose, hydroxymethylcellulose, hydroxyethylcellulose, hydroxyethylcellulose, hydroxypropylcellulose and the like.
Preferred examples of an isotonizing agent in the liquid preparation include, but are not limited to, sodium chloride, glycerin, D-mannitol and the like.
Preferred examples of a buffer in the liquid preparation include, but are not limited to, phosphates, acetates,

carbonates, citrates and the like.

Preferred examples of a soothing agent in the liquid preparation include, but are not limited to, benzyl alcohol, benzalkonium chloride, procaine hydrochloride and the like.

Preferred examples of an antiseptic agent in the liquid preparation include, but are not limited to, parahydroxybenzoic acid esters, chlorobutanol, benzyl alcohol, 2-phenylethyl alcohol, dehydroacetic acid, sorbic acid and the like.

Preferred examples of an antioxidant in the liquid preparation include, but are not limited to, sufites, ascorbic acid, α-tocopherol, cysteine and the like.

When the composition or the like of the present invention is prepared as an injection, the solution and suspension are preferably sterilized and isotonic with the blood or a solvent for other purpose in the site of injection. These are normally sterilized by filtration using a bacteria-retentive filter or the like, blending of a sterilizing agent, irradiation or the like. Furthermore, after such treatment, they may be formed into a solid by a method such as lyophilizing, and aseptic water or aseptic diluents for injection (lidocaine hydrochloride aqueous solution, physiological saline, glucose aqueous solution, ethanol, mixture solution thereof or the like) may be added just before use.

Furthermore, a composition of the present invention may contain a colorant, a preservative, a perfume, a flavoring agent, a sweetening agent or the like, and other agent.

The amount of substance, composition or the like used in the present invention can be readily determined by those skilled in the art in view of the purpose of use, subject disease (type, severity or the like), age, weight, sex, anamnesis, cellular form and type of the subject. The frequency for applying the method of the present invention to a subject can also be readily determined by those skilled in the art in view of the purpose of use, subject disease (type, severity or the like), age, weight, sex, anamnesis and therapeutic process of a subject, and the like. As for frequency, for example, administration of once per day to per several months (for example, once per week to once per month) is included. It is preferable to apply administration of once per week to once per month while observing the process. The dose of administration can be determined by estimating the necessary amount to the site to be treated.

As used herein, the term "magnetic resonance imaging (MRI)" refers to the method of imaging information inside an organism by utilizing the nuclear magnetic resonance phenomenon.

As used herein, the term "nuclear magnetic resonance (NMR)" refers to resonance with oscillating field or electromagnetic wave when spin energy level of an atomic nucleus is separated by the Zeeman effect.

As used herein, the term "contrast medium" refers to an agent used for increasing or decreasing the density (signal intensity) of a tissue in the image diagnosis. A contrast medium for increasing the density is referred to as positive contrast medium, and a contrast medium for decreasing the density is referred to as negative contrast medium. Examples of positive contrast medium include, but are not limited to, for example, gadolinium chelating agent, barium sulfate, water-soluble iodine contrast medium and the like. Examples of negative contrast medium include, but are not limited to, superparamagnetic iron oxide particle (SPIO), air, carbon dioxide and the like.

As used herein, the term "subject" refers to an organism to be the subject of a treatment, detection or diagnosis of the present invention, and is also referred to as "patient." A patient or subject maybe preferably human. An animal to be the subject of the present invention may be, for example, avian, mammal or the like. Preferably, such an animal may be a mammal (for example, Monotremata, Marsupialia, Edentata, Dermoptera, Chiroptera, Carnivora, Insectivora, Proboscidea, Perissodactyla, Artiodactyla, Tubulidentata, Squamata, Sirenia, Cetacea, Primates, Rodentia, Lagomorpha or the like). Examples of subject include, but are not limited to, for example, an animal such as bovine, porcine, equine, chicken, cat, dog or the like. More preferably, a small animal such as mouse, rat, rabbit, hamster, guinea pig or the like may be used.

(Synthesis of Glycolipid)

As used herein, the term "protected sugar" refers to a sugar protected by a protecting group. As the protecting group, any protecting group can be used as long as it can protect the groups of a sugar from a certain reaction. The reason is as follows: generally, a protecting group may be used not only for protection of a hydroxyl or amino group, but also for steric control of glycosylation, however, in the method of the invention, a solvent effect attains steric control, thus the role of protection groups is to protect the sugar from a certain reaction, and therefore, as long as at least it can be attained, a glycolipid can be created. Furthermore, as a protecting group, in view of the reactivity thereof, a variety of protecting groups can be used. Accordingly, examples of protected sugars can include, but are not limited to:

[Chem. 53]

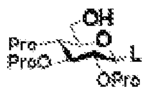
Glucose

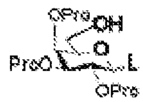
Galactose

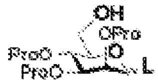
Mannose

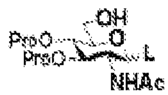
N-Acetyl glucosamine

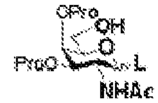
N-Acetyl galactosamine

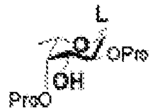
Fucose

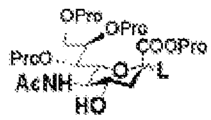
Sialic acid

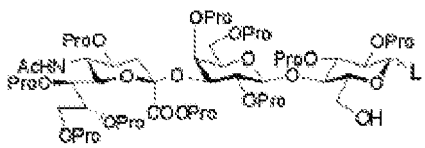
and

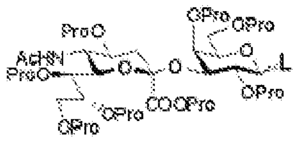

L: Leaving group

Pro: Protecting group

Examples:

Pro: Bz, Ac, Piv, MPM, MP Benzyl, Methyl

L: SPh, SMe, SEt, F, $OPO(OPh)_2$, $OPO(NMe_2)_2$ Trichloroacetimidate

The protected sugar chains exemplified above are those using the main sugars that exist in nature. By combining these, a wide variety of sugar chains can be constructed. Furthermore, it is understood that the representation of oligosaccharides in the formulas is an exemplification, and the number of sugars included may be an arbitrary number of two or more. Furthermore, the above-described sugar may be a sugar derivative. The reason for that is due to an example that was reported in which even a non-naturally occurring type sugar derivative has bioactivity.

In one embodiment, a protected sugar used in the invention, an acetyl, benzoyl, or benzyl group, or the like may be used as a protecting group of a hydroxyl group, and a methyl or benzyl group, or the like maybe used as a protecting group of a carboxyl group, but the protecting groups are not limited thereto. A phenylthio, fluoro, or trichloroacetimidate group, or the like may be used as the leaving group, but the leaving group is not limited threreto.

As used herein, the term "protected lipid amide" is a general term for lipid amides (for example, ceramide) having a protecting group and having the formula:

[Chem. 54]

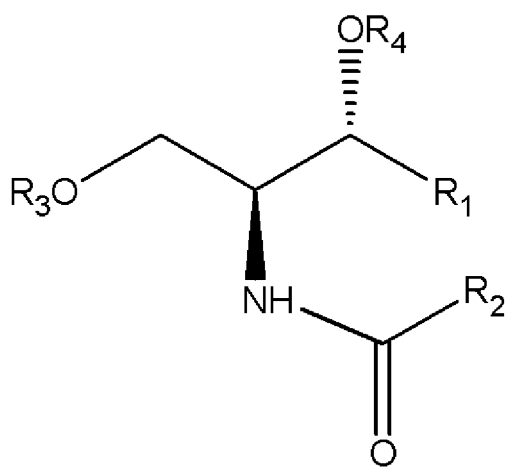

wherein $R_1$ and $R_2$ are independently selected from an alkyl or an alkenyl group; $R_3$ may be TBDPS, TIPS, Tr, isopropylidene ketal, or methoxybenzylidene acetal; and $R_4$ is a protecting group that may be succinyl, malonyl, phthaloyl, oxalyl, carbonyl, benzoyl, acetyl, or pivaloyl. Regarding a protecting group, in view of the reactivity thereof, a variety of protecting groups can be used. It is understood that these protecting groups, as long as the protective function is achieved, may be any protecting groups other than the exemplified protecting groups. The reason is that, in the method of the invention, in the case where only the primary hydroxyl group at 1-position can be glycosylated and the other hydroxyl groups can be protected, even using a protected lipid amide other than the above-described protected lipid amides, a glycolipid can be created.

Examples of protected lipid amides include, but are not limited to:

[Chem. 55]

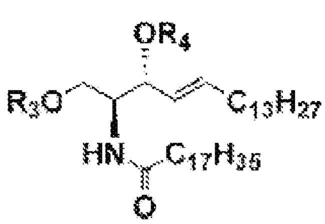

and

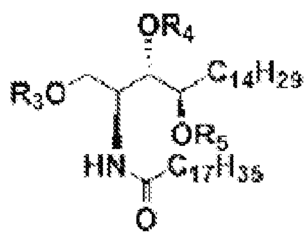

Sphingosine-type ceramide Phytosphingosine-type ceramide

It is understood that these protecting groups, $R_3$, $R_4$, and $R_5$, as long as the protective function is achieved, may be one other than the exemplified protecting groups, or any protecting groups. The reason is that, in the method of the invention, in the case where only the primary hydroxyl group at 1-position can be glycosylated and the other hydroxyl groups can be protected, even using a protected lipid amide other than the above-described protected lipid amides, a glycolipid can be created.

In the present specification, "conditions in which the protected sugar binds with a protected lipid amide" may be any condition under which a protected sugar binds with a protected lipid amide. Those skilled in the art can appropriately practice it while referring to textbooks listed in the Techniques above. Exemplary conditions can be conditions in which an alcohol binds with a carboxylic acid. The reason for this is because a protected sugar has only one free hydroxyl group which is not protected, and a protected lipid amide has a spacer with a carboxyl group. While not wishing to be bound by theory, a rational explanation is presented which is that conditions in which the protected sugar binds with a protected lipid amide may be achieved by any condition, as long as an alcohol binds with a carboxylic acid.

As another exemplary condition, examples of solvents can include, but are not limited to, tetrahydrofuran (THF), $CH_2Cl_2$, benzene, toluene, and N,N-dimethylformamide (DMF), and combinations thereof.

Reagents may be, but are not limited to, triphenylphosphine ($PPh_3$), DEAD, 1-methyl-3-(3-dimethylaminopropyl)-carbodiimide hydrochloride (WSC), 2,4,6-trichlorobenzoylchloride, triethylamine ($Et_3N$), 4-dimethylaminopyridine (DMAP), combinations thereof, or the like.

A reaction time may be from 2 to 4 hours, but may be no more or less than this range. The reason for that is considering the case where a reaction does not proceed completely , the reaction time can be extended, while in the case where a reaction efficiently proceeds, the reaction time is shortened.

A reaction temperature may be, but is not limited to, for example, 0 degree Celsius, 0 degree Celsius to room temperature (forexample, 0 degree Celsius, 5 degrees Celsius, 10 degrees Celsius, 15 degrees Celsius, 20 degrees Celsius, or the like), or room temperature or higher (for example, room temperature to 90 degrees Celsius, reflux at 90 degrees Celsius, or 90 degrees Celsius or higher). The reason is that the reaction temperature may vary according to other conditions such as the solvent.

These reaction conditions can be appropriately selected by those skilled in the art while considering the progress of a reaction.

As used in the present invention, the term "room temperature" refers to temperatures in the range of about 15 degrees Celsius to about 30 degrees Celsius, preferably, about 20 degrees Celsius to about 25 degrees Celsius.

As used herein, the term "sugar-lipid amide acceptor precursor" is not a sugar-lipid amide acceptor, but refers to any substance that is converted to a sugar-lipid amide acceptor by further reacting to this precursor (for example, condensation). Using another expression, it is a general term for compounds in which a sugar binds to a lipid amide acceptor precursor. When the case is that the lipid amide is a ceramide, it can be named sugar-ceramide acceptor

precursor.

A sugar-lipid amide acceptor precursor may be, but is not limited to:

[Chem. 56]

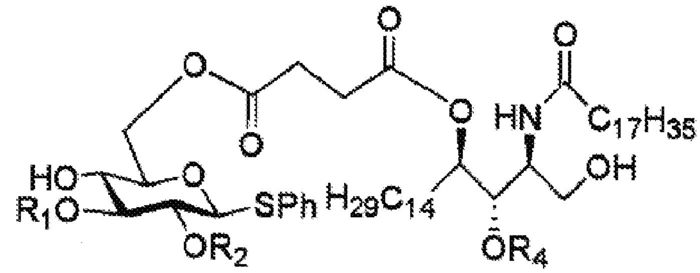

or

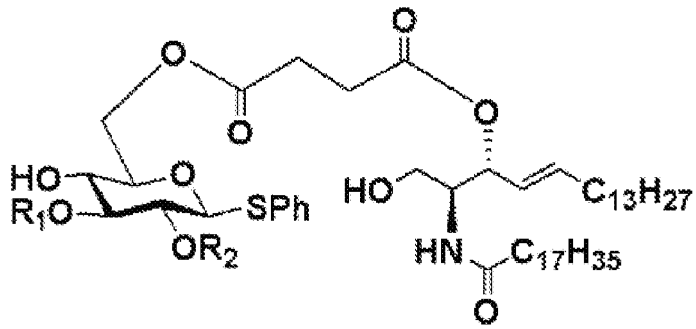

wherein $R_1$ and $R_2$ may be independently an alkyl or an alkenyl group, or the like; $R_3$ may be tert-butyldiphenylsilyl (TBDPS), tert-butyldimethylsilyl(TBDMS),triisopropylsilyl(TIPS), trityl (Tr), isopropylidene ketal, methoxybenzylidene acetal; $R_4$ may be a protecting group such as succinyl, malonyl, phthaloyl, oxalyl, carbonyl, benzoyl, acetyl, pivaloyl. It is understood that these protecting groups, as long as the protective function is achieved, may be other protecting groups than the exemplified protecting groups, or any other protecting groups. The reason for that is, in the method of the invention, a thioglycoside is used as the leaving group, wherein a glycolipid can be created even when using a sugar-lipid amido acceptor precursor having a protecting group other than the above-described protecting groups, and as long as the protecting group is resistant to an activating condition.

In the present specification, "conditions in which an intramolecular condensation reaction in the sugar-lipid amide acceptor precursor proceeds" may be any conditions as long as it is a condition under which an intramolecular condensation reaction of a sugar-lipid amido acceptor precursor proceeds. Those skilled in the art can appropriately practice it while referring to textbooks listed in the Techniques above.

As an exemplary condition, a reaction temperature maybe, but is not limited to, for example, -80 degrees Celsius to room temperature (for example, -40 degrees Celsius to room temperature, -20 degrees Celsius to 0 degree Celsius, -80 degrees Celsius to 0 degree Celsius).

In one embodiment, a reaction temperature may vary during the reaction. Examples in which a reaction temperature varies are that the reaction may start at -80 degrees Celsius, and then warmed up to -60 degrees Celsius, next to -40 degrees Celsius, and then to 0 degree Celsius, successively; that the reaction may be cooled down from -40 degrees Celsius to 0 degree Celsius; and that it may be warmed up from 0 degree Celsius to room temperature. The reason is that, as long as the reaction efficiently proceeds, the reaction temperature may be any temperature. Accordingly, in view of the state of a reaction, those skilled in the art can appropriately change the reaction temperature.

Examples of solvents that can be used include, but are not limited to, $CH_2Cl_2$, diethylether ($(CH_2CH_3)_2O$), diethylether, acetonitrile, diethylether, acetonitrile, propionitrile, toluene, nitromethane, combinations thereof, and the like. The reason is that, as long as an intended reaction proceeds, any solvent may be used. In view of the state of a reaction, those skilled in the art can appropriately select the solvent(s) used.

Examples of reagents that can be used include N-iodosuccinimide (NIS), trifluoromethanesulfonic acid (TfOH), dimethyl(methylthio)sulfonium triflate (DMTST), molecular sieves 4 angstroms (MS4Å), molecular sieves 3 angstroms (MS3Å), and the like. Here, it is understood that, as the reagent used, not only a catalyst, but also a desiccant and the

like can be appropriately present. The reason is that, as long as an intended reaction proceeds, any reagents may be used. In view of the state of a reaction, those skilled in the art can appropriately select the reagent(s) used.

A reaction time may be, for example, 1-48 hours (for example, 1, 1.5, 2, 2.5, 3, 5, 10, 15, 20, 24, 36, or 48 hours) (preferably, 1 to 5 hours), or the like, but it may be in a range other than those above. The reason is that, when a reaction does not efficiently proceed, the reaction time can be extended, while in the case where a reaction efficiently proceeds, the reaction time is shortened.

Asusedherein, the term "sugar-lipid amide acceptor" refers to a compound having a function of accepting a sugar chain and with a sugar-lipid structure in its structure.

A sugar-lipid amide acceptor may be, for example:

[Chem. 57]

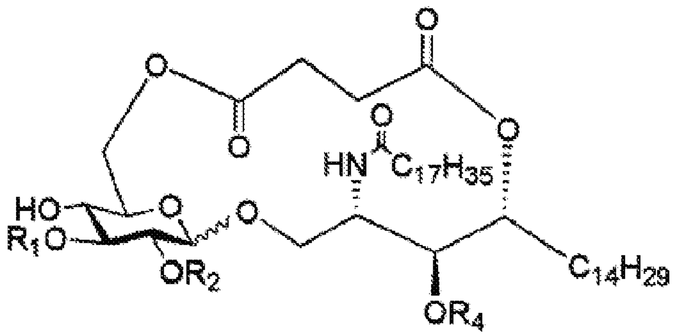

or

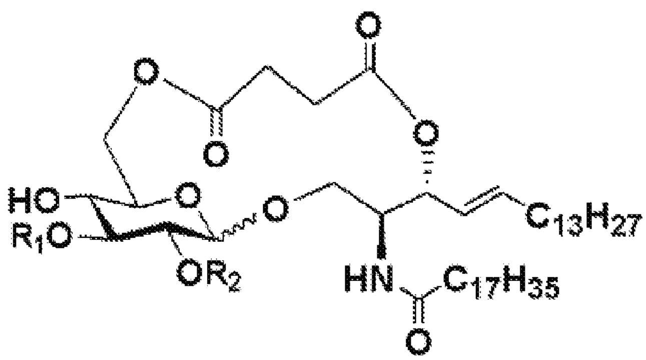

wherein $R_1$ and $R_2$ may be independently an alkyl or an alkenyl group; $R_3$ may be TBDPS, TBDMS, TIPS, Tr, isopropylidene ketal, or methoxybenzylidene acetal; $R_4$ is a protecting group that may be succinyl, malonyl, phthaloyl, oxalyl, carbonyl, benzoyl, acetyl, or pivaloyl. It is understood that these protecting groups, as long as the protective function is achieved, maybe other protecting groups than the exemplified protecting groups, or any other protecting groups. The reason for that is, in the method of the invention, a thioglycoside is used as a leaving group, wherein a glycolipid can be created even by using a sugar-lipid amido acceptor having a protecting group other than the above-described protecting groups, and as long as the protecting group is resistant to an activating condition.

As used herein, the term "protected sugar chain donor" refers to a "sugar chain donor" protected by a protecting group. It is understood that the protecting group used, as long as the protective function is achieved, may be other protecting group than the exemplified protecting groups, or any other protecting group. The protected sugar chain donor is, but is not limited to, for example:

[Chem. 58]

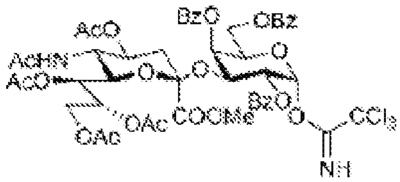

,

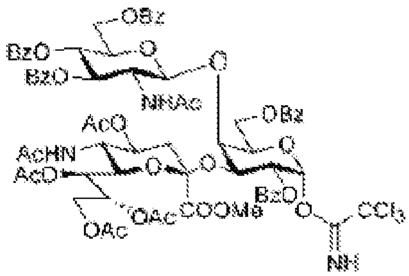

or

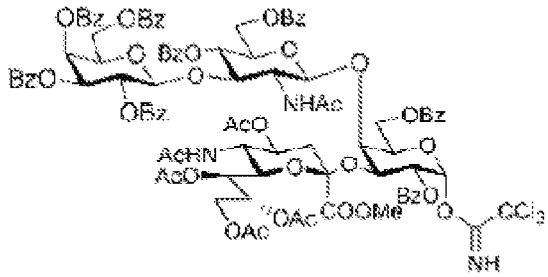

The protected sugar chain donor, as long as it has a leaving group at the reducing terminal of the sugar chain and the other protecting groups are protected, it may be any sugar. The reason is that, in glycosylation by an activating agent, only the anomer position of the sugar residue of the reducing terminal of the sugar chain leaves, and then thereto the sugar-lipid amide acceptor nucleophilically can attack. Thus, examples of such leaving groups can include, but are not limited to, -SPh, $-SCH_3$, $-SCH_2CH_3$, -F, $-OPO(OPh)_2$, and $-OPO(N(CH3)_2)_2$ (wherein Ph is phenyl).

As used herein, the term "sugar chain donor" refers to a compound giving a sugar chain to a sugar acceptor. Thus, a "sugar chain donor" may be any substance that can provide a sugar chain to a sugar acceptor, and it typically consists of a sugar portion and the other portion. In the present specification, the terms "sugar chain donor" and "glycosyl donor" can be interchangeably used.

In the present specification, "conditions in which the sugar-lipid amide acceptor binds with the protected sugar chain donor" maybe any conditions under which the sugar-lipid amide acceptor is connected with the protected sugar chain donor. Those skilled in the art can appropriately practice it while referring to textbooks listed in the Techniques above.

As an example a donor of more than about 2.5 equivalents relative to an acceptor may be used, but the equivalent is not limited thereto. The reason for that is, in view of the other conditions, those skilled in the art can appropriately change the equivalent of donor.

A reaction temperature may be, but is not limited to, for example, about -40 to about 0 degree Celsius (for example, about -40 degrees Celsius, about -35 degrees Celsius, about -30 degrees Celsius, about -25 degrees Celsius, about -20 degrees Celsius, about -15 degrees Celsius, about -10 degrees Celsius, about 5 degrees Celsius, about 4 degrees Celsius, about 3 degrees Celsius, about 2 degrees Celsius, about 1 degrees Celsius, about 0 degree Celsius, or the like) . The reason is that, as long as the reaction efficiently proceeds, the reaction temperature may be any temperature. Accordingly, in view of the state of a reaction, those skilled in the art can appropriately change the reaction temperature.

Examples of solvents include, but are not limited to, $CH_2Cl_2$ and the like. The reason is that, as long as the intended reaction proceeds, any solvent may be used. In view of the state of a reaction, those skilled in the art can appropriately select the solvent to be used.

Examples of reagents include, but are not limited to, trimethylsilyl trifluoromethanesulfonate (TMSOTf) and the like. The reason is that, as long as the intended reaction proceeds, any reagents may be used. In view of the state of a reaction, those skilled in the art can appropriately select the reagent to be used.

A reaction time may be, for example, about 1 to about 48 hours (for example, about 1, about 1.5, about 2, about 2.5, about 3, about 5, about 10, about 15, about 20, about 24, about 36, or about 48 hours) (preferably, 1-5 hours), or the like, but it may be in a range other than the above. The reason is that, in the case where a reaction does not efficientlyproceed, the reaction time can be extended, while in the case where a reaction efficiently proceeds, the reaction time is shortened.

As used herein, the term "protected glycolipid" refers to a glycolipid protected by a protecting group. It is understood that the protecting group used, as long as the protective function is achieved, may be other protecting group

than the exemplified protecting groups, or any other protecting group. The reason is that, in the method of the invention, a thioglycoside is used as the leaving group, wherein a glycolipid can be created even when using a protected glycolipid having a protecting group other than the above-described protecting groups, as long as the protecting group is resistant to an activating condition.

In the present specification, the term "glycolipid" is a general term of a complex lipid including a residue of carbohydrate. According to the type of a lipid portion, glycolipid is classified into glycosphingolipid, glycoglycerolipid, and other glycolipid, and examples of these glycolipids can include, but are not limited to, ganglioside GM3 and ganglioside GM4. Typical glycosphingolipid includes neutral glycosphingolipids such as galactocerebroside, glucocerebroside, and globoside, and acidic glycosphingolipids such as ganglioside. Glycoglycerolipid can include monogalactosyldiacylglycerol, digalactosyldiacylglycerol, and the like. Other kinds of glycolipids, such as glycolipid containing uronic acid (uronic acid-containing glycolipid), phosphonoglycolipid containing phosphonic acid, and phosphoglycolipid containing phosphoric acid have been found.

As used herein, the term "synthetic glycolipid" refers to any synthesized glycolipid. Examples of synthesized glycolipids can include glycosphingolipid, glycoglycerolipid, and the like. Typical glycolipids can include, but are not limited to, GM1, GM2, GM3, GM4, GD3, GD2, GD1a, GD1b, GT3, GT2, GT1a, GT1b, GT1c, GQ1b GQ1c GP1c, and the like.

[Chem. 59]

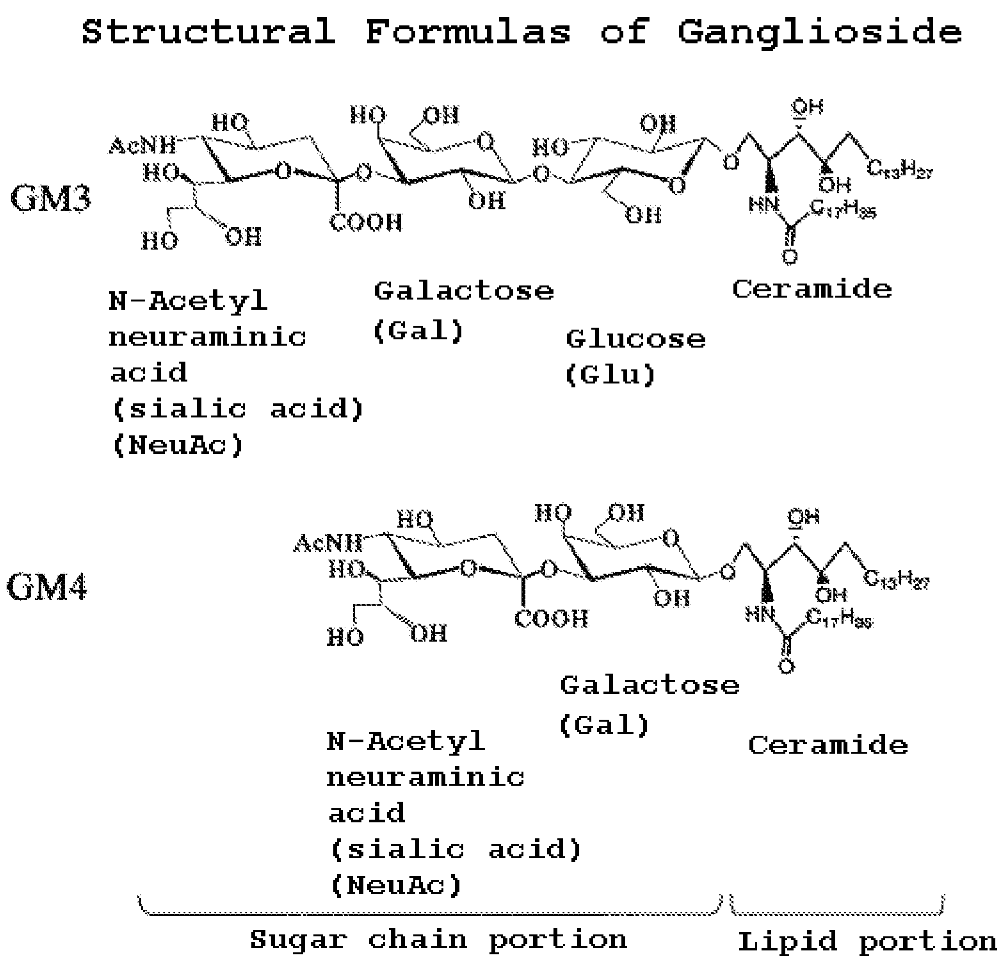


In the present specification, "conditions in which an alcohol binds with a carboxylic acid" may be any condition

as long as it is a condition under which an alcohol reacts with a carboxylic acid. Those skilled in the art can appropriately practice it while taking into consideration textbooks listed in the Techniques above. Exemplary conditions can include the following:

[Table 2]

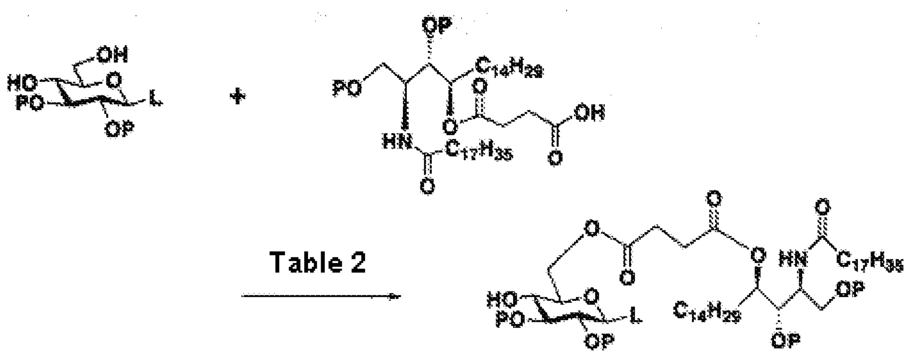


Table 2

| entry | Reagent (eq.) | Solvent | Temperature (˚C) |
|---|---|---|---|
| 1 | $PPh_3$(3.0),DEAD(3.0) | THF | reflux (90) |
| 2 | WSC(3.0) | $CH_2Cl_2$ | r.t. |
| 3 | 2,4,6-trichlorobenzoyl chloride (1.1)<br>$Et_3N$(1.5)<br>DMAP(1.5) | $CH_2Cl_2$ | r.t |

As used herein, the term "spacer precursor" refers to a precursor of a "spacer" that is intercalated between a sugar and a protected glycolipid amide and connects the sugar with the protected glycolipid amide.

As used herein, the term "spacer" refers to a substance that binds to a sugar and to a protected glycolipid amide. Examples of spacers include, but are not limited to, succinic acid, malonic acid, phthalic acid, oxalic acid, carboxylic acid, isopropylidene ketal, and methoxybenzylidene acetal. The reason is that, for an intramolecular reaction, the relative position between the functional groups to be reacted is important. However, in the method of the invention, even when performing a condensation reaction of a molecule having a relatively large degree of freedom (for example, succinic acid and the like), a sufficiently good result was obtained, and therefore it is thought that bridging using a molecule other than the above-described molecules is sufficiently effective.

In the present specification, "conditions in which the protected sugar chain donor is deprotected" may be any condition as long as it is a condition under which a protected sugar chain donor is deprotected. Those skilled in the art can appropriately practice it while referring to textbooks listed in the Techniques above.

As an exemplary, a reaction temperature may be, and is not limited to, for example, room temperature, preferably, room temperature. The reason is that, as long as the reaction efficiently proceeds, the reaction temperature may be any temperature. Accordingly, in view of the state of a reaction, those skilled in the art can appropriately change the reaction temperature.

Examples of solvents include, but are not limited to, $CH_2Cl_2$ and the like. The reason is that, as long as the intended reaction proceeds, any solvent may be used. In view of the state of a reaction, those skilled in the art can appropriately select the solvent to be used.

Examples of reagents include, but are not limited to, trifluoroacetic acid and the like. The reason is that, as long as the intended reaction proceeds, any reagents may be used. In view of the state of a reaction, those skilled in the art can appropriately select the reagent to be used.

The reaction time may be, for example, about 2 to about 12 hours (for example, about 2 hours, about 2.5 hours, about 3 hours, about 5 hours, about 10 hours, and about 12 hours), or the like, but it may be in a range other than the above. The reason is that, when a reaction does not efficiently proceed, the reaction time can be extended, while in the case where a reaction efficiently proceeds, the reaction time is shortened.

In the present specification, the term "under conditions in which a hydroxyl group at the 1-position of the protected lipid amide is deprotected" refers to a condition under which only the hydroxyl group at 1-position of a protected lipid amide is selectively deprotected, and other protecting groups are not affected. For example, in the case where the hydroxyl group at 1-position of a ceramide is protected by a silyl protecting group, this condition can be in THF solvent

in the presence of TBAF and AcOH at 0 degree Celsius.

As used herein, the term "acyl protecting group" refers to a protecting group having an acyl group. Examples thereof include, but are not limited to, acetyl, benzoyl, and pivaloyl groups, and the like.

In the present specification, "conditions in which an acyl protecting group is deprotected" may be any condition as long as it is a condition under which an acyl protecting group is removed by deprotection. Those skilled in the art can appropriately practice it while referring to textbooks listed in the Techniques above.

As an exemplary, a reaction temperature may be, and is not limited to, for example, room temperature to about 100 degrees Celsius (for example, about 20 degrees Celsius, about 25 degrees Celsius, about 30 degrees Celsius, about 35 degrees Celsius, about 40 degrees Celsius, about 45 degrees Celsius, about 50 degrees Celsius, about 55 degrees Celsius, about 60 degrees Celsius, about 65 degrees Celsius, about 70 degrees Celsius, about 75 degrees Celsius, about 80 degrees Celsius, about 85 degrees Celsius, about 90 degrees Celsius, about 95 degrees Celsius, and about 100 degrees Celsius). The reason is that, as long as the reaction efficiently proceeds, the reaction temperature may be any temperature. Accordingly, in view of the state of a reaction, those skilled in the art can appropriately change the reaction temperature.

Examples of solvents include, but are not limited to, methanol (MeOH), water ($H_2O$), and the like. The reason is that, as long as the intended reaction proceeds, any solvent may be used. In view of the state of a reaction, those skilled in the art can appropriately select the solvent to be used.

Examples of reagents include, but are not limited to, sodiummethoxide ($NaOCH_3$), KOH, and the like. The reason is that, as long as the intended reaction proceeds, any reagents may be used. In view of the state of a reaction, those skilled in the art can appropriately select the reagent to be used.

The reaction time may be, for example, about 1 hour-about 1 week (for example, about 1 hour, about 2 hours, about 2.5 hours, about 3 hours, about 5 hours, about 10 hours, about 12 hours, about 24 hours, about 2 days, about 3 days, about 4 days, about 5 days, about 6 days, and about 1 week), or the like, but it may be in a range other than those above. The reason is that, when the reaction does not efficiently proceed, the reaction time can be extended, while in the case where a reaction efficiently proceeds, the reaction time is shortened.

## The Description of the preferred embodiments

Hereinafter, preferable embodiments of the present invention will be described. Embodiments described below are provided only for a more profound understanding of the present invention, and the scope of the present invention shall not be restricted by the following description. It is thus obvious that those skilled in the art can modify the present invention properly within the scope of the present invention with reference to the present description.

### (Glycolipid-containing liposomes)

In one aspect, the present invention provides glycolipid-containing liposomes, the glycolipid including a synthetic glycolipid and not including a component accompanying a naturally-derived glycolipid.

In one embodiment, examples of the glycolipid include, but are not limited to, preferably, GM4, GM3, GM2, GM1, GD3, GD2, GD1a, GD1b, GT3, GT2, GT1a, GT1b, GT1c, GQ1b GQ1c GP1c, preferably, GM1, GM2, GM3, GM4, GD1a, GD1b, and GT1b, more preferably, GM3, GM4, and the like. The reason is that these have a N-acetylneuraminic acid (Neu-5AC) at the terminal, and thus bind by a coupling reaction with a bridging spacer.

In other embodiments, absorbance of the liposome at 680 nm may be, but is not limited to, about 0.5 to about 3.0 (for example, about 0.6-3.0, 0.8-3.0, 1.0-3.0, 1.5-3.0, 2.5-3.0, 0.5-2.5, 0.5-2, 0.5-1.5, 0.5-1.0, 0.5-0.8, 0.5-0.6, 0.6-2.5, 0.6-2.0, 0.6-1.5, 0.6-1.0, 0.6-0.8, 0.7-2.5, 0.7-2.0, 0.7-1.5, 0.7-1.0, 0.7-0.8, 0.8-2.5, 0.8-2.0, 0.8-1.5, 0.8-1.0, 1.0-2.5, 1.0-2.0, 1.0-1.5, 1.5-2.5, 1.5-2.0, and 2.0-2.5). The reason is that the absorbance of the liposome at 680 nm may be in any range as long as it is in a range in which the fluorescence signal *in vivo* can be detected by *in vivo* imaging. Preferably, absorbance of the liposome at 680 nm can be 0.5 or higher. Higher absorbance allows for a fluorescence signal to be detected more sensitively.

In another embodiment, the amount of lipid of the liposome may be, but is not limited to, about 0.5 to about 5.0 (for example, about 0.6 to 5.0, 0.8 to 5.0, 1.0 to 5.0, 2.0 to 5.0, 3.0 to 5.0, 4.0 to 5.0, 0.5 to 4.0, 0.6 to 4.0, 0.8 to 4.0, 1.0 to 4.0, 2.0 to 4.0, 3.0 to 4.0, 0.5 to 3.0, 0.6 to 3.0, 0.8 to 3.0, 1.0 to 3.0, 2.0 to 3.0, 0.5 to 2.0, 0.6 to 2.0, 0.8 to 2.0, 1.0 to 2.0, 0.5 to 1.0, 0.6 to 1.0, 0.8 to 1.0, 0.5 to 0.8, and 0.6 to 0.8) mg/mL. The reason is that, the liposome can be concentrated or the like according to the purpose of usage, and thus the lipid amount can be 5 mg or more.

In another embodiment, the amount of HSA of the liposome may be, but is not limited to, about 0.1 to about 1.0 (for example, about 0.2 to 1.0, 0.4 to 1.0, 0.6 to 1.0, 0.8 to 1.0, 0.1 to 0.8, 0.2 to 0.8, 0.4 to 0.8, 0.6 to 0.8, 0.1 to 0.6, 0.2 to 0.6, 0.4 to 0.6, 0.1 to 0.4, and 0.2 to 0.4) mg/mL. The reason is that the liposome can be concentrated or the like according to the purpose of usage, and thus the amount of protein can be 1 mg or more.

In another embodiment, the mean particle size of liposome can be, but is not limited to, about 50 to about 300

(for example, about 60 to 300, 80 to 300, 100 to 300, 150 to 300, 200 to 300, 250 to 300, 50 to 250, 60 to 250, 80 to 250, 100 to 250, 150 to 250, 200 to 250, 50 to 200, 60 to 200, 80 to 200, 100 to 200, 150 to 200, 50 to 150, 600 to 150, 80 to 150, 100 to 150, 50 to 100, 60 to 100, 80 to 100, 50 to 80, 60 to 80, and 50 to 60) nm. The reason is that, according to the purpose of usage of the liposome, a liposome having a mean particle size of no more than 50 nm or no less than 300 nm may be contemplated, and those skilled in the art, when practicing the invention, can appropriately change the size of liposome on the basis of the description of the present specification.

In another embodiment, a Z electric potential of the liposome may be, but is limited to, about -120 to about -30 (for example, about -110 to -30, -100 to -30, -80 to -30, -50 to -30, -40 to -30, -120 to -40, -110 to -40, -100 to -40, -80 to -40, -50 to -40, -120 to -50, -110 to -50, -100 to -50, -80 to -50, -120 to -60, -110 to -60, -100 to -60, -80 to -60, -120 to -80, -110 to -80, -100 to -80, -120 to -100, -110 to -100, or -120 to -110) mV. The reason is that, according to the use of the liposome, a liposome of which the Z potential is no more than -120 mV or no less than -30mV may be considered, and those skilled in the art, when practicing the invention, can appropriately prepare a liposome of which the Z potential is out of the above-described range, on the basis of the description of the present specification.

In other embodiments, the liposome may encapsulate a desired substance. Examples of desired substances include, but are not limited to, fluorescent substances (for example, cy5.5, cy5, cy7, cy3B, cy3.5, Alexa Fluor 350, Alexa Fluor 488, Alexa Fluor 532, Alexa Fluor 546, Alexa Fluor 555, Alexa Fluor 568, Alexa Fluor 594, Alexa Fluor 633, Alexa Fluor 647, Alexa Fluor 680, Alexa Fluor 700, Alexa Fluor 750, fluorescein-4-isothiocyanate (FITC), europium-containing label), fluorescent proteins (GFP, CFP, YFP), luminescent enzyme (luciferase and the like), proteins (antibody, tPA, β-galactosidase, albumin, botulinus toxin, diphtheria toxin, and the like), compounds (methylprednisolon, prednisolon phosphate, peptide, gold colloid, Gd complex, Fe complex, cisplatin, pravastatin, heparin, Fasudil hydrochloride, clodronic acid, water-soluble iodine, chitin, chitosan, and the like), nucleic acid (plasmid DNA, RNAi, and the like), and the like. The reason is that the glycolipid-containing liposome of the present invention can encapsulate a variety of substances by changing its constitution according to a physical property of an encapsulated substance. Thus, the glycolipid-containing liposome of the present invention can change the encapsulated substance according to a certain purpose.

In another embodiment, the liposome may include target-recognizing probes on the surface of the liposome. Examples of target-recognizing probes include, but are not limited to, sugar chain, antibody, antigen, peptide, nucleic acid (for example, aptamer and the like), hyaluronic acid, and the like. The reason for that is that the glycolipid-containing liposome of the present invention can encapsulate a variety of substances by changing its constitution according to a physical property of an encapsulated substance. Thus, the glycolipid-containing liposome of the present invention can change the encapsulated substance according to a certain purpose.

In a preferred embodiment, an amount of the sugar chain added may be, but is not limited to, about 0.5 to about 500 μg/mL of bond density of sugar chain (for example, about 1 to 500 μg/mL, 5 to 500 μg/mL, 10 to 500 μg/mL, 50 to 500 μg/mL, 100 to 500 μg/mL, 150 to 500 μg/mL, 200 to 500 μg/mL, 300 to 500 μg/mL, 400 to 500 μg/mL, 0.5 to 400 μg/mL, 1 to 400 μg/mL, 5 to 400 μg/mL, 10 to 400 μg/mL, 50 to 400 μg/mL, 100 to 400 μg/mL, 150 to 400 μg/mL, 200 to 400 μg/mL, 300 to 400 μg/mL, 0.5 to 300 μg/mL, 1 to 300 μg/mL, 5 to 300 μg/mL, 10 to 300 μg/mL, 50 to 300 μg/mL, 100 to 300 μg/mL, 150 to 300 μg/mL, 200 to 300 μg/mL, 0.5 to 200 μg/mL, 1 to 200 μg/mL, 5 to 200 μg/mL, 10 to 200 μg/mL, 50 to 200 μg/mL, 100 to 200 μg/mL, 150 to 200 μg/mL, 0.5 to 100 μg/mL, 1 to 100 μg/mL, 5 to 100 μg/mL, 10 to 100 μg/mL, 50 to 100 μg/mL, 0.5 to 50 μg/mL, 1 to 50 μg/mL, 5 to 50 μg/mL, 10 to 50 μg/mL, 0.5 to 50 μg/mL, 1 to 10 μg/mL, 5 to 10 μg/mL, 1 to 5 μg/mL, and 0.5 to 1 μg/mL). The reason is that, as long as the sugar chainsworkasthetarget-recognizingprobes, thebonddensity of a sugar chain may be out of the above-described range.

In another embodiment, the amount of antibody added may be, but is not limited to, about 0.1 to about 100 μg/mL, about 0.1 to about 50 μg/mL, about 0.3 to about 100 μg/mL (for example, about 0.4 to 100 μg/mL, 0.5 to 100 μg/mL, 1 to 100 μg/mL, 5 to 100 μg/mL, 10 to 100 μg/mL, 50 to 100 μg/mL, 90 to 100 μg/mL, 0.3 to 90 μg/mL, 0.4 to 90 μg/mL, 0.5 to 90 μg/mL, 1 to 90 μg/mL, 5 to 90 μg/mL, 10 to 90 μg/mL, 50 to 90 μg/mL, 0.3 to 50 μg/mL, 0.4 to 50 μg/mL, 0.5 to 50 μg/mL, 1 to 50 μg/mL, 5 to 50 μg/mL, 10 to 50 μg/mL, 0.3 to 10 μg/mL, 0.4 to 10 μg/mL, 0.5 to 10 μg/mL, 1 to 10 μg/mL, 5 to 10 μg/mL, 0.3 to 5 μg/mL, 0.4 to 5 μg/mL, 0.5 to 5 μg/mL, 1 to 5 μg/mL, 0.3 to 1 μg/mL, 0.4 to 1 μg/mL, 0.5 to 1 μg/mL, 0.3 to 0.5 μg/mL, 0.4 to 0.5 μg/mL, and 0.3 to 0.4 μg/mL). The reason is that, as long as the antibody works as a target-recognizing probe, the amount of antibody added may be out of the above-described range.

In one embodiment, examples of lipids constituting the glycolipid-containing liposome of the present invention include, but are not limited to, phosphatidylcholines, phosphatidylethanolamines, phosphatidic acids, long-chain-alkyl phosphoric acid salts, glycolipids (gangliosides and the like), phosphatidylglycerols, sphingomyelins, cholesterols, and the like.

Phosphatidylcholines include, but are not limited to, dimyristoylphosphatidylcholine, dipalmitoylphosphatidylcholine, distearoylphosphatidylcholine, and the like.

Phosphatidylethanolamines include, but are not limited to, dimyristoylphosphatidylethanolamine, dipalmitoylphosphatidylethanolamine, distearoylphosphatidylethanolamine, and the like.

Phosphatidic acids include dimyristoylphosphatidic acid, dipalmitoylphosphatidic acid, and distearoylphosphatidic acid. Long-chain-alkyl phosphoric acid salts include, but are not limited to, dicetylphosphate, and the like.

Glycolipids include galactosylceramide, glucosylceramide, lactosylceramide, phosphatide, globoside, gangliosides, and the like. Gangliosides include, but are not limited to, ganglioside GM1 (Galβ1, 3GalNAcβ1, 4(NeuAα2,3) Galβ1, 4Glcβ1, 1'Cer), ganglioside GDla, ganglioside GTlb, and the like.

As phosphatidylglycerols, dimyristoylphosphatidylglycerol, dipalmitoylphosphatidylglycerol, distearoylphosphatidylglycerol, and the like are preferred.

Among of those above, phosphatidic acids, long-chain-alkyl phosphoric acid salts, glycolipids, and cholesterols have an effect of increasing the stability of a liposome, and therefore it is desirable to add them as a component lipid. Examples of lipids constituting the liposome used in the present invention include, but are not limited to, a lipid comprising: one or more kinds of lipids (molar ratio: 0 to 30%) selected from the group consisting of phosphatidylcholines (molar ratio: 0 to 70%), phosphatidylethanolamines (molar ratio: 0 to 30%), phosphatidic acids, and long-chain-alkyl phosphoric acid salts; one or more kinds of lipids (molar ratio: 0 to 40%) selected from the group consisting of glycolipids, phosphatidylglycerols, and sphingomyelins; and cholesterols (molar ratio: 0 to 70%). It is preferred to include ganglioside, glycolipid, or phosphatidylglycerol. The reason is that binding a linker like albumin become easy.

Examples of lipids constituting the glycolipid-containing liposome of the present invention include, but are not limited to, dipalmitoylphosphatidylcholine, cholesterol, ganglioside, dicetylphosphate, dipalmitoylphosphatidylethanolamine, sodium cholate, dicetylphosphatidylethanolamine-polyglycerin 8G, dimyristoylphosphatidylcholine, distearoylphosphatidylcholine, dioleoylphosphatidylcholine, dimyristoylphosphatidylserine, dipalmitoylphosphatidylserine, distearoylphosphatidylserine, dioleoylphosphatidylserine, dimyristoylphosphatidylinositol, dipalmitoylphosphatidylinositol, distearoylphosphatidylinositol, dioleoylphosphatidylinositol, dimyristoylphosphatidylethanolamine, distearoylphosphatidylethanolamine, dioleoylphosphatidylethanolamine, dimyristoylphosphatidic acid, dipalmitoylphosphatidic acid, distearoylphosphatidic acid, dioleoylphosphatidic acid, galactosylceramide, glucosylceramide, lactosylceramide, phosphatide, globoside, GM1 (Galβ1, 3GalNAcβ1, 4(NeuAα2,3)Galβ1, 4Glcβ1, 1'Cer), gangliosideGD1a, gangliosideGD1b, dimyristoylphosphatidylglycerol, dipalmitoylphosphatidylglycerol, distearoylphosphatidylglycerol, dioleoylphosphatidylglycerol, and the like. Preferably, the lipid may be dipalmitoylphosphatidylcholine, cholesterol, ganglioside, dicetylphosphate, dipalmitoylphosphatidylethanolamine, sodium cholate, dicetylphosphatidylethanolamine-polyglycerin 8G, dipalmitoylphosphatidylcholine, or dipalmitoylphosphatidylglycerol.

In a preferred embodiment, a liposome can include ganglioside, glycolipid, orphosphatidylglycerol, which can bind to a linker such as a peptide in order to bind to a sugar chain.

When a liposome is prepared, by adding ganglioside, glycolipid, or phosphatidylglycerol, the liposome includes sugar chains included in the glycolipid above, as a component, and consequently a synthetic ganglioside sugar chain-modified liposome can be prepared.

In another preferred embodiment, the liposome in the present invention preferably includes phosphatidylethanolamine. The reason is that, by including phosphatidylethanolamine, binding to a hydrophilicity-imparting group (tris (hydroxyalkyl)aminoalkane and the like) becomes easier.

In another embodiment, in the present invention, paramagnetism is given as the result of at least one of the components of the glycolipid-containing liposome that has paramagnetism, or that the newly glycolipid further has an element having paramagnetism.

Examples of elements having paramagnetism and being capable of imparting it include, but are not limited to, paramagnetic metals (for example, gadolinium, iron, and the like), contrast medium (for example, iron oxide particle, gadolinium chelating agent, barium sulfate, water-soluble iodine, and the like). The reason is that the element may be any substance as long as it can set a composition under a detectable condition to the magnetic resonance imaging.

In another embodiment, in the present invention, fluorescence is imparted as the result of at least one of the components of the glycolipid-containing liposome that has fluorescence, or that the newly glycolipid-containing liposome further has an element having fluorescent.

Examples of elements having fluorescence include, but are not limited to, fluorochrome, fluorescent protein (for example, GFP, CFP, YFP, and the like), and fluorescent enzyme (for example, luciferase and the like). Examples of fluorochomes are include cy5.5 (for example,

[Chem. 60]

<Cy5. 5>

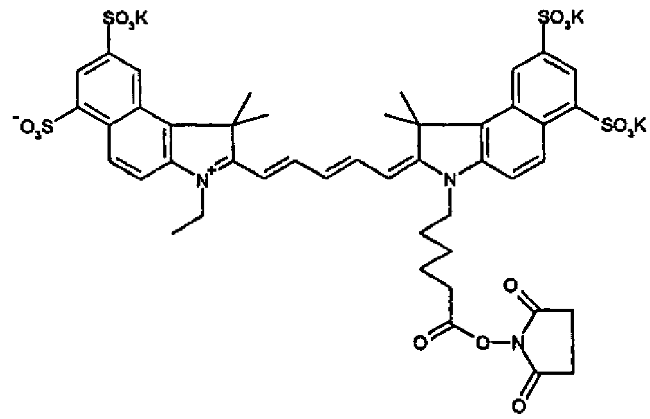

,

[Chem. 61]

<cy3>

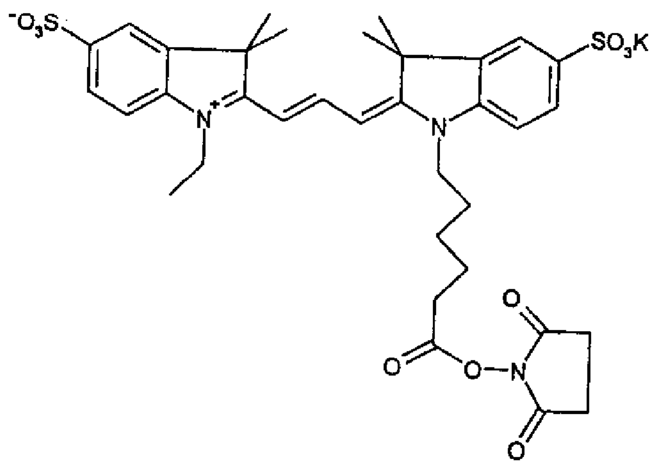

,

cy5, cy7, cy3B, cy3.5, Alexa Fluor 350, Alexa Fluor 488, Alexa Fluor 532, Alexa Fluor 546, Alexa Fluor 555, Alexa Fluor 568, Alexa Fluor 594, Alexa Fluor 633, Alexa Fluor 647, Alexa Fluor 680, Alexa Fluor 700, Alexa Fluor 750, fluorescein-4-isothiocyanate (FITC), europium-containing label, and combinations thereof. By these fluorochomes, fluorescence can be originated.

In another embodiment, the glycolipid-containing liposome (for example, a sugar-chain-modified liposome, an antibody liposome, and the like), which is modified with a target-site-recognition probe of the present invention, may include a target-recognizing probe (for example, sugar chain, antibodies) with an appropriate density for delivery to the intended site.

In the glycolipid-containing liposome of the present invention, the upper limit of the modification bond density may be any density as far as, by a target-site-recognition probe (for example, sugar chain, antibody), the liposome accumulate at the target site. The reason is that the higher the modification bond density the lower is the accumulation

property. For example, in the case of a sugar chain, the upper limit of the modification bond density may be about 500 mg sugar chain/mg lipid, or more about 500 mg sugar chain/mg lipid, or may be about 100 mg sugar chain/mg lipid, 10 mg sugar chain/mg lipid, 5 mg sugar chain/mg lipid, 1 mg sugar chain/mg lipid, 0.9 mg sugar chain/mg lipid, 0.8 mg sugar chain/mg lipid, 0.7 mg sugar chain/mg lipid, 0.6 mg sugar chain/mg lipid, 0.5 mg sugar chain/mg lipid, 0.4 mg sugar chain/mg lipid, 0.3 mg sugar chain/mg lipid, 0.2 mg sugar chain/mg lipid, 0.1 mg sugar chain/mg lipid, 0.09 mg sugar chain/mg lipid, 0.08 mg sugar chain/mg lipid, 0.07 mg sugar chain/mg lipid, 0.06 mg sugar chain/mg lipid, 0.05 mg sugar chain/mg lipid, 0.04 mg sugar chain/mg lipid, 0.03 mg sugar chain/mg lipid, 0.029 mg sugar chain/mg lipid, 0.028 mg sugar chain/mg lipid, 0. 027 mg sugar chain/mg lipid, 0.026 mg sugar chain/mg lipid, or 0.025 mg sugar chain/mg lipid.

In the case of an antibody, converting 1 ml of the liposome solution to 3 mg of lipid amount, the upper limit of the modification bond density may be 0.1 μg/mg lipid, 0.5 μg/mg lipid, 1 μg/mg lipid, 2 μg/mg lipid, 4 μg/mg lipid, 8 μg/mg lipid, 16 μg/mg lipid, or 32 mg/mg lipid.

The lowest limit for the modification bond density may be any density as far as a stimulation property to a target site can be detected. The reason is that, according to the purpose of usage, use, or experiment technique, the lowest limit for the modification bond density varies. In the case of a sugar chain, the lowest limit for the modification bond density, for example, may be about 0.0001 mg sugar chain/mg lipid, or less than about 0.0001 mg sugar chain/mg lipid, or may be about 0.005 mg saccharide/mg lipid, 0.0025 mg saccharide/mg lipid, 0.002 mg sugar chain/mg lipid, 0.001 mg saccharide/mg lipid, 0.09 mg saccharide/mg lipid, 0.08 mg sugar chain/mg lipid, 0.07 mg saccharide/mg lipid, 0.06 mg saccharide/mg lipid, 0.05 mg saccharide/mg lipid, 0.04 mg sugar chain/mg lipid, 0.03 mg saccharide/mg lipid, 0.029 mg saccharide/mg lipid, 0.028 mg saccharide/mg lipid, 0.027 mg saccharide/mg lipid, 0.026 mg saccharide/mg lipid, or 0.025 mg sugar chain/mg lipid.

In the case of an antibody, for converting 1 ml of the liposome solution to 3 mg of lipid amount, the lowest limit for the modification bond density may be about 0.1 μg/mg lipid, 0.5 μg/mg lipid, 1 μg/mg lipid, 2 μg/mg lipid, 4 μg/mg lipid, 8 μg/mg lipid, 16 μg/mg lipid, or 32 mg/mg lipid.

Consequently, as the glycolipid-containing liposome of the present invention, liposomes in a density range combining any of the upper limits and any of the lowest limits described above (for example, about 500 mg sugar chain/mg lipid to about 0.0001 mg sugar chain/mg lipid) may be used. The reason is that, without being bound by theory, if the glycolipid-containing liposome of the present invention can accumulate at the target site, the effect of working as DDS will be expected. Thus, in any ranges combining the upper limit and the lowest limit of the modification bond density described above, the effect that the liposome can be used as DDS is attained. The reason is that, in those ranges, the liposome is stable in blood, and is capable of binding to the target site.

In one embodiment, the hydrophilization can be imparted by a group having many hydroxyl groups and an amino group, for example, tris(hydroxyalkyl)alkylamino group (for example, tris(hydroxymethyl)methylamino group, tris (hydroxymethyl)ethylamino group, tris(hydroxyethyl)ethylamino group, tris(hydroxypropyl)ethylamino group, tris(hydroxymethyl)methylamino group, tris(hydroxyethyl)methylamino group, tris(hydroxypropyl)methylamino group, tris(hydroxymethyl)propylamino group, tris(hydroxyethyl)propylamino group, tris(hydroxypropyl)propylamino group, and the like). The hydrophilization may be imparted by any other group than the above. The reason is that the group may be any group as long as the group can impart such a degree of hydrophilicity that a liposome maintains a stealth property, stability, and an accumulation property to the target site in a blood vessel.

In one embodiment, some of the lipids constituting the liposome used in the glycolipid-containing liposome of the present invention bind to a hydrophilic compound crosslinking group-W-tris(hydroxyalkyl)alkylamino group via NH-C(=O) bond, wherein W may be C(=O)-NHbond. Preferably, tris(hydroxyalkyl)alkylamino group may be tris(hydroxymethyl)methylamino group. Without being bound by theory, the reason is that, as understood from the description of the present specification that the glycolipid-containing liposome of the present invention, when it has the aforementioned constitution, can attain the effect that the liposome can be used as DDS, wherein a liposome hydrophilized by a tris (hydroxymethyl)methylamino group is more stable and has a higher stealth property than a liposome that is not hydrophilized in blood, and thereby the retentivity in blood is improved, the concentration of the liposome in the blood becomes difficult to lower, and accumulation at the disease site is improved.

In one embodiment, in the glycolipid-containing liposome of the present invention, the liposome may bind to the target-recognizing probe group (for example, SLX group) via a serum albumin group (for example, human serum albumin group). Without being bound by theory, in the glycolipid-containing liposome of the present invention, the reason is that, as understood from the description of the present specification that, by binding the liposome with a target-recognizing probe via a serum albumin group, the effect that the liposome can be used as DDS can be attained, the serum albumin existing in the blood by binding onto the surface of the liposome increases the stability and the stealth property of the liposome in the blood as well as it improves the retentivity in the blood, and consequently the accumulation at the disease site is also improved.

In one embodiment, in the glycolipid-containing liposome of the present invention, some of the lipids constituting the liposome may bind to a serum albumin group -A-linker protein crosslinking group -X- target recognizing site group

(for example, SLX group) via $CH_2$-NH bond, wherein A is NH-C(=O) bond and X may be C(=O)-NH bond. A serum albumin group may be, for example, a human serum albumin group. Without being bound by theory, the reason is that, as understood from the description of the present specification that the glycolipid-containing liposome of the present invention, when it has the aforementioned constitution, can attain the effect that it can be used as DDS, the serum albumin existing in the blood by binding onto the surface of the liposome increases the stability and the stealth property of the liposome in the blood as well as it improves the retentivity in the blood, and consequently the accumulation at the disease site is also improved, and furthermore, by binding a sugar chain onto serum albumin via a crosslinking agent, a target-recognizing probe group (for example, SLX group) can be presented on the surface of the liposome under the condition which steric hindrance is low, and consequently the function of the target-recognizing probe groups is not inhibited.

In one preferred embodiment, the glycolipid-containing liposome of the present invention may encompass a liposome consisting of:

(a) phosphatidylcholines(molar ratio: 0 to 70%);
(b) cholesterols (molar ratio: 0 to 70%);
(c) one or more kinds of lipids selected from the group consisting of glycolipids, phosphatidylglycerols, and sphingomyelins (molar ratio 0 to 40%);
(c') one or more kinds of lipid variants selected from the group consisting of variants of glycolipids, variants of phosphatidylglycerols, and variants of sphingomyelins (molar ratio 0 to 40%);
(d) one or more kinds of lipids selected from the group consisting of phosphatidic acids, and long-chain-alkyl phosphoric acid salts (molar ratio 0 to 30%);
(e) phosphatidylethanolamines (molar ratio 0 to 30%); and (e') variants of phosphatidylethanolamines (molar ratio 0 to 30%).

For example, this liposome may include dipalmitoylphosphatidylcholine, cholesterol, ganglioside, dicetylphosphate, dipalmitoylphosphatidylethanolamine, and sodium cholate in the ratio of 35:40:15:5:5:167. This liposome may further include (f) a negatively-charged surfactant. The reason is that, as a result that the liposome includes a negatively-charged component, it becomes difficult for the liposome to be uptaken by negatively-charged cells, and consequently it is thought that non-specific adsorption to endothelial cells and the like can be prevented.

In one embodiment, this liposome may encapsulate (h) a labeling substance as a substance for diagnosing whether or not a disease is present.

In the above-described liposome, the (c') lipid variants are substances in which one or more kinds of lipids selected from the group consisting of glycolipids, phosphatidylglycerols, and sphingomyelins bind to a serum albumin group -A- linker protein crosslinking group -X-target-recognizing probe group (for example, SLX group) via $CH_2$-NH bond, wherein A is NH-C(=O) bond, X may be C(=O)-NH bond. Without being bound by theory, the reason is that, as understood from the description of the present specification that the glycolipid-containing liposome of the present invention, when it has the aforementioned constitution, can attain the effect that it can be used as DDS, binding serum albumin existing in blood onto the surface of the liposome increases the stability and the stealth property of the liposome in blood to improve the retentivity in blood, and consequently the accumulation at the disease site is also improved, and furthermore, by binding a target-recognizing probe (for example, sugar chain) onto serum albumin via a crosslinking agent, a target-recognizing probe group (for example, SLX group) can be presented on the surface of the liposome under the condition which steric hindrance is low, and consequently the function of the target-recognizing probe group is not inhibited.

In the liposome, the (e') variants are substances in which phosphatidylethanolamine binds to a hydrophilic compound crosslinking group -W-tris(hydroxyalkyl)alkylamino group via NH-C(=O) bond, wherein W may be C(=O)-NHbond. Here, the target-recognizing probe group may exist on the surface of the liposome. In the liposome used in the present invention, a liposome hydrophilized by a tris(hydroxymethyl)methylamino group is more stable and has a higher stealth property than a liposome that is not hydrophilized in blood, and thereby the retentivity in blood is improved, and consequently the accumulation at the disease site is also improved, and furthermore, the presence of the target-recognizing probe group on the surface of the liposome make it easier to bind to the target site. For the above reason, it is preferred that the liposome has this form.

In one embodiment, Examples of linker protein crosslinking groups that may be used in the glycolipid-containing liposome of the present invention include, but are not limited to, bis(sulfosuccinimidyl)glutarate-$d_0$ group, bis(sulfosuccinimidyl)2,2,4,4-glutarate-$d_4$ group, bis(sulfosuccinimidyl)suberate group, bis(sulfosuccinimidyl)suberate-$d_0$ group, bis (sulfosuccinimidyl)2,2,7,7-suberate-$d_4$ group, bis(2-[succinimidoxycarbonyloxy]ethyl)sulfone group, disuccinimidylglutarate, dithiobis(succinimidylpropionate) group, disuccinimidyl suberate group, disuccinimidyltartrate group, ethyleneglycolbis(succinimidylsuccinate) group, sulfodisuccinimidyltartrate group, ethyleneglycolbis(sulfo-succinimidylsuccinate) group, tris-(succinimidyl)aminotristearate) group. The reason is that, as long as an amino group of serum albumin can

be crosslinked with an amino group of a sugar chain, the crosslinking group may be any crosslinking group.

In one embodiment, examples of hydrophilic compound crosslinking groups that may be used in the glycolipid-containing liposome of the present invention can include, but are not limited to, bis(sulfosuccinimidyl)glutarate-do group, bis(sulfosuccinimidyl)2,2,4,4-glutarate-d4 group, bis(sulfosuccinimidyl)suberate-do group, bis(sulfosuccinimidyl) 2,2,7,7-suberate-$d_4$ group, bis(2-[succinimidoxycarbonyloxy]ethyl)sulfone group, disuccinimidylglutarate, disuccinimidylsuberate, disuccinimidyltartrate group, ethyleneglycolbis(succinimidylsuccinate) group, ethyleneglycolbis(sulfo-succinimidylsuccinate) group, tris-(succinimidylaminotristearate) group. The reason is that, as long as a peptide bond can be formed between a hydrophilic compound group and a liposome or a linker protein, the crosslinking agent may be any crosslinking agent.

In one embodiment, the glycolipid-containing liposome of the present invention may further contain (g)a biocompatible buffer solution. Examples of (g) biocompatible buffer solutions include, but are not limited to, phosphate buffered physiological saline (PBS), physiological saline, Tris buffer solution, carbonate buffer solution (CBS), tris(hydroxymethyl)methylaminopropane sulfonate buffer solution (TAPS), 2-[4-(2-hydroxylethyl)-1-piperazinyl]ethanesulfonic acid (HEPES), other Good's Buffer Solutions (for example, 2-morpholinoethanesulfonic acid, monohydrate (MES), bis (2-hydroxyethyl)iminotris(hydroxymethyl)methane (Bis-tris), N-(2-acetamide)iminodiacetic acid (ADA), 1,3-bis[tris(hydroxymethyl)methylamino]propane(Bis-tris propane), piperazine-1,4-bis(2-ethanesulfonic acid) (PIPES), N-(2-acetamide)-2-aminoethanesulfonic acid (ACES), cholamine chloride, N,N-bis(2-hydroxyethyl)-2-aminoethanesulfonic acid (BES), 3-morpholinopropanesulfonic acid (MOPS), N-tris(hydroxymethyl)methyl-2-aminoethanesulfonic acid (TES), N-(2-hydroxyethyl)piperazine-N'-3-propanesulfonic acid (HEPPS), N-[tris(hydroxymethyl)methyl]glycin (Tricine), aminoacetamide(glycinamide), N,N-bis(2-hydroxyethyl)glycin (Bicine), N-cyclohexyl-2-aminoethanesulfonic acid (CHES), and N-cyclohexyl-3-aminopropanesulfonic acid (CAPS)). The reason is that the buffer solution included in the liposome may be any buffer solution as long as it is not harmful to a living body.

(Liposomes including plant ceramide portions)

In other aspects, the present invention provides glycolipid-containing liposomes including plant ceramide portions and sugar chain portions as glycolipid. Here, glycolipids, liposomes, target-recognizing probes, desired substances, and the like used in the glycolipid-containing liposomes of the present invention may be used in any forms described in the above sections of (Glycolipid-containing liposome) and the like.

As one embodiment, a plant ceramide portion included in the glycolipid-containing liposome of the present invention may be, but is not limited to, for example:

[Chem. 1-1]

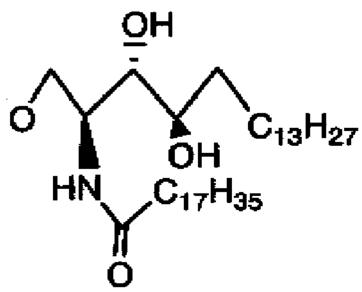

In another embodiment, a sugar chain portion included in the glycolipid-containing liposome of the present invention may be, but is not limited to, for example:

[Chem. 2-1]

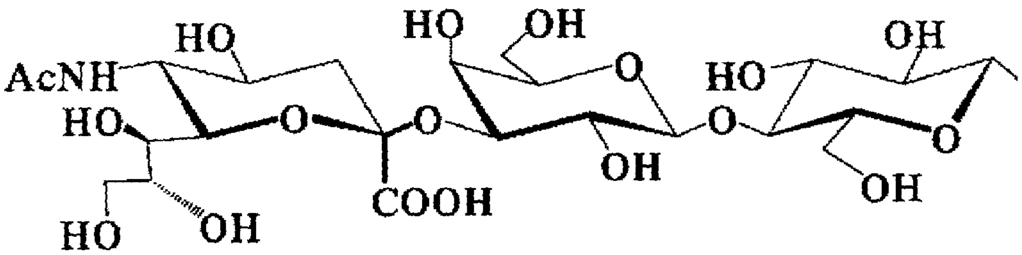

In a preferred embodiment, a glycolipid included in the glycolipid-containing liposome of the present invention may be, but is not limited to, for example:

[Chem. 3-1]

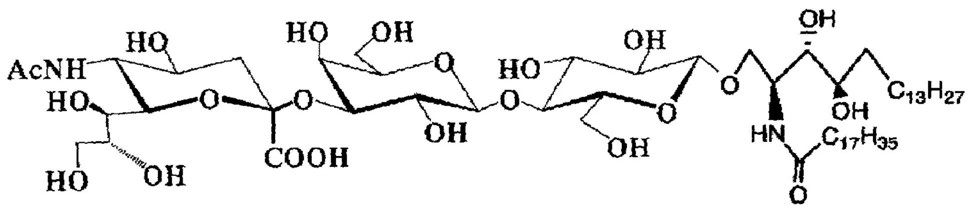

In amore preferred embodiment, a glycolipid included in the glycolipid-containing liposome of the present invention has, for example:

[Chem. 4-1]

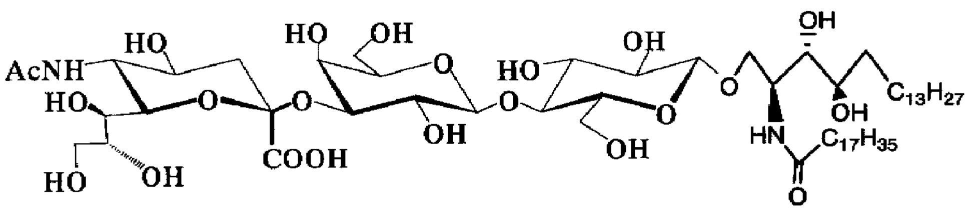

The glycolipid-containing liposome may include, as lipids composing the liposome, dipalmitoylphosphatidylcholine (DPPC), cholesterol, dicetylphosphate (DCP), the glycolipid, dipalmitoylphosphatidylethanolamine (DPPE) at a molar ratio of 35:40:5:15:5.

In other embodiments, with regard to the glycolipid-containing liposome of the present invention, absorbance thereof at 680 may be, but is not limited to, 0.5 to 3.0.

In other embodiments, with regard to the glycolipid-containing liposome of the present invention, lipid amount may be about 0.5 to about 5 mg/mL.

In other embodiments, the glycolipid-containing liposome of the present invention contains human serum albumin (HSA), and the amount thereof may be, for example, about 0.1 to about 1 mg/mL.

In other embodiments, with regard to the glycolipid-containing liposome of the present invention, the mean particle size can be about 50 to about 300 nm.

In other embodiments, with regard to the glycolipid-containing liposome of the present invention, Z potential may be about -30 mV to about -120 mV.

In other embodiments, the glycolipid-containing liposome of the present invention may encapsulate desired substances. Examples of these desired substances include, but are not limited to, cy5.5, cy5, cy7, cy3B, cy3.5, Alexa

Fluor 350, Alexa Fluor 488, Alexa Fluor 532, Alexa Fluor 546, Alexa Fluor 555, Alexa Fluor 568, Alexa Fluor 594, Alexa Fluor 633, Alexa Fluor 647, Alexa Fluor 680, Alexa Fluor 700, Alexa Fluor 750, fluorescein-4-isothiocyanate (FITC), europium-containing label, GFP, CFP, YFP, luciferase, antibody, tPA, β-galactosidase, albumin, botulinus toxin, diphtheria toxin, methylprednisolon, prednisolon phosphate, peptide, gold colloid, Gd complex, Fe complex, cisplatin, pravastatin, heparin, Fasudilhydrochloride, clodronic acid, water-soluble iodine, chitin, chitosan, plasmid DNA, RNAi, and the like.

In one embodiment, the glycolipid-containing liposome of the present invention includes a target-recognizing probe on the surface of the liposome. This target-recognizing probe may be, but is not limited to, for example, sugar chain, antibody, antigen, peptide, nucleic acid, hyaluronic acid, and the like.

In other embodiments, an amount of sugar chain added and included as target-recognizing probe in the glycolipid-containing liposome of the present invention may be, but is not limited to, a bond density of sugar chain of about 0.5 to about 500 μg/mL (for example, about 1 to 500 μg/mL, 5 to 500 μg/mL, 10 to 500 μg/mL, 50 to 500 μg/mL, 100 to 500 μg/mL, 150 to 500 μg/mL, 200 to 500 μg/mL, 300 to 500 μg/mL, 400 to 500 μg/mL, 0.5 to 400 μg/mL, 1 to 400 μg/mL, 5 to 400 μg/mL, 10 to 400 μg/mL, 50 to 400 μg/mL, 100 to 400 μg/mL, 150 to 400 μg/mL, 200 to 400 μg/mL, 300 to 400 μg/mL, 0.5 to 300 μg/mL, 1 to 300 μg/mL, 5 to 300 μg/mL, 10 to 300 μg/mL, 50 to 300 μg/mL, 100 to 300 μg/mL, 150 to 300 μg/mL, 200 to 300 μg/mL, 0.5 to 200 μg/mL, 1 to 200 μg/mL, 5 to 200 μg/mL, 10 to 200 μg/mL, 50 to 200 μg/mL, 100 to 200 μg/mL, 150 to 200 μg/mL, 0.5 to 100 μg/mL, 1 to 100 μg/mL, 5 to 100 μg/mL, 10 to 100 μg/mL, 50 to 100 μg/mL, 0.5 to 50 μg/mL, 1 to 50 μg/mL, 5 to 50 μg/mL, 10 to 50 μg/mL, 0.5 to 50 μg/mL, 1 to 10 μg/mL, 5 to 10 μg/mL, 1 to 5 μg/mL, 0.5 to 1 μg/mL). The reason is that the bond density of sugar chain may be out of the above-described range as long as the sugar chain works as the target-recognizing probe.

In other embodiments, the amount of antibody added and included as the target-recognizing probe in the glycolipid-containing liposome of the present invention may be, but is not limited to, about 0.1 to about 100 μg/mL, about 0.1 to about 50 μg/mL, or about 0.3 to about 100 μg/mL (for example, about 0.4 to 100 μg/mL, 0.5 to 100 μg/mL, 1 to 100 μg/mL, 5 to 100 μg/mL, 10 to 100 μg/mL, 50 to 100 μg/mL, 90 to 100 μg/mL, 0.3 to 90 μg/mL, 0.4 to 90 μg/mL, 0.5 to 90 μg/mL, 1 to 90 μg/mL, 5 to 90 μg/mL, 10 to 90 μg/mL, 50 to 90 μg/mL, 0.3 to 50 μg/mL, 0.4 to 50 μg/mL, 0.5 to 50 μg/mL, 1 to 50 μg/mL, 5 to 50 μg/mL, 10 to 50 μg/mL, 0.3 to 10 μg/mL, 0.4 to 10 μg/mL, 0.5 to 10 μg/mL, 1 to 10 μg/mL, 5 to 10 μg/mL, 0.3 to 5 μg/mL, 0.4 to 5 μg/mL, 0.5 to 5 μg/mL, 1 to 5 μg/mL, 0.3 to 1 μg/mL, 0.4 to 1 μg/mL, 0.5 to 1 μg/mL, 0.3 to 0.5 μg/mL, 0.4 to 0.5 μg/mL, 0.3 to 0.4 μg/mL). The reason is that the amount of antibody added may be out of the above-described range as long as the antibody still works as the target-recognizing probe.

In other embodiments, a glycolipid included in the glycolipid-containing liposomes of the present invention may not include a component accompanying a naturally-derived glycolipid.

(Method for producing liposomes containing plant ceramide portions)

In other aspects, the present invention provides a method for producing a glycolipid-containing liposome including a plant ceramide portion and a sugar chain portion. This method may include: A) providing a glycolipid, wherein the glycolipid including a plant ceramide portion and a sugar chain portion; and B) mixing the provided synthetic glycolipid with a liposome raw material and subjecting the mixture to conditions in which a liposome is formed. Here, glycolipids, liposomes, target-recognizing probes, desired substances, and the like used in the glycolipid-containing liposomes of the present invention may be used in any of the forms described in the above sections of (Glycolipid-containing liposomes), (Liposomes containing plant ceramide portions) and the like.

In one embodiment, step A) in the producing method of the present invention may include: (a) reacting a protected sugar with a protected lipid amide under conditions in which the protected sugar binds with the protected lipid amide, so as to produce a sugar-lipid amide acceptor precursor; (b) allowing the sugar-lipid amide acceptor precursor to react under conditions in which an intramolecular condensation reaction in the sugar-lipid amide acceptor precursor proceeds, so as to produce a sugar-lipid amide acceptor; (c) reacting the sugar-lipid amide acceptor with a protected sugar chain donor under conditions in which the sugar-lipid amide acceptor binds with the protected sugar chain donor, so as to produce a protected glycolipid; and (d) performing deprotection reaction of the protected glycolipid under conditions in which the protected sugar chain donor is deprotected, so as to produce a glycolipid, wherein the protected lipid amide may be:

[Chem. 5-1]

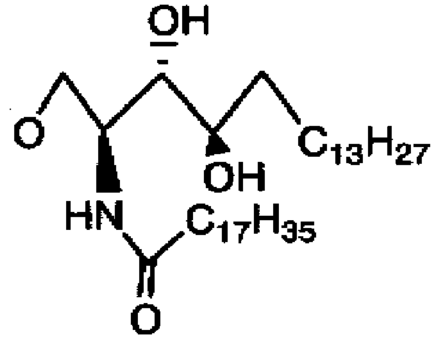

(Use)

In one aspect, the present invention provides glycolipid-containing liposomes for the purpose of using them as a medicinal material. When the present invention is utilized, liposomes that do not include a living-body-derived starting material including an animal-derived starting material, can be provided as a medicinal material. When a living-body-derived starting material is used, high cost and risk (unknown behavior in a living body, contamination, and the like) are involved. Thus, considering that the present invention can avoid those above, the present invention can be regarded as being significantly important.
When the present invention is used, in drugs and the like, improvements of homogeneity and stability can be contemplated. This is based on the removal of impurities.
When the present invention is used, it is possible to supply drugs and the like safely and stably. The reason is that, for example, since a necessary scale up is made possible, contamination or shortage of them is reduced.
By homogenously binding a target-site-recognition probe to the glycolipid-containing liposome of the present invention, it is expected to be used as a DDS not having accumulation variation.
Furthermore, the present invention exhibits an improved physical property in comparison with naturally-derived conventional glycolipid-containing liposomes rather than at least in the same level as them, and thus can be used as a substitute for naturally-derived glycolipid-containing liposomes.
In other aspects, the present invention provides a glycolipid-containing liposome for producing a medicament.
These liposomes can be obtained by preparation using a synthesized glycolipid (for example, synthetic GM3, synthetic GM4) as a glycolipid by any technique publicly known in the relevant field. They may be prepared by, for example, an ultrasonication method, an ethanol injection method, a French press method, an ether injection method, a cholate method, a freeze drying method, or a reverse phase evaporization method (see, e.g., "New development in liposome Application-For development of an artificial cell-, Kazunari Akiyoshi and Kaoru TSUJII Ed., NTS p33 to 45 (2005)" and "Liposomes, Shoshichi NOJIMA, p. 21-40 Nankodo (1988)"). The glycolipid-containing liposome of the present invention may be administered intravenously, intra-arterially, intrabdominally, directly, or orally, but the administration routes thereof are not limited thereto.
Here, the liposome of the present invention to be used as a medicinal material may be used in any forms described in the above sections of (Glycolipid-containing liposome), (Composition), and the like.

(Method for producing a glycolipid-containing liposome)

The present invention provides a method for producing glycolipid-containing liposomes, and the producing method may include:

A) providing a glycolipid, wherein the glycolipid including a plant ceramide portion and a sugar chain portion; and
B) mixing the provided synthetic glycolipid with a liposome raw material and subjecting the mixture to conditions in which a liposome is formed.

In one embodiment, the step A) may include:

(a) reacting a protected sugar with a protected lipid amide under conditions in which the protected sugar binds with the protected lipid amide, so as to produce a sugar-lipid amide acceptor precursor;
(b) allowing the sugar-lipid amide acceptor precursor to react under conditions in which an intramolecular condensation reaction in the sugar-lipid amide acceptor precursor proceeds, so as to produce a sugar-lipid amide acceptor;
(c) reacting the sugar-lipid amide acceptor with a protected sugar chain donor under conditions in which the sugar-lipid amide acceptor binds with the protected sugar chain donor, so as to produce a protected glycolipid; and
(d) performing deprotection reaction of the protected glycolipid under conditions in which the protected sugar chain donor is deprotected, so as to produce a glycolipid.

Here, the glycolipid used in the method for producing the glycolipid-containing liposome of the present invention may be used in any of the forms described in the following sections of (Method for producing a glycolipid) and the like.

(Method of producing a glycolipid)

A glycolipid used as the material for producing the glycolipid-containing liposome of the present invention can be created by, for example, the following producing method. This method includes: (a) reacting a protected sugar with a protected lipid amide under conditions in which the protected sugar binds with the protected lipid amide, so as to produce a sugar-lipid amide acceptor precursor; (b) allowing the sugar-lipid amide acceptor precursor to react under conditions in which an intramolecular condensation reactioninthesugar-lipid amide acceptor precursor proceeds, so as to produce a sugar-lipid amide acceptor; (c) reacting the sugar-lipid amide acceptor with a protected sugar chain donor under conditions in which the sugar-lipid amide acceptor binds with the protected sugar chain donor, so as to produce a protected glycolipid; and (d) performing deprotection reaction of the protected glycolipid under conditions in which the protected sugar chain donor is deprotected, so as to produce a glycolipid.

Step (a) of reacting a protected sugar with a protected lipid amide under conditions in which the protected sugar binds with the protected lipid amide, so as to produce a sugar-lipid amide acceptor precursor, can be performed as follows.

On an appropriately protected sugar and a protected lipid amide in dichloromethane solvent, the appropriate reagents such as WSC and DMAP act at room temperature to bind the carboxyl group of the protected lipid amide to the hydroxyl group of the sugar. Then, the protecting group at 1-position of the protected lipid amide is removed by deprotection. It is understood that those skilled in the art can optionally design these conditions on the basis of the present specification in view of well-known techniques.

Step (b) of allowing the sugar-lipid amide acceptor precursor to react under conditions in which an intramolecular condensation reaction in the sugar-lipid amide acceptor precursor proceeds, so as to produce a sugar-lipid amide acceptor, can be performed as follows.

An intramolecular glycosylation reaction of the sugar-lipid amide acceptor precursor is carried out (for example, in dichloromethane solvent at 0 degree Celsius, NIS (2 equivalents) and TfOH (0.3 equivalents) are used as activating agents). It is understood that those skilled in the art can optionally design these conditions on the basis of the present specification in view of well-known techniques.

Step (c) of reacting the sugar-lipid amide acceptor with a protected sugar chain donor under the conditions in which the sugar-lipid amide acceptor binds with the protected sugar chain donor, so as to produce a protected glycolipid, can be performed as follows.

On the sugar-lipid amide acceptor obtained and a protected sugar chain donor, in the appropriate solvent (for example, dichloromethane solvent) and at an appropriate temperature (for example, 0 degree Celsius), an activating agent (for example, 0.04 equivalents of TMSOTf (trimethylsilyl trifluoromethanesulfonate) is used) act, and thereby a condensation reaction is carried out. It is understood that those skilled in the art can optionally design these conditions on the basis of the present specification in view of well-known techniques.

Step (d) of performing deprotection reaction of the protected glycolipid under conditions in which the protected sugar chain donor is deprotected, so as to produce a glycolipid, can be performed as follows.

On the protected glycolipid obtained, in the appropriate solvent (for example, dichloromethane solvent) at room temperature, a deprotection agent (for example, TFA (trifluoromethanesulfonic acid)) acts, and thereby a protecting group (for example, MPM group) is removed by deprotection. Then, in the appropriate solvent (for example, methanol solvent) at room temperature, another deprotection agent (for example, NaOMe (sodium methoxide)) acts, and thereby another protecting group (for example, in this case, acyl protection group) is removed by deprotection. At last, in the case where there is a methyl ester of a sialic acid, by adding an appropriate hydrolysis agent (for example, water ($H_2O$)) to perform an alkaline hydrolysis, the methyl ester of the sialic acid is deprotected and to be lead to a glycolipid. It is understood that those skilled in the art can optionally design these conditions on the basis of the present specification in view of well-known techniques.

In one embodiment, examples of protected sugars that can be used by the present method can include, but

are not limited to:

[Chem. 62]

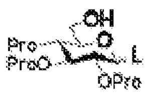 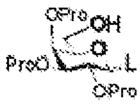 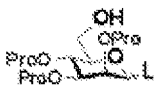 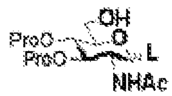 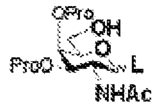

Glucose Galactose Mannose N-Acetyl glucosamine N-Acetyl galactosamine

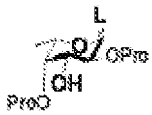

Fucose

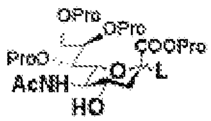

Sialic acid

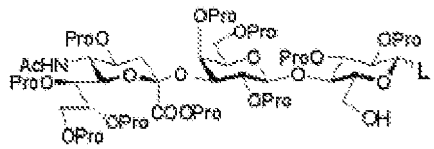

and

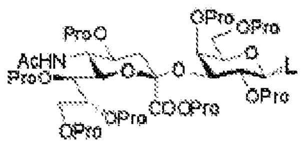

L: Leaving group

Pro: Protecting group

Examples:

Pro: Bz, Ac, Piv, MPM, MP **Benzyl, Methyl**

L: SPh, SMe, SEt, F, $OPO(OPh)_2$, $OPO(NMe_2)_2$, **Trichloroacetimidate**

In one embodiment, in protected sugars used in the present invention, an acetyl, a benzoyl, or a benzyl group, or the like may be used as the protecting group of a hydroxyl group and a methyl or a benzyl group, or the like may be used as the protecting group of a carboxyl group, but the protecting groups are not limited to those above. A phenylthio, fluoro, or trichloroacetimidate group, or the like may be used as the leaving group, but the leaving groups are not limited thereto.

In other embodiments, a protected lipid amide that may be used in the present method may be, for example:

[Chem. 63]

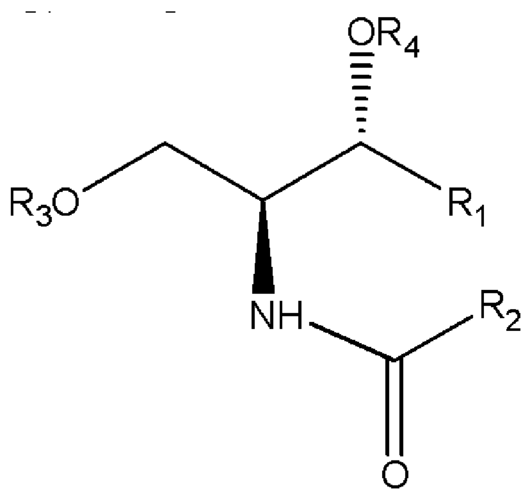

wherein $R_1$ and $R_2$ are independently selected from an alkyl or an alkenyl group; $R_3$ may be TBDPS, TBDMS, TIPS, Tr, isopropylidene ketal, or methoxybenzylidene acetal; and $R_4$ is a protecting group that maybe succinyl, malonyl, phthaloyl, oxalyl, carbonyl, benzoyl, acetyl, or pivaloyl. It is understood that these protecting groups may be any other protecting groups as long as the protective function is achieved. The reason is that, in the method of the invention, in the case where only the primary hydroxyl group at 1-position can be glycosylated and the other hydroxyl groups can be protected, a glycolipid can be created, even when using a protected lipid amide other than the above-described protected lipid amides.

In a preferred embodiment, a protected lipid amide that may be used in the present invention may be:

[Chem. 64]

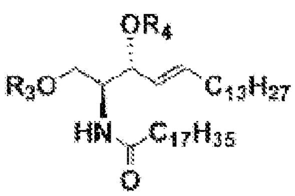

or

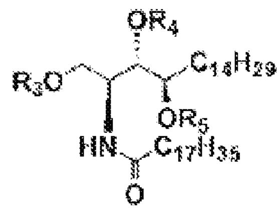

Sphingosine-type ceramide Phytosphingosine-type ceramide

A sugar-lipid amide acceptor precursor that may be used in the present invention may be:

[Chem. 65]

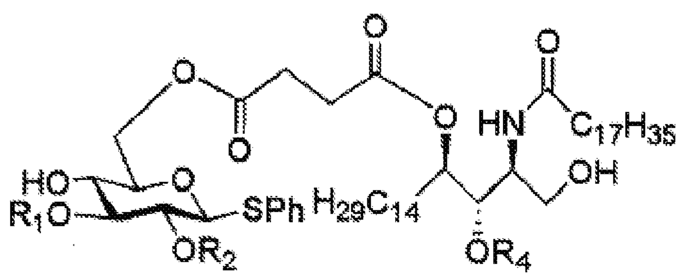

or

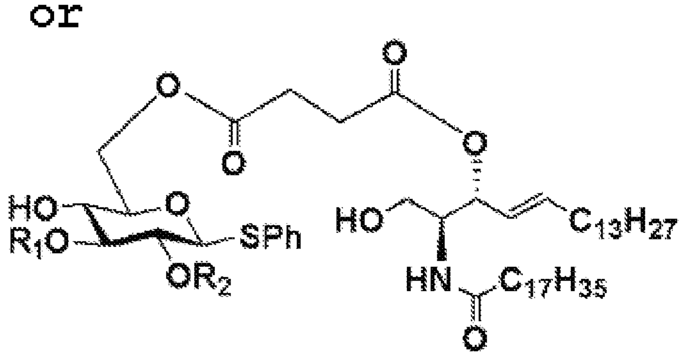

and a sugar-lipid amide acceptor that may be used in the present invention may be:

[Chem. 66]

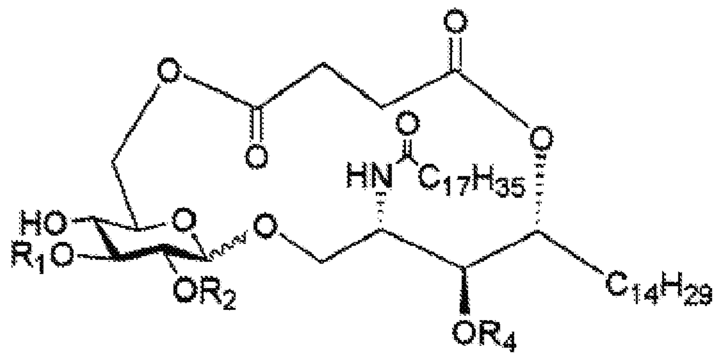

or

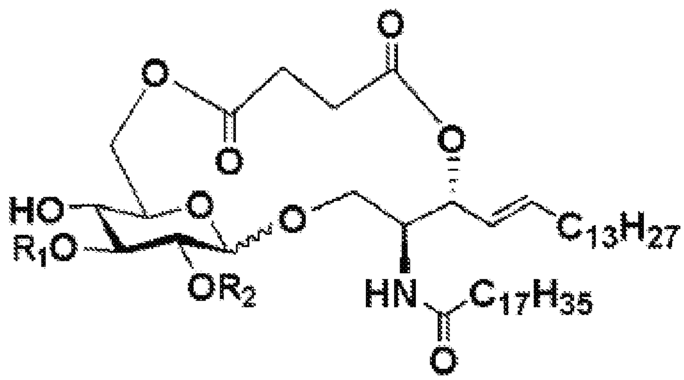

wherein $R_1$ is MPM; $R_2$ is MPM or Bz; $R_3$ is selected from the group consisting of TBDPS, TBDMS, TIPS, Tr, isopropylidene ketal, and methoxybenzylidene acetal; $R_4$ and $R_5$ are independently protecting groups that may be succinyl, malonyl, phthaloyl, oxalyl, carbonyl, benzoyl, acetyl, or pivaloyl. It is understood that these protecting groups may be any other protecting groups as long as the protective function is achieved. The reason is that, in the method of the invention, a thioglycoside is used as the leaving group, wherein a glycolipid can be created even when using a sugar-lipid amido acceptor having a protecting group other than the above-described protecting groups, as long as the protecting group is resistant to an activating condition.

In another embodiment, in the present method, the conditions in which the protected sugar binds with a protected lipid amide maybe, for example, conditions in which an alcohol binds with a carboxylic acid. The reason is that the protected sugar has only one free hydroxyl group which is not protected, and the protected lipid amide has a spacer with a carboxyl group. While not wishing to be bound by theory, a rational explanation is presented which is that conditions in which the protected sugar binds with a protected lipid amide may be any condition as long as an alcohol binds with a carboxylic acid.

In another embodiment, the step (a) includes mixing and reacting the protected sugar chain with the lipid amide in a solvent in the presence of a reagent at a predetermined reaction temperature for a predetermined reaction time. Here, the solvent may be tetrahydrofuran (THF), $CH_2Cl_2$, benzene, toluene, or N,N-dimethylformamide (DMF), or combinations thereof; the reagent may be triphenylphosphine ($PPh_3$), DEAD, 1-methyl-3-(3-dimethylaminopropyl)-carbodiimide hydrochloride (WSC), 2,4,6-trichlorobenzoyl chloride, triethylamine ($Et_3N$), 4-dimethylaminopyridine (DMAP), or any combination thereof; and the reaction time may be 2 to 4 hours.

In another embodiment, in the step (a), the reaction temperature may be room temperature or higher, preferably, room temperature - 90 degrees Celsius. However, these vary according to the solvent to be used or the like.

One exemplified embodiment can include a condition in which the reagents are $PPh_3$ and DEAD, the solvent is THF, and the reaction temperature is 90 degrees Celsius or higher.

In a preferred embodiment, in step (a), the solvent is tetrahydrofuran (THF), the reagents are triphenylphosphine ($PPh_3$: 3.0 equivalents) and DEAD (3.0 equivalents), and the temperature maybe at reflux (90 degrees Celsius).

In another preferred embodiment, in step (a), the solvent is $CH_2Cl_2$, the reagents are 1-methyl-3-(3-dimethylaminopropyl)-carbodiimide hydrochloride (WSC: 3.0 equivalents) and 2,4,6-trichlorobenzoyl chloride (1.1 equivalents), and the temperature may be room temperature. Here, the equivalent is an equivalent with respect to the main substance (for example, protected sugar chain) in the reaction.

In another preferred embodiment, in step (a), the solvent is $CH_2Cl_2$, the reagents are triethylamine ($Et_3N$: 1.5 equivalents) and 4-dimethylaminopyridine (DMAP: 3.0 equivalents), and the temperature is room temperature.

In another embodiment, the step (a) may include further adding a spacer precursor to the sugar and the protected lipid amide, so that the protected lipid amide binds with the sugar via a spacer. Examples of the conditions under which the protected lipid amide binds to the sugar via the spacer can include, but are not limited to, (1) the condition being in the presence of WSC, DMAP, and $CH_2Cl_2$ at room temperature; (2) the condition being in the presence of $PPh_3$, DEAD, and THF at reflux; (3)the condition being in the presence of 2, 4, 6-trichlorobenzoyl chloride, $Et_3N$, DMAP, and $CH_2Cl_2$ at room temperature; and the like. This spacer may be previously bound with the sugar or the protected lipid amide.

In yet another embodiment, step (a) may include allowing the protected lipid amide under conditions in which a hydroxyl group at the 1-position of the protected lipid amide is deprotected, so as to deprotect the hydroxyl group at the 1-position. Conditions under which the hydroxyl group at the 1-position of the protected lipid amide is deprotected can include, in the case of protecting the hydroxyl group at the 1-position of a ceramide with a silyl protecting group, and the condition being in THF solvent in the presence of TBAF and AcOH at 0 degree Celsius.

In another embodiment, the spacer precursor is succinic acid. When said succinic acid binds to $R_3$ of the protected lipid amide, $R_3$ may be isopropylidene ketal or methoxybenzylidene acetal, $R_4$ may be:

[Chem. 67]

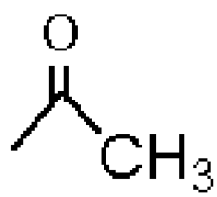

(A c)

or

[Chem. 68]

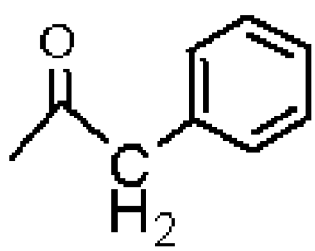

(B x)

and $R_5$ may be Ac or Bx, but the Rs are not limited to those above.

In another embodiment, the spacer precursor is succinic acid. When said succinic acid binds to $R_4$ of the protected lipid amide, $R_3$ may be Tr, TBDPS, or TBDMS, $R_4$ may besuccinyl,malonyl,oxalyl,carbonyl,glutaryl,phthaloyl, or the like, and $R_5$ may be acetyl or benzoyl, but the Rs are not limited to those above.

In another embodiment, the spacer precursor is succinic acid. When said succinic acid binds to $R_5$ of the protected lipid amide, $R_3$ may be Tr, TBDPS, TBDMS, or the like, $R_4$ may be succinyl, malonyl, oxalyl, carbonyl, glutaryl, phthaloyl or the like, and $R_5$ may be acetyl or benzoyl group, or the like,but the Rs are not limited to those above. The reason is that, in an intramolecular reaction, the relative position between the functional groups to be reacted is important. However, in the method of the invention, even when performing a condensation reaction of a molecule having a relatively large degree of freedom (for example, succinic acid and the like), a sufficiently good result was obtained, and therefore it is thought that bridging using a molecule other than the above-described molecules is sufficiently effective.

In another embodiment, a sugar residue at a reducing terminal side of the oligosaccharide may bind with the protected lipid amide via a spacer.

In one embodiment, the step (b) is performed in the presence of an activating agent for activating the intramolecular condensation reaction. The activating agent is any activating agent as long as it activates a SPh group of a sialic acid. The activating reagents can include, but are not limited to, N-iodosuccinimide (NIS), trifluoromethanesulfonic acid (TfOH), TMSOTf, dimethyl(methylthio)sulfonium triflate (DMTST), N-bromosuccinimide (NBS), and combinations thereof. The reason is that, in the case where a SPh group of a sialic acid can be activated, the activation of the intramolecular condensation reaction can be attained, and simultaneously a sugar-lipid amide acceptor can be created.

In other embodiments, the step (b) can be performed at a reaction temperature of -80 degrees Celsius to room temperature, in a solvent such as $CH_2Cl_2$, diethylether ($(CH_2CH_3)_2O$), diethylether, acetonitrile, diethylether, acetonitrile, propionitrile, toluene, nitromethane, and combinations thereof, in the presence of N-iodosuccinimide (NIS), trifluoromethanesulfonic acid (TfOH), dimethyl(methylthio)sulfonium triflate (DMTST), molecular sieves 4 angstroms (MS4Å), and molecular sieves 3 angstroms (MS3Å) as reagents (wherein it is understood that, as reagents to be used, not only a catalyst, but also a desiccant and the like can be appropriately present), for a reaction time of 1 to 48 hours.

In a preferred embodiment, the reaction temperature may be, but is not limited to, -20 to 0 degree Celsius. While not wishing to be bound by theory, the reasons are that the reaction proceeds advantageously, and a glycosylation is generally performed at such a degree of low temperature as not to inhibit the progress of the reaction wherein the temperature is set as low as possible.

In a preferred embodiment, the solvent may be, but limited to, dichloromethane. While not wishing to be bound by theory, the reason is that, in the case of dichloromethane, the highest reactivity was exhibited. In addition, it is understood that diethylether, acetonitrile, propionitrile, toluene, or nitromethane may be used.

In a preferred embodiment, the reagents may be, but are not limited to, N-iodosuccinimide (NIS) and trifluoromethanesulfonic acid (TfOH).

In a preferred embodiment, the reaction time may be, but limited to, 1 to 5 hours. However, it is understood to vary depending on the reaction solvent or temperature.

In another embodiment, in the step (b), the reaction temperature is 0 degree Celsius; the solvent is $CH_2Cl_2$; the reagents are N-iodosuccinimide (NIS), trifluoromethanesulfonic acid (TfOH), TMSOTf, and molecular sieves 4 angstroms (MS4Å); and the reaction time may be 1.5 hours.

In another embodiment, in the step (b), the reaction temperature is -40 degrees Celsius; the solvent is $CH_2Cl_2$; the reagents are N-iodosuccinimide (NIS), trifluoromethanesulfonic acid (TfOH), and molecular sieves 4 angstroms (MS4Å); and the reaction time may be 5 hours.

In another embodiment, in the step (b), the reaction temperature is -80 degrees Celsius → -60 degrees Celsius → -40 degrees Celsius → 0 degree Celsius; the solvent is $CH_2Cl_2$; the reagents are N-iodosuccinimide (NIS), trifluoromethanesulfonic acid (TfOH), and molecular sieves 4 angstroms (MS4Å); and the reaction time may be 36 hours.

In another embodiment, in the step (b), the reaction temperature is -40 degrees Celsius → 0 degree Celsius; the solvent is acetonitrile (MeCN); the reagents are N-iodosuccinimide (NIS), trifluoromethanesulfonic acid (TfOH), and molecular sieves 3 angstroms (MS3Å); and the reaction time may be 48 hours.

In another embodiment, in the step (b), the reaction temperature is -0 degree Celsius; the solvent is acetonitrile (MeCN); the reagents are N-iodosuccinimide (NIS), trifluoromethanesulfonic acid (TfOH), and molecular sieves 3 angstroms (MS3Å); and the reaction time may be 1.5 hours.

In another embodiment, in the step (b), the reaction temperature is -20 degrees Celsius; the solvent is acetonitrile (MeCN); the reagents are N-iodosuccinimide (NIS), trifluoromethanesulfonic acid (TfOH), and molecular sieves 3 angstroms (MS3Å); and the reaction time may be 3 hours.

In another embodiment, in the step (b), the reaction temperature is 0 degree Celsius → room temperature; the solvent is diethylether ($Et_2O$); the reagents are N-iodosuccinimide (NIS), trifluoromethanesulfonic acid (TfOH), and molecular sieves 3 angstroms (MS3Å); and the reaction time may be 25 hours.

In another embodiment, in the step (b), the reaction temperature is 0 degree Celsius; the solvent is acetonitrile

(MeCN); the reagents are dimethyl(methylthio)sulfonium triflate (DMTST), and molecular sieves 3 angstroms (MS3Å); and the reaction time may be 1 hour.

In another embodiment, in the step (b), the reaction temperature is 0 degree Celsius; the solvent is $CH_2Cl_2$; the reagents are N-iodosuccinimide (NIS), trifluoromethanesulfonic acid (TfOH), and molecular sieves 4 angstroms (MS4Å); and the reaction time may be 5 hours.

In another embodiment, in the step (b), the reaction temperature is -20 degrees Celsius; the solvent is $CH_2Cl_2$; the reagents are N-iodosuccinimide (NIS), trifluoromethanesulfonic acid (TfOH), and molecular sieves 4 angstroms (MS4Å); and the reaction time may be 1.5 hours.

In another embodiment, in the step (b), the reaction temperature is 0 degree Celsius; the solvent is $CH_2Cl_2$; the reagents are dimethyl(methylthio)sulfonium triflate (DMTST), and molecular sieves 4 angstroms (MS4Å); and the reaction time may be 2 hours.

The "condition under which an intramolecular condensation reaction in the sugar-lipid amide acceptor precursor proceeds" in step (b) may be any condition as long as an intramolecular condensation reaction in a sugar-lipid amide acceptor precursor proceeds, even if it is not the above-described conditions. The reason is that the purpose of the present method is to efficiently introduce lipid portions, and thus the condition is no object. Furthermore, it is because, when the intramolecular glycosylation is achieved, it means that simultaneously a sugar-lipid amide acceptor is synthesized.

In another embodiment, the intramolecular condensation reaction may be, but is not limited to, glycosylation. In the present invention, it has been found possible to appropriately introduce a lipid amide to a sugar by using glycosylation.

In one embodiment, step (c) of the present method may include performing the reaction using a donor of more than 2.5 equivalents relative to an acceptor, at a reaction temperature of -40 to 0 degree Celsius, in $CH_2Cl_2$ solvent, in the presence of trimethylsilyl trifluoromethanesulfonate (TMSOTf) as a reagent, for a reaction time of 1 to 48 hours.

In a preferred embodiment, instep (c), the equivalent of the donor relative to the acceptor is 2.5 equivalents; the reaction temperature is 0 degree Celsius; the solvent is $CH_2Cl_2$; and the reagent is TMSOTf; and the reaction time may be 7 hours.

In other embodiments, the protected sugar chain donor may be, but is not limited to, for example:

[Chem. 69]

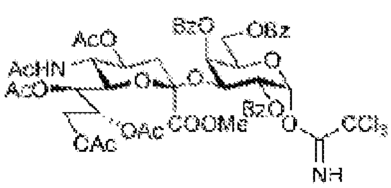

,

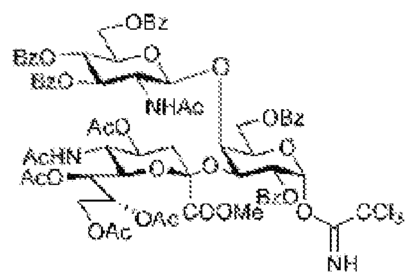

or

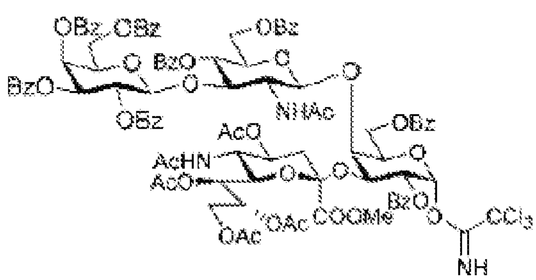

The protected sugar chain donor may be any sugar as long as it has a leaving group at the reducing terminal of the sugar chain and other protecting groups are also protected. The reason is that, in glycosylation by an activating agent, only the anomer position of a sugar residue of the reducing terminal of the sugar chain leaves, and then thereto a sugar-lipid amide acceptor nucleophilically can attack. Thus, examples of such leaving groups can include, but are not limited to, -SPh, $-SCH_3$, $-SCH_2CH_3$, -F, $-OPO(OPh)_2$, and $-OPO(N(CH_3)_2)_2$ (wherein Ph is phenyl).

In one embodiment, step (d) of the present method may be performed in $CH_2Cl_2$ solvent, in the presence of

trifluoroacetic acid as a reagent, at a reaction temperature of room temperature, for a reaction time of 2 to 12 hours, but the condition of step (d) is not limited to those above. Step (d) maybe under any condition as long as it is a condition under which the protected sugar chain donor is deprotected.

In a preferred embodiment, in step (d), the solvent is $CH_2Cl_2$; the reagent is trifluoroacetic acid; the reaction temperature is room temperature; and the reaction time may be 2 hours.

In one embodiment, step (e) of the present method may further include reacting the product of the step (d) under the condition wherein an acyl protecting group is removed by deprotection, and thereby deprotecting said product.

In another embodiment, the step (e) can be performed in methanol (MeOH) or water ($H_2O$) solvent, in the presence of sodiummethoxide ($NaOCH_3$) or KOH as a reagent, at a reaction temperature of room temperature to 100 degrees Celsius, for a reaction time of 1 hour to 1 week, but the condition is not limited to this condition. The reason is that step (e) may be any condition as long as it is a condition under which an acyl protecting group can be removed by deprotection.

In a preferred embodiment, in the condition under which step (e) is performed, the solvent is methanol (MeOH); the reagent is sodium methoxide (NaOMe); the reaction temperature is room temperature; and the reaction time may be 12 hours.

(Method for synthesizing a sugar chain)

In one aspect, the present invention provides a method for producing:

[Chem. 70]

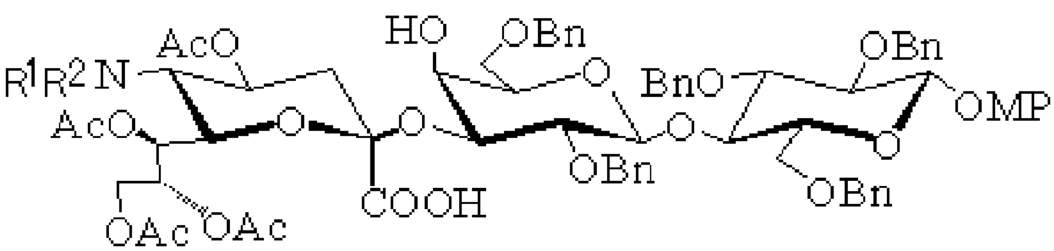

This method includes (A) reacting an acceptor compound:

[Chem. 71]

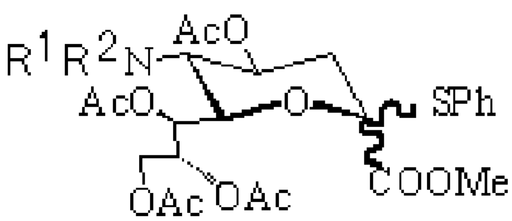

and a donor compound:

[Chem. 72]

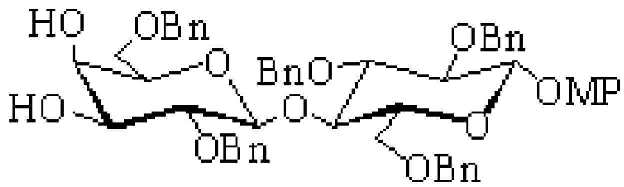

under conditions in which the acceptor compound binds with the donor compound, wherein $R^1$ is Ac or H; $R^2$ is Ac or Troc; SPh is:

[Chem. 73]

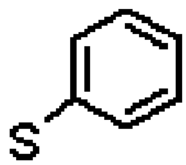

**[0570]** ; MP is:

[Chem. 74]

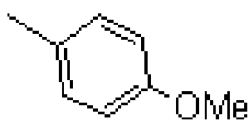

**[0572]** ; SE is:

[Chem. 75]

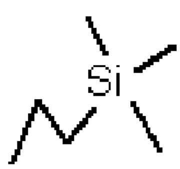

**[0574]** ; Ac is:

[Chem. 76]

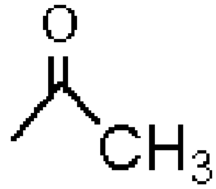

[0576] ; Bz is:
[Chem. 77]

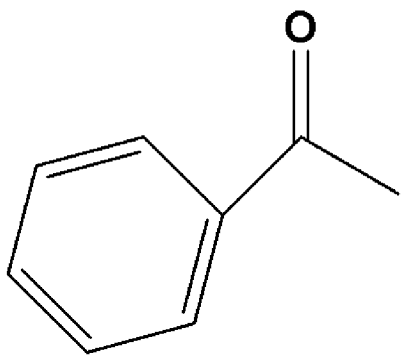

[0578] ; Bn is:
[Chem. 78]

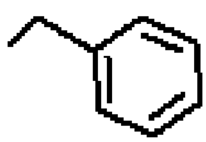

[0580] ; and Me is methyl.
In other embodiments, the step (A) may be performed at a reaction temperature of -40 degrees Celsius to room temperature, in solvent of mixture solution of ; in $CH_3CN$, $CH_2Cl_2$, diethylether, acetonitrile, propionitrile, toluene, nitromethane, and a mixture solution of any combination thereof as the solvent; in the presence of a catalyst of N-iodosuccinimide (NIS), trifluoromethanesulfonic acid (TfOH), TMSOTf, or a catalyst of a combination thereof, for a reaction time of 1 hour to 3 days, but the condition is not limited to those above.
In a preferred embodiment, the catalyst is, but is not limited to, N-iodosuccinimide (NIS), or trifluoromethanesulfonic acid (TfOH), or a combination thereof. The catalyst that can be used in the present method may be any catalyst as long as the catalyst is well dissolved together with a sialyl donor and a lactosyl acceptor and the catalyst attains good steric control (alpha-sialylation) during condensation. The reason is that, as long as at least an activated sialyl donor and a lactosyl acceptor exist, the condensation reaction proceeds.
In a preferred embodiment, examples of the solvent can include, but are not limited to, $CH_3CN$, $CH_2Cl_2$, andamixture solution of $CH_3CN$ and $CH_2Cl_2$. The solvent that may be used in the present method may be any solvent

as long as the solvent activates a SPh group of a sialyl acid. The reason is that, when a sialyl donor is activated, due to the nucleophilicity of a free hydroxyl group of lactose, the reaction rapidly proceeds.

In a preferred embodiment, the reaction temperature maybe, and is not limited to, for example, -30 degrees Celsius to 0 degree Celsius. In other preferred embodiments, the reaction temperature may be -50 degrees Celsius.

In a preferred embodiment, the reaction temperature may be, and is not limited to, for example, 2 to 3 hours, about 4 to 8 hours, preferably about 6 hours. Glycosylation usually takes 1 hour to within 1 day. However, according to the progress of the reaction, it is possible to optionally set a reaction temperature, and the reaction temperature is not limited to those above.

In another embodiment, the step (A) may include: reacting at temperatures of -30 degrees Celsius → room temperature, using $CH_3CN$ → a mixture solution of $CH_3CN$ and $CH_2Cl_2$ as solvent, using N-iodosuccinimide (NIS) and trifluoromethanesulfonic acid (TfOH) as catalysts, for a reaction time of 2 days.

In another embodiment, in the step (A), the reaction temperature is -30 degrees Celsius → room temperature; the solvent is a mixture solution of $CH_3CN$ and $CH_2Cl_2$; the catalyst is N-iodosuccinimide (NIS), trifluoromethanesulfonic acid (TfOH), and TMSOTf; and the reaction time may be 3 days.

In another embodiment, in step (A), the reaction temperature is -30 degrees Celsius → 0 degree Celsius; the solvent is a mixture solution of $CH_3CN$ and $CH_2Cl_2$; the catalyst is N-iodosuccinimide (NIS) and trifluoromethanesulfonic acid (TfOH); and the reaction time may be 2 days.

In another embodiment, in step (A), the reaction temperature is -30 degrees Celsius; the solvent is a mixture solution of $CH_3CN$ and $CH_2Cl_2$; the catalyst is N-iodosuccinimide (NIS) and trifluoromethanesulfonic acid (TfOH); and the reaction time may be 3 days.

In another embodiment, in step (A), the reaction temperature is room temperature; the solvent is $CH_2Cl_2$; the catalyst is N-iodosuccinimide (NIS) and trifluoromethanesulfonic acid (TfOH) ; and the reaction time may be 1 day.

In another embodiment, in the step (A), the reaction temperature is -50 degrees Celsius; the solvent is a mixture solution of propionitrile and $CH_2Cl_2$; the catalyst is N-iodosuccinimide (NIS) and trifluoromethanesulfonic acid (TfOH); and the reaction time may be about 6 hours.

In one aspect, the present invention provides a method for synthesizing an oligosaccharide, including the step of reacting an amino sugar having an amino group protected by trichloroethoxycarbonyl (Troc) with a sugar protected by methoxyphenyl (MP) under conditions in which the amino sugar protected by Troc binds with the sugar protected by MP.

In one embodiment, the amino sugar that may be used in the present method may have a leaving group L.

In other embodiments, the leaving group L may be, and is not limited to, for example, -SPh, $-SCH_3$, $-SCH_2CH_3$, -F, $-OPO(OPh)_2$ (wherein, Ph is phenyl), $-OPO(N(CH_3)_2)_2$, Br, or Cl. The reason is that, if the leaving group is a leaving group used for a glycoside of a sugar, it will be capable of working in a similar way.

In a preferred embodiment, the amino sugar protected by Troc may be, for example:

[Chem. 79]

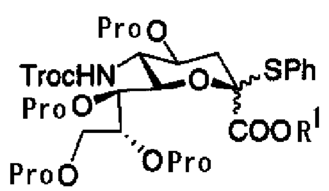

wherein Pro may be, and is not limited to, for example, independently, acetyl(Ac), benzyl (Bn), benzoyl (Bz), pivaloyl (Piv), MPM (p-methoxybenzyl), methoxyphenyl (MP), or the like; and
R1 may be, for example, an alkyl, or an aryl.

In other preferred embodiment, the sugar protected by MP may be, for example:

[Chem. 80]

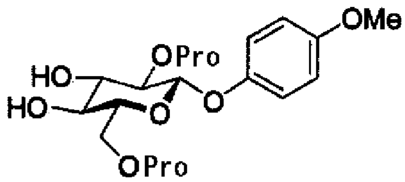

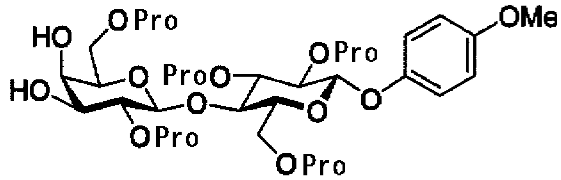

wherein Pro may be, for example, independently a protecting group that is an acetyl (Ac), a benzyl (Bn), a benzoyl (Bz), a pivaloyl (Piv), a MPM (*p*-methoxybenzyl), or a methoxyphenyl (MP).

In another embodiment, the present methodmay include the step of deprotecting the Troc group in the presence of Zn (Cu).

(Intermediate of glycolipid)

In one aspect, the present invention may provide, for example, compounds having the following structures:

[Chem. 81]

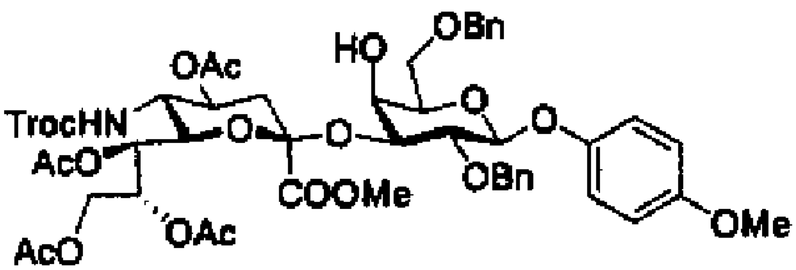

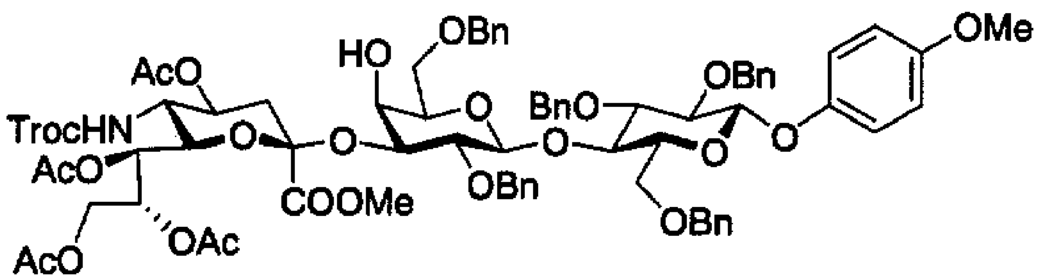

and the like, the compounds are not limited thereto. The reason is that, as long as the compound has sialic acid and a base sugar chain and the reducing terminal thereof is protected by a protecting group that can be chemoselectively removed, a lipid is introduced by a similar method and thereby a ganglioside can be derived.

In another aspect, the present invention is, for example, a compound represented by the formula:

[Chem. 82]

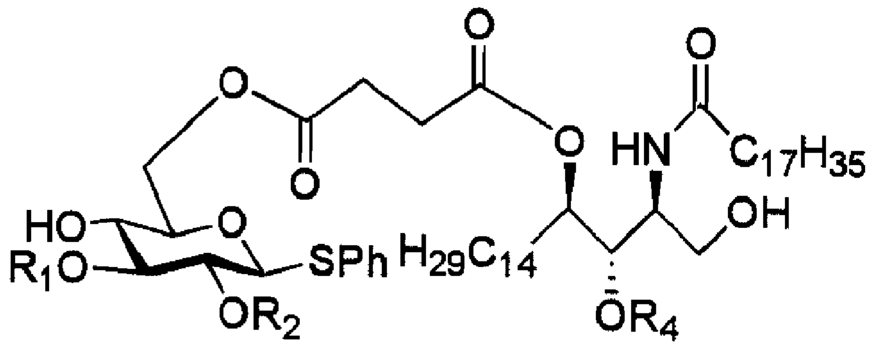

wherein $R_1$ and $R_2$ may be, for example, independently an alkyl or an alkenyl group, or the like; $R_4$ may be, and is not limited to, for example, a benzoyl, acetyl, or pivaloyl group. The reason is that, after a glycolipid is constructed, if the group can be removed under a mild condition, it will not cause a problem.

In another aspect, the present invention is, for example, a compound represented by the formula:

[Chem. 83]

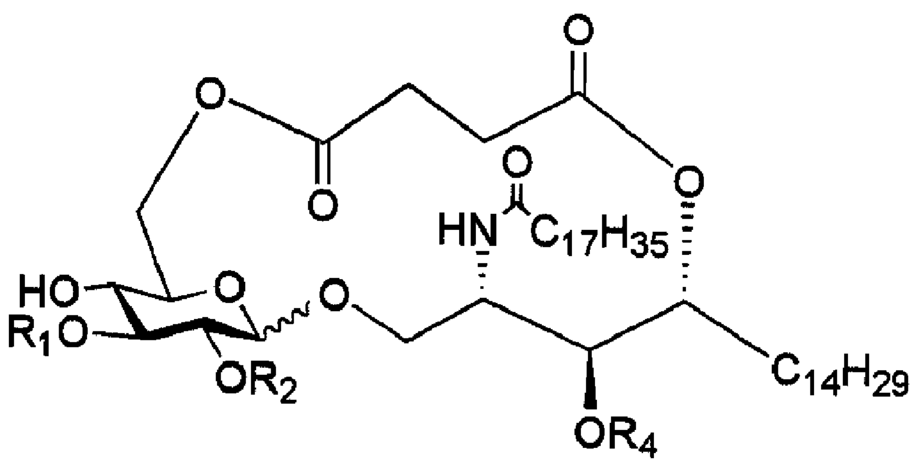

wherein $R_1$ and $R_2$ may be, for example, independently an alkyl or an alkenyl group; $R_4$ may be, for example, a protecting group like benzoyl, acetyl, or pivaloyl.

(Sugar chain)

In one aspect, the present invention provides a sugar chain produced by the method synthesizing the sugar chain in the present method. Here, in the method synthesizing a sugar chain, any form described in the above section of (Method for synthesizing a sugar chain) can be used.

Recently, Glycoconjugates such as glycoprotein, proteoglycan, glycolipid, and the like have gained attention from the biochemistry field. Moreover, the different physiological roles of polysaccharides existing in the cell wall of plants and bacterium have been gradually clarified. Following it, construction of oligosaccharide portions (sugar chains) thereof has been recognized as an organic-synthetic-chemically important problem (Paulsen, H. Angew. Chem. Int. Ed. Engl. 1982, 21, 155-.) Structures of sugar chains, in the case where it includes the kinds of sugars constituting sugar chains, the number, the order of a sequence , stereochemistry of a glycosidic bond, the position of a bond between sugar residues, is extremely various. Accordingly, in order to efficiently synthesize a sugar chain, a means for stereoselectively and regioselectively forming a glycosidic bond is necessary. A lot of studies regarding o-glycosylation reaction, which is a basic methodology to efficiently synthesize a sugar chain, have been carried out. However, it is difficult to say that a factor controlling the stereochemistry thereof is sufficiently understood. Furthermore, in the present circumstance, there is no omnipotent reaction that can apply to every kind of o-glycosylation. However, glycosylation reactions already known have slightly different properties with regard to a factor affecting stereoselectivity. Thus, by combining reaction conditions such as a leaving group and a protecting group, an activating agent, temperature, and the like, with regard to stereoselectivity, the glycosylation reaction can have a wide range of variability. In fact, regarding pyranosides, in most of o-glycosylation, currently it is getting possible to highly control stereochemistry (Wulff, G.; Rohl, G. Angew. Chem. Int. Ed. 1974, 13, 157-.).

(Stereochemical control in the glycosylation reaction)

An o-glycosylation reaction is represented by the following general formula (o-Glycosylation reaction). Usually, (1) is called glycosyl donor, (2) is called glycosyl acceptor or aglycon. A substituent X at the anomer position of the glycosyl donor is called the leaving group. In order to perform a glycosylation reaction, a catalytic or stoichiometric amount of activating agent (activator or promoter) is necessary for the purpose of increasing the leaving ability of the glycosyl donor. Furthermore, an activating agent can fill a role as a scavenger of an acid that is created accompanying the reaction.

(o-Glycosylation reaction)

[Chem. 84]

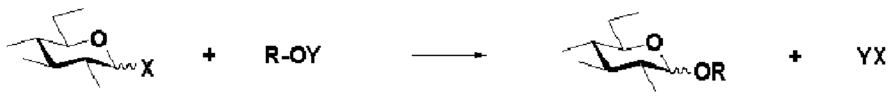

(1) (2)

From the viewpoint of synthetic chemistry, o-glycoside is roughly classified into the 2-hydroxy system, 2-amino system, and 2-deoxy system. Among these, the 2-hydroxy system glycoside and 2-amino system glycoside are classified into 1,2-cis type and 1,2-trans type. Moreover, for the synthesis of 2-hydroxy system glycosides, regarding each of the 1,2-cis type and 1,2-trans type, different methodologies are used for a mannose type (manno type) and a glucose type (gluco type). When roughly classifying the methodologies used for stereochemical control in an o-glycosylation reaction, there are three methodologies as follow: (1) synthesis of α-glycoside by *in situ* anomerization; (2) synthesis of β-glycoside by $S_N2$ type inversion; and (3) synthesis of 1,2-trans-glycoside utilizing neighboring group participation. In the following table (Classification and synthesis method of o-glycoside), the basic structures of o-glycosides and the methodologies utilized in the syntheses thereof are summarized. Hereinafter, while glycosyl halides having Br or Cl as the leaving group are shown as examples, a way of thinking, which is the basis, will be mentioned.

(Classification and synthesis method of o-glycoside)

[Chem. 85]

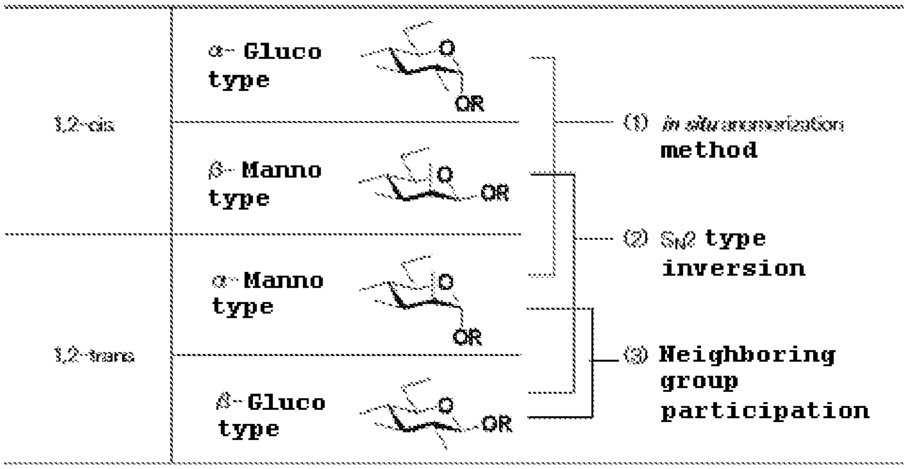


(a. *in situ* Anomerization method)

Regarding glycosyl halides, most of them usually exist as α body (3a), which is thermodynamically stable. However, when the α body (3a) is activated by a heavy metal salt, a quaternary ammonium salt, or the like, an oxocarbenium ion (4a) is created, and equilibrium is established between it and β-halide (3b) via the β-type ion pair. Here, if an alcohol (R-OH) exists in the system, it will be thought that (4a) gives β-glycoside (5b) and (4b) gives α-glycoside (5a) . A glycosyl donor that appears not to perform a neighboring group participation reacts in such a way, the stereochemistry thereof is determined by relative velocities of a series of these reactions. Here, in view of an anomeric effect, it is thought that (4b) is more reactive than (4a) ($K_2 > K_1$). Consequently, if this equilibrium is fast ($K_3 >> K_1$), the reaction proceeds mainly via (4b) to give α-glycoside (5a). This kind of reaction is useful in the synthesis of an α-gluco type glycoside and an α-manno type glycoside. As a protecting group of the hydroxyl group at C-2 position, in order to prevent neighboring group participation, an ether protecting group such as benzyl group, an allyl group, and the like are used. Regarding a 2-amino system glycoside, by selecting a kind of a substituent at C-2 position equivalent to an amino group, similarly, the reaction can proceed α-selectively. What should be noted is that, in order to obtain high selectivity, in view of the reactivity of a substrate, it is necessary to select a reaction condition as mild as possible. That is to say, if an activating agent is too strong or a reaction temperature is too high in comparison to the reactivity of a substrate, the reaction will proceed along the route of (3a) → (4a) → (5b), and consequently can considerably give the β body as a by-product. On the other hand, a method in which a β-halide is prepared in advance and directly used for the reaction is also known. This method is useful for a α-glycosylation of a highly reactive substance.

(Route of glycosylation reaction (1))

[Chem. 86]

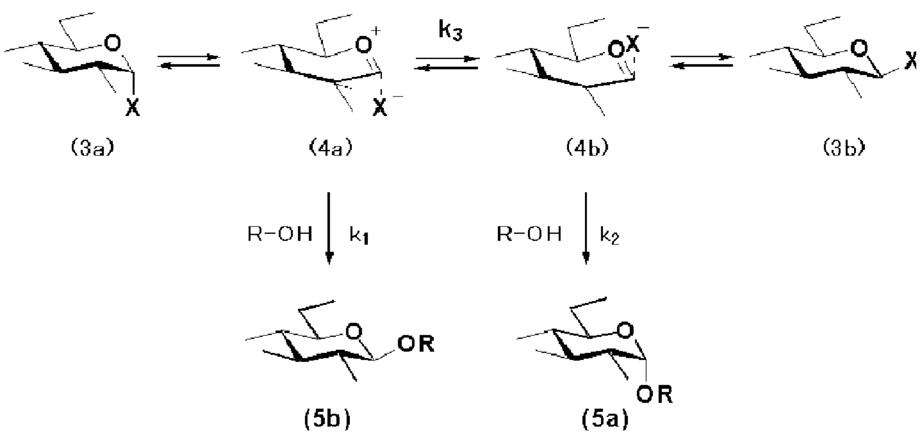


(3a) (4a) (4b) (3b)

(5b) (5a)

(b. Glycosylation with $S_N2$ type inversion)

When a sort of insoluble catalyst is used as an activating agent, an ion pair created by the activation of a halide is immobilized on the surface of the catalyst, and therefore equilibrium between the anomers of the halides is suppressed. Under such a condition, the α-halide is reacted with $S_N2$ type inversion of stereochemistry to give the β-glycoside. This type of reaction is particularly useful in the synthesis of a β-manno type glycoside. Furthermore, when using a highly strong activating agent and a glycosyl donor that has high leaving ability, the speed of the substitution reaction is larger than the speed of the anomerization ($K_1 > K_3$), and thus the β body can be obtained as the main product.

(c. Stereochemistry control by neighboring group participation)

When using an acyl group such as an acetyl and a benzoyl group and the like as a protecting group of the hydroxyl group at C-2 position of a glycosyl donor, a β-gluco type glycoside and an α-manno type glycoside can be selectively synthesized. This phenomenon is explained by assuming the neighboring group participation of an acyl group. That is to say, an acyloxy group that performs neighboring group participation to an oxocarbenium ion (7) and created from a glycosyl halide (6), consequently (7) isomerizes to a more stable cyclic acyloxonium ion (8). In (8), a direction of the nucleophilic attack of an alcohol to the anomer position is limited (route (a)), and consequently only a product (9), which has the 1,2-trans configuration, is obtained. From the viewpoint of stereochemistry control, this method has a highly reliable reaction. However, a problem often occurs which is that route (b) often competes to give an orthoester (10) as a by-product. Furthermore, an acyl group, which is an electron-withdrawing, that exists next to the anomer position, and therefore the reactivity of the halide is lower. Thus, generally, a strong reaction condition is necessary.

Regarding a 2-amino sugar, neighboring group participation by N-acyl group, N-phthaloyl group, or the like is utilized. On the other hand, a 2-deoxy sugar cannot utilize neighboring group participation in the case that the 2-deoxy sugar is as it is. However, control of stereochemistry by introducing a substituent, such as a halogen, sulfur, selen, and the like, to the C-2 position has been actively tried.

(Route of glycosylation reaction (2))

[Chem. 87]

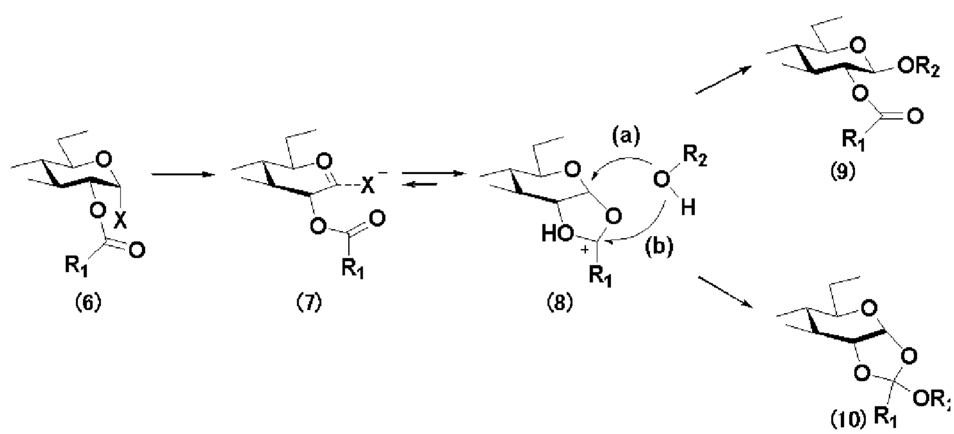

There is another problem, which is the control of the biding position between sugar residues. An oligosaccharide has many hydroxyl groups in its molecule, and thus it is necessary to strictly distinguish them from one another, and react only an intended hydroxyl group. One of means for achieving it is a method of utilizing the difference in reactivity of the hydroxyl groups. For example, the primary hydroxyl group at C-6 position is much more reactive than other hydroxyl groups, and thus a sugar residue can be selectively introduced. Furthermore, taking advantage of the fact that an equatorial hydroxyl group is less sterically hindered than an axial hydroxyl group, a regioselective glycosylation can be often performed. However, these cases are special examples. More generally, it is desirable to selectively introduce a protecting group, and thereby increase the solubility of a saccharide to an organic solvent, and only make a specific hydroxyl group free. When a complicated oligosaccharide is synthesized, it is necessary to use a variety of different protecting groups according to their properties. Appropriate selection thereof is decisively important with respect to the success or failure of the synthesis.

(Setting of reaction condition)

Regarding the glycosylation reaction, it is naturally desirable to perform it under a strictly anhydrous condition. Accordingly, it is necessary to remove water from all the solvents, reagents, substrates, and reaction containers as much as possible. Particularly, as silver salt is highly hygroscopic, and it is thus required to carefully handle it. Currently, when performing the reaction,the general method is the one in which a solution is injected with a syringe under the atmosphere of nitrogen or argon . Regarding a reaction using a silver salt, it is preferred to perform it with the light cut off. Meanwhile, most glycosylation reactions are not inhibited by molecular sieves (MS)or anhydrouscalciumsulfate(Drierite). Particularly, in precise synthesis, in order to reduce the technical difficulty thereof, a reaction is often performed in the presence of these desiccants. Solvents used for many purposes include halogenated hydrocarbon such as dichloromethane, 1,2-dichloroethane, and the like, aromatic hydrocarbon such as toluene, benzene, and the like, and diethylether. Polar solvents such as nitromethane, acetonitrile, and the like are also often used. It is difficult to uniformly understand the solvent effect in glycosylation reactions. However, generally, in polar solvents such as nitromethane, the reaction is accelerated. A lot of data is accumulated with regard to the relationship with stereoselectivity. What is significantly interesting is the solvent effect of acetonitrile and diethylether. That is to say, in a kind of reaction, in the case of being in acetonitrile, a β body is obtained as the main product, while in the case of being in diethylether, an α body is obtained as the main product. In comparison with other nonpolar solvents such as dichloromethane, in the case of ether, it is often recognized that α-selectivity is improved. Thus, it does not appear that the difference of a directional property from acetonitrile simply resultsfromthe difference of polarity. Reaction temperatures are as extremely wide as -70 degrees Celsius to about 100 degrees Celsius according to the reactivity of a substrate. However, it is usually desirable to perform a reaction at low temperature as far as the progress of the reaction is not inhibited. Regarding an effect of concentration, systematic studies have not been carried out. However, as common practice of intermolecular reactions, it is believed that the reaction should be performed in high concentration. On the other hand, among of them, there is an example in which only a highly diluted condition gave a good result (Nicolaou, K. C.; Daines, R. A.; Ogawa, Y.; Chakraboty, T. K. J. Am. Chem. Soc. 1988, 110, 4696-.). Those skilled in the art can select an appropriate condition as necessary on the basis of the description of the present specification in view of the aforementioned information.

(Design of a preferred production example on a new synthesis strategy for introducing a ceramide)

As described above, when synthesizing a complicated ganglioside, the inventors have used a method for introducing a ceramide portion using an azide sphingosine (see below) for various purposes. One reason is that the larger the molecular weight of a sugar chain is, the much more difficult it is to introduce a lipid by other methods. By conversion to an azide body, the steric hindrance of two alkyl chains of the ceramide is reduced, and moreover, by suppressing the reduction of the nucleophilicity of the primary hydroxyl group of the ceramide caused by hydrogen bond between the primary hydroxyl group and an amide, it becomes easy to introduce the ceramide skeleton.

Method of introducing a lipid after completing the construction of a sugar chain (Improvement of the lipid acceptor)

[Chem. 88]

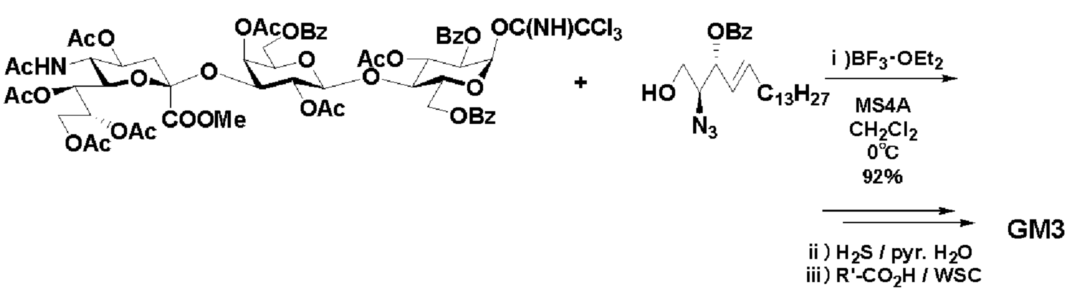


However, by this conventional method, a satisfactory introduction yield has not been obtained. Additionally, the present method requires complicated synthesis steps before and after preparation of an azide sphingosine, reduction of an azide group, and introduction and condensation of a fatty acid. Thus, as a result of minutely investigating these problems, it is thought that the points for improvement are three points as follows: (1) improvement of the condensation

yield of a sugar chain and a lipid; (2) stereoselectivity in introducing a lipid; and (3) simplification of preparation and conversion of a ceramide. In the present invention, taking the above points into consideration, a new synthesis strategy using an intramolecular condensation reaction was designed. It is thought that an intramolecular glycosylation appropriately designed can improve all of the above problems.

The main advantages of the intramolecular glycosylation are improvements of reactivity, stereoselectivity, and regioselectivity. Moreover, it becomes possible to apply it to solid-phase synthesis or connective synthesis. An intramolecular reaction is more entropically advantageous than an intermolecular reaction because two molecules are changed to one molecule. As a result, it is thought the reactivity largely increases. By appropriately designing compounds, it can be expected that two molecules collide more frequently at the reaction site and thus it makes the equilibrium state of the reaction shift to the side of the target compound. Furthermore, with regard to stereoselectivity and regioselectivity, by combining to consider a position and a type of a spacer used (length, properties of its functional group, and flexibility) or solvent and reaction temperature, both of the molecules are variously, sterically controlled, and consequently it becomes possible to improve the selectivity.

In the field of synthetic carbohydrate chemistry, many synthesis making the most of the aforementioned property have been utilized. Intramolecular glycosylation has a long history, and a variety of reactions have been examined in the past (Jung, K. H.; Muller, M.; Schmidt, R. R. Chem. Rev. 2000, 100, 4423-4442.). Intramolecular glycosylations can be roughly classified into three types on the basis of a spacer connecting a glycosyl donor and a glycosyl acceptor.

(Classification of intramolecular glycosylation)

[Chem. 89]

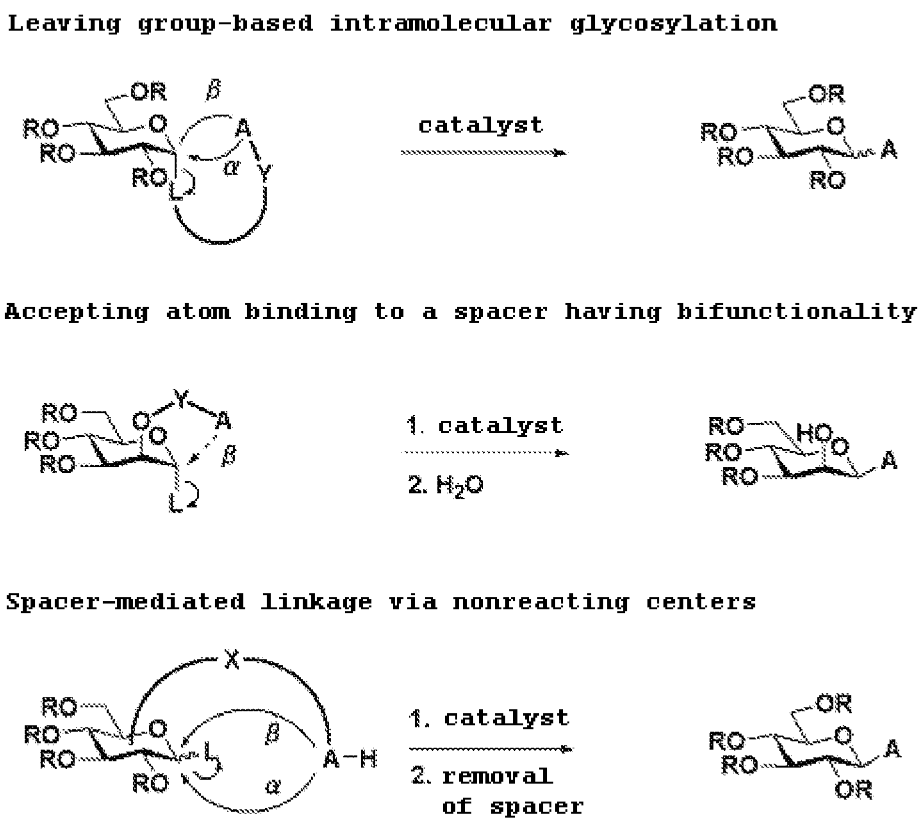


In the case of the Leaving group-based intramolecular glycosylation, a glycosyl acceptor is linked with the leaving group of a glycosyl donor, and at the same time when the leaving group leaves, the acceptor attacks the anomer carbon of the donor.

In the case of the accepting atom binding to a spacer having bifunctionality (Linkage of the accepting atom via a bifunctional group), an acceptor is linked to a donor (generally, to the hydroxyl group at 2-position) via a spacer having bifunctionality, and in work-up or at the same time when the leaving group leaves, the accepting atom removes the spacer.

In the case of Spacer-mediated linkage via nonreacting centers, an acceptor is linked with a glycosyl donor via the spacer that is not related to the reaction regardless of functionality or a position thereof, and by one (or more) non-protected hydroxyl group(s), which generally exists in the acceptor, the reaction proceeds.

One of the most famous proposals utilizing an intramolecular glycosylation is the synthesis of β-mannoside by Ito and Ogawa, et *al*. (Ito, Y.; Ogawa, T.; J. Am. Chem. Soc. 1997, 119, 5562-.). Regarding β-mannoside, because of the structure of mannose, neither the aforementioned *in situ* anomerization method nor the steric control method by a neighboring group can be used. Moreover, β-mannoside must form β-glycoside, which is thermodynamically and kinetically unstable. Consequently, even in this day by which a variety of steric control methods have been reported, it still is regarded as one of the most difficult problems. The binding mode and the degree of difficulty in synthesis of the main glycosides in higher animals are shown below.

(The binding mode and the degree of difficulty in synthesis of the main glycosides in higher animals)

[Chem. 90]

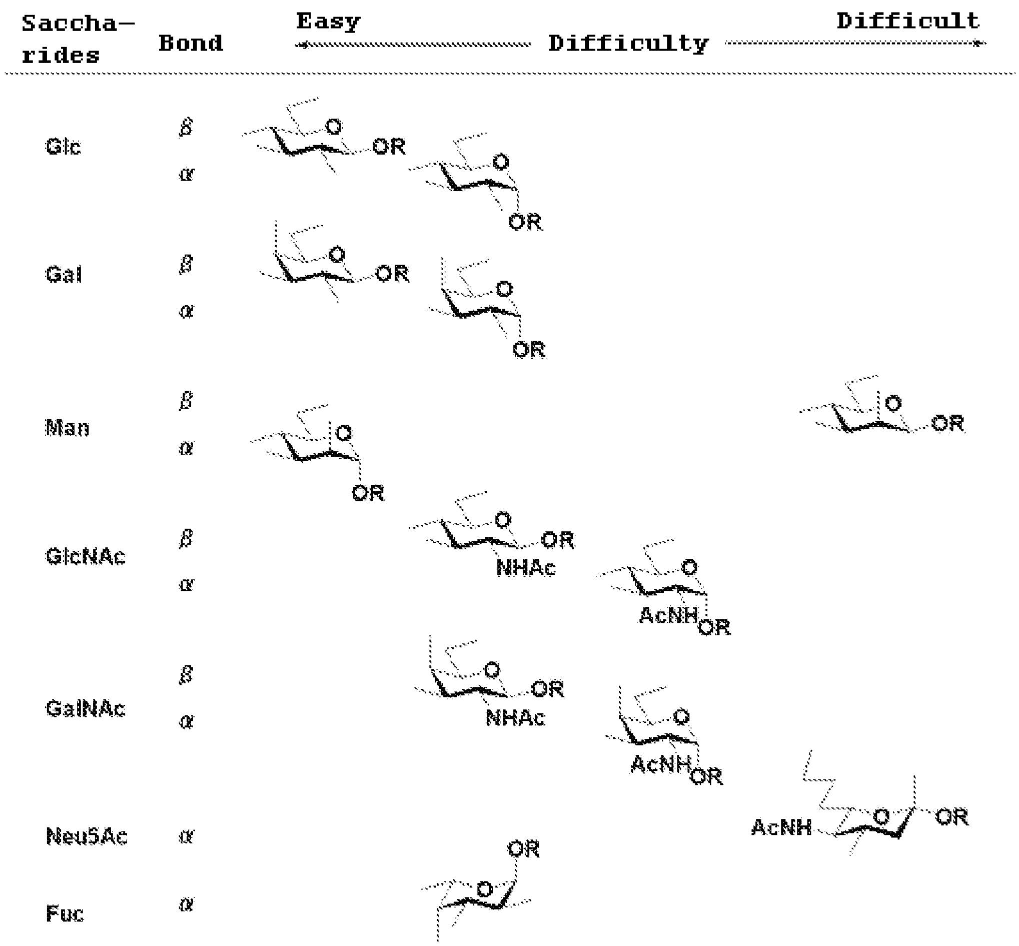


Ito and Ogawa, *et al*. applied an intramolecular glycosylation to solid-phase synthesis, and successively attained highly-stereoselective and efficient synthesis of β-mannoside (Ito, Y.; Ogawa, T.; J. Am. Chem. Soc. 1997, 119, 5562-.).

(Synthesis of β-mannoside utilizing an intramolecular glycosylation by Ito·and Ogawa, *et al*.)

[Chem. 91]

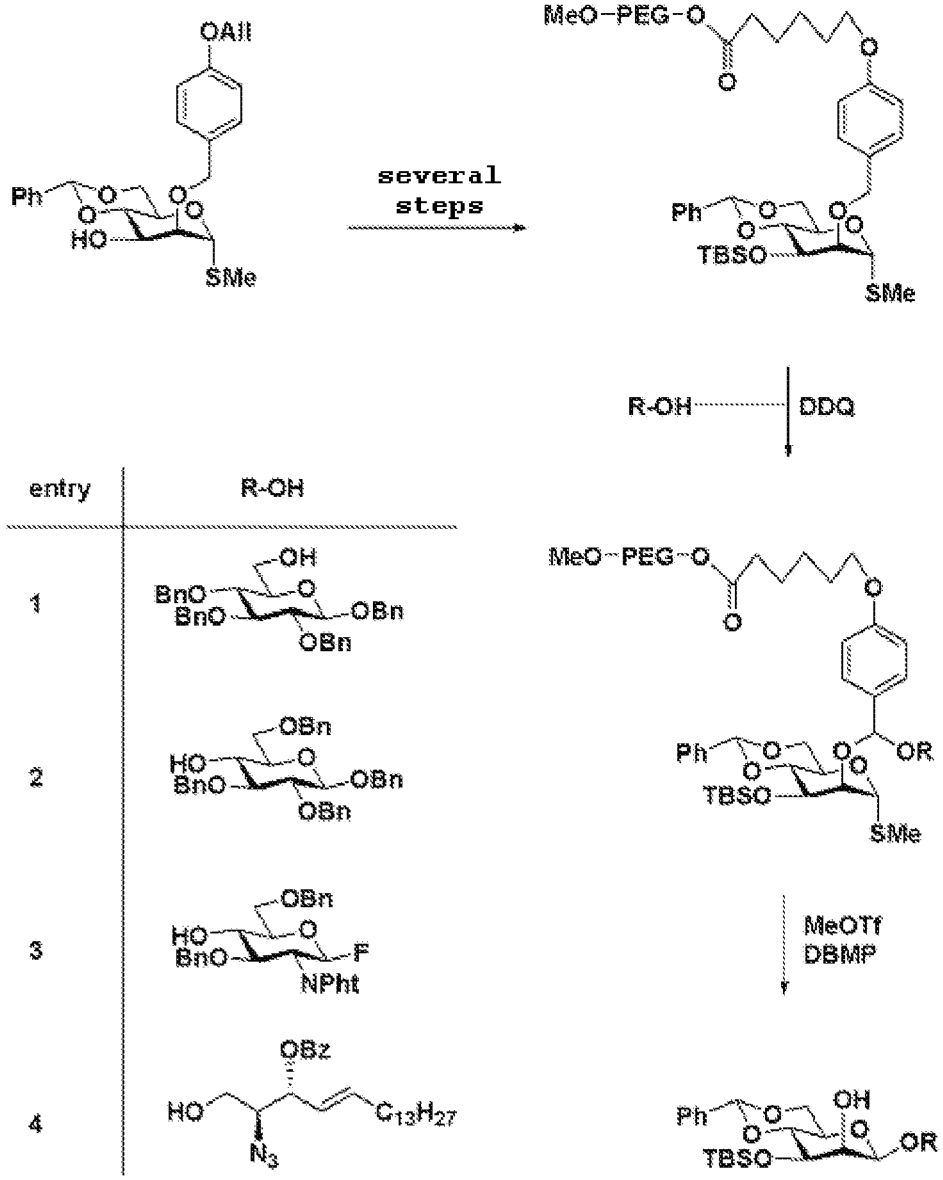


In the above reactions, by bonding glycosyl donor to resin, purification using conventional column chromatography was omitted. Furthermore, by an appropriately-designed structure, formation of β-mannoside, which believed to be difficult, is stereoselectively performed.

The present invention has been exemplified so far with reference to preferable embodiments of the present invention, but it should not be construed that the present invention is restricted by the embodiments of the present invention. It should be understood that the scope of the present invention should be construed only by the claims. It would be understood that those skilled in the art can perform an invention practically equivalent to the present invention, based on the description of the present specification and technical common sense from the description of typical preferable embodiments of the present invention. It would be understood that the patents, patent applications and literatures cited herein are incorporated herein by reference to the present specification, similarly to the case where the description is described specifically herein.

Hereinafter, the configurations of the present invention will be described more specifically with reference to Examples, but it should be understood that the present invention is not limited thereby. Reagents used below were commercially available products, unless specified otherwise.

[Examples]

(Synthesis examples)

In the present invention, when using an intramolecular glycosylation, it was examined which hydroxyl group of a sugar (glucose) at the reducing terminal and which hydroxyl group of a ceramide is crosslinked, what is used as a spacer, and which method of the three kinds of intramolecular glycosylation types is used. First, with regard to which hydroxyl group of a sugar (glucose) at the reducing terminal and which hydroxyl group of a ceramide is crosslinked, as a result of considering a binding yield, it is thought to be preferred to bind the 6-position of glucose, which is a highly reactive primary hydroxyl group, with the hydroxyl group at 3-position of the ceramide. It is possible to variously examine what is used as a spacer. First, it was determined to use succinic acid, since succinic acid is used for many purposes in intramolecular glycosylations, is easy to be handled, and, in a last deprotection, is possible to be efficiently removed by deprotection. For the above reasons, among three kinds of intramolecular glycosylation types, it was determined to use (Spacer-mediated linkage via nonreacting centers).

With regard to introduction of a ceramide using an intramolecular glycosylation, two kinds of synthesis strategies are proposed. One is a method in which a sugar chain is constructed and then an intramolecular glycosylation is used (the following formula). The other is a method in which, first, a ceramide is introduced to glucose to be the reducing terminal of a sugar chain by an intramolecular condensation via succinic acid and then a glucosyl ceramide acceptor is introduced to a sugar chain (the following formula).

(Method in which a sugar chain is constructed and then an intramolecular glycosylation is used)

[Chem. 92]

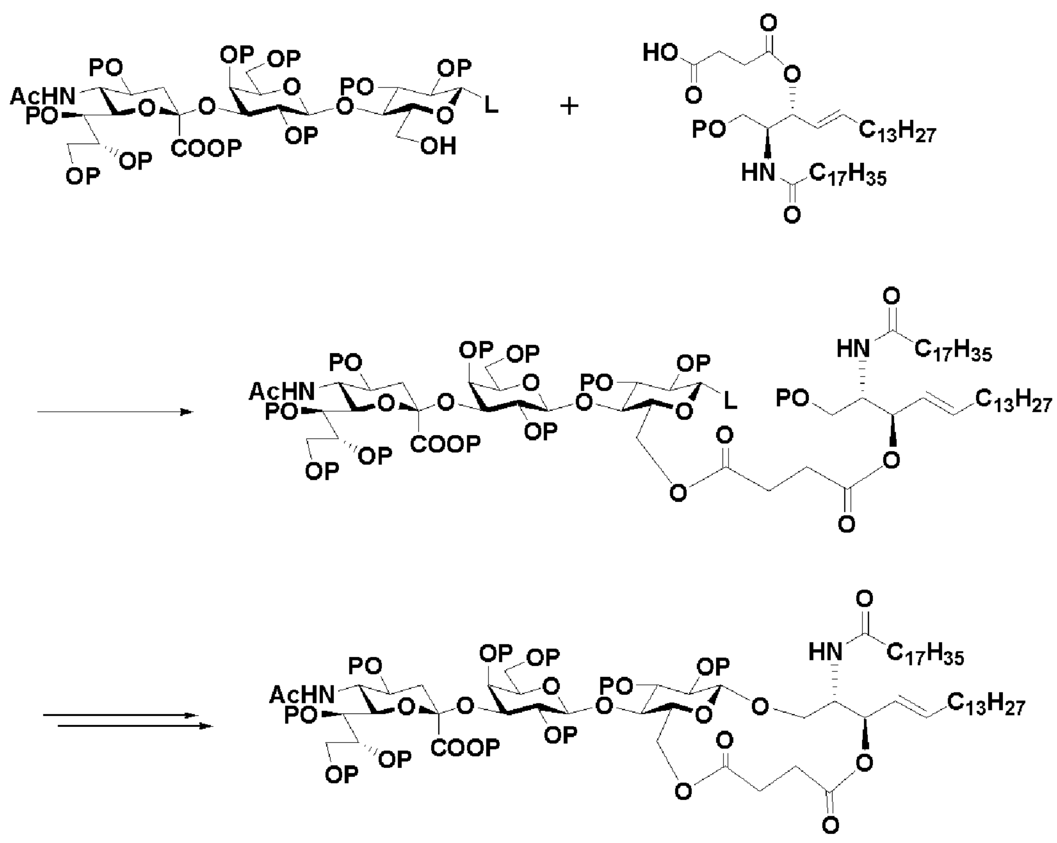

(Method in which an intramolecular glycosylation is used to glucose and then glucosylceramide acceptor is utilized)

[Chem. 93]

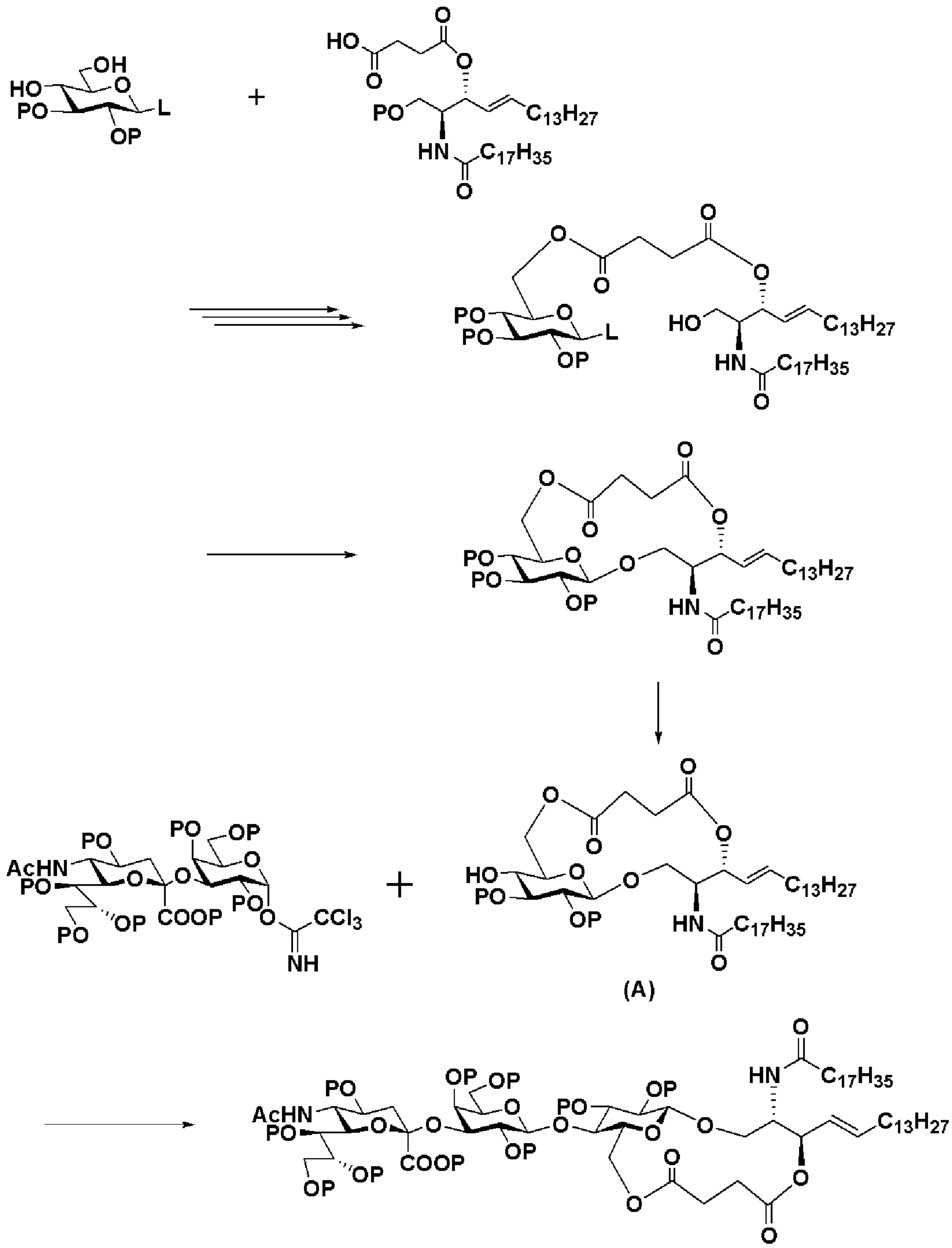

(A)

First, it was verified whether a ceramide could be introduced to a sugar by an intramolecular glycosylation. Here, glucose, which is a monosaccharide, was used as a sugar chain. After glucose was crosslinked with a ceramide and then an intramolecular glycosylation was performed, results of the intramolecular glycosylation were discussed. Then, two synthesis strategies were each examined.

For the above reason, first, an appropriately-protected glucose donor and a ceramide acceptor are prepared, and then GlcH-6 and CerH-3 are crosslinked with succinic acid. Then, an intramolecular glycosylation is performed. The result is discussed, and then, using the resulting glucosyl ceramide acceptor, introduction to a sugar chain is performed. Then, it was determined that, with regard to more complicated sugar chains, an intramolecular glycosylation is examined

(Scheme 1).

(Scheme 1)

[Chem. 94]

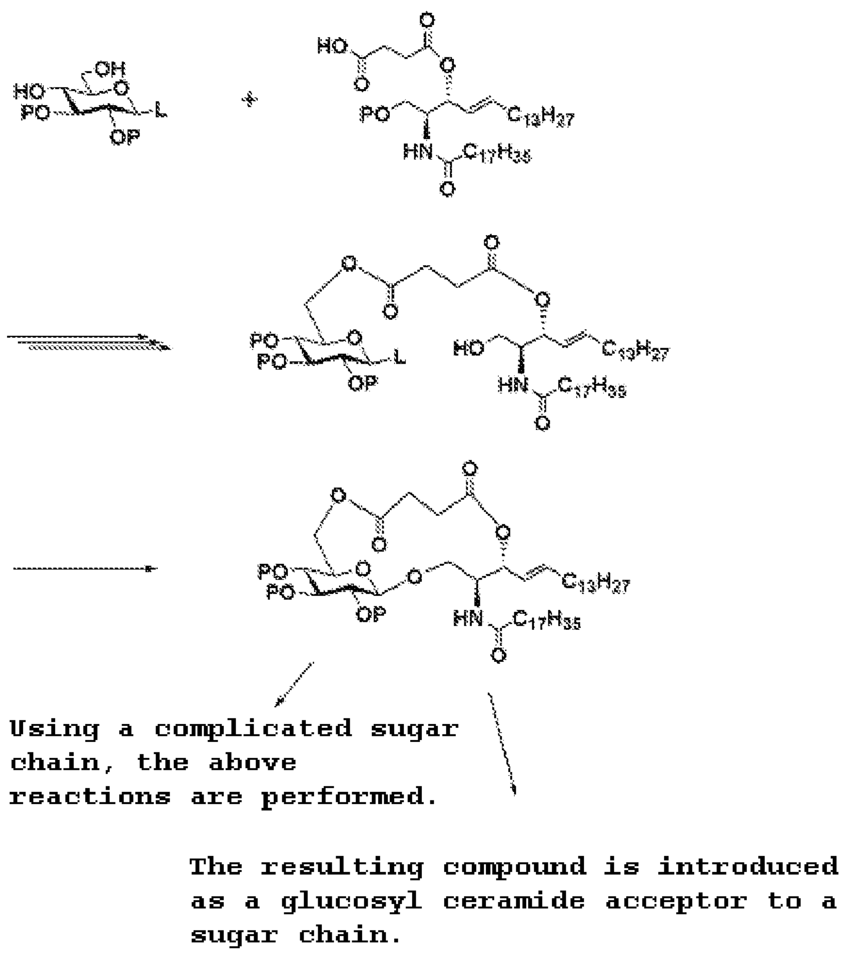


(Preparation of a glucose donor)

In natural gangliosides, glucose is positioned at the reducing terminal of a sugar chain, most gangliosides other than the gala-series (glycolipids in which a β-galactoside bond is constituted between a sugar chain and a ceramide) have the structure of Galβ(1-4)Glcβ(1-1)Cer.

(Basic sugar chain structure of a typical glycosphingolipid)

[Chem. 95]

| | |
|---|---|
| Galβ(1-4)Glcβ(1-1)Cer | Hematoside-series |
| GalNAcβ(1-4)Galβ(1-4)Glcβ(1-1)Cer | Ganglio-series |
| Galβ(1-3)GalNAcβ(1-4)Galβ(1-4)Glcβ(1-1)Cer | Ganglio-series |
| [Galβ(1-3)GlcNAcβ(1-3)]nGalβ(1-4)Glcβ(1-1)Cer | Lacto-series (Lacto-type 1) |
| [Galβ(1-4)GlcNAcβ(1-3)]nGalβ(1-4)Glcβ(1-1)Cer | Neolacto-series (Lacto-type 2) |
| GalNAcβ(1-3)Galα(1-4)Galβ(1-4)Glcβ(1-1)Cer | Globo-series |
| GalNAcβ(1-3)Galα(1-3)Galβ(1-4)Glcβ(1-1)Cer | Isoglobo-series |

In view of the structures described above, it is thought that the most reliable method is to introduce a benzyl group, which is an acyl group of which the neighboring group participation can be expected, as a protecting group at 2-position of a glucose donor. Furthermore, since a sugar chain is introduced to the 4-position of glucose, it is more preferable that a protecting group at the 3-position of glucose does not decrease the reactivity of the hydroxyl group at 4-position when the sugar chain is introduced to the 4-position. For the above reason, it is thought appropriate that a protecting group at 3-position is Bn or MPM group or the like, which is an electron-donating group and an ether group, wherein the utility thereof has been confirmed. For removal of the Bn group by deprotection, catalytic hydrogenation is mainly used. However, when the method is used, an unsaturated bond of a ceramide is reduced. It therefore appears more appropriate to use the MPM group than the Bn group. Regarding the4- and 6-positions of glucose, it was determined that: when a crosslink with a ceramide using succinic acid is performed, the hydroxyl groups at 4- and 6-positions of glucose are free; and, by utilizing the difference of reactivity between the most highly reactive primary hydroxyl group at 6-position and the low-reactive secondary hydroxyl group at 4-position, a crosslink with succinic acid is performed at the 6-position selectively. It was determined that after the crosslink using succinic acid, the hydroxyl group at 4-position of glucose is protected by the chloroacetyl group, which allows selective deprotection and then it is lead to an intramolecular glycosylation. Furthermore, with regard to protection of the anomeric position, a variety of protecting groups are expected. However, it was determined to use the SPh group, which is relatively stable as a protecting group and, when condensation with a ceramide is performed, it can be utilized as a leaving group as it is. The SPh group has characteristics which are that there are so many kinds of activating agents for SPh group, that other leaving groups can be easily derived from the SPh group, and the like (the following formula: a required protection mode of a glucose acceptor).

(Required protection mode of a glucose acceptor)

[Chem. 96]

4- and 6-positions are free hydroxyl groups.

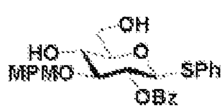

As both a leaving group and a protecting group

- Not to decrease the reactivity of 4-position.
- Possible to be removed in the presence of a ceramide in deprotection.

Neighboring group participation

Moreover, it was determined to design and synthesize a more efficient glucose acceptor. Specifically, the Bz group at 2-position of a glucose acceptor is converted to an electron-donating MPM group, and thereby preparation of

the glucose acceptor is simplified. Additionally, as an armed sugar, improvement of the leaving ability of the SPh group at the 1-position was expected as well as the improvement of the reactivity by introducing a sugar chain to the 4-position (the following formula: a required protection mode 2 of a glucose acceptor). By conversion to the MPM group, the neighboring group participation at 2-position is lost. However, it is thought that, by utilizing the property of high stereoselectivity in an intramolecular glycosylation using succinic acid, it is desired to skillfully control stereoselectivity by introducing a ceramide.

(Required protection mode 2 of a glucose acceptor)

[Chem. 97]

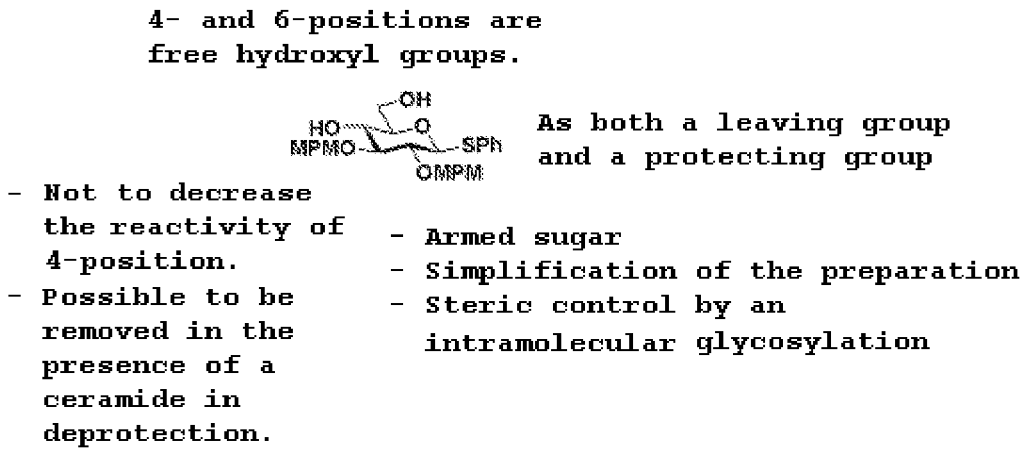


(Preparation example of a glucose donor (2-Bz))

At 45 degrees Celsius, $Ac_2O$ and pyridine (pyr.) worked on Compound 1 to yield Compound 2 quantitatively. Then, to protect the anomeric position of glucose with the SPh group, under argon atmosphere, in $CH_2Cl_2$ solvent at room temperature, PhSH and $BF_3 \cdot OEt_2$ worked on Compound 2 to give Compound 3 with a 67% yield. Then, under argon atmosphere, in MeOH solvent at room temperature, MeONa worked on Compound 3 to remove the acetyl groups at 2, 3, 4, 6-positions by deprotection, consequently giving Compound 4 in a 93% yield. Then, under argon atmosphere, in MeCN solvent at room temperature, BDA and *p*-TsOH worked on Compound 4 to give Compound 5 with a 93% yield. Here, to determine the structure of the product, Compound 5 was acetylated and then the $^1$H-NMR spectrum was checked. Signals δ(ppm) 3.4(m, 1H) of the proton at 2-position and 3.8(m, 1H) of the proton at 3-position of Compound 5 exhibited downfield shifts to 5.0(t, 1H) and 5.3-5.4(t, 1H), respectively. Based on that, the introduction of a benzylidene group to the 4- and 6-positions was confirmed.

Next, in toluene solvent under reflux, DBTO, TBAB, and MPMC1 worked on Compound 5 to give Compound 6 with a 77% yield. It is said that $Bu_2SnO$ forms a cyclic stannylene compound, and then first activates cis-glycols , and subsequently a reaction to equatorial hydroxyl groups among them easily occurs. Compound 5 does not have a *cis*-glycol, and the 4- and 6-positions are protected with the benzylidene group, and therefore it is thought that, a stannylene compound was formed at 2- and 3-positions, and then protection with MPM proceeded at the 3-position selectively. To determine the structure of the product, Compound 6 was acetylated and the $^1$H-NMR spectrum thereof was checked. Signal δ (ppm) 3.5(m, 1H) of the proton at 2-position of Compound 6 was downfield shifted to 5.2-5.3(t, 1H). Based on that, it is confirmed that the 2-position was acetylated, in other words, the MPM group was introduced to the 3-position.

Next, under argon atmosphere, in pyridine (pyr.) solvent at room temperature, benzoyl chloride and DMAP worked on Compound 6 to give Compound 7 with a 80% yield. Compound 7 was deprotected in aqueous 85%-AcOH solution at 40 degrees Celsius to remove the benzylidene group at 4- and 6-positions, consequently giving Compound 8 with a 82% yield (Scheme 2).

(Scheme 2)

[Chem. 98]

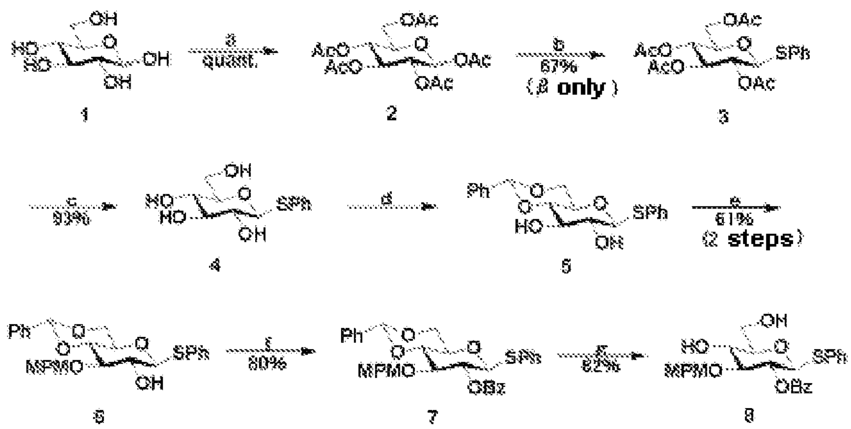


**Reagents and conditions;** (a) $Ac_2O$, Pyr., 45°C (b) PhSH, $BF_3 \cdot OEt_2$, $CH_2Cl_2$, r.t. (c) MeONa, MeOH, r.t. (d) BDA, p-TsOH, MeCN, DMF, r.t. (e) DBTO, TBAB, MPMCl, **toluene, reflux** (f) BzCl, Pyr., r.t. (g) 85% AcOHaq., 40°C

(Preparation example of a glucose donor (2-MPM))

In order to efficiently prepare a glucose acceptor, it was determined to prepare a glucose acceptor of which the Bz group at 2-position is converted to an electron-withdrawing MPM group.
By the conversion to MPM group at 2-position, preparation of the glucose acceptor is simplified. Additionally, as an armed sugar, improvements of the leaving ability of the SPh group at 1-position and the reactivity by introducing a sugar chain to the 4-position were expected. By the conversion to MPM group, the neighboring group participation at 2-position is lost. However, it is thought that, by utilizing the property of stereoselectivity in an intramolecular glycosylation using succinic acid, it is desired to skillfully control stereoselectivity of the anomeric position by introducing a ceramide.
In the preparation of glucose donor (2-MPM), in MeOH/THF mixed solvent at room temperature, a catalytic amount of NaOMe worked on prepared Compound 7 to remove the Bz group at 2-position by deprotection, and subsequently, in DMF solvent at room temperature, NaH and MPMC1 reacted to introduce MPM to the 2-position, consequently preparing Compound 9 with a 70% yield for 2 steps. Then, 83% acetic acid worked on Compound 9 at 30 degrees Celsius to prepare a glucose donor (2-MPM) with a 86% yield (Scheme 3).
(Scheme 3)
[Chem. 99]

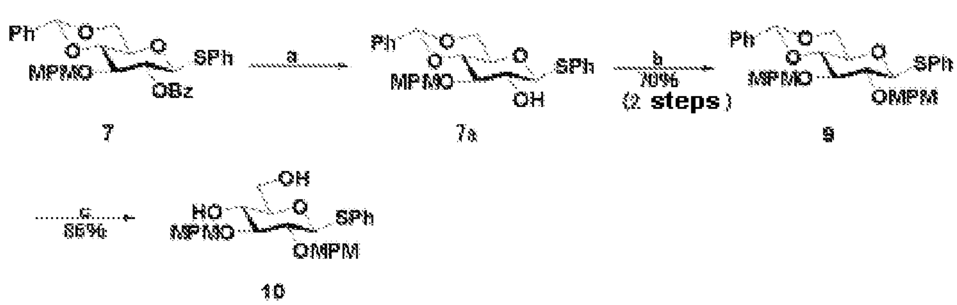


**Reagents and conditions** : (a) MeONa, MeOH / THF = 2 / 1, r.t. (b) MPMCl, NaH, DMF, r.t. (c) AcOH / $H_2O$ = 5 / 1, 30°C

(Preparation example of ceramide)

In the present invention, at the beginning, the research usingsphingosinetype ceramides,which widely exist in mammals, proceeded. However, while the utility of an intramolecular glycosylation was confirmed, because of an unsaturated bond of a sphingosine type ceramide, in carrying out the research, a variety of difficulty was imposed. Specifically, there are the following points: catalytic hydrogenation, which is used when the Bn group and the like are removed by deprotection, which reduces an unsaturated bond, and accordingly cannot be easily used; and, in glycosylation, an activating agent that can be added to an unsaturated bond, such as NIS, cannot be used. Furthermore, we suffered from shortage of samples of ceramides. Currently, some methods for preparing ceramide have been reported ((a)Berg, R. V.; Korevaar, C.; Overkleeft, H.; Marel, G. V. ; BoomJ. V. J. Org. Chem. 2004, 69, 5699-5704. (b) Alexander, M.; Richard, J.K. T.;Robert, J. W. ; Norman, Lewis. Synthesis. 1994, 31-33. (c) Murakami, T.; Furusawa. K.; Tetrahedron. 2002, 58, 9257-9263.). However, it was immediately desired to corroborate the utility of an intramolecular glycosylation. Thus, first, it was determined to develop a method for introducing a ceramide using a phytosphingosine-type ceramide, which can be obtained in a large amount, by which the limitation of protecting groups, activating agents, and the like is small, and for which detailed conditions can be examined.

The present examples mainly describe examples in which a phytosphingosine-type ceramide is used. However, as described in other portions of the present specification, in the present invention, it was clarified that, even using a sphingosine-type ceramide, which widely exists in mammals, a glycolipid can be synthesized by an intramolecular condensation. Therefore, the invention can be recognized as being significant on the point of finding that, from ceramides in general, particularly, a sphingosine-type ceramide, a glycolipid can be produced by intramolecular condensation, wherein the point could not be expected in the past.

A sphingosine-type ceramide has not only a primary hydroxyl group at 1-position but also a secondary hydroxyl group at 3-position. Thus, in the past, succinic acid was bound to the hydroxyl group at 3-position, the primary hydroxyl group was protected with a protecting group that can selectively protect and deprotect the primary hydroxyl group, and thereby the ceramide was used as a ceramide acceptor. On the other hand, a phytosphingosine-type ceramide has not only a primary hydroxyl group at 1-position but also secondary hydroxyl groups at 3- and 4-positions. The present invention is intended to develop a method for introducing a ceramide using a phytosphingosine-type ceramide wherein the method is applicable to a sphingosine-type ceramide. Thus, in a phytosphingosine-type ceramide, it was determined to prepare a ceramide acceptor having succinic acid at 3-position. Furthermore, with regard to the protection at 1-position of a ceramide, a protecting group was required that can protect the primary group selectively and, in deprotection, has selectivity which distinguishes other protecting groups. In a glucose donor, the MPM group, which is weak against an acidic condition, was used and therefore the Tr group, which is used as a protecting group of a primary hydroxyl group for many purposes, was not suitable. Thus, a protecting group was required that can protect and deprotect the hydroxyl group under basic condition. As a protecting group that satisfies with the above requests, the TBDPS or TBDMS group, which is a silyl protecting group, was expected. In deprotection, Silyl protecting groups can be selectively removed by nucleophilic attack of the fluoro anion. The TBDPS and TBDMS groups are bulky and thus are used in the selective protection of a primary hydroxyl group for many purposes. With regard to the hydroxyl group at 4-position of a ceramide, it was determined to use the Bz group, which is an acyl protecting group, which is easily introduced, and, in the last deprotection, is simultaneously removed under the Zemplen condition (the following formula).

(Example of the required protection mode of a ceramide)

[Chem. 100]

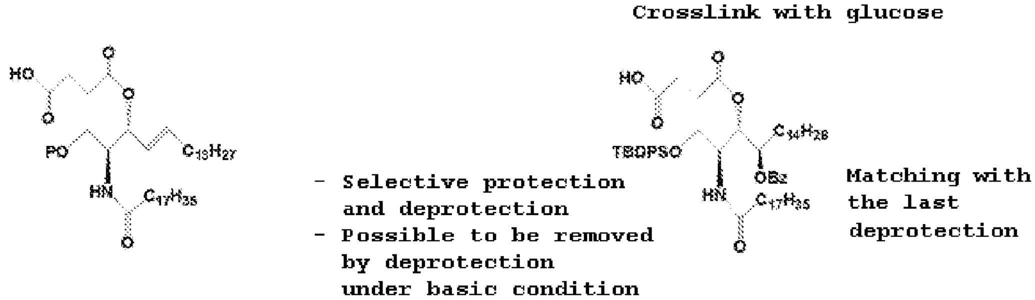


Sphingosine-type ceramide

Phytosphingosine-type ceramide

(Preparation 1 of ceramide)

A phytosphingosine was adopted as the starting material, and stearic acid was introduced to the amino group at 2-position. Then, CerH-1 and CerH-3 were protected with the benzylidene group, and consequently the 1, 3-benzylidene body and the 3, 4-benzylidene body were yielded in the ratio of 1 to 1. Moreover, regarding the 1,3-benzylidene body, when, in pyridine (pyr.) solvent at room temperature, 1 equivalent of benzoic anhydride worked on it to benzoylate the hydroxyl group at 4-position of the ceramide, it was confirmed that the reaction only slightly proceeded. Based on that, it was expected that the steric hindrance around the hydroxyl group at 4-position of the ceramide inhibits the benzoylation (Scheme 4).

(Scheme 4)

[Chem. 101]

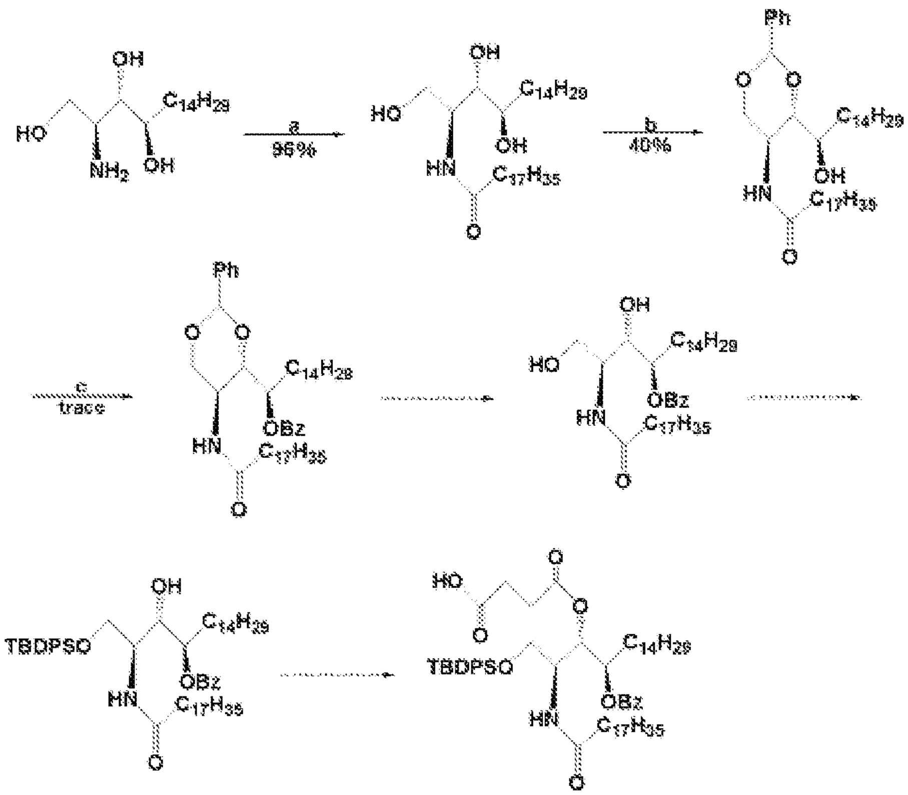


**Reagents and conditions** : (a) stearic acid , WSC, $CH_2Cl_2$, r.t.
(b) BDA, *p*-TsOH, MeCN, r.t. (c) $Bz_2O$, Pyr., r.t.

Thus, the original preparation plan was changed. It is expected that TBDPS group is introduced to the 1-position of a phytosphingosine ceramide, and then, when in pyridine (pyr.) solvent at room temperature, 1 equivalent of succinic anhydride works on the free hydroxyl groups at 3- and 4-positions, because of the steric hindrance around the hydroxyl group at 4-position, succinic acid can be introduced to the 3-position selectively. However, contrary to expectation, the result was that succinic acid was introduced to the 4-position preferentially. Based on that, a ceramide derivative having succinic acid at 3-position of the ceramide, which was originally expected, was not yielded, but a ceramide derivative having succinic acid at 4-position was yielded (Scheme 5).

(Scheme 5)

[Chem. 102]

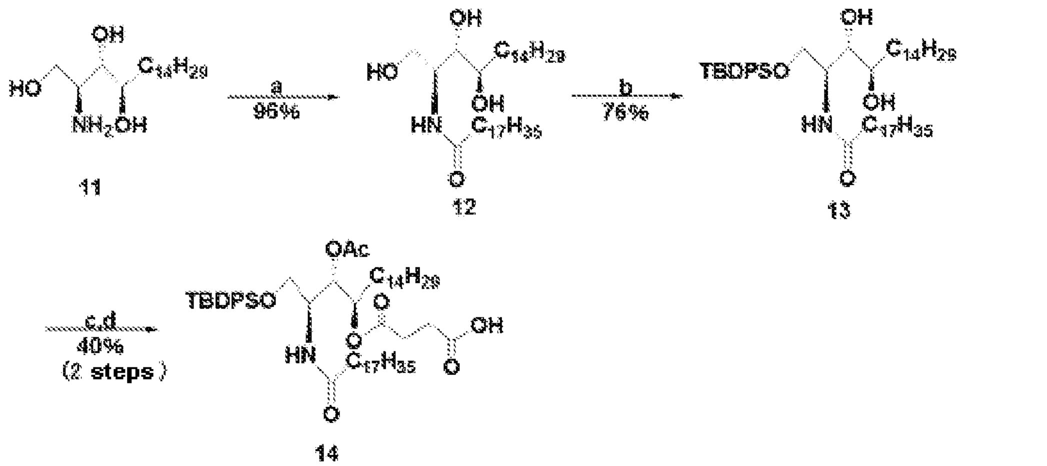


Reagents and conditions : (a) stearic acid , WSC, $CH_2Cl_2$, r.t. (b) TBDPSCl, DMAP, TEA, $CH_2Cl_2$, r.t.
(c) succinic anhydride , DMAP, Pyr., r.t.→40°C (d) $Ac_2O$

(Preparation example 2 of Ceramide)

In the above-described preparation 1 of the ceramide acceptor (Scheme 5), the problems regarding the selectivity of the 3- and 4-positions, and the efficiency of the synthesis remained. However, the introduction of 1, 3-benzylidene was examined, and consequently the problems were diminished. In the past, BDA and p-TsOH worked on the protection with benzylidene group at 1- and 3-positions of a ceramide in acetonitrile solvent at room temperature. Here, focusing on the fact that benzylidene group forms a six-membered ring at 1- and 3-positions of a ceramide, and forms a five-membered ring at 3- and 4-positions, the reaction was performed by heating to 40 degrees Celsius. Because of that, the benzylidene group preferentially formed a thermodynamically stable six-membered ring structure, the improvement of the yield of the protection with 1,3-benzylidene group was attained. After that, in pyridine (pyr.) solvent at 40 degrees Celsius, succinic anhydride and DMAP worked on Compound 15 to introduce succinic acid to the 4-position. Compound 16 has a carboxyl group and thus is extremely polar. Therefore, purification thereof was extremely difficult. Accordingly, after the carboxyl group was protected with theBn group, purification was performed. Then, using 80% acetic acid solution for Compound 17, removal of benzylidene group was performed by deprotection. Here, compounds in which succinic acid transferred to the hydroxyl groups at 1- or 3- position where benzylidene group was removed by deprotection, were each identified. It is thought that, under acidic condition, a reaction temperature of 60 degrees Celsius promoted the transfer. Then, in dichloromethane solvent at 40 degrees Celsius, TBDPSC1, TEA, and DMAP worked on Compound 18 to selectively protect the hydroxyl group at 1-position of the ceramide. Then, the 3-position of the ceramide was protected with an Ac group to give Compound 20. At the end, the hydrogenation gave a ceramide acceptor (Scheme 6).

(Scheme 6)

[Chem. 103]

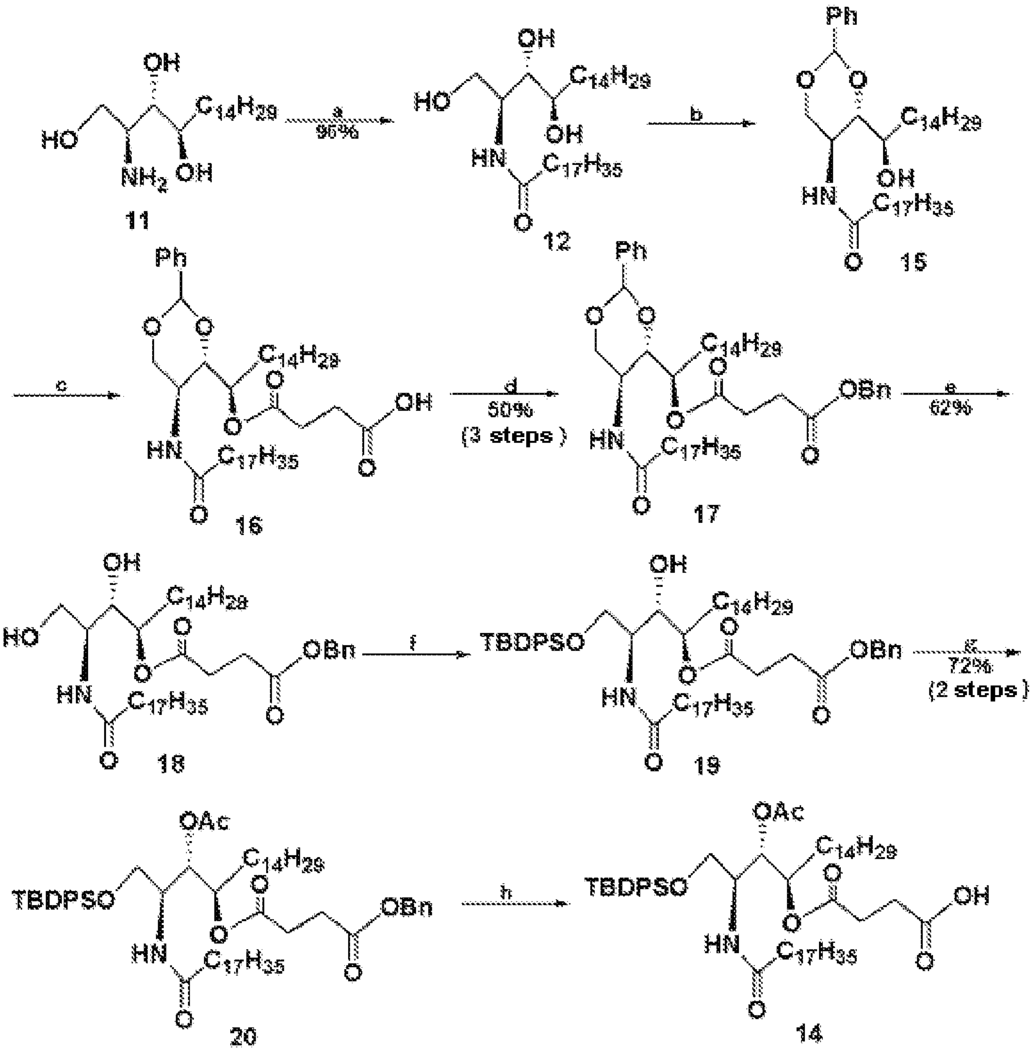


**Reagents and conditions** : (a) **stearic acid** , WSC, $CH_2Cl_2$, r.t. (b) BDA, *p*-TsOH, MeCN, 40°C
(c) **succinic anhydride** , DMAP, Pyr., 40°C (d) BnBr, $K_2CO_3$, MeCN / DMF = 1 / 1, r.t.
(e) 80%-AcOH aq, 60°C (f) TBDPSCl, TEA, DMAP, $CH_2Cl_2$, 40°C (g) $Ac_2O$, pyr., r.t.
(h) $H_2$, $Pd(OH)_2$, EtOH, 40°C

(Synthesis example of a glucosyl ceramide acceptor)

(Condensation of the glucose donor and ceramide)

As mentioned above, the ceramide derivative having succinic acid at 3-position of the ceramide, which was originally expected, was not yielded, but the ceramide derivative having succinic acid at 4-position was yielded. However, it is thought that it will be sufficiently informative for intramolecular glycosylations that form a macrocycle with Glc, and for the development of a new glucosyl ceramide acceptor, and thus it was determined that the ceramide derivative having succinic acid at 4-position that was yielded was used as it was.

(Condensation of glucose(2-Bz 4-CA) and ceramide)

Using the glucose donor 8 and ceramide acceptor 14 previously prepared, in dichloromethane solvent at room temperature, 2,4,6-trichlorobenzoylchloride, TEA and DMAP worked on them to bond glucose with the ceramide via succinic acid with a 75% yield (Scheme 7). A condensation product of the ceramide and the hydroxyl group at 4-position

of the glucose, which was a concern, was not created. Then,the 4-position of Glc was protected with the chloroacetyl group. Then, in deprotection, removal of the TBDPS group at 1-position of the ceramide was performed. In entry 1, in THF solvent at room temperature, the deprotection was performed under the condition in which 2 equivalents of TBAF worked (Table 3). 3 hours after the reaction started, the starting material was all consumed. However, at nearly 60 percent, the compound in which the acetyl group at 3-position of the ceramide was transferred to the 1-position, and the chloroacetyl group at 4-position of Glc left, was created. Thus, in the condition of entry 2, 7.5 equivalents of acetic acid, which buffers TBAF, was added, and then 1. 5 equivalents of TBAF worked. In this case, it took 18 hours to complete the reaction. However, a by-product was not observed, the yield was as high as 92%. In entry 3, after the scale was slightly increased, the reaction was performed under the same condition as entry 2. However, even after 18 hours passed, the reaction was not completed. Thus, 0.5 equivalents of TBAF were added. Additionally, the reaction was performed for another 18 hours, and then a large percentage of by-product in which the CA group at Glc 4-position was removed by deprotection, was created. As a result, the yield of the target compound was 36%. Detailed causes by which the reproducibility was not obtained here cannot be mentioned. However, 7.5 equivalents of acetic acid relative to 1.5 equivalents of TBAF were used, and thus the progress of the reaction was slow. It is therefore thought, accompanying the scale-up, such a tendency was more strongly developed.

(Scheme 7)

[Chem. 104]

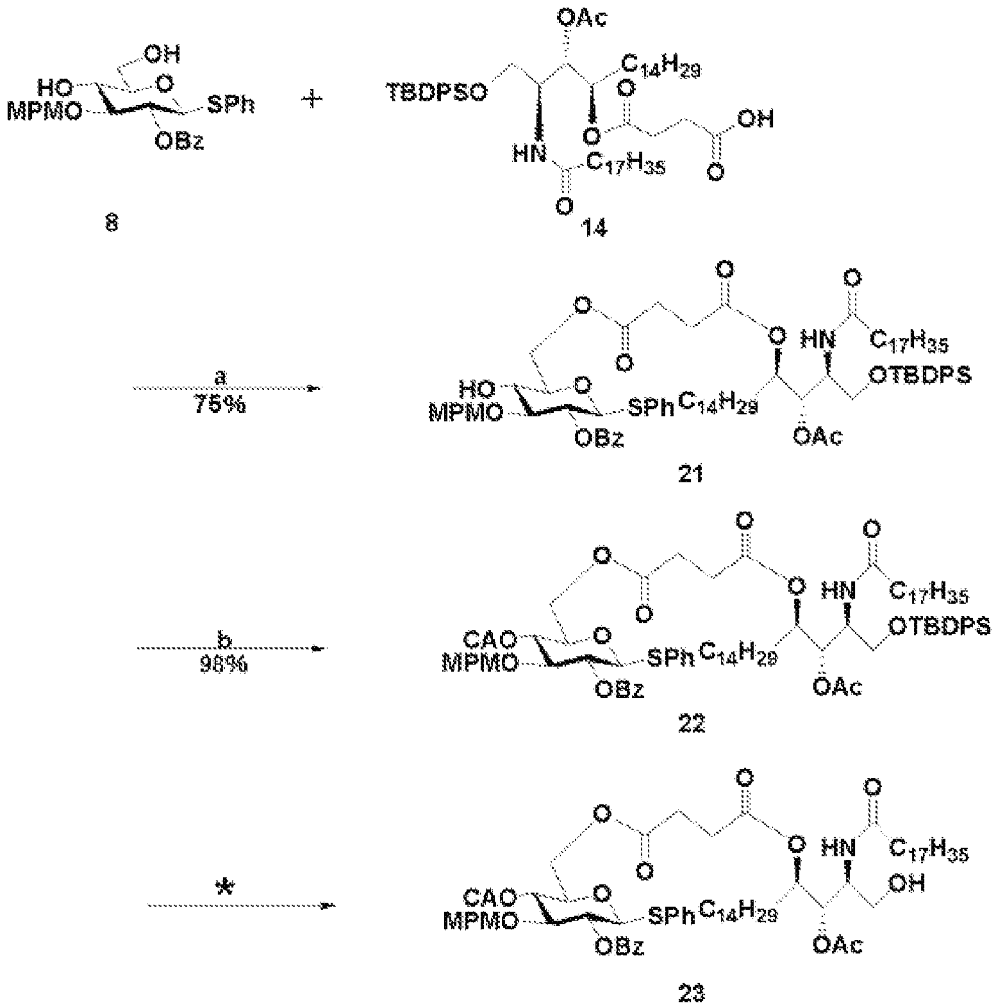


8

14

21

22

23

Reagents and conditions: (a) 2,4,6- trichlorobenzoylchloride, TEA, DMAP, $CH_2Cl_2$, r.t.
(b) $ClAc_2O$, TEA, $CH_2Cl_2$, r.t.

(Removal of TBDPS group at 1-position of ceramide by deprotection)

[Table 3]

| * entry | Reagents | | Solvent | Temperature | Time | Yield |
|---|---|---|---|---|---|---|
| | TBAF(eq.) | AcOH(eq.) | | (˚C) | (h) | (%) |
| 1 | 2.0 | - | THF | r.t. | 3 | 32 |
| 2 | 1.5 | 7.5 | THF | r.t. | 18 | 92 |
| 3 | 1.5+0.5 | 7.5 | THF | r.t. | 36 | 36 |

(Condensation example of the glucose (2-MPM 4-CA) and ceramide)

In dichloromethane solvent at room temperature, WSC and DMAP worked on the glucose donor 10 and ceramide acceptor 14 to give Compound 24 with a 61% yield for 2 steps. Then, the chloroacetylation of the Glc 4-position was performed with a 95% yield. Then, in deprotection, removal of TBDPS group at 1-position of the ceramide was performed (Scheme 8). In the above-described removal of the TBDPS group, it was found that the longer reaction time tends to increase the production of a by-product. Thus, in entry 1, in THF solvent at room temperature, each 1 equivalent of

acetic acid and TBAF is used, the reaction was performed (Table 4). 2 hours after the reaction started, the starting material was not all consumed. However, the reaction was quenched, and then products were checked. Mainly, two products were observed. One of them was target Compound 26 with a 22% yield. The by-product was slightly more polar than the target Compound, and the molecular weight thereof was 1342. This compound was analyzed by $^1$H-NMR, and consequently the result was almost identical to the $^1$H-NMR of the target compound. However, only the protons of the methylene ($-CH_2$) of CA group are different, and were downfield shifted. Because of that, it was expected that a compound of which the Cl of CA group was substituted with F was produced. However, a molecular weight obtained from the mass spectrum did not match that of an expected compound, and consequently it was not identified. Then, in entry 2, in order to suppress the transfer of the Ac group at 3-position and an influence on the CA group at 4-position of glucose, the reaction was performed under a low temperature. In THF solvent at 0 degree Celsius, the reaction was performed using 1 equivalent of acetic acid and 1 equivalent of TBAF. On TLC, it appeared that the reaction proceeded with a relatively high yield. However, a by-product in which the Ac group at 3-position of the ceramide transferred to 1-position of the ceramide was also yielded. It is expected that this by-product not only was produced during the reaction, but also increased during purification by acidity of silica gel chromatography. As a result of setting the reaction temperature at a low temperature, the transfer of the Ac group at 3-position was more or less suppressed.

(Scheme 8)

[Chem. 105]

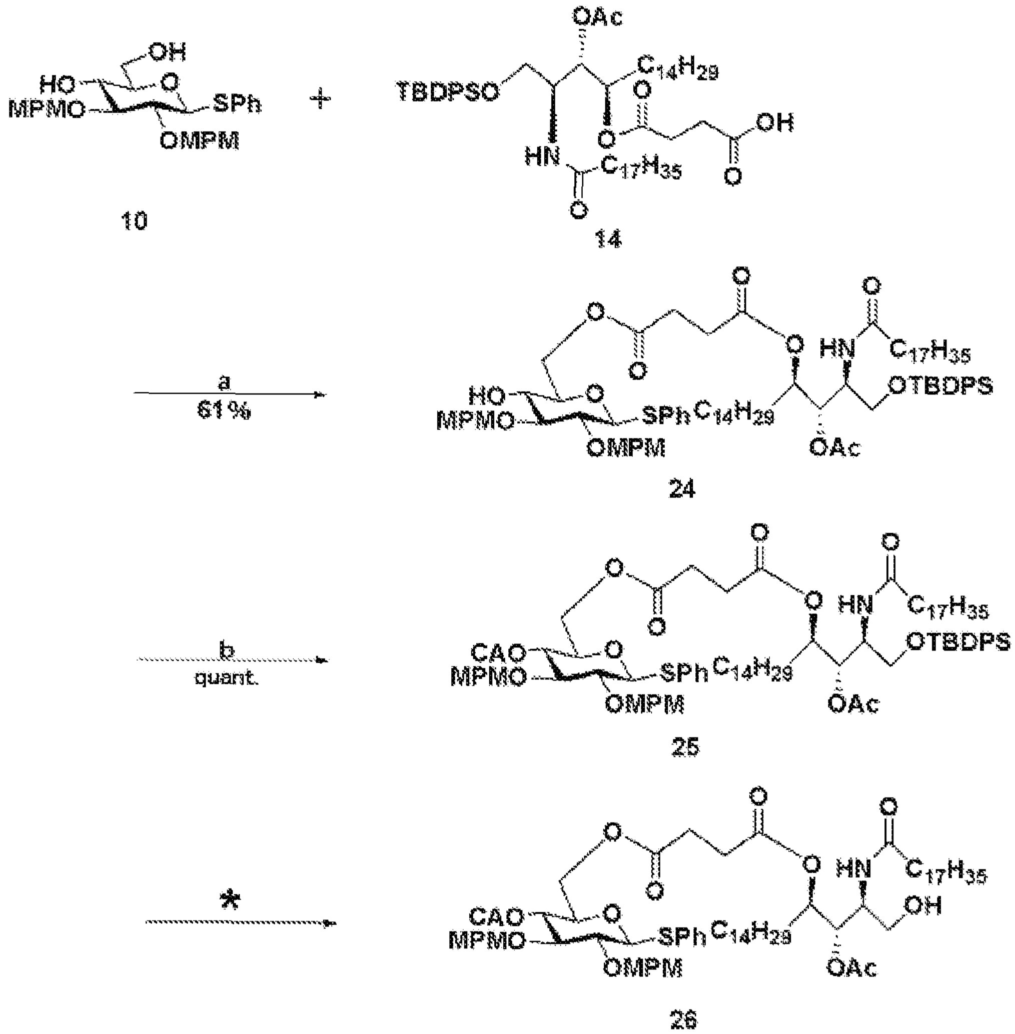

10
14
24
25
26

Reagents and conditions : (a) WSC, DMAP, $CH_2Cl_2$, r.t. (b) $ClAc_2O$, TEA, $CH_2Cl_2$, r.t.

(Table 4 Removal of the TBDPS group at 1-position of ceramide by deprotection)
[Table 4]

| * entry | Reagents | | Solvent | Temperature | Time | Yield |
|---|---|---|---|---|---|---|
| | TBAF(eq.) | AcOH(eq.) | | (˚C) | (h) | (%) |
| 1 | 1.0 | 1.0 | THF | r.t. | 2 | 22 |
| 2 | 1.0 | 1.0 | THF | 0 | 3 | 58 |

(Condensation of glucose(2-Bz 4-OH) and ceramide)

In the above, prior to an intramolecular glycosylation, the 4-position of glucose was protected with the CA group. However, in view of efficiency, its need was examined. The presence of the CA group caused various side reactions when the TBDPS group at 1-position of the ceramide was removed, and thus that was the problem. Then, the following differences were examined: the difference of reactivity between the primary hydroxyl group at 1-position of the

ceramide and the secondary hydroxyl group at 4-position of glucose; the difference of steric circumstance when succinic acid crosslinked; and the difference between reactivities in an intermolecular reaction and an intramolecular reaction. As a result, even in the case where the hydroxyl group at 4-position of glucose is not protected, it is expected that an intended intramolecular glycosylation is attained. Thus, it was determined to perform an intramolecular glycosylation without protecting the 4-position of glucose (Scheme 9).

In solvent at room temperature, WSC and DMAP worked on the glucose donor 8 and ceramide acceptor 14 to give Compound 21 with a 68% yield for 2 steps. Then, by deprotection, removal of the TBDPS group at 1-position of the ceramide was performed. As mentioned above, it became clear that, by removing TBDPS under a low-temperature condition, the transfer of the Ac group at 3-position of the ceramide to 1-position is suppressed. Accordingly, the reaction temperature was set at 0 degree Celsius. Compound 21 was reacted in THF solvent at 0 degree Celsius with 3.0 equivalents of acetic acid and 3.0 equivalents of TBAF. After 12 hours, the reaction was quenched, and then purified using silica gel column chromatography to give a target compound with a 75% yield. In this reaction condition, a compound of which the Ac group at 3-position of the ceramide was transferred to 1-position was mostly not observed.

(Scheme 9)

[Chem. 106]

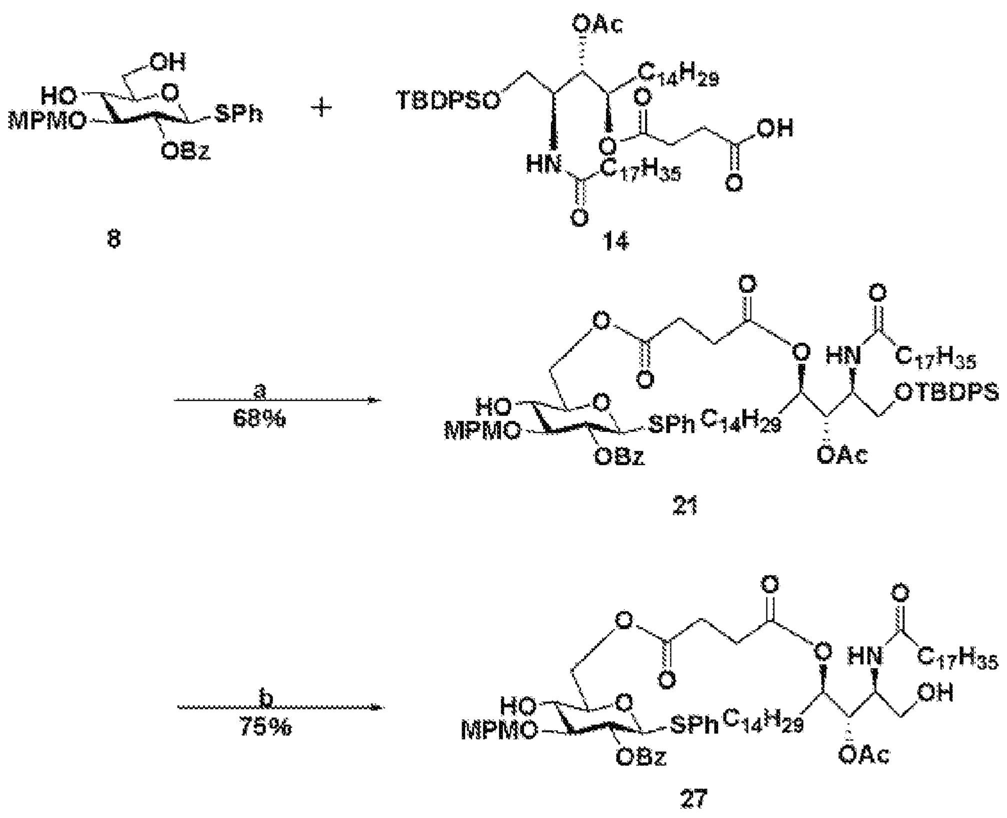


8

14

21

27

Reagents and conditions : (a) WSC, DMAP, $CH_2Cl_2$, r.t. (b) TBAF, AcOH, THF, 0°C

(Condensation example of glucose(2-MPM 4-OH) and ceramide)

In solvent at room temperature, WSC and DMAP worked on the glucose donor 10 and ceramide acceptor 14 to give Compound 24 with a 61% yield for 2 steps. Then, by deprotection, removal of the TBDPS group at 1-position of the ceramide was performed. In THF solvent at 0 degree Celsius, the reaction using 1 equivalent of acetic acid and 1

equivalent of TBAF was performed. On TLC, it appeared that the reaction proceeded with a relatively high yield. However, the yield was 58%. As a by-product, a by-product in which the Ac group at 3-position of the ceramide transferred to 1-position of the ceramide was also yielded. It is expected that this by-product not only was produced during the reaction, but also increased during purification by acidity of silica gel chromatography (Scheme 10).

(Scheme 10)

[Chem. 107]

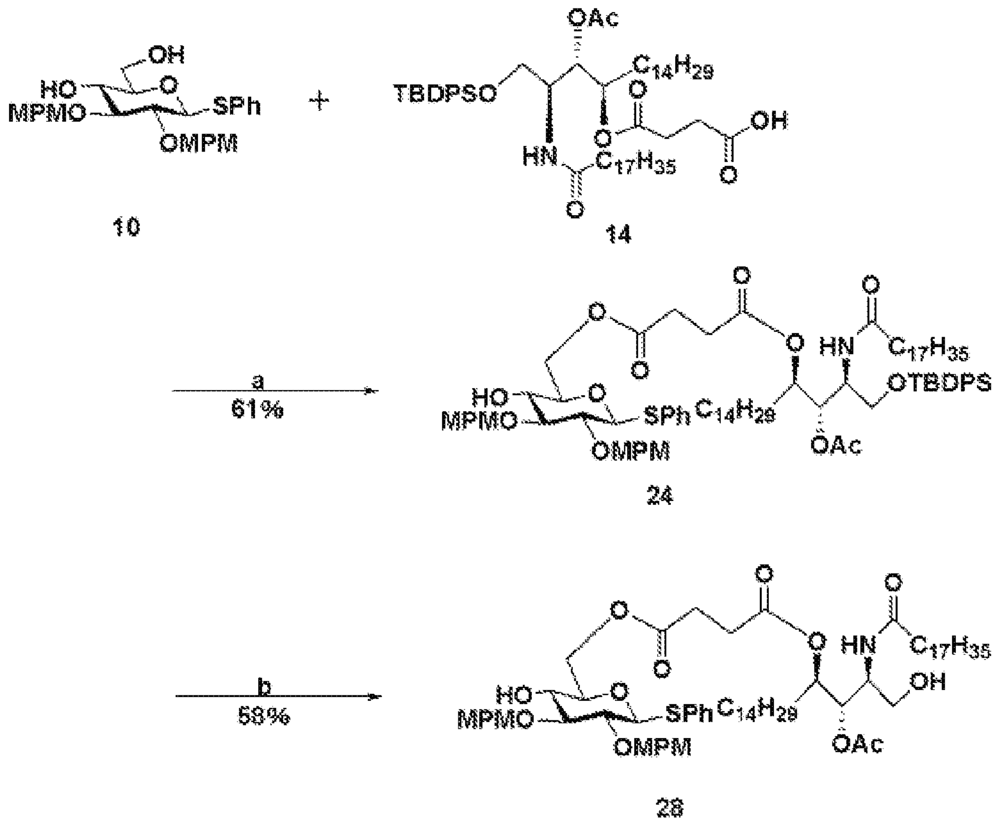


Reagents and conditions : (a) WSC, DMAP, $CH_2Cl_2$, r.t. (b) TBAF, AcOH, THF, 0°C

(Example of a intramolecular glycosylation)

(Intramolecularglycosylationofglucose(2-Bz4-CA) and ceramide)

Compound 23 was reacted in dichloromethane solvent at 0 degree Celsius with NIS and TfOH as activating agents, and then the reaction was completed in 6 hours to give target Compound 29 with a 60% yield. In the $^1$H-NMR, the coupling constant between protons of the 1- and 2-positions of glucose was 7.813, it was confirmed to be a β body. Because of the Bz group at 2-position of glucose, a completely β-selective glycosylation proceeded. In order to obtain reproducibility, the intramolecular glycosylation was performed again under the same conditions. Consequently, the reaction was completed in 3 hours and the yield was 69% (the following formula).

(Intramolecular glycosylation)

[Chem. 108]

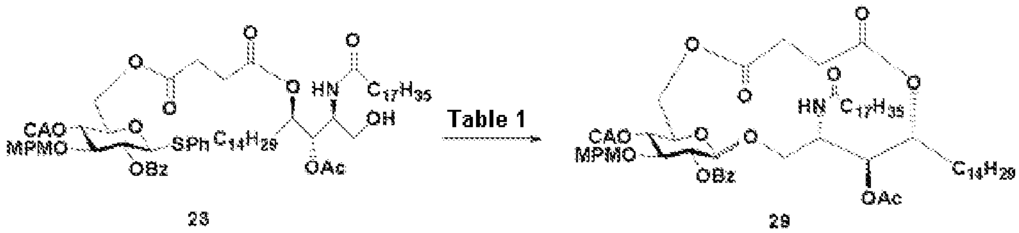


28 29

Table 1

| entry | Reagent (eq.) | Solvent | Temperature (°C) | Time (h) | Yield (%) | (α/β)*1 |
|---|---|---|---|---|---|---|
| 1 | NIS(2.0), TfOH(0.2), MS4 Å | $CH_2Cl_2$ | 0 | 6 | 60 | (β only) |
| 2 | NIS(2.0), TfOH(0.2), MS4 Å | $CH_2Cl_2$ | 0 | 3 | 69 | (β only) |

*1: Determined by $^1$H NMR spectrum

(Example of the intramolecular glycosylation of glucose(2-MPM 4-CA) and ceramide)

Compound 26 was reacted in dichloromethane solvent at 0 degree Celsius with 2 equivalents of NIS and 0.2 equivalents of TfOH as activating agents. It was a compound of which the reactivity was expected as an armed sugar, but the progress of the reaction was not observed. After 2 hours, 0.2 equivalents of TfOH were added, and then the temperature was warmed up to room temperature. However, a slight amount of lactol was merely produced.

(Intramolecular glycosylation)

[Chem. 109]

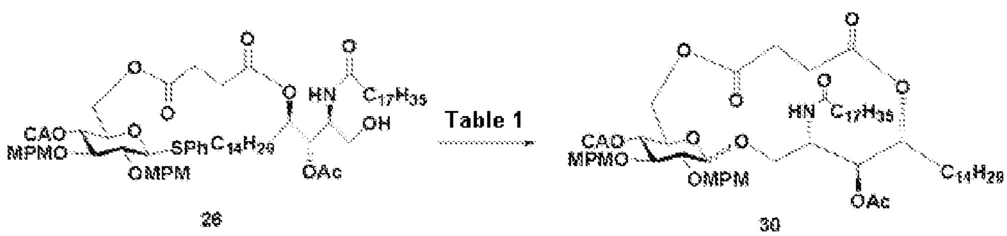


26 30

Table 1

| entry | Reagent (eq.) | Solvent | Temperature (°C) | Time (h) | Yield (%) | (α/β) |
|---|---|---|---|---|---|---|
| 1 | NIS(2.0), TfOH(0.2), MS4 Å | $CH_2Cl_2$ | 0→r.t. | 6 | - | |

(Example of the intramolecular glycosylation of glucose(2-Bz 4-OH) and ceramide)

Compound 27 was reacted in dichloromethane solvent at 0 degree Celsius with 2.0 equivalents of NIS and 0.3 equivalents of TfOH as activating agents. This reaction was completed in 5 hours to give target Compound 31 with an 85% yield. From the $^1$H-NMR, the coupling constant between GlcH-1 and GlcH-2 was 7.8 Hz, and thus it was confirmed to be a β body. Subsequently, Compound 27 was reacted in dichloromethane solvent at -20 degrees Celsius with 3.0 equivalents of NIS and 0.3 equivalents of TfOH as activating agents (entry 2) to give target Compound 31 with a 85% yield. In the $^1$H-NMR, the coupling constant between GlcH-1 and GlcH-2 was 7.8 Hz, and thus it was confirmed to be a β body. In entry 3, in dichloromethane solvent at 0 degree Celsius, the reaction was performed with 1.5 equivalents

of DMTST as an activating agent, and was completed in 2 hours to give target Compound 31 with a 75% yield. In the $^1$H-NMR, the coupling constant between GlcH-1 and GlcH-2 was 7.8 Hz, and thus it was confirmed to be a β body. In the system using DMTST as an activating agent (entry 3), the reaction proceeded. Therefore, as a result, although a sphingosine-type ceramide has an unsaturated bond and thus NIS cannot be used as an activating agent for the sphingosine-type ceramide, it can be expected to apply the present method to a sphingosine-type ceramide. In all of entry 1-3, by the neighboring group participation of the Bz group at 2-position of glucose, the target compound was yielded β-selectively. However, in all reactions of entry 1-3, the presence of a slight amount of a by-product was observed. While this compound had the same molecular weight as the target compound, a difference appeared on the $^1$H-NMR spectrum. GlcH-2 was upfield shifted in spite of the presence of the Bz group, and GlcH-3 and GlcH-5 were slightly downfield shifted in comparison to those of the target compound. Additionally, GlcH-4 remained free, but GlcH-6 was downfield shifted. Moreover, the coupling constant between GlcH-1 and GlcH-2 was 10.9 Hz. From the above points, the by-product was expected to be an orthoester. Because of the production of the orthoester, the carbonyl group of the Bz group at 2-position disappeared, and thus it is expected that the electron-withdrawing property decreased. Furthermore, it is guessed that the downfield shifts of GlcH-3, GlcH-5, and GlcH-6 are caused by strain of the pyranose ring by the crosslink of succinic acid. In the case where the orthoester was produced, the coupling constant between GlcH-1 and GlcH-2 was 10. 9Hz, which was slightlylarge. However, it is expected that these are also affected by the crosslink of succinic acid.

(Intramolecular glycosylation)

[Chem. 110]

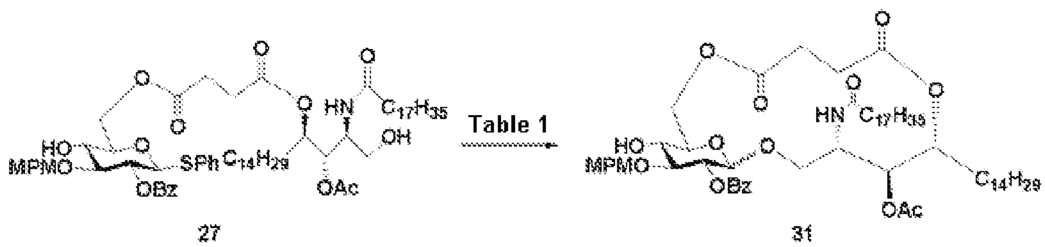


27 31

Table 1

| entry | Reagent (eq.) | Solvent | Temperature (°C) | Time (h) | Yield (%) | ($\alpha/\beta$)*1 |
|---|---|---|---|---|---|---|
| 1 | NIS(2.0), TfOH(0.3), MS4Å | $CH_2Cl_2$ | 0 | 5 | 85 | (β only) |
| 2 | NIS(3.0), TfOH(0.3), MS4Å | $CH_2Cl_2$ | -20 | 1.5 | 85 | (β only) |
| 3 | DMTST(1.5), MS4Å | $CH_2Cl_2$ | 0 | 2 | 75 | (β only) |

* 1: Determined by $^1$H NMR spectrum

(Example of the intramolecular glycosylation of glucose(2-MPM 4-OH) and ceramide)

First, Compound 28 was reacted in dichloromethane solvent at 0 degree Celsius with 2.0 equivalents of NIS and 0.2 equivalents of TfOH as activating agents. This reaction was completed in about 1 and half hours to give target Compound 32 with a 55% yield (the ratio of α/β is 1/1). Then, in entry 2, in dichloromethane solvent at -40 degrees Celsius, the reaction was performed with 2.0 equivalents of NIS and 0.2 equivalents of TfOH as activating agents. The reaction was completed in 5 hours to give target Compound 32 with a 74% yield (the ratio of α/β is 1/1). Then, in entry 3, in dichloromethane solvent at -80 degrees Celsius, the reaction was performed with 2.0 equivalents of NIS and 0.2 equivalents of TfOH as activating agents. In entry 3, the progress of the reaction was not observed, and thus 2.0 equivalents of NIS and 0.2 equivalents of TfOH were added, and then the reaction temperature was gradually warmed up to -60 degrees Celsius, -40 degrees Celsius, and then 0 degree Celsius. Although the reaction was performed for 36 hours, the starting material was not all consumed, and a small amount of a hemiacetal body as a by-product was observed. Then, in entry 4, in acetonitrile solvent at -40 degrees Celsius, the reaction was performed with 2.0 equivalents of NIS and 0.2 equivalents of TfOH as activating agents. The progress of the reaction was slow, and thus, after 35 hours, the reaction temperature was warmed up to 0 degree Celsius. The reaction was completed in 48 hours to give target

Compound 32 with a 28% yield (the ratio of α/β is 1/6.3). This time, a large amount of lactol was observed as a by-product. By a solvent effect of acetonitrile, in comparison with dichloromethane solvent, β-selectivity was improved. However, in acetonitrile solvent, probably, because the reaction temperature of -40 degrees Celsius was low, the progress of the reaction was slow. It is thought that a reaction using acetonitrile, which is a polar solvent, isusuallyaccelerated, and in combination with a nitrile solvent effect, β-selectivity is increased. However, it was considered that the reaction slowly proceeded and therefore the original β-selectivity was not exhibited. Then, in entry 5, in acetonitrile solvent at 0 degree Celsius, the reaction was performed with 3.0 equivalents of NIS and 0.3 equivalents of TfOH as activating agents. The reaction was completed in 1.5 hours to give target Compound 32 with a 81% yield (the ratio of α/β is 1/8.4). In this case, it is thought that the reaction rapidly proceeded, without proceeding via a β-coordinating counter anion, in combination with the solvent effect of acetonitrile, high β-selectivity was attained. Then, in entry 6, in acetonitrile solvent at -20 degrees Celsius, the reaction was performed with 3.0 equivalents of NIS and 0.3 equivalents of TfOH as activating agents. The reaction was finished in 3 hours to give target Compound 32 with a 47% yield (the ratio of α/β is β only). In comparison with entry 5 of 0 degree Celsius, the progress of the reaction was slow, and a hemiacetal body as a by-product was observed. In entry 7, in order to attain α-selectivity, in diethylether solvent at 0 degree Celsius, the reaction was performed with 3.0 equivalents of NIS and 0.3 equivalents of TfOH as activating agents. The progress of the reaction was slow, and thus, after 20 hours, the reaction temperature was warmed up to room temperature. The reaction was completed in 25 hours to give target Compound 32 with a 44% yield (the ratio of α/β is 1/1.9). When diethylether is used, usually, the reaction slowly proceeded, and, by *in situ* anomerization, a α-selective glycosylation occurs. However, probably, because the crosslink using succinic acid was inappropriate, outstanding α-selectivity was not observed. In entry 8, in acetonitrile solvent at 0 degree Celsius, the reaction was performed with 4.0 equivalents of DMTST as an activating agent. The reaction was completed in 1 hour to give target Compound 32 with a 76% yield (the ratio of α/β is 1/6). In the system using DMTST as an activating agent, the reaction proceeded. Therefore, as a result, although a sphingosine-type ceramide has an unsaturated bond and thus NIS cannot be used as an activating agent for the sphingosine-type ceramide, it can be expected to apply the present method to a sphingosine-type ceramide.

In an intramolecular glycosylation using a glucose donor having the MPM group at 2-position, high reactivity as an armed sugar, *i.e.*, a high yield was expected. However, in comparison with a glucose donor having the Bz group at 2-position, particularly remarkable improvement of the reactivity was not observed. Furthermore, by the crosslink using succinic acid, high stereoselectivity was expected. However, stereoselectivity by the crosslink was mostly not observed. As a result, only stereoselectivity by an effect of solvent such as acetonitrile and the like was attained. Accordingly, it became clear that the present invention can be practiced by appropriately designing as necessary on the basis of the above information

(Intramolecular glycosylation)

[Chem. 111]

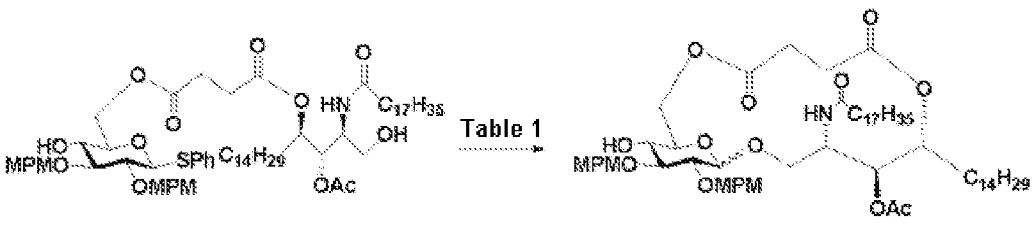


28

32

**Table 1**

| entry | Reagent (eq.) | Solvent | Temperature (°C) | Time (h) | Yield (%) | $(\alpha/\beta)$ *1 |
|---|---|---|---|---|---|---|
| 1 | NIS(2.0), TfOH(0.2), MS4 Å | $CH_2Cl_2$ | 0 | 1.5 | 55 | (1 / 1) |
| 2 | NIS(2.0), TfOH(0.2), MS4 Å | $CH_2Cl_2$ | -40 | 5 | 74 | (1 / 1) |
| 3 | NIS, TfOH, MS4 Å (2.0→4.0)(0.2→0.4) | $CH_2Cl_2$ | -80→-60→-40→0 | 36 | trace | ( - / - ) |
| 4 | NIS(2.0), TfOH(0.2), MS3 Å | MeCN | -40→0 | 48 | 28 | (1 / 6.3) |
| 5 | NIS(3.0), TfOH(0.3), MS3 Å | MeCN | 0 | 1.5 | 81 | (1 / 8.4) |
| 6 | NIS(3.0), TfOH(0.3), MS3 Å | MeCN | -20 | 3 | 47 | ($\beta$ only) |
| 7 | NIS(3.0), TfOH(0.3), MS4 Å | $Et_2O$ | 0→r.t. | 25 | 44 | (1 / 1.9) |
| 8 | DMTST(4.0), MS3 Å | MeCN | 0 | 1 | 76 | (1 / 6) |

*1: Determined by $^1$H NMR spectrum

(Synthesis examples of the GM3 analog)

(Condensation of the sialyl galactose donor and glucosyl ceramide acceptor)

With regard to the introduction of a ceramide using an intramolecular glycosylation, two kinds of synthesis strategies are proposed. One is a method in which a sugar chain is constructed and then an intramolecular glycosylation is used (the above formula: Method in which a sugar chain is constructed and then an intramolecular glycosylation is used). Furthermore, the other is a method in which, first, a ceramide is introduced to glucose to be the reducing terminal of a sugar chain by an intramolecular condensation via succinic acid and then a glucosyl ceramide acceptor is introduced to a sugar chain (the above formula: Method in which an intramolecular glycosylation is used to glucose and then glucosylceramide acceptor is utilized). It was stated that, in the above strategies, first, glucose, which was monosaccharide, was used, and was crosslinked with a ceramide, an intramolecular glycosylation was performed, and then, on the basis of the results, two strategies were each examined. Thus, first, it was determined to introduce a sugar chain (Neuα(2-3)Gal) using an obtained glucosyl ceramide acceptor of the second method of the synthesis strategies.

2. 5 equivalents of sialyl α(2-3)galactose donor 33, relative to the acceptor, and the glucosyl ceramide acceptor 32 are reacted in dichloromethane solvent at 0 degree Celsius with 0.02 equivalents of TMSOTf as an activating agent. The progress of the reaction did not become exhibited. Thus, after 2 hours, 0.02 equivalents of TMSOTf were added. After 7 hours, the reaction was completed and the yield was 72% (β only). In the $^1$H-NMR, it was confirmed that the coupling constant between GalH-1 and GalH-2 was 8.1 Hz, and it was a β body. As a by-product, a lactol of sialyl galactose was identified. When 2.5 equivalents of the sialyl galactose donor were used, despite of mild reactivity of the acceptor, since sufficient amount of the donor existed, the reaction was completed.

1.2 equivalents of sialyl α(2-3) galactose donor 33, relative to the acceptor, and the glucosyl ceramide acceptor 32 were reacted in dichloromethane solvent at 0 degree Celsius with 0.02 equivalents of TMSOTf as an activating agent. After 4 hours, 0.02 equivalents of TMSOTf were added. After 6 hours, the reaction was completed, and the yield was 18% (β only). The acceptor was not all consumed, and the donor was converted to a lactol. As a by-product, a lactol of sialyl galactose and a compound in which the Bz group at 2-position of galactose of sialyl galactose transferred to 1-position were observed. In the $^1$H-NMR, it was confirmed that the coupling constant between GalH-1 and GalH-2 was 8.1Hz, and it was a β body. When 1. 2 equivalents of the sialyl galactose donor were used, before an attack of the acceptor occurred, the donor was converted to a lactol and thus the reaction was not completed. Based on the above result, it is difficult to say that the glucosyl ceramide acceptor has high reactivity. However, in entry 1 in which a sufficient amount of the donor was used, a glycosylation with a high yield was attained. Furthermore, in entry 2, an unreacted

acceptor can be retrieved and therefore the stability of the acceptor is regarded to be high. Accordingly, it is thought that, by converting an imidate, which is very unstable as a leaving group of a donor, to another stable leaving group, improvement of a yield of a glycosylation could be expected.

(NeuGal donor + GlcCer acceptor)

[Chem. 112]

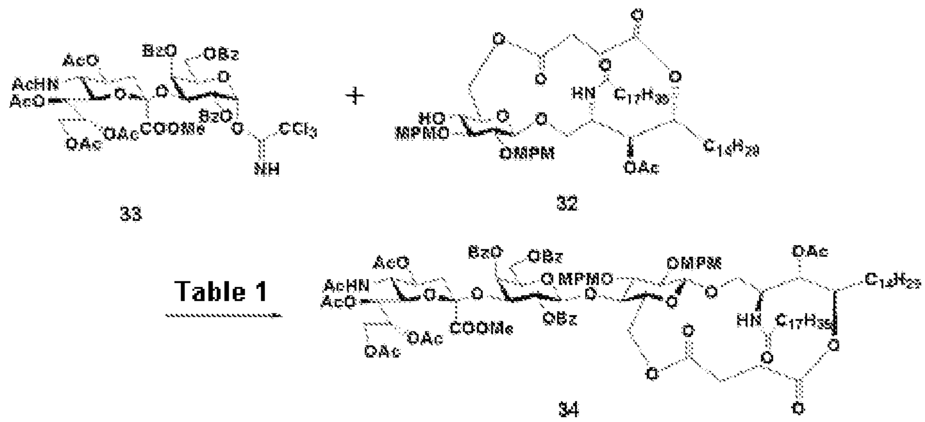


33 32 34

**Table 1**

| entry | Donor (eq. of donor) | Reagent (eq. of acceptor) | **Solvent** | **Temp.** (˚C) | **Time** (h) | **Yield** (%) |
|---|---|---|---|---|---|---|
| 1 | 2.5 | TMSOTf(0.02-0.04, AW300 | $CH_2Cl_2$ | 0 | 7 | 72(β) |
| 2 | 1.2 | TMSOTf(0.02-0.04), AW300 | $CH_2Cl_2$ | 0 | 6 | 18(β) |

(Example of deprotection)

[Chem. 113]

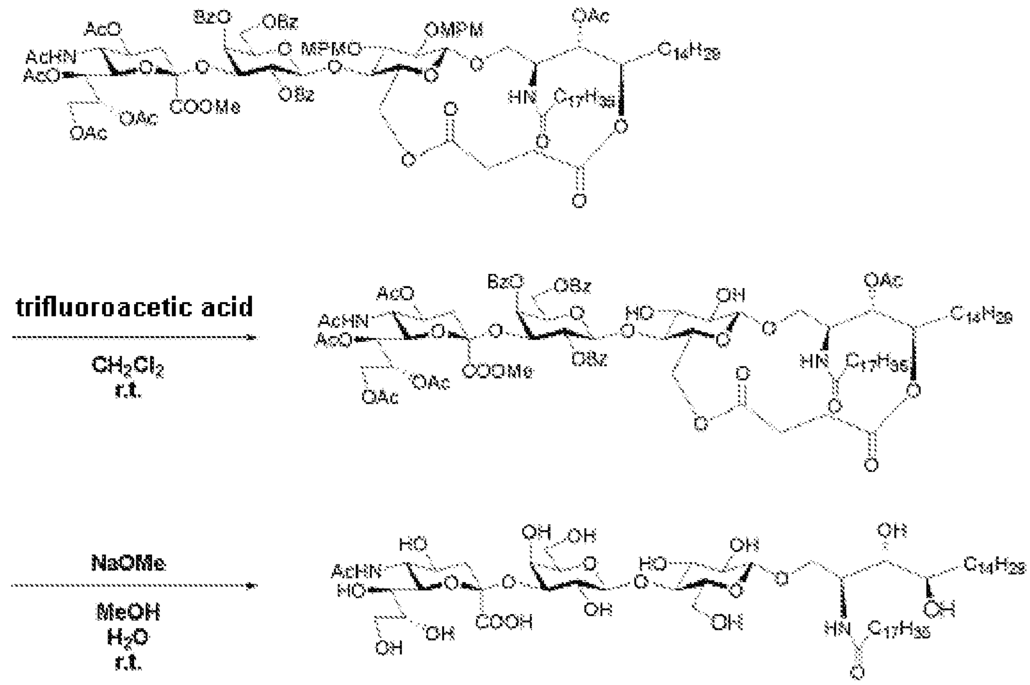


(Example 1. General procedure)

$^1$H and $^{13}$C NMR spectra were measured by Varian INOVA 400 and 500. Chemical shifts are shown as ppm (δ) that is relative to a signal of $Me_4Si$ that is adjusted to be δ 0 ppm. MALDI-TOF MS spectra were recorded in a presumed ion format in the Bruker Autoflex using α-cyano-4-hydroxycinnamic acid (CHCA) as a matrix. Molecular sieves were purchased from Wako Chemicals Inc., and before use, they were dried in a muffle furnace at 300 degrees Celsius for 2 hours. Solvents as reaction medium were dried with molecular sieves, and then were used without purification. TLC analyses were performed on Merck TLC (silica gel 60 $F_{254}$ on a glass plate) . Silica gel (80 mesh and 300 mesh), manufactured by Fuji Silysia Co., was used in flash column chromatography. An amount of silica gel was usually estimated to be 200-400 times the weight of the sample to be filled. Solvent system in column chromatography was described in v/v. Evaporation and concentration was performed under reduced pressure. Specific rotatory power was measured by the Horiba SEPA-300 high-sensitive polarimeter.

(Preparation example of the glucose donor (2-Bz))

First, $Ac_2O$ and pyridine (pyr.) worked on Compound 1 at 45 degrees Celsius to quantitatively yield Compound 2. Then, to protect the anomeric position of glucose with the SPh group, under argon atmosphere, in $CH_2Cl_2$ solvent at room temperature, PhSH and $BF_3$•$OEt_2$ worked on Compound 2 to give Compound 3 with a 67% yield. Then, under argon atmosphere, in MeOH solvent at room temperature, MeONa worked on Compound 3 to remove the Ac groups at 2-, 3-, 4-, and 6-positions by deprotection, consequently giving Compound 4 with a 93% yield. Then, under argon atmosphere, MeCN solvent at room temperature, BDA and *p*-TsOH worked on Compound 4 to give Compound 5 with a 93% yield. To determine the structure of the product, Compound 5 was acetylated, and then the $^1$H-NMR spectrum thereof was checked. Signals δ (ppm) 3.4(m, 1H) of the proton at 2-position and 3.8(m, 1H) of the proton at 3-position of Compound 5 exhibited downfield shifts to 5.0(t, 1H) and 5.3-5.4 (t, 1H), respectively. Based on that, the introduction of the benzylidene group to 4- and 6-positions was confirmed.

Next, toluene solvent under reflux, DBTO, TBAB, and MPMCl worked on Compound 5 to give Compound 6 with a 77% yield. It is said that $Bu_2SnO$ forms a cyclic stannylene compound, and then first activates *cis*-glycols, and subsequently a reaction to equatorial hydroxyl groups among them easily occurs. Compound 5 does not have a *cis*-glycol, and the 4- and 6-positions are protected with the benzylidene group, and therefore it was thought that, a stannylene compound was formed at 2- and 3-positions, and then protection with MPM proceeded at the 3-position selectively. To

determine the structure of the product, Compound 6 was acetylated and then the $^1H$-NMR spectrum thereof was checked. Signal δ (ppm) 3.5(m, 1H) of the proton at 2-position of Compound 6 was downfield shifted to 5.2-5.3 (t, 1H). Based on that, it is confirmed that the 2-position was acetylated, in other words, the MPM group was introduced to the 3-position.

(Preparation of Phenyl 2-0-benzoyl-4,6-0-benzylidene -1-deoxy-3-O-p-methoxybenzyl-1-thio-β-D-glucopyranoside (7))

To a solution of Compound 6 (171 mg, 0.356 mmol) in pyridine (3.56 mL) was added benzoylchloride (62.0 μL, 0.534 mmol) and DMAP (4.35 mg, 0.036 mmol). Then, the mixture was stirred at room temperature for 4 hours. By TLC analysis (toluene/MeOH 50/1), the starting material was confirmed to be completely consumed. To this reaction mixture was added methanol at 0 degree Celsius, and then was co-evaporated with toluene. This mixture was diluted with $CHCl_3$, washed with 2 M HCl, $H_2O$, saturated aqueous $NaHCO_3$, and brine, dried ($Na_2SO_4$), and then concentrated. The residue was purified by silica gel column chromatography ($CHCl_3$) to yield 7 (166 mg, 80%)

[number 1-1]

$$[\alpha]_D = +47.3^\circ \quad (c\ 1.0, CHCl_3)$$

$^1H$ NMR (400 MHz, $CDCl_3$):

δ 6.55-8.01 (m, 19 H, 4 Ph)
5.60 (s, 1 H, PhC*H*)
5.26 (near t, 1 H, $J_{1,2}$ = 9.9 Hz, H-2)
4.84 (d, 1 H, $J_{1,2}$ = 9.9 Hz, H-1)
4.73 (d, 1 H, $J_{gem}$ = 11.7 Hz, $PhCH_2$)
4.59 (d, 1 H, $J_{gem}$ = 11.7 Hz, $PhCH_2$)
4.41 (t, 1 H, H-4)

[number 1-2]

3.68-3.89 (m, 3 H, H-6, H-6', H-3)
3.68 (s, 3 H, OMe)
3.56 (m, 1 H, H-5)
$^{13}C$ NMR (100 MHz, $CDCl_3$):

δ 164.9, 159.1, 137.2, 133.1, 132.9, 132.2, 129.9, 129.8, 129.7, 129.0, 128.9, 128.3, 128.2, 128.1, 126.0, 113.5, 101.2, 87.0, 81.4, 78.7, 73.8, 72.0, 70.6, 68.6, 55.0.

MALDI-TOF-MS: $m/z$ = 607 $[M+Na]^+$.

(Preparation of phenyl 2-O-benzoyl-1-deoxy-3-O-p-methoxybenzyl-1-thio-β-D-gluc opyranoside (8))

To a solution of Compound 7 (200 mg, 0.330 mmol) in AcOH (15.0 mL) was added $H_2O$ (3.00 mL). After stirring at 40 degrees Celsius for 12 hours (TLCmonitoring: toluene/EtOAc 1/1), this mixture was diluted with $CHCl_3$, washed with $H_2O$, saturated aqueous $NaHCO_3$, and brine, dried ($Na_2SO_4$), and then concentrated. This residue was purified by silica gel chromatography (toluene/EtOAc 3/1) to yield 8 (145 mg, 86%).

[number 2-1]

$$[\alpha]_D = +0.35^\circ \quad (c\ 1.0, CHCl_3)$$

$^1H$ NMR (400 MHz, $CDCl_3$):

δ 6.60-8.10 (m, 14 H, 3 Ph)
5.23 (t, 1 H, $J_{1,2}$ = 9.9 Hz, H-2)
4.83 (d, 1 H, $J_{1,2}$ = 9.9 Hz, H-1)
4.63 (d, 1 H, $J_{gem}$ = 11.7 Hz, $PhCH_2$)
4.59 (d, 1 H, $J_{gem}$ = 11,7 Hz, $PhCH_2$)
3.90 (t, 1 H, H-6')
3.83 (dd, 1 H, H-6)
3.74 (m, 1 H, H-4)
3.70 (m, 1 H, H-3)
3.65 (s, 3 H, OMe)
3.44 (m, 1 H, H-5)
3.42 (d, 1 H, 4-OH)
2.77 (t, 1 H, 6-OH)

[number 2-2]

$^{13}C$ NMR (100 MHz, $CDCl_3$):

δ 165.2, 159.1, 133.2, 132.8, 132.0, 129.8, 129.6, 129.6, 128.8, 128.3, 127.8, 113.7, 86.3, 83.1, 79.5, 74.3, 72.2, 70.2, 62.3, 55.0.

MALDI-TOF-MS: $m/z$ = 519 $[M+Na]^+$.

(Preparation of phenyl 4,6-0-benzylidene -1-deoxy-2,3-di-O-p-methoxybenzyl-1-thio-β-D-glucopyran oside (9))

Based on the description of Hiromune Ando, Yusuke Koike, Hideharu Ishida, Makoto Kiso, et al., Tetrahedron Letters 44 (2003) 6883-6886, the following experiments were performed.

For example, a solution of Compound 7 in MeOH/THF=2/1 containing MeONa was stirred at room temperature to yield Compound 7a.

To a solution of Compound 7a (4.11 g, 8.56 mmol) in DMF (85.6 mL) was added NaH (307 mg, 12.8 mmol). After stirring at room temperature for 1 hour, at 0 degree Celsius, to this mixture was added MPMCl (1.74 mL, 12.5 mmol). This mixture was stirred at room temperature for 12 hours. By TLC analysis (toluene/EtOAc 20/1), the starting material was confirmed to be completely consumed. This mixture was diluted with EtOAc, washedwith2MHCl, $H_2O$, saturated aqueous $NaHCO_3$, and brine, dried ($Na_2SO_4$), and then concentrated. This residue was purified by silica gel chromatography (toluene/EtOAc 200/1) to yield 9 (3.60 g, 70%).

[number 3]

$$[\alpha]_D = -9.3^\circ \quad (c\ 1.2, CHCl_3)$$

$^1H$ NMR (400 MHz, $CDCl_3$):

δ 6.80-7.60 (m, 18 H, 4 Ph)
5.58 (s, 1 H, PhC*H*)
4.70→4.87 (m, 5 H, 2 $PhCH_2$,H-1)
4.37 (near t, 1 H, H-4)
3.81 and 3.79 (2 s, 6 H, 2 OMe)
3.77-3.82 (m, 2 H, H-6', H-3)
3.67 (t, 1 H, H-6)
3.45-3.50 (m, 2 H, H-5, H-2)

$^{13}C$ NMR (100 MHz, $CDCl_3$):

δ 159.4, 159.3, 137.3, 133.2, 132.3, 130.3, 130.0, 130.0, 129.0, 129.0, 128.2, 127.8, 126.0, 113.8, 113.8, 101.1, 88.3, 82.7, 81.5, 80.2, 75.5, 75.0, 70.3, 68.7, 55.3, 55.3.

MALDI-TOF-MS: $m/z$ = 623 [$M$+Na]$^+$.

(Preparation of the glucose donor 10 (2-MPM))

Using a similar method to that in the preparation of Compound 8, the following experiments were performed. For example, 83% acetic acid worked on Compound 9 at 30 degrees Celsius to prepare the glucose donor 10 (2-MPM) with a 86% yield.

(Preparation of (2S,3S,4R)-1-O-tert-butyldiphenylsilyl-2-octadecanamide -octadecane (13))

To a solution of Compound 11 in $CH_2Cl_2$, in the presence of WSC, was added stearic acid. Stirring at room temperature yielded 12 (96%).

To a solution of Compound 12 (500 mg, 0.857 mmol) and triethylamine (10.0 mL) in $CH_2Cl_2$ (8.57 mL) was added TBDPSCl (0.260 mL, 1.03 mmol) and DMAP (209 mg, 1.71 mmol). After stirring at room temperature for 20 hours (TLC monitoring: $CHCl_3$/MeOH 50/1), to this mixture was added MeOH at 0 degree Celsius. Concentration and purification by silica gel column chromatography ($CHCl_3$/MeOH 100/1) yielded 13 (533 mg, 76%).

[number 4]

$$[\alpha]_D = +1.25^\circ \quad (c\ 1.0, CHCl_3)$$

$^1$H NMR (400 MHz, $CDCl_3$):

δ 7.30-7.80 (m, 10 H, 2 Ph)
6.15 (d, 1 H, $J_{2,NH}$=8.4 Hz, NH)
4.17 (m, 1 H, H-2)
4.05 (dd, 1 H, $J_{gem}$ = 10.6 Hz, H-1)
3.79 (dd, 1 H, $J_{gem}$ = 10.6 Hz, H-1')
3.58 (m, 2H, H-3, H-4)
3.46 (d, 1 H, 4-OH)
2.80 (d, 1 H, 3-OH)
1.00-1.80 (m, 58 H, $-CH_2-$)
0.85 (t, 6 H, 2 $-CH_3$)

$^{13}$C NMR (100 MHz, $CDCl_3$):

δ 173.1, 135.5, 135.4, 134.8, 132.5, 132.2, 130.0, 127.9, 127.5, 75.6, 73.3, 63.8, 51.3, 36.7, 33.3, 31.9, 29.7, 29.6, 29.5, 29.3, 29.3, 29.3, 26.9, 26.5, 25.9, 25.6, 22.7,19.1, 14.1.

MALDI-TOF-MS: $m/z$ = 844 [$M$+Na]$^+$.

(Preparation 1 of the ceramide derivative 14)

Based on the description of Numata, M.; Sugimoto, M.; Koike, K. ; Ogawa, T.; Carbohydr. Res. 1990, 203, 205-217, the following experiments were performed. For example, in pyridine solvent at room temperature, in the presence of DMAP, 1 equivalent of succinic anhydride worked on Compound 13, then warmed up to 40 degrees Celsius. This product was treated with $Ac_2O$ to yield the ceramide derivative 14 having succinic acid at 4-position (40% yield for the 2 steps).

(Preparation of the Compound 16)

Based on the description of Numata, M.; Sugimoto, M.; Koike, K.; Ogawa, T.; Carbohydr. Res. 1990, 203, 205-217, the following experiments were performed. For example, to Compound 12 in acetonitrile solvent was added BDA and *p*-TsOH, and then heated to 40 degrees Celsius to yield Compound 15. In pyridine solvent at 40 degrees Celsius, succinic anhydride and DMAP worked on Compound 15 to yield Compound 16, in which succinic acid was introduced to the 4-position.

(Preparation of (2s,3S,4R)-1,3-O-benzylidene-1-deoxy-4-O-succinoylbenzylester-2-octadecanamide-octa decane (17))

To a solution of Compound 16 (10.0 g, 13.0 mmol) in MeCN (130 mL) and DMF (130 mL) was added $K_2CO_3$ (1.80 g, 13.0 mmol) and BnBr (1.70 mL, 14.3 mmol). This mixture was stirred at room temperature for 16 hours (TLC monitoring: toluene/EtOAc2/1). This reaction mixture was co-evaporated with toluene, and then extracted with $CHCl_3$. The organic phase was washed with 2 M HCl, $H_2O$, saturated aqueous $NaHCO_3$, and brine, dried ($Na_2SO_4$), and then concentrated. Purification by silica gel chromatography (toluene/EtOAc 40/1) yielded 17 (9.0 g, 80%).

[number 5]

$$[\alpha]_D = +28.0^\circ \quad (c\ 1.4, CHCl_3)$$

$^1H$ NMR (500 MHz, $CDCl_3$):

δ 7.30-7.50 (m, 10 H, 2 Ph)
5.62 (d, 1 H, $J_{2,NH}$ = 8.3 Hz, NH)
5.41 (s, 1 H, PhC*H*)
5.07-5.13 (2 d, 2 H, $J_{gem}$ = 12.5 Hz, PhC$H_2$)
4.88 (dd, 1 H, H-4)
4.37 (dd, H, $J_{1,2}$ = 10.5 Hz, H-1')
4.06-4.13 (m, 1 H, $J_{1,2}$ = 10.5 Hz, H-2)
3.81 (dd, 1 H, H-3)
3.46 (t, 1 H, $J_{1,2}$ = 10.5 Hz, H-1)
2.65-2.75 (m, 4 H, $-OCOCH_2CH_2COO-$)
1.20-2.25 (m, 58 H, $-CH_2-$)
0.90 (t, 6 H, 2 $-CH_3$)

$^{13}C$ NMR (125 MHz, $CDCl_3$):

δ 173.3, 173.0, 172.0, 137.6, 135.7, 129.0, 128.6, 128.3, 128.2, 128.1, 126.2, 69.5, 66.6, 43.6, 31.9, 29.7, 29.7, 29.6, 29.5, 29.5, 29.4, 29.4, 29.2, 22.7, 14.1.

MALDI-TOF-MS: *m*/*z*= 884 $[M+Na]^+$.

(Preparation of (2s,3S,4R)-4-O-succinoylbenzylester-2-octadecanamide-oc tadecane (18))

To a solution of Compound 17 (20.3 mg, 0.023 mmol) in AcOH (0.8 mL) was added $H_2O$ (0.2 mL). After stirring at 60 degrees Celsius for 12 hours (TLCmonitoring: toluene/EtOAc 2/1), this mixture was diluted with $CHCl_3$, washed with $H_2O$, saturated aqueous $NaHCO_3$, and brine, dried ($Na_2SO_4$), and then concentrated. Purification by silica gel chromatography (toluene/EtOAc 2/1) yielded 18 (11.0 mg, 62%).

[number 6-1]

$$[\alpha]_D = +4.0^\circ \quad (c\ 1.0, CHCl_3)$$

$^1H$ NMR (400 MHz, $CDCl_3$):

δ 7.30-7.40 (m, 5 H, Ph)

[number 6-2]

6.56 (d, 1 H, $J_{2,NH}$ = 7.7 Hz, NH)
5.10-5.17 (2 d, 2 H, $J_{gem}$ =12.1 Hz, PhC*H₂*)
4.93 (m, 1 H, H-4)
3.99 (m, 1 H, H-1')
3.97 (m, 1 H, H-2)
3.81 (m, 1 H, H-3)
3.71 (m, 1 H, H-1)
2.55-2.81 (m, 4 H, $-OCOCH_2CH_2COO-$)
1.20-2.21 (m, 58 H, $-CH_2-$)
0.90 (t, 6 H, 2 $-CH_3$)
$^{13}C$ NMR (100 $MH_2$, $CDCl_3$):

δ 174.1, 172.9, 172.4, 135.4, 128.6, 128.4, 128.1, 75.7, 73.3, 66.8, 62.8, 52.6, 36.6, 31.9, 29.7, 29.6, 29.6, 29.5, 29.5, 29.5, 29.4, 29.3, 29.3, 29.1, 25.7, 25.3, 22.7, 14.1.

MALDI-TOF-MS: *m*/*z* = 796 $[M+Na]^+$.

(Preparation of (2s,3S,4R)-3-O-acetyl-1-O-tert-butyldiphenylsilyl-4-O-s uccinoylbenzylester-2-octadecanamide-octadecane (20))

To a solution of Compound 18 (100 mg, 0.129 mmol) and triethylamine (1.0 mL) in 1,2-dichloroethane (1.3 mL) was added TBDPSCl (66.5 μL, 0.259 mmol) and DMAP (30.6 mg, 0.250 mmol). This mixture was stirred at 40 degrees Celsius for 12 hours. By TLC analysis (toluene/EtOAc 1/1), the starting material was confirmed to be completely consumed, and then MeOH was added at 0 degree Celsius. This reaction mixture was evaporated, and then was under vacuum line condition for 30 hours. This residue was dissolved in pyridine (3.0 mL), and then $Ac_2O$ was added. This mixture was stirred at room temperature for 16 hours (TLC monitoring: toluene/EtOAc2/1). This reaction mixture was co-evaporated with toluene, and then extracted with $CHCl_3$. The organic phase was washed with 2M HCl, $H_2O$, saturated aqueous $NaHCO_3$, and brine, dried ($Na_2SO_4$), and then concentrated. Purification by silica gel chromatography (toluene/EtOAc 4/1) yielded 20 (98.0 mg, 72%).

[number 7-1]

$$[\alpha]_D = +3.7^\circ \quad (c\ 1.0, CHCl_3)$$

$^1H$ NMR (500 MHz, $CDCl_3$):

[number 7-2]

δ 7.30-7.65 (m, 15 H, 3 Ph)
5.90 (d, H, $J_{2,NH}$ = 9.5 Hz, NH)
5.34 (dd, 1 H, H-3)
5.09-5.13 (2 d, 2 H, $J_{gem}$ = 12.5 Hz, PhC*H₂*)
4.94 (m, 1 H, H-4)

4.24 (m, 1 H, H-2)
3.62-3.69 (m, 2 H, H-1, H-1')
2.56-2.73 (m, 4 H, $-OCOCH_2CH_2COO-$)
1.90 (s, 3 H, OAc)
1.00-2.17 (m, 58 H, $-CH_2-$)
0.90 (t, 6 H, 2 $-CH_3$)
$^{13}$C NMR (100 MHz, $CDCl_3$):

δ 172.6, 172.1, 172.0, 169.7, 135.9, 135.7, 135.6, 135.5, 133.0, 132.7, 129.9, 128.6, 128.5, 128.3, 128.2, 128.2, 127.8, 127.8, 73.9, 73.2, 71.3, 66.5, 66.4, 62.4, 62.3, 49.3, 36.8, 31.9, 29.7, 29.7, 29.6, 29.5, 294, 29.3, 29.2, 29.1, 29.0, 27.8, 26.8, 25.7, 25.6, 22.7, 20.9, 20.7, 19.2, 14.1.

MALDI-TOF-MS: $m/z$ = 1076 $[M+Na]^+$.

(Preparation 2 of the ceramide derivative 14)

Based on the description of Numata, M. ; Sugimoto, M. ; Koike, K. ; Ogawa, T. ; Carbohydr. Res. 1990, 203, 205-217, the following experiments were performed. For example, for Compound 20, a catalytic hydrogenation using $Pd(OH)_2$ in EtOH at 40 degrees Celsius yielded the ceramide acceptor 14.

(Preparation 1 of phenyl 6-O-[{(2S,3S,4R)-3-O-acetyl-1-O-tert-butyldiphenylsilyl -2-octadecanamide-octadecane-4-yloxy}carbonylpropanoyl] -2-O-benzoyl-1-deoxy-3-O-p-methoxybenzyl-1-thio-β-D-glu copyranoside (21))

To a solution of Compound 14 (204 mg, 0.212 mmol) in $CH_2Cl_2$ (2.12 mL) was added 2, 4, 6-trichlorobenzoyl chloride (50.0 μL, 0.318 mmol), DMAP (39.0 mg, 0.318 mmol), triethylamine (44.4 μL, 0.318 mmol), and Compound 8 (105 mg, 0.212 mmol). This mixture was stirred at room temperature for 2 hours. By TLC analysis (toluene/EtOAc 2/1), the starting material was confirmed to be completely consumed. This mixture was diluted with $CHCl_3$, washed with saturated aqueous $NaHCO_3$, $H_2O$, and brine, dried ($Na_2SO_4$), and then concentrated. The residue was purified by silica gel chromatography (EtOAc/hexane 1/6) to yield 21 (229 mg, 75%).

[number 8-1]

$$[\alpha]_D = +0.39^\circ \quad (c\ 1.9, CHCl_3)$$

$^1$H NMR (500 MHz, $CDCl_3$):

δ 6.60-8.10 (m, 24 H, 5 Ph)
5.95 (s, 1 H, $J_{2,NH}$=9.5 Hz, NH)
5.35 (dd, 1 H, H-$3^{Cer}$)
5.19 (t, 1 H, $J_{1,2}$ = 9.9 Hz, H-$2^{Glc}$)
4.95 (dt, 1 H, H-$4^{Cer}$)
4.76 (d, 1 H, $J_{1,2}$ = 9.9 Hz, H-$1^{Glc}$)
4.66 (d, 1 H, $J_{gem}$ = 11.0 Hz, $PhCH_2$)
4.63 (d, 1 H, $J_{gem}$ = 11.0 Hz, $PhCH_2$)
4.46 (dd, 1 1 H, H-$6'^{Glc}$)
4.39 (dd, 1 H, H-$6'^{Glc}$)
4.22 (m, 1 H, H-$2^{Cer}$)
3.72 (m, 1 H, H-$4^{Glc}$)
3.71 (s, 3 H, OMe)
3.63 (m, 1 H, H-$3^{Glc}$)
3.53 (m, 1 H, H-$5^{Glc}$)
2.60-2.66 (2 d, 4 H, $-OCOCH_2CH_2COO-$)

[number 8-2]

1.00-2.20 (m, 58 H, $-CH_2-$)
0.90 (t, 6 H, 2 -CH3)
$^{13}C$ NMR (125 MHz, $CDCl_3$):

δ 172.7, 172.2, 171.9, 170.5, 165.1, 159.3, 135.7, 135.5, 129.7, 128.7, 128.4, 127.9, 127.8, 127.8, 113.8, 86.3, 82 55.1, 49.2, 36.8, 31.9, 29.7, 29.7, 29.6, 29.5, 29.4, 29.4, 20.7, 19.2, 14.1.

MALDI-TOF-MS: $m/z$ = 1464 $[M+Na]^+$.

(Preparation of phenyl 6-O-[{(2S,3S,4R)-3-O-acetyl-1-O-tert-butyldiphenylsilyl -2-octadecanamide-octadecane-4-yloxy} carbonylpropanoyl] -2-O-benzoyl-4-O-chloroacetyl-1-deoxy-3-O-p-methoxybenz yl-1-thio-β-D-glucopyranoside (22))

To a solution of Compound 21 (200 mg, 0.138 mmol) in $CH_2Cl_2$ (2.20 mL) was added triethylamine (38.7 μL, 0.277 mmol), and chloroacetic acid anhydride (47.4 mg, 0.277 mmol). This mixture was stirred at room temperature for 2 hours. By TLC analysis (EtOAc/hexane 1/3), the starting material was confirmed to be completely consumed. To this reaction mixture was added methanol at 0 degree Celsius. This mixture was diluted with $CHCl_3$, washed with 2M HCl, $H_2O$, saturated aqueous $NaHCO_3$, and brine, dried ($Na_2SO_4$), and then concentrated. The residue was purified by silica gel chromatography (EtOAc/hexane 1/8) to yield 22 (208 mg, 98%).

[number 9-1]

$$[\alpha]_D = +0.60^\circ \quad (c\ 1.9, CHCl_3)$$

$^1H$ NMR (400 MHz, $CDCl_3$):

δ 6.60-8.10 (m, 24 H, 5 Ph)
5.92 (d, 1 H, $J_{2,NH}$=9.5 Hz, NH)
5.36 (dd, 1 H, H-$3^{Cer}$)
5.28 (t, 1 H, $J_{1,2}$ = 9.9 $H_z$, H-$2^{Glc}$)
5.12 (t, 1 H, H-$4^{Glc}$)
4.94 (dt, 1 H, H-$4^{Cer}$)
4.80 (d, 1 H, $J_{1,2}$ = 9.9 Hz, H-$1^{Glc}$)

[number 9-2]

4.4-4.53 (2 d, 2 H, $J_{gem}$ = 11.4 Hz, $PhCH_2$)
4.18-4.28 (m, 3 H, H-$2^{Cer}$, H-$6^{Glc}$, H-$6'^{Glc}$)
3.84-3.92 (m, 1 H, H-$3^{Glc}$)
3.78-3.92 (2 d, 2 H, $-CH_2Cl$)
3.70-3.77 (m, 2 H, H-$1^{Cer}$, H-$5^{Glc}$)
3.71 (s, 3 H, OMe)
3.66 (dd, 1 H, H-$1'^{Cer}$)
2.50-2.70 (m, 4 H, $-OCOCH_2CH_2COO-$)
1.95 (s, 3 H, OAc)
1.00-2.30 (m, 58 H, $-CH_2-$)
0.90 (t, 6 H, 2 $-CH_3$)
$^{13}C$ NMR (100 MHz, $CDCl_3$):

δ 172.6, 172.0, 171.8, 169.7, 165.8, 164.8, 135.6, 135.5, 133.4, 132.9, 132.7, 132.2, 129.9, 129.8, 129.6, 129.5, 128.8, 128.5, 128.2, 127.8, 127.8, 113.7, 86.3, 80.7, 75.9, 74.1, 74.0, 72.1, 71.5, 71.2, 62.7, 62.4, 55.1, 49.3, 40.4, 36.8, 31.9, 29.7, 29.6, 29.5, 29.4, 29.3, 29.1, 28.8, 27.7, 26.8, 25.7, 22.7, 20.7, 19.2, 14.1.

MALDI-TOF-MS: $m/z$ = 1540 $[M+Na]^+$.

(Preparation of phenyl 6-O-[{(2S,3S,4R)-3-O-acetyl-2-octadecanamide-octadecane -4-yloxy}carbonylpropanoyl]-2-O-benzoyl-4-O-chloroacety 1-1-deoxy-3-O-p-methoxybenzyl-1-thio-β-D-glucopyranosid e (23))

To a solution of Compound 22 (17.0 mg, 0.011 mmol) in THF (224 μL) was added AcOH (5.00 μL, 0.084 mmol) and tetrabutylammonium fluoride (16.8 μL, 0.017 mmol). This mixture was stirred at room temperature for 18 hours. By TLC analysis (EtOAc/hexane 1/1), the starting material was confirmed to have completely disappeared. This mixture was diluted with $CHCl_3$, washed with 2 M HCl, saturated aqueous $NaHCO_3$, dried ($Na_2SO_4$), and then concentrated. This residue was purified by silica gel chromatography (EtOAc/hexane 2/3) to yield 23 (13.2 mg, 92%).

[number 10-1]

$$[\alpha]_D = -18.5^\circ \quad (c\ 0.7, CHCl_3)$$

[number 10-2]

$^1H$ NMR (500 MHz, $CDCl_3$):

δ 6.70-8.10 (m, 14 H, 3 Ph)
6.17 (d, H, $J_{2,NH}$=9.5 Hz, NH)
5.30 (t, 1 H, $J_{1,2}$ = 10.0 Hz, H-$2^{Glc}$)
5.14 (t, 1 H, H-$4^{Glc}$)
5.02-5.07 (m, 2 H, H-$3^{Cer}$, H-$4^{Cer}$)
4.81 (d, 1 H, $J_{1,2}$ = 10.0 Hz, H-$1^{Glc}$)
4.43-4.53 (2 d, 2 H, $J_{gem}$ = 11.2 Hz, $PhCH_2$)
4.16-4.32 (m, 3 H, H-$2^{Cer}$, H-$6^{Glc}$, H-$6'^{Glc}$)
3.90 (t, 1 H, H-$3^{Glc}$)
3.79-3.95 (2 d, 2 H, $J_{gem}$ = 14.7 Hz, $-CH_2Cl$)
3.72-3.76 (m, 1 H, H-$5^{Glc}$)
3.72 (s, 3 H, OMe)
3.63 (dd, 1 H, H-$1'^{Cer}$)
3.55 (t 1 H, H-$1^{Cer}$)
2.56-2.72 (m, 4 H, $-OCOCH_2CH_2COO-$)
2.13 (s, 3 H, OAc)
1.10-2.30 (m, 58 H, $-CH_2-$)
0.90 (t, 6 H, 2 $-CH_3$)

$^{13}C$ NMR (125 MHz, $CDCl_3$):

δ 188.0, 183.9, 179.0, 176.8, 132.8, 129.9, 129.7, 128.9, 128.8, 128.5, 113.7, 75.8, 74.2, 73.4, 68.1, 62.7, 55.2, 49.5, 40.5, 38.7, 36.8, 31.9, 30.4, 29.7, 29.6, 29.5, 29.4, 29.1, 28.9, 28.3,25.7,23.7,23.0,22.7,20.9,14.1,14.0,11.0.

MALDI-TOF-MS: $m/z$= 1302 $[M+Na]^+$.

(Preparation of phenyl 6-O-[{(2S,3S,4R)-3-O-acetyl-1-O-tert-butyldiphenylsilyl -2-octadecanamide-octadecane-4-yloxy} carbonylpropanoyl] -1-deoxy-2,3-di-O-p-methoxybenzyl-1-thio-β-D-glucopyran oside (24))

To a solution of Compound 10 (490 mg, 0.957 mmol) and Compound 14 (1.11g, 1.15 mmol) in $CH_2Cl_2$ (9. 6 mL) was added WSC (550 mg, 2.87 mmol). This mixture was stirred at room temperature for 6 hours. The completion of the reaction was confirmed by TLC (toluene/EtOAc 3/1). This reaction mixture was $CHCl_3$, washed with $H_2O$ and brine, dried ($Na_2SO_4$), and then concentrated. Purification by silica gel chromatography (EtOAc/hexane 1:3) yielded 24 (851 mg, 61%).

[number 11-1]

$$[\alpha]_D = -6.5^\circ \quad (c\ 2.3, CHCl_3)$$

[number 11-2]

$^1H$ NMR (500 MHz, $CDCl_3$):

δ 6.90-7.60 (m, 23 H, 5 Ph)
5.95 (d, 1 H, $J_{2,NH}$=9.5 Hz, NH)
5.35 (dd, 1 H, H-3$^{Cer}$)
4.95 (dt, 1 H, H-4$^{Cer}$)
4.68-4.87 (4 d, 4 H, $J_{gem}$ = 11.0 Hz, 2 PhC$H_2$)
4.64 (d, 1 H, $J_{1,2}$ = 9.5 Hz, H-1$^{Glc}$)
4.32-4.40 (m, 2 H, H-6$^{Glc}$, H-6'$^{Glc}$)
4.23 (m, 1 H, H-2$^{Cer}$)
3.80 and 3.79 (2 s, 6 H, 2 OMe)
3.61-3.69 (m, 2 H, H-1$^{Cer}$, H-1'$^{Cer}$)
3.41-3.57 (m, 4 H, $J_{1,2}$ = 9.5 Hz, H-2$^{Glc}$, H-5$^{Glc}$, H-3$^{Glc}$, H-4$^{Glc}$)
3.00 (d, 1 H, OH-4)
2.55-2.70 (m, 4 H, $-OCOCH_2CH_2COO-$)
1.95 (s, 3 H, OAc)
1.01-2.15 (m, 58 H, $-CH_2-$)
0.90 (t, 6 H, 2 $-CH_3$)

$^{13}C$ NMR (100 MHz, $CDCl_3$):

δ 172.6, 172.2, 171.9, 170.2, 159.4, 135.6, 135.5, 133.6, 132.9, 132.6, 132.1, 131.8, 130.5, 130.1, 129.9, 129.6, 129.5, 128.8, 127.8, 127.7, 127.5, 113.9, 113.8, 87.7, 87.6, 85.5, 80.1, 77.5, 75.2, 75.0, 73.8, 71.4, 69.8, 63.4, 62.3, 55.2, 55.2, 49.2, 36.8, 31.9, 29.7, 29.6, 29.5, 29.5, 29.3, 29.3, 29.3, 29.2, 29.0, 27.8, 26. 8, 25.6, 22.6, 20.9, 20.7, 19.2, 14.1.

MALDI-TOF-MS: *m*/*z* = 1480 [*M*+Na]$^+$.

(Preparation of phenyl 6-O-[{(2S,3S,4R)-3-O-acetyl-1-O-tert-butyldiphenylsilyl -2-octadecanamide-octadecane-4-yloxy} carbonylpropanoyl] -4-O-chloroacetyl-1-deoxy-2,3-di-O-p-methoxybenzyl-1-th io-β-D-glucopyranoside (25))

To a solution of Compound 24 (150 mg, 0.103 mmol) in $CH_2Cl_2$ (1.6 mL) was added triethylamine (28.8 μL, 0.206 mmol) and chloroacetic acid anhydride (35.1 mg, 0.205 mmol). This mixture was stirred at room temperature for 2 hours. By TLC analysis (EtOAc/hexane 1/3), the starting material was confirmed to be completely consumed, and then MeOH was added at 0 degree Celsius. This mixture was diluted with $CHCl_3$, washed with 2 M HCl, $H_2O$, saturated aqueous $NaHCO_3$, and brine, dried ($Na_2SO_4$), and then concentrated. Purification by silica gel chromatography (EtOAc/hexane 2/9) yielded 25 (158 mg, quantitative).

[number 12]

$$[\alpha]_D = -2.2^\circ \quad (c\ 0.9, CHCl_3)$$

$^1H$ NMR (400 MHz, $CDCl_3$):

δ 6.85-7.60 (m, 23 H, 5 Ph)
5.95 (d, 1 H, $J_{2,NH}$=9.5 Hz, NH)
5.36 (dd, 1 H, H-$3^{Cer}$)
5.00 (t, 1 H, H-$4^{Glc}$)
4.91 (dt, 1 H, H-$4^{Cer}$)
4.84 (d, 1 H, $J_{gem}$ = 9.9 Hz, $PhCH_2$)
4.78 (d, 1 H, $J_{gem}$ = 11.0 Hz, $PhCH_2$)
4.66 (d, 1 H, $J_{gem}$ = 9.9 Hz, $PhCH_2$)
4.63 (d, 1 H, $J_{1,2}$ = 8.8 Hz, H-$1^{Glc}$)
4.56 (d, 1 H, $J_{gem}$ = 11.0 Hz, $PhCH_2$)
4.19-4.30 (m, 2 H, H-$2^{Cer}$, H-$6'^{Glc}$)
4.13 (dd, 1 H, H-$6^{Glc}$)
3.83 (d, 1 H, $-CH_2Cl$)
3.81 and 3.80 (2 s, 6 H, 2 OMe)
3.58-3.77 (m, 5 H, H-$1^{Cer}$, H-$1'^{Cer}$, H-$3^{Glc}$, H-$5^{Glc}$, $-CH_2Cl$)
3.53 (t, 1 H, $J_{1,2}$ = 8.79 Hz, H-$2^{Glc}$)
2.50-2.65 (m, 4 H, $-OCOCH_2CH_2COO-$)
1.95 (s, 3 H, OAc)
1.00-2.18 (m, 58 H, $-CH_2-$)
0.90 (t, 6 H, 2 $-CH_3$)

$^{13}C$ NMR (100 MHz, $CDCl_3$):

δ 172.7, 172.0, 171.8, 169.7, 166.1, 159.5, 159.4, 135.6, 135.5, 133.1, 133.0, 132.6, 132.3, 132.2, 130.1, 130.0, 129.9, 129.5, 129.0, 127.9, 127.8, 127.8, 113.9, 87.7, 83.1, 80.5, 75.5, 75.3, 75.1, 73.9, 71.3, 71.1, 62.8, 62.4, 55.3, 49.3, 40.4, 36.8, 31.9, 29.7, 29.7, 29.6, 29.5, 29.4, 29.4, 29.3, 29.1, 28.8, 27.7, 26.8, 25.7, 22.7, 20.7, 19.2, 14.1.

MALDI-TOF-MS: $m/z$ = 1556 $[M+Na]^+$.

(Preparation of phenyl 6-O-[{(2S,3S,4R)-3-O-acetyl-2-octadecanamide-octadecane -4-yloxy}carbonylpropanoyl]-4-O-chloroacetyl-1-deoxy-2, 3-di-O-p-methoxybenzyl-1-thio-β-D-glucopyranoside (26))

To a solution of Compound 25 (157 mg, 0.102 mmol) in THF (1.0 mL) was added AcOH (6.4 μL, 0.102 mmol) and tetrabutylammonium fluoride (102 μL, 0.102 mmol). After stirring at 0 degree Celsius for 3 hours (TLC monitoring: EtOAc/hexane 1/1), this mixture solution was diluted with $CHCl_3$, washed with saturated aqueous $NaHCO_3$ and brine, dried ($Na_2SO_4$), and then concentrated. Purification by silica gel chromatography (EtOAc/hexane 1/4) yielded 26(76 mg, 58%).

[number 13-1]

$$[\alpha]_D = -7.6^\circ \quad (c\ 1.6, CHCl_3)$$

$^1H$ NMR (400 MHz, $CDCl_3$):

δ 6.86-7.57 (m, 13 H, 3 Ph)
6.20 (d, 1 H, $J_{2,NH}$=9.0 Hz, NH)
4.99-5.06 (m, 3 H, H-$3^{Cer}$, H-$4^{Cer}$, H-$4^{Glc}$)
4.85 (d, 1 H, $J_{gem}$ = 10.0 Hz, $PhCH_2$)
4.78 (d, 1 H, $J_{gem}$ = 11.5 Hz, $PhCH_2$)
4.67 (d, 1 H, $J_{gem}$ = 10.0 Hz, $PhCH_2$)
4.65 (d, H, $J_{1,2}$ = 8.8 Hz, H-$1^{Glc}$)
4.57 (d, 1 H, $J_{gem}$ = 11.5 Hz, $PhCH_2$)
4.26 (dd, 1 H, $J_{gem}$ = 12.5 Hz, H-$6'^{Glc}$)
4.19 (m, 1 H, H-$2^{Cer}$)

4.13 (dd, 1 H, $J_{gem}$ = 12.5 Hz, H-$6^{Glc}$)
3.86 (d, 1 H, $J_{gem}$ = 12.0 Hz, $CH_2Cl$)
3.82 and 3.81 (2 s, 6 H, 2 OMe)
3.69 (d, 1 H, $J_{gem}$ = 12.0 Hz, $CH_2Cl$)
3.54-3.66 (m, 5 H, H-$1^{Cer}$, H-$1'^{Cer}$, H-$2^{Glc}$, H-$3^{Glc}$, H-$5^{Glc}$)
2.55-2.70 (m, 4 H, $-OCOCH_2CH_2COO-$)
2.15 (s, 3 H, OAc)
1.20-2.22 (m, 58 H, $-CH_2-$)
0.90 (t, 6 H, 2 $-CH_3$)

[number 13-2]

$^{13}C$ NMR (100 MHz, $CDCl_3$):
δ 173.3, 172.1, 172.0, 171.4, 166.2, 159.5, 159.4, 133.1, 132.2, 130.1, 130.0, 129.8, 129.6, 128.9, 127.8, 113.9, 87.7, 83.0, 80.4, 75.5, 75.2, 75.0, 73.5, 72.7, 71.2, 62.7, 61.5, 55.3, 50.6, 49.5, 40.4, 36.6, 31.9, 29.7, 29.6, 29.5, 29.5, 29.3, 29.3, 29.0, 28.8, 28.1, 25.7, 25.6, 22.6, 20.8,14.1.
MALDI-TOF-MS: *m*/*z*= 1318 $[M+Na]^+$.

(Preparation of phenyl 6-O-[{(25,35,4R)-3-O-acetyl-2-octadecanamide-octadecane -4-yloxy}carbonylpropanoyl]-2-O-benzoyl-1-deoxy-3-O-p-m ethoxybenzyl-1-thio-β-D-glucopyranoside (27))

To a solution of Compound 21 (48.8 mg, 0.034 mmol) in THF (338 μL) was added AcOH (6.0 μL, 0.101 mmol) and tetrabutylammonium fluoride (102 μL, 0.102 mmol). After stirring at 0 degree Celsius for 12 hours (TLC monitoring: EtOAc/hexane 1/2), this mixture was diluted with $CHCl_3$, washed with saturated aqueous $NaHCO_3$ and brine, dried ($Na_2SO_4$), and then concentrated. Purification by silica gel chromatography (toluene/EtOAc1.5:1) yielded27 (30mg, 75%).

[number 14]

$$[\alpha]_D = +7.4^\circ \quad (c\ 0.6, CHCl_3)$$

$^1H$ NMR (500 MHz, $CDCl_3$):

δ 6.66-8.05 (m, 13 H, 3 Ph)
6.23 (d,1 H, $J_{2,NH}$ = 9.0 Hz, NH)
5.21 (t, 1 H, $J_{1,2}$ = 10.0 Hz, H-$2^{Glc}$)
5.01-5.06 (m, 2 H, H-$3^{Cer}$, H-$4^{Cer}$)
4.78 (d, 1 H, $J_{1,2}$ = 10.0 Hz, H-$1^{Glc}$)
4.63 (2 d, 2 H, $J_{gem}$ = 11.5 Hz, $PhCH_2$)
4.48 (dd, 1 H, $J_{gem}$ = 12.0 Hz, H-$6'^{Glc}$)
4.39 (dd, 1 H, $J_{gem}$ = 12.0 Hz, H-$6^{Glc}$)
4.19 (m, 1 H, H-$2^{Cer}$)
3.65-3.74 (m, 6 H, H-$1'^{Cer}$, H-$3^{Glc}$, H-$4^{Glc}$, OMe)
3.54-3.59 (m, 2 H, H-$1^{Cer}$, H-$5^{Glc}$)
2.60-2.75 (m, 4 H, $-OCOCH_2CH_2COO-$)
2.15 (s, 3 H, OAc)
1.20-2.20 (m, 58 H, $-CH_2-$)
0.89 (t, 6 H, 2 $-CH_3$)

$^{13}C$ NMR (100 MHz, $CDCl_3$):

δ 173.3, 172.6, 171.9, 171.5, 165.2, 159.3, 133.2, 132.8, 132.5, 129.8, 129.8, 129.7, 129.0, 128.7, 128.4, 128.2, 127.8, 125.3, 113.7, 86.4, 82.9, 77.8, 74.5, 73.4, 73.2, 72.0, 69.7, 63.3, 61.4, 55.1, 49.6, 36.7, 31.9, 29.7, 29.6, 29.5, 29.3, 29.3, 29.1, 28.6, 25.6, 25.5, 22.7, 20.9, 14.1.

MALDI-TOF-MS: $m/z$ = 1226 $[M+Na]^+$.

(Preparation of phenyl 6-O-[{(2S,3S,4R)-3-O-acetyl-2-octadecanamide-octadecane -4-yloxy}carbonylpropanoyl]-1-deoxy-2,3-di-O-p-methoxyb enzyl-1-thio-β-D-glucopyranoside (28))

To a solution of Compound 24 (157 mg, 0.108 mmol) in THF (1.1 mL) was added AcOH (6.5 μL, 0.108 mmol) and tetrabutylammonium fluoride (110 μL, 0.110 mmol). After stirring at 0 degree Celsius for 3 hours (TLC monitoring: EtOAc/hexane 1/1), this mixture solution was diluted with $CHCl_3$, washed with saturated aqueous $NaHCO_3$ and brine, dried ($Na_2SO_4$), and then concentrated. Purification by silica gel chromatography (toluene/EtOAc 3/2) yielded 28 (76 mg, 58%).

[number 15-1]

$$[\alpha]_D = -5.7^\circ \quad (c\ 3.1, CHCl_3)$$

$^1$H NMR (400 MHz, $CDCl_3$):

δ 6.86-7.56 (m, 13 H, 3 Ph)
6.22 (d, 1 H, $J_{2,NH}$=9.2 Hz, NH)
5.00-5.05 (m, 2 H, H-3$^{Cer}$, H-4$^{Cer}$)
4.64-4.87 (4 d, 4 H, 2 PhC$H_2$)
4.65 (d, 1 H, $J_{1,2}$ = 9.6 Hz, H-1$^{Glc}$)
4.40 (dd, 1 H, $J_{gem}$ = 9.60 Hz, H-6'$^{Glc}$)
4.34 (dd, 1 H, $J_{gem}$ = 9.60 Hz, H-6$^{Glc}$)
4.17 (m, 1 H, H-2$^{Cer}$)
3.81 and 3.80 (2 s, 6 H, 2 OMe)
3.80 (s, 3 H, OMe)
3.42-3.59 (m, 6 H, H-1$^{Cer}$, H-1'$^{Cer}$, H-2$^{Glc}$, H-3$^{Glc}$, H-4$^{Glc}$, H-5$^{Glc}$)

[number 15-2]

3.12 (d, 1 H, OH-4$^{Glc}$)
2.61 (d, 1 H, OH-1$^{Cer}$)
2.59-2.70 (m, 4 H, $-OCOCH_2CH_2COO-$)
2.10 (s, 3 H, OAc)
1.20-2.20 (m, 58 H, $-CH_2-$)
0.90 (t, 6 H, 2 $-CH_3$)
$^{13}$C NMR (100 MHz, $CDCl_3$):

δ 173.3, 172.5, 171.9, 171.5, 159.4, 133.7, 131.9, 130.4, 130.1, 129.9, 129.6, 128.9, 127.5, 114.0, 113.9, 87.7, 85.6, 80.2, 77.5, 75.2, 75.0, 73.4, 73.3, 69.8, 63.7, 61.5, 55.3, 55.2, 49.6, 36.7, 31.9, 29.7, 29.7, 29.5, 29.5, 29.4, 29.3, 29.0, 28.6, 25.6, 25.5, 22.7, 20.8, 14.1.

MALDI-TOF-MS: m/z = 1242 $[M+Na]^+$.

(Phenyl 6-O-[{(2S,3S,4R)-3-O-acetyl-1-O-tert-butyldiphenylsilyl -2-octadecanoylamino-octadecane-4-yloxy}carbonylpropano yl]-2-O-benzoyl-3-O-p-methoxybenzyl-1-thio-β-D-glucopyr anoside (7)

[Chem. 114]

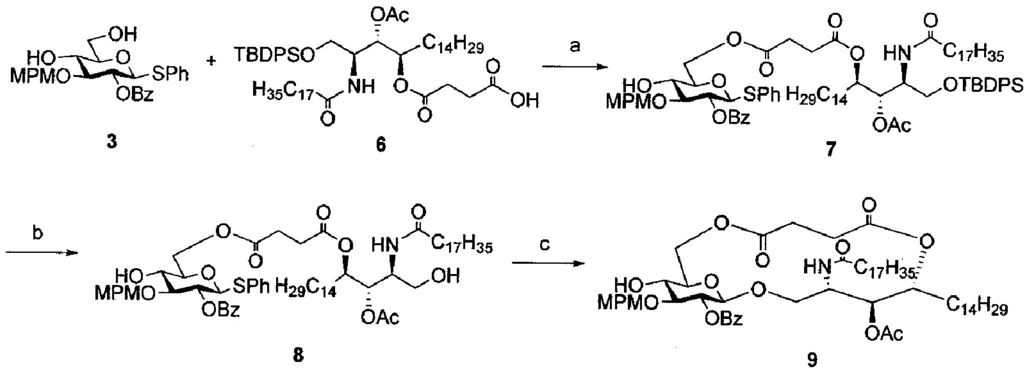

3 6 7 8 9

To a solution of Compound 6 (204 mg, 0.212 mmol) in $CH_2Cl_2$ (2.1 mL) was added 2,4,6-trichlorobenzoyl chloride (50.0 μL, 0.318 mmol), DMAP (39.0 mg, 0.318 mmol), triethylamine (44.4 μL, 0.318 mmol), and Compound 3 (105 mg, 0.212 mmol). This mixture was stirred for 2 hours at room temperature. After the consumption of the starting material was checked by TLC analysis (toluene/EtOAc 2/1), this mixture was diluted with $CHCl_3$, washed with saturated $NaHCO_3$, $H_2O$, and brine, dried ($Na_2SO_4$), and then concentrated. The residue was subjected to silica gel column chromatography (EtOAc/hexane 1/6) to yield Compound 7 (229 mg, 76%):

$[\alpha]_D$ +0.39° (c 1.9, $CHCl_3$); $^1$H NMR (500 MHz, $CDCl_3$) :

δ 6.60-8.10 (m, 24H, 5Ph), 5.95 (s, 1H, $J_{2,NH}$-9.5Hz, NH), 5.35 (dd, 1H, H-$3^{Cer}$), 5.19 (t, 1H, $J_{1,2}$=$J_{2,3}$=9.9Hz, H-$2^{Glc}$), 4.95 (dt, 1H, H-$4^{Cer}$), 4.76 (d, 1H, $J_{1,2}$=9.9Hz, H-$1^{Glc}$), 4.66 (d, 1H, $J_{gem}$=11.0Hz, $PhCH_2$), 4.63 (d, 1H, $J_{gem}$=11.0Hz, $PhCH_2$), 4.46 (dd, 1H, H-$6'^{Glc}$), 4.39 (dd, 1H, H-$6'^{Glc}$), 4.22 (m, 1H, H-$2^{Cer}$), 3.72 (m, 1H, H-$4^{Glc}$), 3.71 (s, 3H, OMe), 3.63 (m, 1H, H-$3^{Glc}$) 3.53 (m, 1H, H-$5^{Glc}$), 2.60-2.66 (2d, 4H, -$OCOCH_2CH_2COO$-), 1.00-2.20 (m, 58H, -$CH_2$-), 0.90 (t, 6H, 2-$CH_3$); $^{13}$CNMR (125MHz, $CDCl_3$): δ 172.7, 172.2, 171.9, 170.5, 165.1, 159.3, 135.7, 135.5, 133.2, 132.8, 132.6, 129.9, 129.8, 129.7, 128.7, 128.4, 127.9, 127.8, 127.8, 113.8, 86.3, 82.9, 73.9, 72.1, 71.6, 69.8, 63.0, 62.3, 55.1, 49.2, 36.8, 31.9, 29.7, 29.7, 29.6, 29.5, 29.4, 29.4, 29.4, 29.1, 27.9, 26.8, 25.7, 22.7, 20.7, 19.2, 14.1; MALDI-TOF-MS: m/z=1464$[M+Na]^+$.

(Phenyl

6-O-[{(2S,3S,4R)-3-O-acetyl-2-octadecanoylamino-octadec ane-4-yloxy}carbonylpropanoyl]-2-O-benzoyl-3-O-4-methox ybenzyl-1-thio-β-D-glucopyranoside (8)

To a solution of Compound 7 (48.8 mg, 0.034 mmol) in THF (338 μL) was added AcOH (6.0 μL, 0.101 mmol) and tetrabutylammonium fluoride (102 μL, 0.102 mmol). After stirring at 0 degree Celsius for 12 hours (TLC monitoring: EtOAc/hexane 1/2), this mixture was diluted with $CHCl_3$, washed with saturated $NaHCO_3$ and brine, dried ($Na_2SO_4$), and then concentrated. This residue was subjected to silica gel column chromatography (toluene/EtOAc 1.5/1) to yield Compound 8 (30 mg, 77%):

$[\alpha]_D$ +7.4° (c 0.6, $CHCl_3$); $^1$H NMR (500 MHz, $CDCl_3$) :

δ 6.66-8.05 (m, 13H, 3 Ph), 6.23 (d, 1H, $J_{2,NH}$=9.0Hz, NH), 5.21 (t, 1H, $J_{1,2}$=$J_{2,3}$=10.0Hz, H-$2^{Glc}$), 5.01-5.06 (m, 2H, H-$3^{Cer}$, H-$4^{Cer}$), 4.78 (d, 1H, $J_{1,2}$=10.0Hz, H-$1^{Glc}$), 4.63 (2 d, 2H, $J_{gem}$=11.5Hz, $PhCH_2$), 4.48 (dd, 1H, $J_{gem}$=12.0Hz, H-$6'^{Glc}$), 4.39 (dd, 1H, $J_{gem}$=12.0Hz, H-$6^{Glc}$), 4.19 (m, 1H, H-$2^{Cer}$), 3.65-3.74 (m, 6H, H-$1'^{Cer}$, H-$3^{Glc}$, H-$4^{Glc}$, OMe), 3.54-3.59 (m, 2H, H-$1^{Cer}$, H-$5^{Glc}$), 2.60-2.75 (m, 4H, -$OCOCH_2CH_2COO$-), 2.15 (s, 3H, OAc), 1.20-2.20 (m, 58H, -$CH_2$-), 0.89 (t, 6H, 2-$CH_3$); $^{13}$CNMR (100MHz, $CDCl_3$) : δ 173.3, 172.6, 171.9, 171.5, 165.2, 159.3, 133.2, 132.8, 132.5, 129.8, 129.8, 129.7, 129.0, 128.7, 128.4, 128.2, 127.8, 125.3, 113.7, 86.4, 82.9, 77.8, 74.5, 73.4, 73.2, 72.0, 69.7, 63.3, 61.4, 55.1, 49.6, 36.7, 31.9, 29.7, 29.6, 29.5, 29.3, 29.3, 29.1,

28.6, 25.6, 25.5, 22.7, 20.9, 14.1; MALDI-TOF-MS: m/z=1226$[M+Na]^+$.

(2-O-benzoyl-3-O-4-methoxybenzyl-β-D-glucopyranosyl -(1(R)1)-(2S,3S,4R)-3-O-acetyl-2-octadecanoylamino-octa decane-4,6-succinate(9)

To a solution of Compound 8 (43 mg, 0.036 mmol) in $CH_2Cl_2$ (1.2 mL) was added MS4Å (40 mg). After stirring for 1 hour, to this suspension was added NIS (16.0 mg, 0.071 mmol) andTfOH (0.6μL, 0.0079mmol). Thismixturewas stirred for 5 hours. The completion of the reaction was confirmed by TLC (toluene/EtOAc 1/1). The reaction mixture was filtered through a Celite® . The filtrate and the wash solution were combined, extracted with $CHCl_3$, washed with saturated $NaHCO_3$, saturated $Na_2S_2O_3$, and brine, dried ($Na_2SO_4$), and then concentrated. The residue was subjected to silica gel column chromatography (toluene/EtOAc 3:1) to yield Compound 9 (33 mg, 85%):

$[\alpha]_D$ +3.7˚ (c 1.1, $CHCl_3$); $^1$H NMR (500 MHz, $CDCl_3$) :δ 6.67-8.00 (m, 9H, 2 Ph), 6.02 (d, 1H, $J_{2,NH=}$9.0Hz, NH), 5.18 (m, 2H, $J_{1,2}$=7.5Hz, H-$4^{Cer}$, H-$2^{Glc}$), 5.10 (t, 1H, H-$3^{Cer}$), 4.66 (d, 1H, $J_{gem}$=11.5Hz, $PhCH_2$), 4.55 (d, 1H, $J_{gem}$=11.5Hz, $PhCH_2$), 4.47 (d, 1H, $J_{1,2}$=7.5Hz, H-$1^{Glc}$), 4.39 (m, 3H, $J_{1,2}$=5.5Hz, H-$2^{Cer}$, H-$6^{Glc}$, H-$6'^{Glc}$), 3.81 (dd, 1H, $J_{gem}$=11.5Hz, $J_{1,2}$=5.5Hz, H-$1^{Cer}$) 3.73 (s, 3H, OMe), 3.61-3.69 (m, 3H, H-$1^{Cer}$, H-$3^{Glc}$, H-$5^{Glc}$) 3.49 (dt, 1H, H-$4^{Glc}$), 2.50-2.80 (m, 4H, $-OCOCH_2CH_2COO-$), 2.10 (s, 3H, OAc), 1.10-2.00 (m, 58H, $-CH_2-$), 0.84 (t, 6H, 2-$CH_3$);$^{13}$C NMR (100MHz, $CDCl_3$): δ 172.9, 171.4, 171.2, 170.8, 165.0, 159.3, 133.3, 129.8, 129.7, 129.6, 129.5, 128.5, 114.0, 113.9, 100.1, 81.5, 74.7, 74.3, 73.8, 73.7, 72.7, 70.4, 63.8, 55.1, 47.4, 37.4, 37.1, 36.5, 33.5, 32.7, 31.9, 30.2, 30.0, 29.7, 29.6, 29.6, 29.5, 29.5, 29.4, 29.3, 29.3, 29.2, 27.4, 27.1, 25.4, 25.0, 24.4, 22.7, 21.0, 19.7, 14.1; MALDI-TOF-MS: m/z=1116$[M+Na]^+$.

(Methyl

5-acetamide-4,7,8,9-tetra-O-acetyl-3,5-dideoxy-D-glycer o-β-D-galacto-2-nonulopyranosylonate)-(2→3)-(2,4,6-triO-benzoyl-β-D-galactopyranosyl)-(1→4)-2-O-benzoyl-3-O-4 -methoxybenzyl-β-D-glucopyranosyl-(1→1)-(2S,3S,4R)-3-O-acetyl-2-octadecanoylaminooctadecane-4,6-succinate(11)

[Chem. 115]

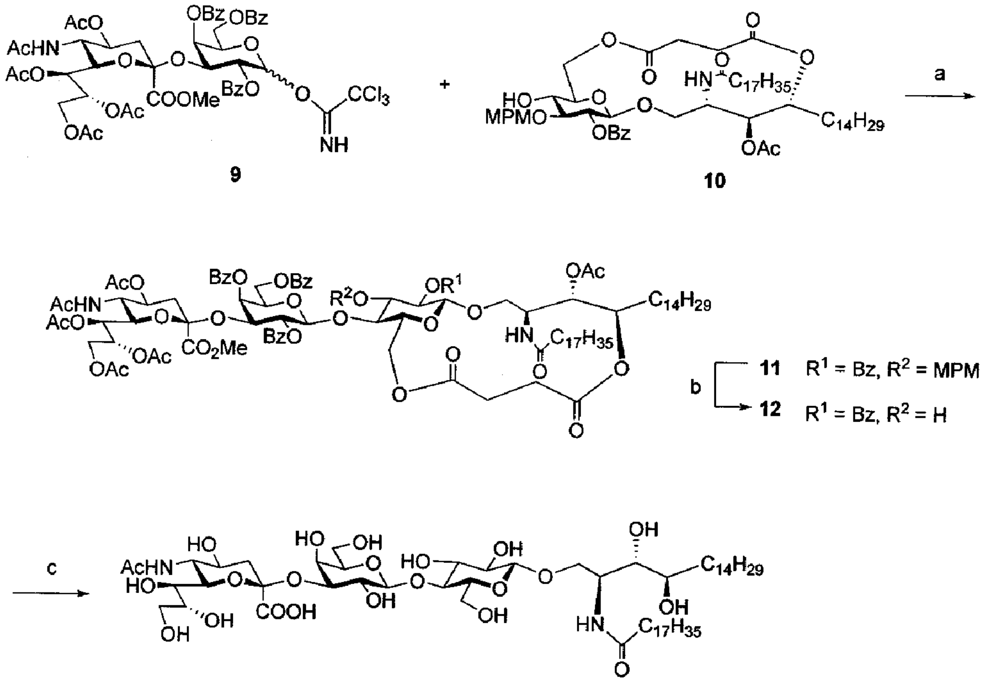


9

10

11 $R^1$ = Bz, $R^2$ = MPM

12 $R^1$ = Bz, $R^2$ = H

To a solution of Compound 9 (63 mg, 0.0578 mmol) and Compound 10 (152 mg, 0.137 mmol) in $CH_2Cl_2$ (1.1 mL) was added molecular sieves (AW-300) (200 mg). After stirring for 1 hour, to this suspension was added TMSOTf (1.0 μL, 0.00548 mmol). The progress of the reaction was monitored by TLC (toluene/EtOAc 1/3). After stirring for 4 hours, this reaction mixture was filtered through a a Celite® pad pad. The filtrate and the wash solution were combined, extracted with $CHCl_3$, washed with saturated $NaHCO_3$ and brine, dried ($Na_2SO_4$), and then concentrated. The residue was subjected to silica gel chromatography (toluene/EtOAc 1/2) to yield Compound 11 (83 mg, 70%):

$[\alpha]_D$ +24.4° (c 0.7, $CHCl_3$);$^1$H NMR(400 MHz, $CDCl_3$) δ 7.34-8.21(m, 20H, 4Ph), 6.43-7.01(2d, 4H, PMB), 5.94(d, 1H, $J_{2,NH}$=8.2Hz, $NH^{Cer}$), 5.68(m, 1H, H-$8^{Neu}$), 5. 55 (near t, 1H, $J_{1,2}$=8.2Hz, H-$2^{Gal}$), 5.36(d, 1H, H-$4^{Gal}$), 5.24(dd, 1H, H-$7^{Neu}$), 5.04-5.12(m, 3H, $J_{1,2}$=8.2Hz, H-$1^{Gal}$ H-$3^{Cer}$, $J_{1,2}$=7.8Hz, H-$2^{Glc}$), 4.91-4.98(m, 3H, H-$4^{Cer}$, $NH^{Neu}$, H-$3^{Gal}$), 4.81(m, 1H, H-$4^{Neu}$), 4.66-4.87 (2d, 2H, $J_{gem}$=11.0Hz, $PhCH_2$), 4.42 (dd, 1H, $J_{gem}$=12.4Hz, H-$9'^{Neu}$), 4.14-4.37 (m, 7H, H-$5^{Gal}$, H-$6^{Gal}$, H-$6'^{Gal}$, H-$6^{Glc}$, H-$6'^{Glc}$, $J_{1,2}$=7.8Hz, H-$1^{Glc}$, H-$2^{Cer}$), 4.05(dd, 1H, $J_{gem}$=12.4Hz, H-$9^{Neu}$), 3.71-3.82(m, 8H, H-$1^{Cer}$, H-$1'^{Cer}$, H-$3^{Glc}$, H-$4^{Glc}$, H-$5^{Neu}$, OMe), 3.61(dd, 1H, H-$6^{Neu}$), 3.57(s, 3H, OMe), 3.50(t, 1H, H-$5^{Glc}$), 2.36-2.67(m, 4H, $-OCOCH_2CH_2COO-$), 2.48(dd, 1H, H-$3eq^{Neu}$), 1.64(m, 1H, H-$3ax^{Neu}$), 1.52-2.18(6s, 18H, OAc), 1.10-1.40(m, 58H, $-CH_2-$), 0.87(t, 6H, 2-$CH_3$); $^{13}$C NMR (100 MHz, $CDCl_3$) : δ 172.84, 170.97, 170.90, 170.75, 170.67, 170.61, 170.25, 170.08, 168.16, 165.73, 165.56, 165.05, 158.82, 133.33, 133.12, 130.36, 130.15, 129.88, 129.78, 129.72, 129.63, 129.35, 128.56, 128.53, 128.31, 113.37, 101.29, 98.78, 96.94, 79.50, 78.72, 77.66, 74.53, 74.36, 73.36, 73.12, 73.06, 71.95, 71.55, 71.41, 70.94, 69.39, 68.23, 67.61, 66.59, 63.33, 62.35, 61.72, 54.97, 53.23, 48.82, 46.55, 37.35, 36.34, 31.93, 30.80, 30.04, 29.67, 29.62, 29.55, 29.45, 29.38, 29.20, 25.30, 25.15, 23.16, 22.70, 21.47, 20.93, 20.83, 20.73, 20.41, 14.13; MALDI-TOF-MS: m/z=2064$[M+Na]^+$.

[Table 5]

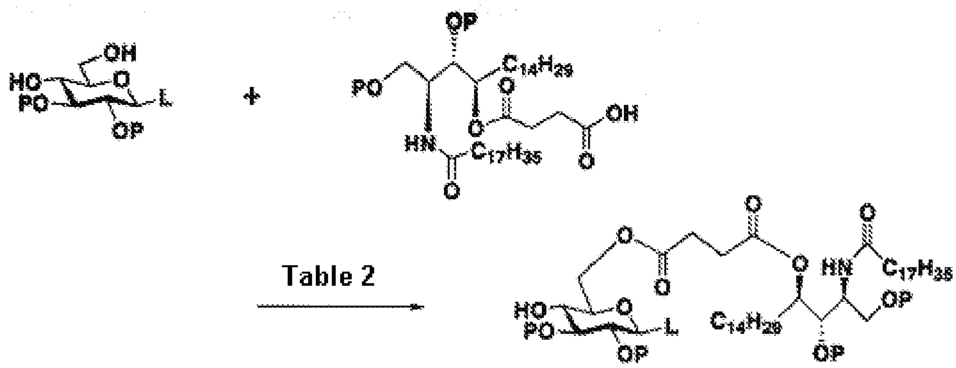


Table 2

| entry | Reagent (eq.) | Solvent | Temperature (˚C) |
|---|---|---|---|
| 1 | $PPh_3$(3.0),DEAD(3.0) | THF | reflux (90) |
| 2 | WSC(3.0) | $CH_2Cl_2$ | r.t. |
| 3 | 2,4,6-trichlorobenzoylchloride (1.1)<br>$Et_3N$(1.5)<br>DMAP(1.5) | $CH_2Cl_2$ | r.t. |

By reacting Glc and Cer under the condition described in Table 2 above, they were crosslinked.

(Preparation of Compound 29)

[Chem. 116]

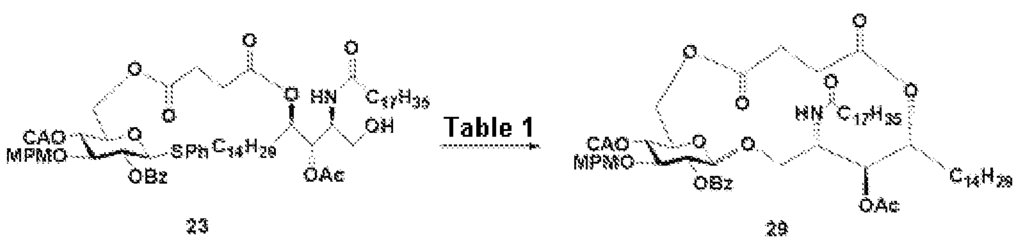


23 29

Table 1

| entry | **Reagent** (eq.) | **Solvent** | **Temperature** (˚C) | **Time** (b) | **Yield** (%) | (α/β)*1 |
|---|---|---|---|---|---|---|
| 1 | NIS(2.0), TfOH(0.2), MS4 A | $CH_2Cl_2$ | 0 | 6 | 60 | (β only ) |
| 2 | HIS(2.0), TfOH(0.2), MS4 A | $CH_2Cl_2$ | 0 | 3 | 69 | (β only ) |

*1; **Determined by** $^1$H NMR spectrum

For example, using a similar method to that described above, Compound 29 was treated in EtOH in the presence of DABCO at 55 degrees Celsius to yield Compound 35 (60% yield) .

[Chem. 117]

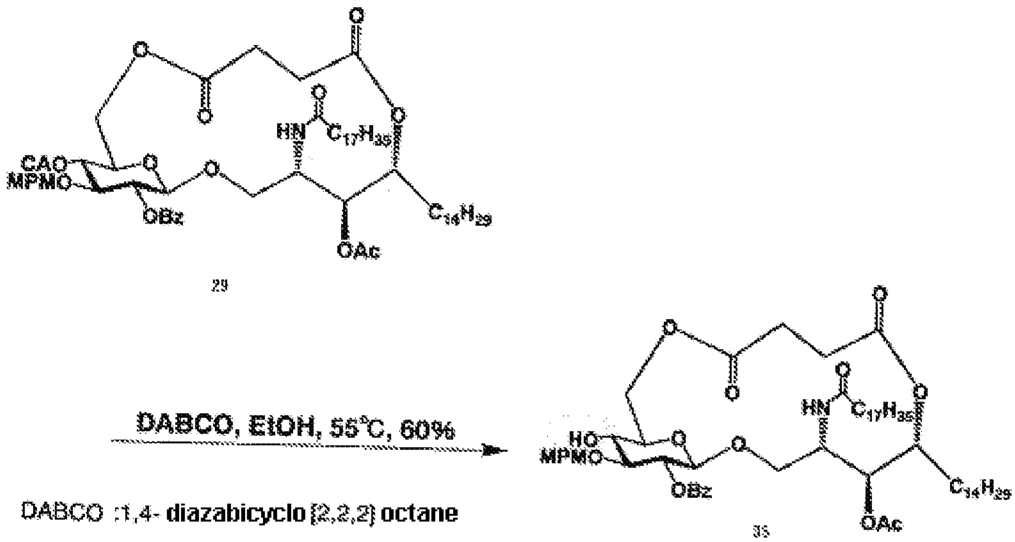


29

35

(Preparation of Compound 30)

For example, using a similar method to that described above, the reaction under the condition described below yielded Compound 30.

[Chem. 118]

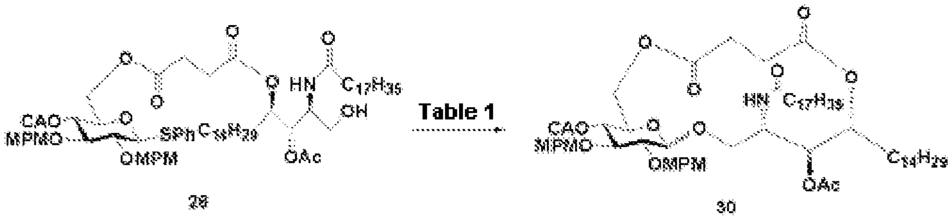


29

30

Table 1

| entry | Reagent (eq.) | Solvent | Temperature (°C) | Time (h) | Yield (%) | (α/β) |
|---|---|---|---|---|---|---|
| 1 | NIS(2.0), TfOH(0.2), MS4 Å | $CH_2Cl_2$ | 0→r.t. | 5 | - | |

(Preparation of 2-O-benzoyl-3-O-p-methoxybenzyl-β-D-glucopyranoside-(1→ 1)-(2S,3S,4R)-3-O-acetyl-2-octadecanamide-octadecane-4, 6-succinate(31))

To a solution of Compound 27 (43 mg, 0.036 mmol) in $CH_2Cl_2$ (1.2 mL) was added molecular sieves 4Å (MS4Å) (40 mg) . After stirring for 1 hour, to this suspension was added NIS (16.0 mg, 0.071 mmol) and TfOH (0.6 μL, 0.0079 mmol). This mixture solution was stirred for 5 hours. The completion of the reaction was confirmed by TLC (toluene/EtOAc 1/1). This reaction mixture was filtrated through a Celite®. The filtrate and the wash were combined, extracted with $CHCl_3$, washed with saturated aqueous $NaHCO_3$, saturated aqueous $Na_2S_2O_3$, and brine, dried ($Na_2SO_4$), and then concentrated. Purification by silica gel chromatography (toluene/EtOAc 3:1) yielded 31 (33 mg, 85%).

[number 16]

$$[\alpha]_D = +3.7^\circ \quad (c\ 1.1, CHCl_3)$$

$^1$HNMR (500 MHz, $CDCl_3$):

δ 6.67-8.00 (m, 9 H, 2 Ph)
6.02 (d, 1 H, $J_{2,NH}$ = 9.0 Hz, NH)
5.18 (m, 2 H, $J_{1,2}$ = 7.5 Hz, H-4$^{Cer}$, H-2$^{Glc}$)
5.10 (t, 1 H, H-3$^{Cer}$)
4.66 (d, 1 H, $J_{gem}$ = 11.5 Hz, PhC$H_2$)
4.55 (d, 1 H, $J_{gem}$ = 11.5 Hz, PhC$H_2$)
4.47 (d, 1 H, $J_{1,2}$ = 7.5 Hz, H-1$^{Glc}$)
4.39 (m, 3 H, $J_{1,2}$ = 5.5 Hz, H-2$^{Cer}$, H-6$^{Glc}$, H-6'$^{Glc}$)
3.81 (dd, 1 H, $J_{gem}$ = 11.5 Hz, $J_{1,2}$ = 5.5 Hz, H-1$^{Cer}$)
3.73(s, 3H, OMe)
3.61-3.69 (m, 3 H, H-1$^{Cer}$, H-3$^{Glc}$, H-5$^{Glc}$)
3.49 (dt, 1 H, H-4$^{Glc}$)
2.50-2.80 (m, 4 H, $-OCOCH_2CH_2COO-$)
2.10 (s, 3 H, OAc)
1.10-2.00 (m, 58 H, $-CH_2-$)
0.84 (t, 6 H, 2 $-CH_3$)

$^{13}$C NMR (100 MHz, $CDCl_3$):

δ 172.9, 171.4, 171.2, 170.8, 165.0, 159.3, 133.3, 129.8, 129.7, 129.6, 129.5, 128.5, 114.0, 113.9, 100.1, 81.5, 74.7, 74.3, 73.8, 73.7, 72.7, 70.4, 63.8, 55.1, 47.4, 37.4, 37.1, 36.5, 33.5, 32.7, 31.9, 30.2, 30.0, 29.7, 29.6, 29.6, 29.5, 29.5, 29.4, 29.3, 29.3, 29.2, 27.4, 27.1, 25.4, 25.0, 24.4, 22.7, 21.0, 19.7, 14.1.

MALDI-TOF-MS: $m/z$= 1116 $[M+Na]^+$.

(Preparation of 2,3-di-O-p-methoxybenzyl-α-D-glucopyranoside-(1→1)-(2S, 3S,4R)-3-O-acetyl-2-octadecanamide-octadecane-4,6-succi nate(32))

To a solution of Compound 28 (32 mg, 0.026 mmol) in MeCN (870μL) was added molecular sieves 3Å (MS3Å) (30 mg). After stirring for 1 hour, to this suspension was added NIS (17.7 mg, 0.079 mmol) and TfOH (0.7 μL, 0.0079 mmol), subsequently stirring for 1.5 hours. The completion of the reaction was confirmed by TLC (toluene/EtOAc 1/1). This reaction mixture was filtered through a Celite® . The filtrate and the wash were combined, extracted with $CHCl_3$, washed with saturated aqueous $NaHCO_3$, saturated aqueous $Na_2S_2O_3$, and brine, dried ($Na_2SO_4$), and then concentrated. Purification by silica gel column chromatography (toluene/EtOAc 3/1) yielded 32 (2.5 mg, 9%).

[number 17-1]

$$[\alpha]_D = +1.9^\circ \quad (c\ 1.0, CHCl_3)$$

$^1$HNMR (500 MHz, $CDCl_3$):

δ 6.86-7.29 (m, 8 H, 2 Ph)
5.90 (d, 1 H, $J_{2,NH}$= 10.5 Hz, NH)
5.14 (dd, 1 H, H-3$^{Cer}$)

5.00 (dt, 1 H, H-$4^{Cer}$)

[number 17-2]

4.55-4.87 (4 d, 4 H, 2 Ph$CH_2$)
4.68 (d, 1 H, $J_{1,2}$ = 4.0 Hz, H-$1^{Glc}$)
4.46 (m, 1 H, H-$2^{Cer}$)
4.33 (neart, 1 H, H-$6'^{Glc}$)
4.24 (dd, 1 H, H-$6^{Glc}$)
4.02 (t, 1 H, H-$5^{Glc}$)
3.81 and 3.80 (2 s, 6 H, 2 OMe)
3.80 (m, 1 H, H-$1'^{Cer}$)
3.71 (t, 1 H, H-$1^{Cer}$)
3.61 (t, 1 H, H-$3^{Glc}$)
3.38 (dd, 1 H, $J_{1,2}$ = 4.0 Hz, H-$2^{Glc}$)
3.20 (t, 1 H, H-$4^{Glc}$)
2.55-2.87 (2 d, 4 H, -OCOC$H_2$C$H_2$COO-)
2.15 (s, 3 H, OAc)
1.11-2.40 (m, 58 H, -$CH_2$-)
0.89 (t, 6 H, 2 -$CH_3$)

$^{13}$C NMR (100 MHz, $CDCl_3$):

δ 173.5, 170.9, 130.6, 129.7, 129.5, 114.0, 114.0, 113.9, 113.9, 97.6, 80.8, 78.6, 75.0, 73.8, 72.9, 71.3, 70.4, 65.3, 55.3, 49.5, 36.9, 31.9, 31.3, 29.7, 29.7, 29.6, 29.6, 29.5, 29.4, 29.4, 29.1, 25.7, 24.8, 22.7, 20.8,14.1.

MALDI-TOF-MS: m/z = 1132 [$M$+Na]$^+$.

(Preparation of 2,3-di-O-p-methoxybenzyl-β-D-glucopyranoside-(1→1)-(2S, 3S,4R)-3-O-acetyl-2-octadecanamide-octadecane-4,6-succi nate(32))

To a solution of Compound 28 (32 mg, 0.026 mmol) in MeCN (870μL) was added MS3Å (30 mg). After stirring for 1 hour, to this suspension was added NIS (17.7 mg, 0.079 mmol) and TfOH (0.7 μL, 0.0079 mmol), subsequently stirring for 1.5 hours. The completion of the reaction was confirmed by TLC (toluene/EtOAc 1/1). This reaction mixture was filtrated through a Celite®. The filtrate and the wash were combined, extracted with $CHCl_3$, washed with saturated aqueous $NaHCO_3$, saturated aqueous $Na_2S_2O_3$, and brine, dried ($Na_2SO_4$), and then concentrated. Purification by silica gel column chromatography (toluene/EtOAc 3/1) yielded 32 (21 mg, 72%).

[number 18]

$$[\alpha]_D = -1.8^\circ \quad (c\ 1.0, CHCl_3)$$

$^1$H NMR (500 MHz, $CDCl_3$):

δ 6.86-7.30 (m, 8 H, 2 Ph)
6.26 (d, 1 H, $J_{2,NH}$= 11.0 Hz, NH)
5.22 (m, 1 H, H-$4^{Cer}$)
5.01 (m, 1 H, H-$3^{Cer}$)
4.85 (d, 1 H, $J_{gem}$ = 14.0 Hz, Ph$CH_2$)
4.75 (d, 1 H, $J_{gem}$ = 13.5 Hz, Ph$CH_2$)
4.64 (d, 1 H, $J_{gem}$ = 13.5 Hz, Ph$CH_2$)
4.59 (d, 1 H, $J_{gem}$ = 14.0 Hz, Ph$CH_2$)

4.46 (m, 1 H, H-$2^{Cer}$)
4.35 (t, 1 H, H-$6'^{Glc}$)
4.26 (m, 1 H, H-$6^{Glc}$)
4.25 (d, 1 H, $J_{1,2}$ = 8.5 Hz, H-$1^{Glc}$)
3.92 (dd, 1 H, $J_{gem}$ = 14.0 Hz, H-$1'^{Cer}$)
3.80 and 3.79 (2 s, 6 H, 2 OMe)
3.73 (dd, 1 H, $J_{gem}$ = 14.0 Hz, H-$1^{Cer}$)
3.54 (t, 1 H, H-$5^{Glc}$)
2.24-3.36 (m, 3 H, H-$2^{Glc}$, H-$3^{Glc}$, H-$4^{Glc}$)
2.50-2.80 (m, 4 H, $-OCOCH_2CH_2COO-$)
2.15 (s, 3 H, OAc)
1.10-2.23 (m, 58 H, $-CH_2-$)
0.90 (t, 6 H, 2 $-CH_3$)

$^{13}C$ NMR (100 MHz, $CDCl_3$):

δ 173.0, 171.4, 171.2, 171.0, 159.4, 159.4, 130.4, 130.1, 129.8, 129.6, 114.0, 113.8, 103.6, 83.1, 81.6, 74.9, 74.4, 73.3, 73.0, 70.2, 64.3, 55.2, 48.4, 36.7, 31.9, 29.7, 29.7, 29.6, 29.6, 29.5, 29.4, 29.3, 29.3, 25.6, 25.6, 25.4, 25.0, 22.7, 21.1, 14.1.

MALDI-TOF-MS: m/z = 1132 $[M+Na]^+$.

(Preparation of the sialyl α (2-3) galactose donor 33)

Based on the description of Hiromune Ando, Yusuke Koike, Hideharu Ishida, Makoto Kiso, et al., Tetrahedron Letters 44 (2003) 6883-6886, the following experiments were performed.

For example, sialyl α(2-3)galactose donor 33 was prepared using a similar method to that in (Preparation of Compound 49A).

((Preparation of methyl 5-acetamide-4,7,8,9-tetra-O-acetyl-3,5-dideoxy-D-glycer o-α-D-galacto-2-nonulopyranosylonate-(2→3)-(2,4,6-tri-O -benzoyl-β-D-galactopyranosyl)-(1→4)-2,3-di-O-p-methoxy benzyl-β-D-glucopyranoside-(1→1)-(2S,3S,4R)-3-O-acetyl-2-octadecanamide-octadecane-4,6-succinate(34))

To a solution of Compound 32 (23.9 mg, 0.022 mmol) and Compound 33 (60.0 mg, 0.054 mmol) in $CH_2Cl_2$ (430 μL) was added molecular sieves (AW300) (84 mg). After stirring for 1 hour, to this suspension was added TMSOTf (0.55 M solution in $CH_2Cl_2$, 0.2μL, 0.001 mmol). The progress of the reaction was monitored by TLC (toluene/EtOAc 1/3). After stirring for 7 hours, this reaction mixture was filtered through a Celite® pad. This filtrate was diluted with $CHCl_3$, and the organic phase was washed with saturated aqueous $NaHCO_3$, saturated aqueous $Na_2S_2O_3$, and brine, dried ($Na_2SO_4$), and then concentrated. Purification by silica gel column chromatography (toluene/EtOAc 2/5) yielded 34 (32 mg, 72%).

[number 19-1]

$$[\alpha]_D = +17.4^\circ \quad (c\ 0.5, CHCl_3)$$

$^1H$ NMR (500 MHz, $CDCl_3$):

δ 6.60-8.20 (m, 23 H, 5 Ph)
6.19 (d, H, $J_{2,NH}$= 8.6 Hz, $NH^{Cer}$)
5.67 (m, 1 H, H-$8^{Neu}$)
5.49 (t, 1 H, $J_{1,2}$ = 8.1 Hz, H-$2^{Gal}$)
5.36 (dd, 1 H, H-$4^{Gal}$)
5.24 (dd, 1 H, H-$7^{Neu}$)
5.15 (m, 1 H, H-$4^{Cer}$)

5.04 (d, H, $J_{1,2}$ = 8.1 Hz, H-$1^{Gal}$)
4.90-4.94 (m, 2 H, $J_{gem}$ = 10.5 Hz, PhC$H_2$, $NH^{Neu}$)
4.79-4.86 (m, 3 H, H-$3^{Gal}$, H-$4^{Neu}$, H-$3^{Cer}$)
4.74 (d, H, $J_{gem}$ = 10.5 Hz, PhC$H_2$)
4.54-4.65 (2 d, 2 H, $J_{gem}$ = 10.5 Hz, PhC$H_2$)
4.44 (m, 1 H, H-$2^{Cer}$)
4.40 (dd, 1 H, $J_{gem}$ = 12.5 Hz, H-$9'^{Neu}$)
4.17-4.32 (m, 4 H, H-$6^{Glc}$, H-$6'^{Glc}$, H-$6^{Gal}$, H-$6'^{Gal}$)
4.14 (d, H, $J_{1,2}$ = 7.8 Hz, H-$1^{Glc}$)
4.11 (t, 1 H, H-$5^{Gal}$)

[number 19-2]

4.03 (dd, 1 H, $J_{gem}$ = 12.5 Hz, H-$9^{Neu}$)
3.73-3.83 (m, 9 H, H-$5^{Neu}$, H-$1^{Cer}$, H-$1'^{Cer}$, 2 OMe)
3.58-3.66 (m, 5 H, H-$4^{Glc}$, H-$6^{Neu}$, OMe)
3.54 (t, 1 H, H-$3^{Glc}$)
3.41 (t, 1 H, H-$5^{Glc}$)
3.23 (t, 1 H, $J_{1,2}$ = 7.8 Hz, H-$2^{Glc}$)
2.40-2.70 (m, 4 H, $-OCOCH_2CH_2COO-$)
2.35 (dd, 1 H, H-3$eq^{Neu}$)
1.60 (m, 1 H, H-3$ax^{Neu}$)
1.51-2.20 (6 s, 18 H, OAc)
1.20-2.10 (m, 58 H, $-CH_2-$)
0.90 (t, 6 H, 2 $-CH_3$)

$^{13}$C NMR (100 MHz, $CDCl_3$):

δ 172.9, 171.1, 170.8, 170.7, 170.6, 170.3, 170.2, 170.0, 168.1, 165.6, 165.5, 165.0, 159.2, 158.8, 133.3, 133.1, 133.0, 130.9, 130.3, 129.9, 129.8, 129.7, 129.6, 129.6, 129.3, 128.8, 128.5, 128.2, 113.7, 113.4, 102.1, 101.2, 96.9, 82.0, 81.4, 78.7, 75.0, 74.5, 73.2, 72.9, 71.9, 71.6, 71.5, 70.9, 69.4, 68.1, 67.5, 66.5, 62.3, 61.6, 55.2, 55.1, 53.2, 48.8, 37.3, 36.6, 31.9, 30.6, 29.7, 29.7, 29.7, 29.6, 29.6, 29.5, 29.4, 29.4, 29.3, 25.5, 25.1, 23.1, 22.7, 21.4, 21.1, 20.8, 20.7, 20.4,14.1.

MALDI-TOF-MS: $m/z$ = 2080 $[M+Na]^+$.

To a solution of Compound 29 and a protected sugar chain donor (for example, Compound 33) in $CH_2Cl_2$ was added molecular sieves (AW300). After stirring, to this suspension was added TMSOTf (in $CH_2Cl_2$). The progress of the reaction was monitored by TLC. After stirring, this reaction mixture was filtered through a Celite® pad. The filtrate was diluted, and the organic phase was washed with saturated aqueous $NaHCO_3$, saturated aqueous $Na_2S_2O_3$, and brine, dried ($Na_2SO_4$), and then concentrated. Purification by silica gel column chromatography (toluene/EtOAc) yielded a product.

To a solution of Compound 30 and a protected sugar chain donor (for example, Compound 33) in $CH_2Cl_2$ was added molecular sieves (AW300). After stirring, to this suspension was added TMSOTf (in $CH_2Cl_2$). The progress of the reaction was monitored by TLC. After stirring, this reaction mixture was filtered through a Celite® pad. This filtrate was diluted, and then the organic phase was washed with saturated aqueous $NaHCO_3$, saturated aqueous $Na_2S_2O_3$, and brine, dried ($Na_2SO_4$), and then concentrated. Purification by silica gel column chromatography (toluene/EtOAc) yielded a product.

To a solution of Compound 31 and a protected sugar chain donor (for example, Compound 33) in $CH_2Cl_2$ was added molecular sieves (AW300). After stirring, to this suspension was added TMSOTf (in $CH_2Cl_2$). The progress of the reaction was monitored by TLC. After stirring, this reaction mixture was filtered through a Celite® pad. This filtrate was diluted, and the organic phase was washed with saturated aqueous $NaHCO_3$, saturated aqueous $Na_2S_2O_3$, and brine, dried ($Na_2SO_4$), and then concentrated. Purification by silica gel column chromatography (toluene/EtOAc) yielded a product.

(Deprotection)

(Deprotection of phytosphingosine GM3)

[Chem. 119]

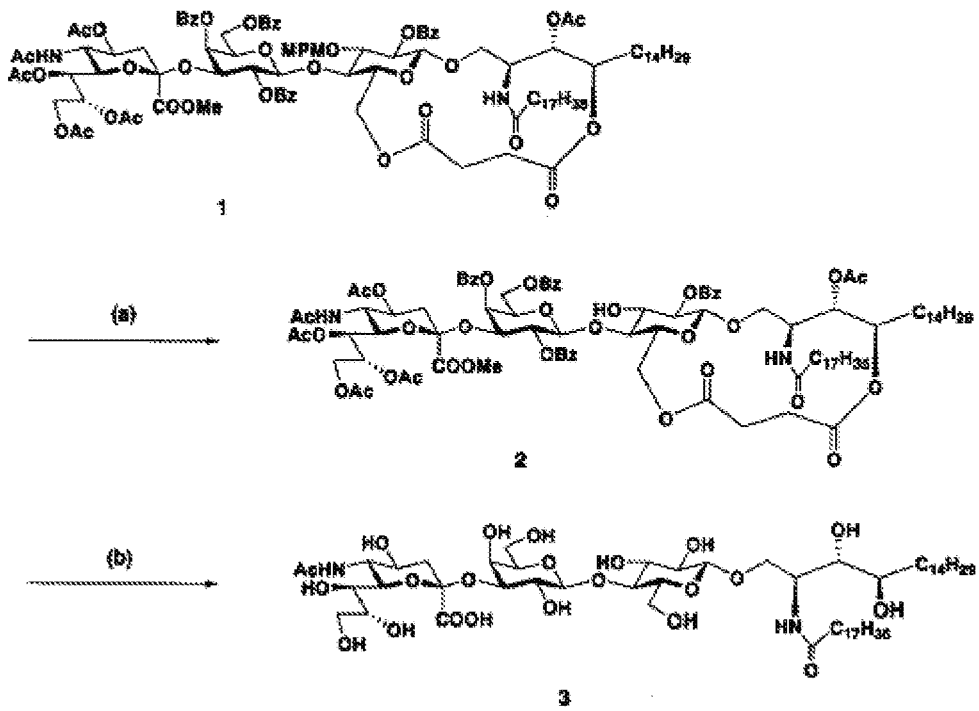


1

2

3

(a) TFA, $CH_2Cl_2$, r.t. (b) NaOMe, MeOH, and then $H_2O$, r.t. quant. (2 steps)

O-(5-acetamide-3,5-dideoxy-D-glycero-α-D-galacto-2-nonulopyranosylonic acid)-(2→3)-(O-β-D-galactopyranosyl)-(1→4)-O-β-D-glucop yranosyl-(1→1)-(2S,3S,4R)-2-octadecanamide-octadecane-1 ,3,4-triol(3).

To a solution of Compound 1 (38 mg, 0.0184 mmol) in 1,2-dichloroethane (800 μL) was added trifluoroacetic acid (370 μL). This mixture was stirred for 7 hours at room temperature. By TLC analysis (EtOAc/hexane 6/1), the starting material was confirmed to be completely consumed, and then $Et_3N$ was added at 0 degree Celsius. The reaction mixture was evaporated, and then co-evaporated with toluene. After evaporation, the residue was under reduced pressure for 30 hours using a pump. The residue was dissolved in MeOH (1.0 mL), and then NaOMe (7.2 mM solution in MeOH, 100 μL, 0.00518 mmol) was added at 0 degree Celsius. After stirring at room temperature for 26 hours (TLC monitoring: BuOH/MeOH/$H_2O$ 10/1/1), to this mixture was added $H_2O$ at 0 degree Celsius. This mixture was stirred at room temperature for 10 hours (TLC monitoring: BuOH/MeOH/$H_2O$ 10/1/1). This reaction mixture was neutralized using Dowex ($H^+$), and then filtrated. The filtrate was combined with the wash solution, and then concentrated. The residue was subjected to column chromatography of 200 (g) of Sephadex LH-20 (MeOH) to yield Compound 3 (22.1 mg, quant.).

[Chem. 120]

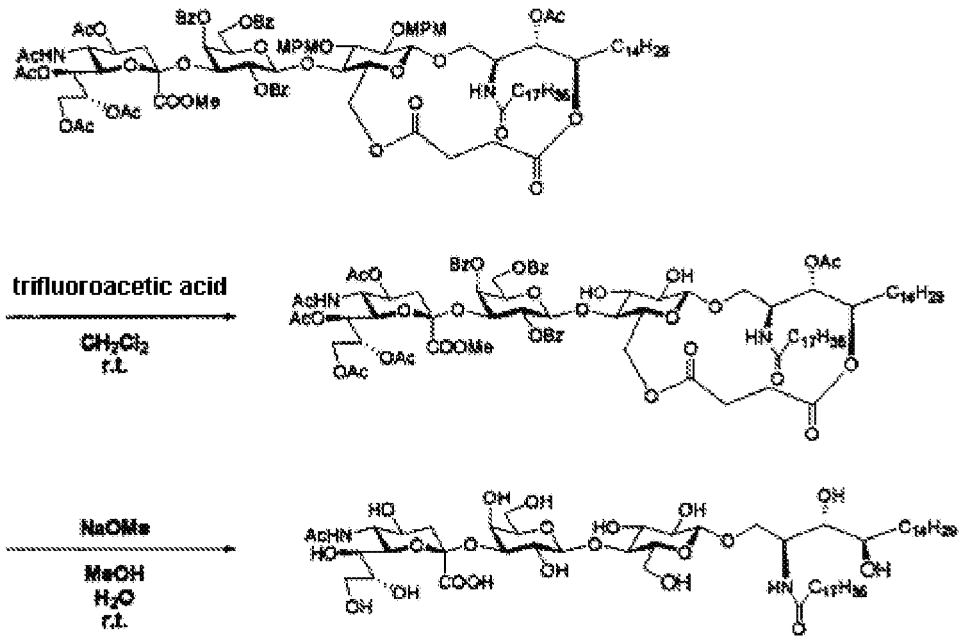


Using a similar method to that described above, for example, Compound 34 in the trifluoroacetic acid in $CH_2Cl_2$ at room temperature is stirred, and then treated with NaOMe in MeOH and $H_2O$ at room temperature to yield a deprotected product.

(Crosslink of sphingosine-type ceramide with glucose (Glc))

[Chem. 121]

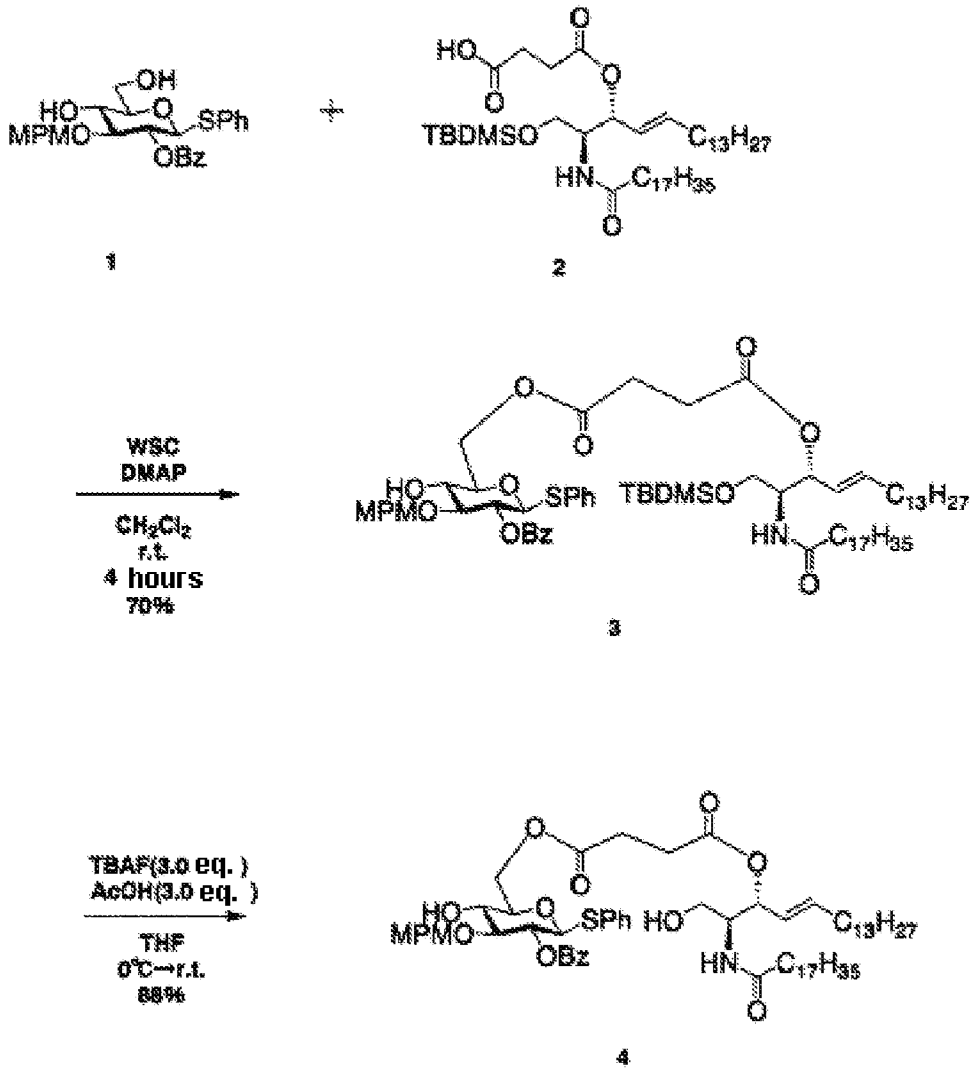


1 2 3 4

Under argon atmosphere, Compound 2 (1.6g, 2. 07 mmol) was dissolved in $CH_2Cl_2$ (19 mL), and then WSC (1.1 g, 5.64 mmol), DMAP (69 mg, 0.564 mmol), and Compound 1 (935 mg, 1.88 mmol) were added thereto, subsequently stirring at room temperature for 4 hours. The reaction solution was diluted with chloroform, washed with water and brine, and then purified by column chromatography (AcOEt/hexane=1/3) to yield Compound 3 (1.64g).

Under argon atmosphere, Compound 3 (280 mg, 0.223 mmol) was dissolved in THF (2.3 mL), and then AcOH (40 μL, 0.669 mmol) and TBAF (670 μL, 0.669 mmol) were added at 0 degree Celsius, subsequently reacting at 0 degree Celsius for 1 hour. After 1 hour, the reaction temperature was warmed up to room temperature, subsequently stirring for 4 hours. The reaction solution was diluted with chloroform, washed with sodium hydrogen carbonate, and brine, and then purified by column chromatography (AcOEt/hexane=1/1.3) to yield Compound 4 (225 mg).

(Intramolecular glycosylation of sphingosine-type ceramide-succinic acid -glucose(Glc))

[Chem. 122]

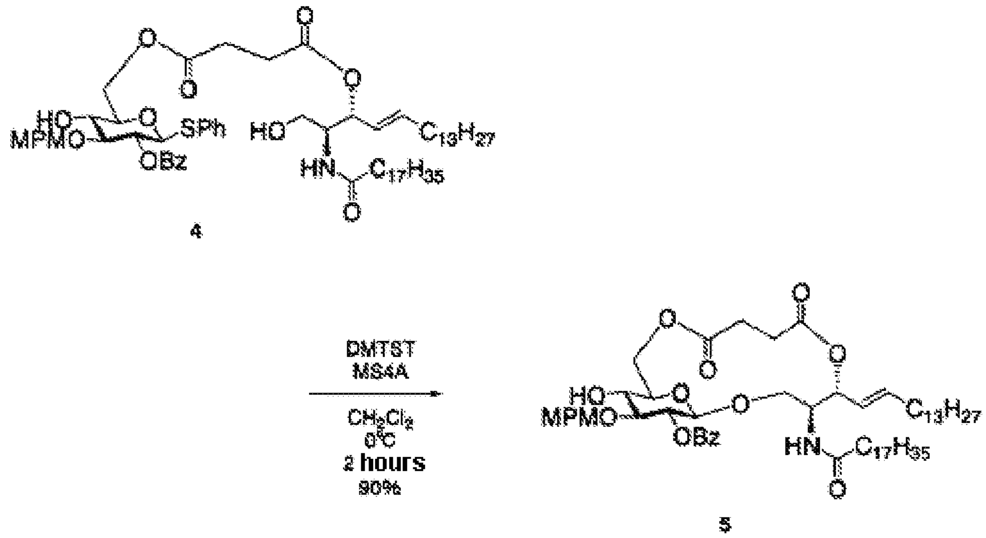


4

5

Under argon atmosphere, Compound 4 (72 mg, 0.0630 mmol) was dissolved in $CH_2Cl_2$ (13 mL), and then MS4A (70 mg) was added thereto, followed by stirring at room temperature for 1 hour. After one hour, the reaction temperature was set to 0 degree Celsius, and then DMTST (102 mg, 0.189 mmol) was added thereto. After stirring at 0 degree Celsius for 2 hours, the reaction was completed, followed by filtration through a Celite®. The filtrate was diluted with chloroform, washed with sodium hydrogen carbonate, and brine, and then purified by column chromatography ($CHCl_3$/MeOH=130/1) to yield Compound 5 (58 mg).

(Synthesis of GM3: (NeuGal donor + GlcCer acceptor))

[Chem. 123]

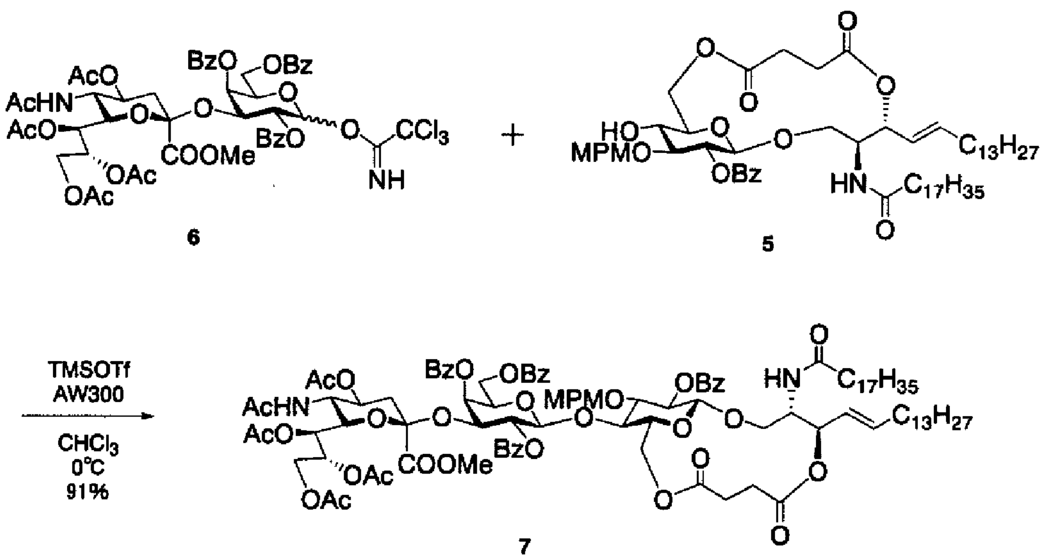


6

5

7

Under argon atmosphere, Compound 6 (21 mg, 0.0189 mmol) and Compound 5 (13 mg, 0.0126 mmol) were dissolved in $CHCl_3$ (420 μL), AW300 (100 mg) was added thereto, followed by stirring at room temperature for 1 hour. After 1 hour, the reaction temperature was set to 0 degree Celsius, and then was added TMSOTf (0.3μL, 0.00151mmol), subsequently stirring at 0 degree Celsius for 2 and half hours. The reaction was completed, followed by filtration through

a Celite®. The filtrate was diluted with chloroform, washed with sodium hydrogen carbonate, and brine, and then purified by column chromatography (toluene/AcOEt/MeOH=50/20/1) to yield Compound 7 (23 mg).

(Deprotection of GM3)

[Chem. 124]

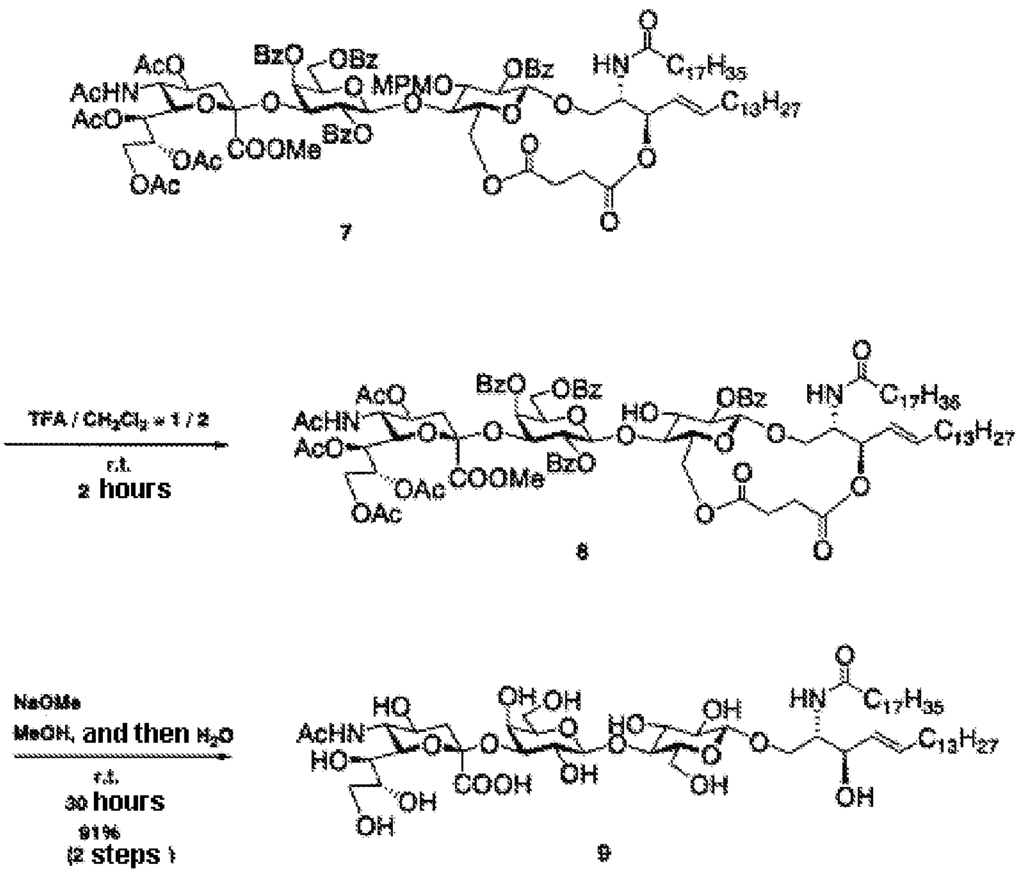


7

8

9

Under argon atmosphere, Compound 7 (35 mg, 0.0177 mmol) was dissolved in $CH_2Cl_2$ (700 μl), TFA (350 μl) was added at 0 degree Celsius, followed by stirring at room temperature for 2 hours. After the reaction was completed, $Et_3N$ (2 mL) was added thereto at 0 degree Celsius. After azeotropy with toluene, the residue was dried *in vacuo* for 12 hours (Compound 8). Under argon atmosphere, Compound 8 was dissolved in MeOH (1.0 mL), NaOMe (0.34 mg, 0.00177 mmol) was added thereto at 0 degree Celsius, subsequently stirring at room temperature for 17 hours. After 17 hours, $H_2O$ (0.5 mL) was added, followed by stirring for 13 hours. The reaction was completed, neutralized with Dowex ($H^+$), and then filtered through cotton. The filtrate was gel-filtrated with Sephadex LH-20 (MeOH) to yield Compound 9 (19 mg).

(Synthesis of GM2: (GM2 donor + GlcCer acceptor))

[Chem. 125]

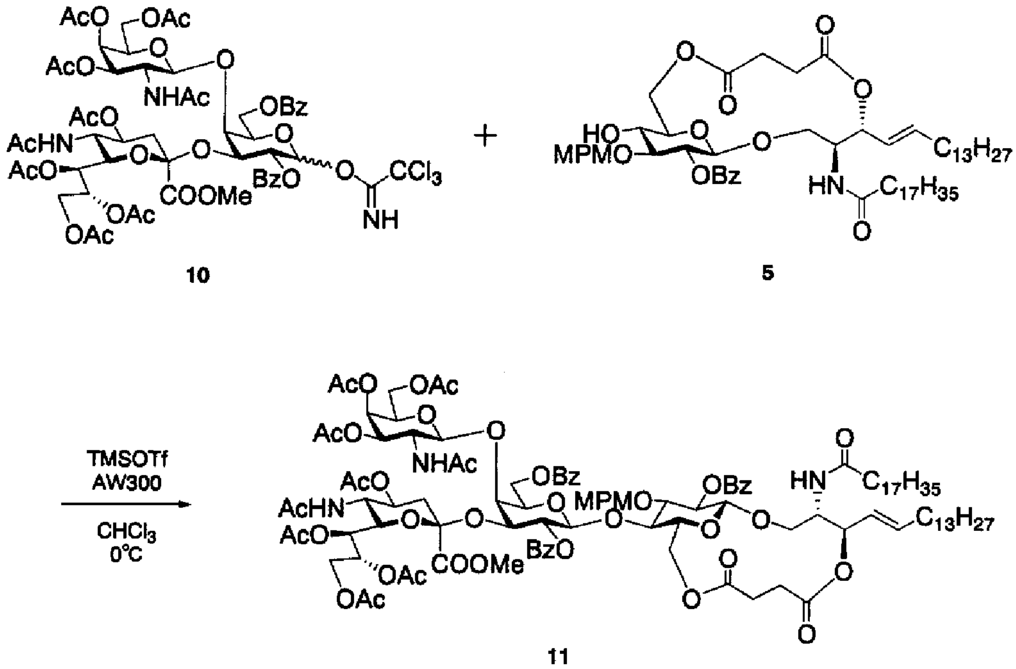


10

5

11

Under argon atmosphere, Compound 10 and Compound 5 was dissolved in $CHCl_3$, AW300 was added thereto, followed by stirring at room temperature. The reaction temperature was set to 0 degree Celsius, and then TMSOTf was added thereto, subsequently stirring at 0 degree Celsius. The reaction was completed, followed by filtration through a Celite®. The filtrate was diluted with chloroform, washed with sodium hydrogen carbonate, and brine, and then purified by column chromatography (toluene/AcOEt/MeOH) to yield Compound 11.

(Deprotection of GM2)

[Chem. 126]

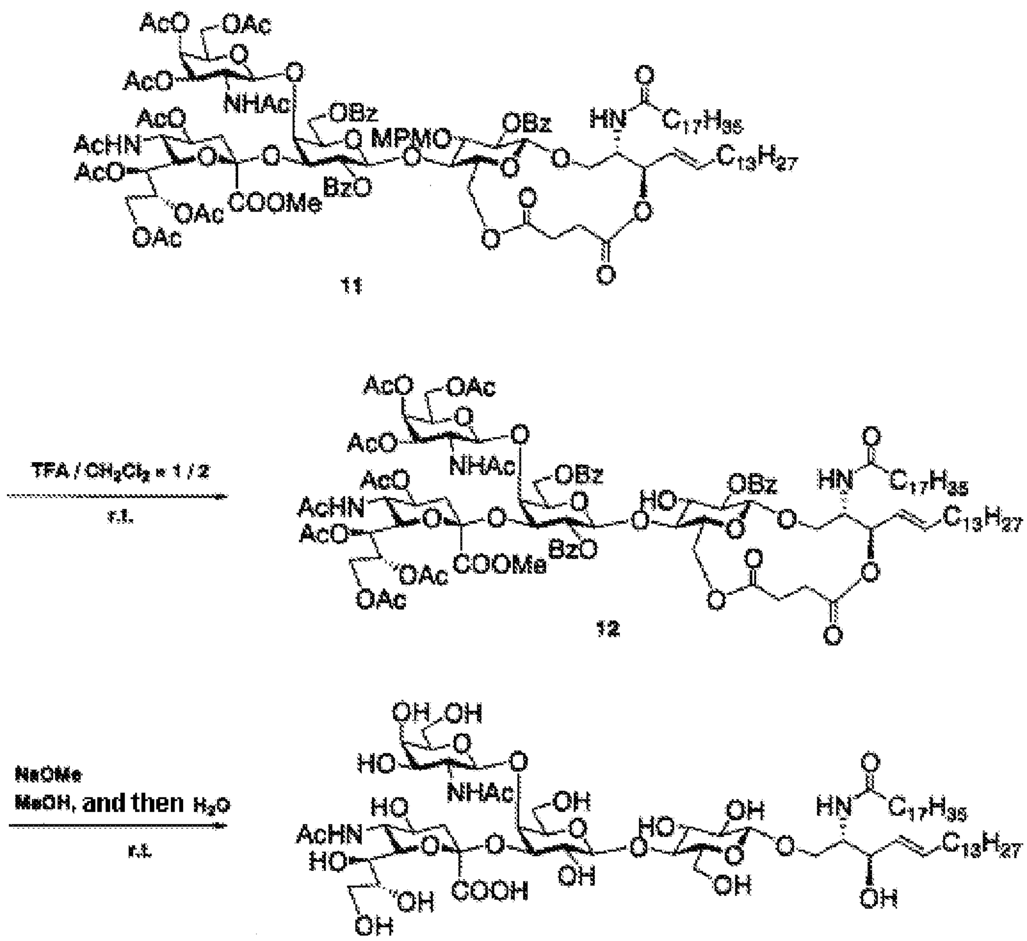


11

12

13

Under argon atmosphere, Compound 11 was dissolved in $CH_2Cl_2$, and then TFA was added at 0 degree Celsius, subsequently stirring at room temperature. After the reaction was completed, $Et_3N$ was added thereto at 0 degree Celsius, followed by azeotropy with toluene. Then, the residue was dried *in vacuo* (Compound 12). Then, under argon atmosphere, Compound 12 was dissolved in MeOH, and then NaOMe was added thereto at 0 degree Celsius, subsequently stirring at room temperature. Then, $H_2O$ was added, and then stirred. The reaction was completed, neutralized with Dowex ($H^+$), and then filtered through cotton. The filtrate was gel-filtrated with Sephadex LH-20 (MeOH) to yield Compound 13.

(Synthesis of GM1: (GM1 donor + GlcCer acceptor)

[Chem. 127]

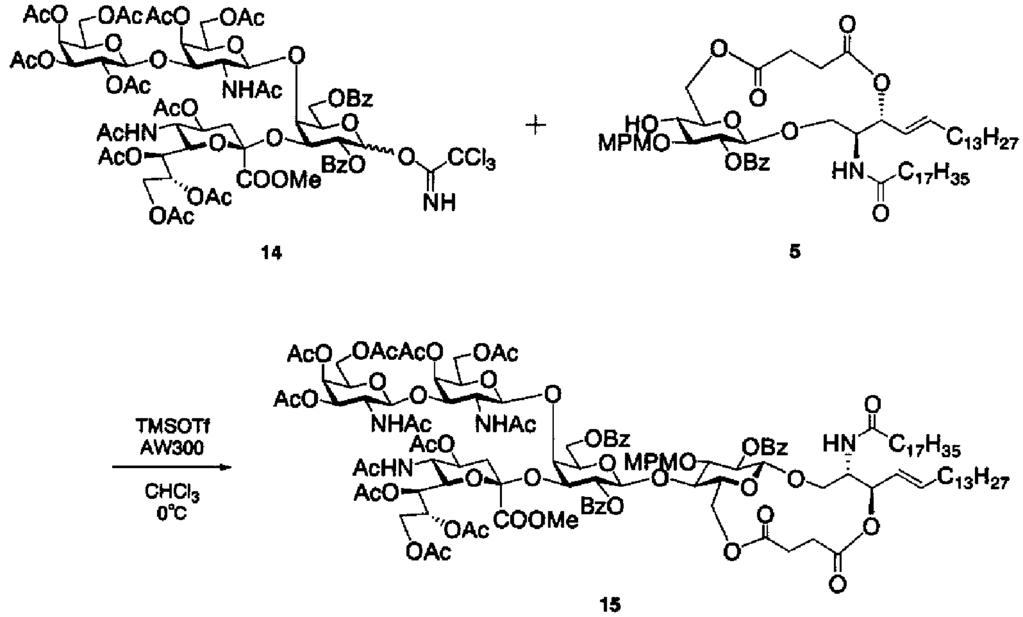


14

5

15

Under argon atmosphere, Compound 14 and Compound 5 were dissolved in $CHCl_3$, and then AW300 was added thereto, subsequently to stirring at room temperature. The reaction temperature was set to 0 degree Celsius, and then TMSOTf was added, subsequently stirring at 0 degree Celsius. The reaction was completed, followed by filtration through a Celite®. The filtrate was diluted with chloroform, washed with sodium hydrogen carbonate, and brine, and then purified by column chromatography (toluene/AcOEt/MeOH) to yield Compound 15.

(Deprotection of GM1)

[Chem. 128]

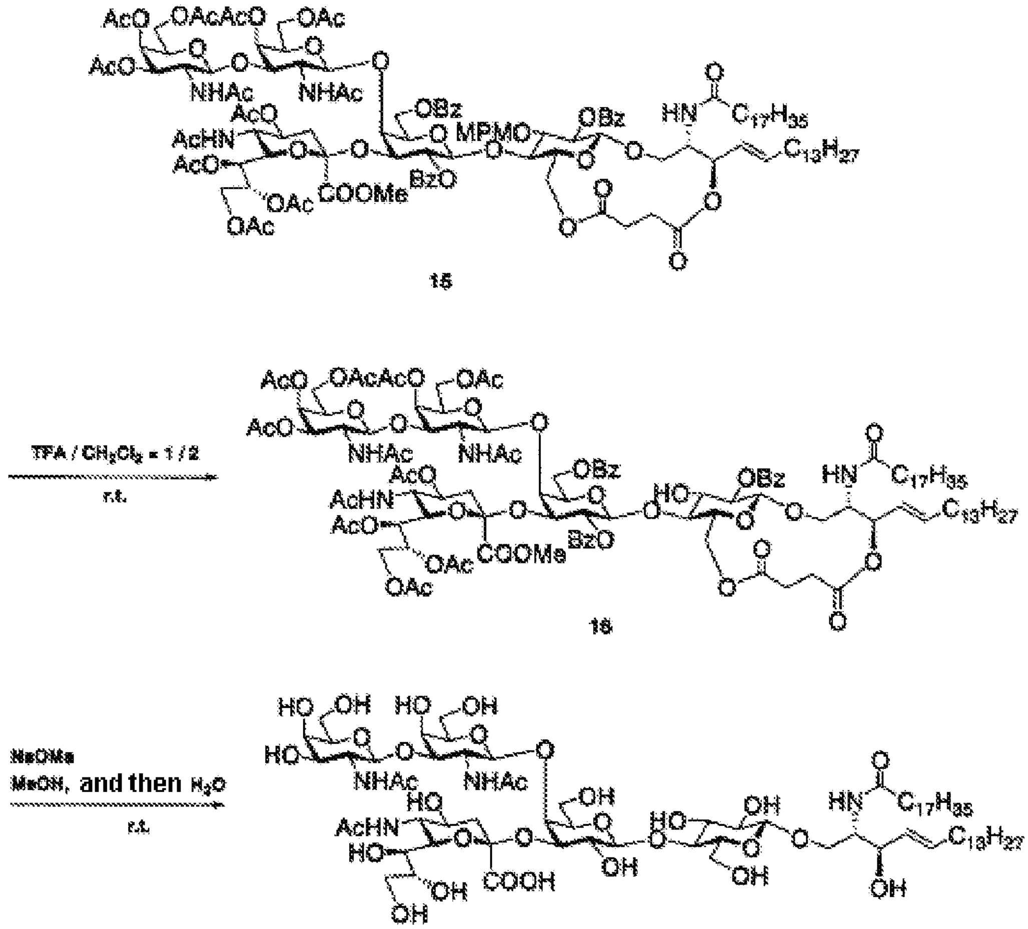


15

16

17

Under argon atmosphere, Compound 15 was dissolved in $CH_2Cl_2$, and then TFA was added thereto at 0 degree Celsius, subsequently stirring at room temperature. After the reaction was completed, Et3N was added thereto at 0 degree Celsius, followed by azeotropy with toluene. Then, the residue was dried in vacuo (Compound 16). Then, under argon atmosphere, Compound 16 was dissolved in MeOH, and then NaOMe was added thereto at 0 degree Celsius, subsequently stirring at room temperature. Then, $H_2O$ was added, and then stirred. The reaction was completed, neutralized with Dowex ($H^+$), and then filtered through cotton. The filtrate was gel-filtrated with Sephadex LH-20 (MeOH) to yield Compound 17.

(Synthesis of GM4: (GM4 donor + GlcCer acceptor))

[Chem. 129]

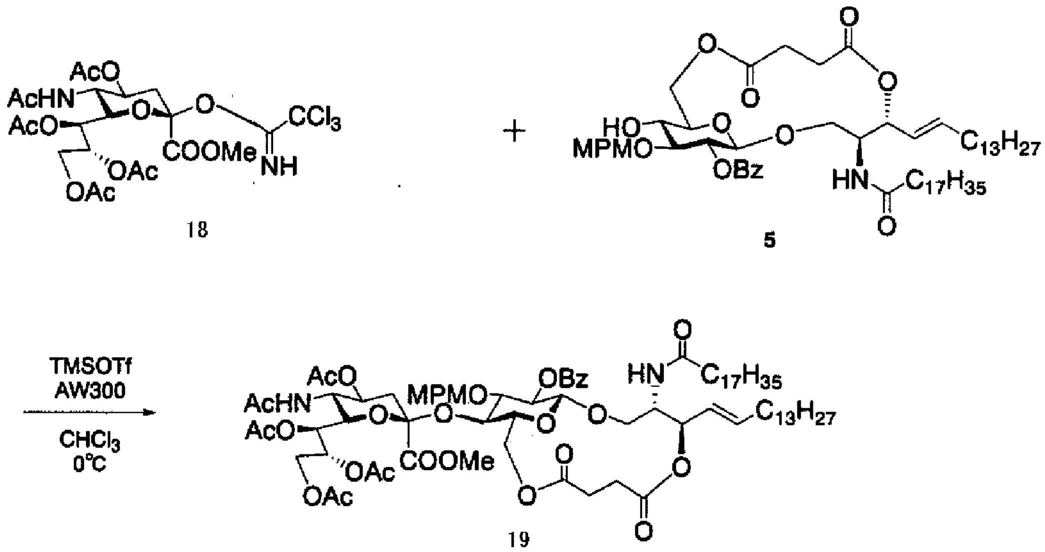


18

5

19

Under argon atmosphere, Compound 18 and Compound 5 was dissolved in $CHCl_3$, AW300 was added thereto, subsequently stirring. Then, TMSOTf was added, subsequently stirring. The reaction was completed, followed by filtration through a Celite®. The filtrate was diluted with chloroform, washed with sodium hydrogen carbonate, and brine, and then purified by column chromatography to yield Compound 19.

(Deprotection of GM4)

[Chem. 130]

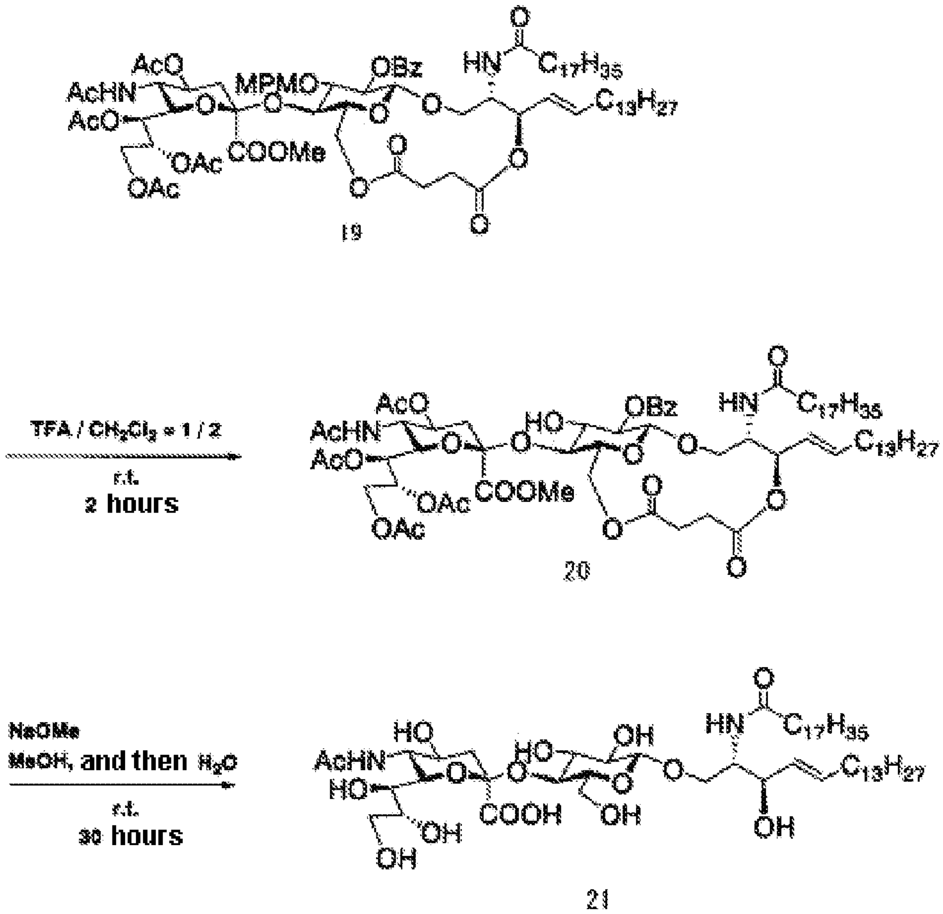


19

20

21

Under argon atmosphere, Compound 19 was dissolved in $CH_2Cl_2$, and then TFA was added thereto, subsequently stirring. The reaction was completed, and then $Et_3N$ was added, followed by azeotropy with toluene. The residue was dried *in vacuo* (Compound 20). Then, under argon atmosphere, Compound 20 was dissolved in MeOH, and then NaOMe was added thereto, subsequently stirring. $H_2O$ was added, and then stirred. The reaction was completed, neutralized with Dowex ($H^+$), and then filtered through cotton. The filtrate was gel-filtrated with Sephadex LH-20 (MeOH) toyieldCompound 21.

(Preparation of the lactose portion)

[Chem. 131]

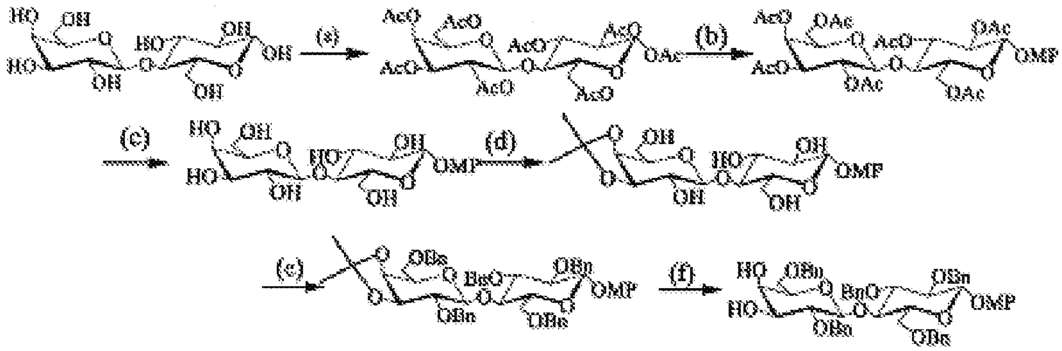


(a) $Ac_2O$, pyridine, r.t.
(b) p- methoxyphenol, $BF_3OEt_2$, triethylamine, $CH_2Cl_2$, 0 °C→60 °C
(c) NaOMe, MeOH, r.t., 61% (3 steps)
(d) 2,2- dimethoxypropane, (±)- camphor-10- sulfonic acid, DMF, 80 °C
(e) NaH, BnBr, DMF, 0 °C→r.t., 61% (2 steps) (f) 80% AcOHaq, 50 °C, 81%

Based on the description of Hiromune Ando, Yusuke Koike, Hideharu Ishida, Makoto Kiso, et al., Tetrahedron Letters 44 (2003) 6883-6886, the reactions were performed under the conditions described in the above table, the lactose portion was prepared.

(Preparation of the sialic acid portion)

[Chem. 132]

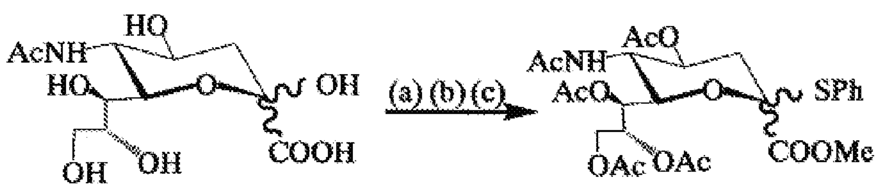


(a) Dowex, MeOH, pH 2, r.t., (b) $AC_2O$, pyridine, r.t.
(c) PhSH, $BF_3$ $OEt_2$, $CH_2Cl_2$, 0°C→r.t., 49% (3 steps)

Based on the description of Hiromune Ando, Yusuke Koike, Hideharu Ishida, Makoto Kiso, et al., Tetrahedron Letters 44 (2003) 6883-6886, the reactions were performed under the conditions described in the above table, the sialic acid portion was prepared.

(Preparation of the sialic acid portion)

(Synthesis of trisaccharide)

[Chem. 133]

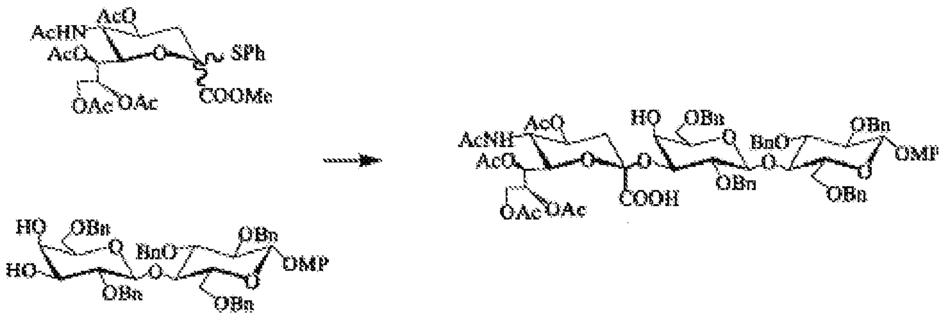

Table 1 Examination of synthesis condition

| Entry | **Acceptor** | **Donor** | MS | TfOH | TMSOTf | **Solvent** | **Temp.**(˚C) | **Time** |
|---|---|---|---|---|---|---|---|---|
| 1 | 500 mg | 1.2 | 1.4-2.9 | 0.14-0.42 | | CH3CN→CHECN: CH2Cl2=5:1 | -30-r.t | 2 days |
| 2 | 100 mg | 1.2 | 1.4 | 0.14-0.42 | 0.14 | CH3CH:CH2Cl2-5:1 | -30-r.t. | 3 days |
| 3 | 100 mg | 1.2 | 2.6 | 0.8 | | CH3CN:CH2Cl2=5:1 | -30-D | 2 days |
| 4 | 100 mg | 1.5 | 3 | 0.75 | | CH3CH:CH2Cl2=5:1 | -30 | 3 days |
| 5* | 5 mg | 1.5 | 3 | 0.75 | | CH2Cl2 | r.t. | 1 day |

Based on the description of Hiromune Ando, Yusuke Koike, Hideharu Ishida, Makoto Kiso, et al., Tetrahedron Letters 44 (2003) 6883-6886, the reactions were performed under the conditions described in the above table, the sialic acid portion was prepared.

(Change 1 of sialic acid)

[Chem. 134]

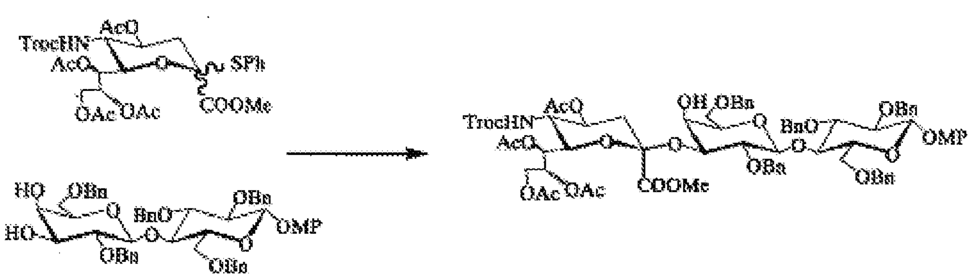

NIS-TfOH 1.5 eq. , MS-3Å, $CH_3CN:CH_2Cl_2$=5:1, -30 °C, 58%, α : β =7:2

Based on the description of Hiromune Ando, Yusuke Koike, Hideharu Ishida, Makoto Kiso, et al., Tetrahedron Letters 44 (2003) 6883-6886, the reaction was performed under the conditions described in the above table, the sialic acid portion was changed.

(Change 2 of the sialic acid portion)

[Chem. 135]

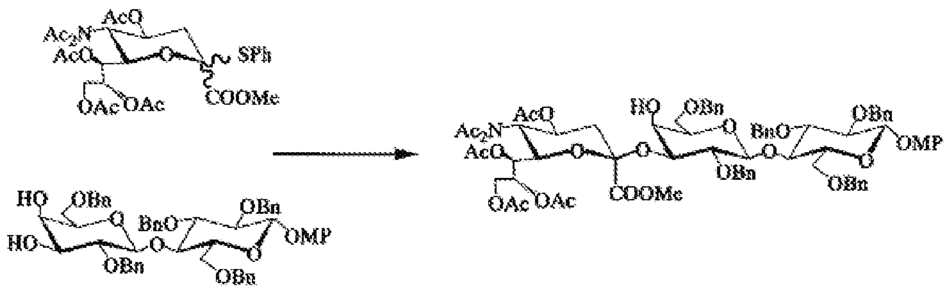

NIS-TfOH 1.5 eq. , MS-3 Å, $CH_3CN:CH_2Cl_2$=5:1, -30 °C, 60%, α : β =3:1

Based on the description of Hiromune Ando, Yusuke Koike, Hideharu Ishida, Makoto Kiso, et al., Tetrahedron Letters 44 (2003) 6883-6886, the reaction was performed under the conditions described in the above table, the sialic acid portion was changed.

(Example 2. Preparation of GM3)

(Preparation of the Compound 31A)

[Chem. 136]

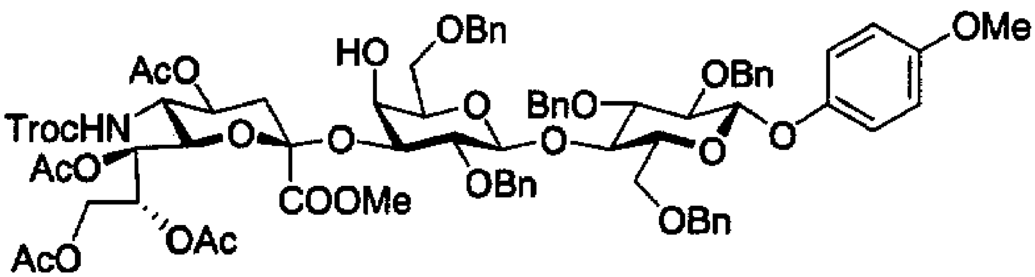

4-methoxyphenyl[methyl4,7,8,9-tetra-O-acetyl-3,5-dideox y-5-(2,2,2-trichloroethoxycarbamoyl)-D-glycero-α-D-gala cto-2-nonulopyranosylonate]-(2→3)-(2,6-O-di-benzyl-β-D-galactopyranosyl)-(1→4)-2,3,6-0-tri-benzyl-β-D-glucopyr anoside (31A)

[Chem. 137]

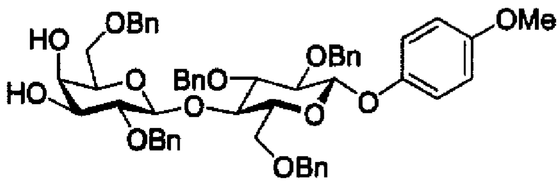

25A

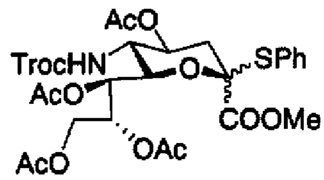

30A

Compound 25A (500 mg, 0.557 mmol: prepared according to Borbas, A.; Csavas, M.; Szilagyi, L.; Majer, G.; Liptak, A. J. Carbohydr. Chem. 2004, 23, 133-146.) and Compound 30A (796 mg, 1.11 mmol: prepared according to Ando, H.; Koike, Y.; Ishida, H.; Kiso, M. Tetrahedron Lett. 2003, 44, 6883-6886.) was dissolved in 10:1 $C_2H_5CN-CH_2Cl_2$ (8.5 mL), subsequently stirring in the presence of MS-4A (1.3g) at room temperature for 1 hour. Then, N-iodosuccinimide (501 mg, 2.22 mmol) was added, subsequently cooling to -50 degrees Celsius, and then trifluoromethanesulfonic acid (24 μl, 0.274 mmol) was added, followed by stirring at -50 degrees Celsius for 6 hours. After TEA was added to neutralize the reaction mixture, the solid was filtrated through a Celite®, and then washed with chloroform. The filtrate and the wash solution were combined, and then washed with $NaHCO_3$, sat., $H_2O$, $Na_2S_2O_3$, $H_2O$, sat., and brine, successively. The resulting organic layer was dried with $Na_2SO_4$, and then filtrated to separate the organic layer from the solid. The filtrate and the washing solution were combined, and then evaporated under reduced pressure. The resulting syrup was purified by silica gel chromatography (toluene:AcOEt=6:1) to yield Compound 31α (468 mg, 45%) and further Compound 31β (146 mg, 14%) as a by-product.

[Chem. 138]

[a]D-15.7° ($c$ = 0.3 $CHCl_3$);

$^1$H-NMR(500MHz,$CDCl_3$):δ(ppm)1.87,1.99,2.09(3s,12H,4AcO), 1.98(t,1H,$J_{3em,3ax,4}$=12.6Hz,H-3$ax^{Neu}$), 2.57(dd,1H,$J_{3eq,4}$=4.6Hz,H-3$eq^{Neu}$), 2.67(d,1H,$J_{4,OH}$=2.8Hz,$OH^{Gal}$), 3.47-3.51(m,3H,H-$4^{Glc}$,H-$6^{Glc}$,H-$6^{Gal}$), 3.55(dd,1H,$J_{2,3}$=9.1Hz,H-$2^{Gal}$), 3.60-3.72(m,4H,H-$5^{Glc}$,H-$6'^{Glc}$,H-$6'^{Gal}$,H-$5^{Neu}$), 3.64(t,1H,$J_{2,3}$=8.0Hz,H-$2^{Glc}$), 3.76(s,3H,OMe), 3.77(s,3H,COOMe), 3.81-3.83(m,1H,H-$5^{Gal}$), 3.83(s,1H,H-$4^{Gal}$), 3.98(dd,1H,$J_{8,9}$=5.7Hz,H-$9^{Neu}$), 4.00(t,1H,$J_{3,4}$=7.4Hz,H-$3^{Glc}$), 4.06(dd,1H,$J_{3,4}$=2.9Hz,H-$3^{Gal}$), 4.09(dd,1H,$J_{6,7}$=1.7Hz,H-$6^{Neu}$),4.27(dd,1H,$J_{gem}$=12.6Hz,H-$9'^{Neu}$), 4.34(d,1H,$J_{gem}$=12Hz,O$CH_2$Ph), 4.42-4.49(m,4H,2O$CH_2$Ph,O$CH_2Cl_2$), 4.58(d,1H,$J_{1,2}$=7.4Hz,H-$1^{Gal}$), 4.68(d,1H,$J_{gem}$=11.4Hz,O$CH_2$Ph), 4.75-4.81(m,4H,4O$CH_2$Ph), 4.83(d,1H,$J_{1,2}$=7.4Hz,H-$1^{Glc}$), 4.87(d,1H,$J_{gem}$=11.5Hz,O$CH_2$Ph), 4.94(m,1H,H-$4^{Neu}$), 4.98(d,1H,$J_{gem}$=11.4Hz,NH), 5.00(d,1H,$J_{gem}$=10.9Hz,O$CH_2$Ph), 5.36(dd,1H,$J_{7,8}$=8.0Hz,H-$7^{Neu}$), 5.42(m,1H,H-$8^{Neu}$), 6.76-7.34(m,29H,$OCH_2$*Ph*)

$^{13}$C-NMR(125MHz,$CDCl_3$):δ(ppm)36.5, 51.4, 53.0, 55.5, 62.1, 67.2, 67.8, 68.4, 68.5, 72.2, 72.4, 73.0, 73.3, 74.5, 74.9, 75.0, 75.2, 75.4, 76.4, 76.5, 78.3, 81.6, 82.9, 95.2, 98.1, 102, 102, 114 ,118, 127, 127, 127, 127, 127, 128, 128, 128, 128, 128, 138, 138, 138, 138, 139, 151, 154, 155, 168, 169, 170, 170;
MS(Positive ion MALDI-TOF MS.):$C_{75}H_{84}C_{13}NO_{25}$ : $m/z$ calcd. for $[M+Na]^+$:1526.43, found :1526.46.

[Chem. 139]

[a]D-6.02° ($c$ = 0.2 $CHCl_3$);

$^1$H-NMR(500MHz, $CDCl_3$): δ (ppm)1.94, 2.02, 2.07, 2.15(4s, 12H, 4AcO), 1.99-2.04(m, 1H, H-3ax$^{Neu}$), 2.35(s, 1H, OH$^{Gal}$), 2.57(dd, 1H, $J_{gem}$=13.7Hz, $J_{3eq,4}$=4.5Hz, H-3eq$^{Neu}$), 3.42-3.44(m, 2H, H-6$^{Glc}$, H-3$^{Gal}$), 3.52-3.56(m, 2H, H-4$^{Glc}$, H-5$^{Gal}$), 3.64-3.86(m, 8H, H-2$^{Glc}$, H-5$^{Glc}$, H-6'$^{Glc}$, H-2$^{Gal}$, H-4$^{Gal}$, H-6$^{Gal}$, H-6'$^{Gal}$, H-5$^{Neu}$), 3.69(s, 3H, OMe), 3.75(s, 3H, COOMe), 3.93(dd, 1H, $J_{gem}$=9.7Hz, H-9$^{Neu}$), 4.01(m, 1H, H-8$^{Neu}$), 4.07-4.14(m, 3H, H-3$^{Glc}$, 2OC$H_2$Ph), 4.20(dd, 1H, $J$=1.7Hz$^{Neu}$, H-6$^{Neu}$), 4.38-4.47(m, 3H, 3OC$H_2$Ph), 4.58-4.62(m, 4H, H-1$^{Gal}$, H-9'$^{Neu}$, NH, OC$H_2$Ph), 4.79(dd, 1H, $J$gem=10.9Hz, OC$H_2$Ph), 4.84(d, 1H, $J_{1,2}$=7.4Hz, H-1$^{Glc}$), 4.88(d, 1H, $J$gem=12.0Hz, OC$H_2$Ph), 4.97-5.02(m, 3H, 3OC$H_2$Ph), 5.10(m, 1H, H-4$^{Neu}$), 5.20(s, 1H, H-7$^{Neu}$), 6.76-7.39(m, 29H, OCH2$Ph$)

$^{13}$C-NMR(125MHz, $CDCl_3$): δ(ppm)14.1, 20.7, 21.0, 21.1, 21.4 29.6, 34.5, 50.8, 53.1, 55.6, 60.3, 62.5, 67.2, 68.0, 68.4, 68.4, 68.7, 70.6, 71.4, 72.6, 73.0, 73.4, 74.5, 75.1, 75.2, 75.3, 75.5, 76.4, 77.9, 78.5, 79.1, 81.6, 82.5, 95.4, 99.0, 102, 102, 114, 118, 127, 127, 127, 127, 127, 128, 128, 128, 128, 128, 128, 128, 128, 128, 128, 129, 138, 138, 138, 138, 138, 138, 138, 138, 138, 138, 151, 154, 155, 168, 169, 170, 170, 171.

MS(Positive ion MALDI-TOF MS.): $C_{75}H_{84}C_{13}NO_{25}$ : $m/z$ calcd. for $[M+Na]^+$:1526.43, found :1526.41.

(Preparation of Compound 33A)

[Chem. 140]

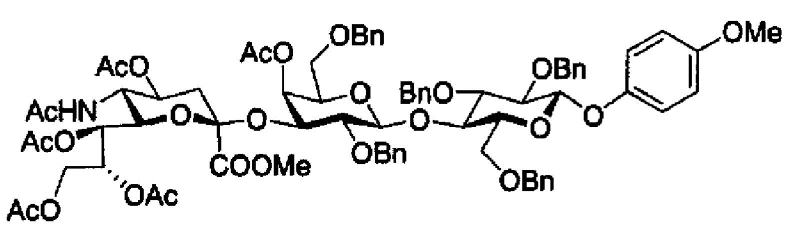

4-Methoxyphenyl[methyl5-acetamide-4,7,8,9-tetra-O-a cetyl-3,5-dideoxy-D-glycero-a-D-galacto-2-nonulopyranos ylonate]-(2→3)-(4-O-acetyl-2,6-O-di-benzyl-O-D-galactop yranosyl)-(1→4)-2,3,6-O-tri-benzyl-β-D-glucopyranoside (33A)

Compound 31A (50 mg, 0.0333 mmol) was dissolved in AcOH-$(CH_2Cl)_2$ (3:1) (1 mL), and then stirred in the

presence of Zn (Cu) (250 mg) at 50 degrees Celsius for 1.5 hours. The solid was filtered through a Celite®, and then washed with chloroform. The filtrate and the wash solution were combined, and then washed with $H_2O$, $NaHCO_3$ saturated, $H_2O$, and brine, successively. The resulting organic layer was dried with $Na_2SO_4$, and then the organic layer was separated from the solid by filtration. After concentrating with toluene under reduced pressure, the resulting syrup was dried *in vacuo* for 1 hour, and then dissolved in pyridine. After cooling in an ice bath, acetic anhydride (50 μl) and a catalytic amount of 4-dimethylaminopyridine (DMAP) were added. The ice bath was removed, followed by stirring for 2.5 hours. Then, MeOH was added to quench the reaction, and subsequently concentrated under reduced pressure. The resulting syrup was washed with 2N HCl, $H_2O$, $NaHCO_3$ saturated, $H_2O$, and brine, successively. The resulting organic layer was dried with $Na_2SO_4$, and then the organic layer was separated from the solid by filtration. The filtrate and the wash solution were then combined, followed by concentration under reduced pressure. The resulting syrup was purified by silica gel chromatography ($CHCl_3$:MeOH=60:1) to yield Compound 33A (35 mg, 75%).

[Chem. 141]

$[a]_D^0$ -45.0(c = 0.2 $CHCl_3$);

$^1$H-NMR(600MHz,$CDCl_3$):δ(ppm)1.75, 1.85, 1.97, 2.00, 2.01, 2.10 (6s, 18H, 5AcO, AcN), 1.83-1.87 (m, 1H, H-3ax$^{Neu}$), 2.60 (dd, 1H, $J_{gem}$=12.3Hz, $J_{3eq,4}$=4.6Hz, H-3eq$^{Neu}$), 3.28-3.29 (m, 2H, H-5$^{Gal}$, H-6$^{Gal}$), 3.44-3.48 (m, 2H, H-4$^{Glc}$, H-2$^{Gal}$), 3.57-3.76 (m, 5H, H-2$^{Glc}$, H-5$^{Glc}$, H-6$^{Glc}$, H-6'$^{Gal}$, H-6$^{Neu}$), 3.75 (s, 3H, OMe), 3.81-3.83 (m, 1H, H-6$^{Glc}$), 3.83 (s, 3H, COOMe), 3.96-4.01 (m, 2H, H-3$^{Glc}$, H-9$^{Neu}$), 4.06 (d, 1H, $J_{gem}$=10.2Hz, $OCH_2Ph$), 4.09 (d, 1H, $J_{gem}$=10.2Hz, $OCH_2Ph$), 4.20 (d, 1H, $J_{gem}$=12.3Hz, $OCH_2Ph$), 4.32-4.38 (m, 3H, H-9$^{Neu}$, 2$OCH_2Ph$), 4.45 (d, 1H, $J_{gem}$=11.7Hz, $OCH_2Ph$), 4.52 (dd, 1H, $J_{3,4}$=3.42Hz, H-3$^{Gal}$), 4.65 (d, 1H, $J_{gem}$=12.3Hz, $OCH_2Ph$), 4.77 (d, 1H, $J_{1,2}$=7.5Hz, H-1$^{Gal}$), 4.78 (d, 1H, $J_{gem}$=12.3Hz, $OCH_2Ph$), 4.78 (d, 1H, $J_{gem}$=12.3Hz, $OCH_2Ph$), 4.81 (d, 1H, $J_{1,2}$=7.5Hz, H-1$^{Glc}$), 4.92 (m, 1H, H-4$^{Neu}$), 4.96-5.02 (m, 3H, $OCH_2Ph$), 5.05 (d, 1H, $J_{3,4}$=1Hz, H-4$^{Gal}$), 5.10 (d, 1H, $J_{gem}$=10.3Hz, NH), 5.32(dd,1H,$J_{7,8}$=1.7Hz,H-7$^{Neu}$), 5.59-5.62(m,1H,H-8$^{Neu}$), 6.75-7.41(m,29H,$OCH_2Ph$);

$^{13}$C-NMR(150MHz,$CDCl_3$):δ(ppm)20.4, 20.7, 20.7, 21.2, 23.1, 29.6, 37.5, 49.1, 53.0, 55.5, 62.0, 67.0, 67.6, 68.4, 68.6, 68.8, 69.4, 71.4, 72.1, 72.7, 73.1, 73.8, 74.8, 75.0, 75.1, 76.6, 79.4, 81.6, 82.8, 97.2, 102, 102, 114, 118, 127, 127, 127, 127, 127, 127, 127, 127, 128, 128, 128, 128, 138, 138, 138, 139, 139, 151, 155, 167, 169, 169, 169, 170, 170, 170;

MS(Positive ion MALDI-TOF MS.):$C_{76}H_{87}NO_{23}$ : m/z calcd. for $[M+Na]^+$: 1436.54, found :1436.66.

(Preparation of Compound 35A)

[Chem. 142]

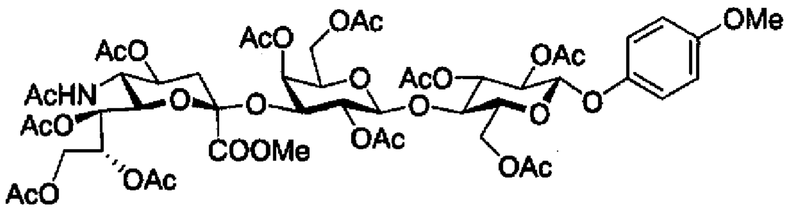

4-methoxyphenyl[methyl5-acetamide-4,7,8,9-tetra-O-a cetyl-3,5-dideoxy-D-glycero-α-D-galacto-2-nonulopyranos ylonate]-(2→3)-(2,4,6-O-acetyl-β-D-galactopyranosyl)-(1 →4)-2,3,6-O-acetyl-β-D-glucopyranoside (35A)

Compound 33A (450 mg, 0.318 mmol) was dissolved in 1:1 EtOH-THF (5mL), and then $Pd(OH)_2$-C(1.0g) was added thereto, subsequently stirring under hydrogen gas stream at 40 degrees Celsius for 1 hour. After the reaction was completed, the solid was filtered through a Celite®, and then washed with chloroform. The filtrate and the wash solution were combined, and then concentrated with toluene under reduced pressure. The resulting syrup was dried *in vacuo* for 1 hour, followed by substituting Ar, and then dissolved in pyridine. After cooling in an ice bath, acetic anhydride (1 mL) and a catalytic amount of 4-dimethylaminopyridine (DMAP) were added, and then the ice bath was removed, subsequently stirring for 5 hours. After cooling in an ice bath, MeOH was added to quench the reaction, followed by concentration under reduced pressure. The resulting syrup was then washed with 2N HCl, $H_2O$, $NaHCO_3$ saturated, $H_2O$, and brine, successively. The resulting organic layer was dried with $Na_2SO_4$, and then the organic layer was separated from the solid by filtration. The filtrate and the wash solution were combined, and then concentrated under reduced pressure. The resulting syrup was purified by silica gel chromatography ($CHCl_3$:MeOH=50:1) to yield Compound 35A (358 mg, 96%).

[Chem. 143]

$[\alpha]_D^{25}$ 8.67 (c = 0.2 $CHCl_3$);

$^1$H-NMR (600MHz, $CDCl_3$): δ(ppm) 1.68 (t, 1H, $J_{gem}$=12.3Hz, H-3ax$^{Neu}$), 1.85, 2.00, 2.06, 2.07, 2.07, 2.08, 2.08, 2.08, 2.09, 2.16, 2.25 (9s, 33H, 10AcO, AcN), 2.58 (dd, 1H, $J_{3eq,4}$=4.8Hz, H-3eq$^{Neu}$), 3.64 (dd, 1H, $J_{6,7}$=2.7Hz, H-6$^{Neu}$), 3.71-3.74 (m, 1H, H-5$^{Glc}$), 3.76 (s, 3H, COOMe), 3.84 (s, 3H, OMe), 3.84-3.87 (m, 1H, H-5$^{Gal}$), 3.95-4.06 (m, 5H, H-4$^{Glc}$, H-6$^{Gal}$, H-6'$^{Gal}$, H-5$^{Neu}$, H-9$^{Neu}$), 4.22 (dd, 1H, $J_{gem}$=11.6Hz, H-6$^{Glc}$), 4.42 (dd, 1H, $J_{gem}$=12.3Hz, H-9'$^{Neu}$), 4.48 (d, 1H, $J_{gem}$=11.6Hz, H-6'$^{Glc}$), 4.53 (dd, 1H, $J_{3,4}$=3.4Hz, H-3$^{Gal}$), 4.74 (d, 1H, $J_{1,2}$=8.2Hz, H-1$^{Gal}$), 4.86-4.90 (m, 1H, H-4$^{Neu}$), 4.88 (d, 1H, $J_{4,5}$=2.7Hz, H-4$^{Gal}$), 4.92 (d, 1H, $J_{1,2}$=8.1Hz, H-1$^{Glc}$), 5.13 (t, 1H, $J_{2,3}$=7.5Hz, H-2$^{Glc}$), 5.24-5.27 (m, 2H, H-3$^{Glc}$, NH), 5.39 (dd, 1H, $J_{7,8}$=8.9, H-7$^{Neu}$), 5.54-5.57 (m, 1H, H-8$^{Neu}$), 6.80-6.94 (m, 4H, $MeOC_6H_4$);

$^{13}$C-NMR (150MHz, $CDCl_3$): δ(ppm) 20.5, 20.5, 20.6, 20.6, 20.6, 20.7, 20.8, 21.4, 23.0, 37.3, 48.9, 53.0, 55.5, 61.4, 62.2, 66.8, 67.2, 67.7, 69.2, 69.8, 70.4, 71.2, 71.6, 71.9, 72.7, 73.3, 76.2, 96.7, 99.9, 100, 114, 118, 150, 155, 167, 169, 169, 169, 169, 170, 170, 170, 170, 170, 170;
MS (Positive ion MALDI-TOF MS.): $C_{51}H_{67}NO_{30}$ : m/z calcd. for $[M+Na]^+$: 1196.36, found: 1196.39.

(Preparation of Compound 37A)

[Chem. 144]

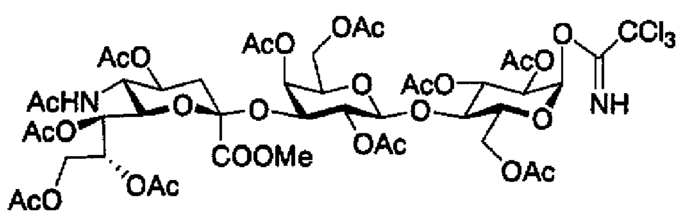

Methyl 5-acetamide-4,7,8,9-tetra-O-acetyl-3,5-dideoxy-D-glycer o-α-D-galacto-2-nonulopyranosylonate]-(2→3)-(2,4,6-O-ac etyl-β-D-galactopyranosyl)-(1→4)-2,3,6-O-acetyl-α-D-glu copyranosyltrichloroacetimidate (37A)

Compound 35A (53 mg, 0.0452 mmol) was dissolved in 6:5:3 toluene-MeCN-$H_2O$ (2 mL), and cooled in an ice bath, and then cerium(IV) diammonium nitrate (CAN) (74 mg) was added thereto. After 15 minutes, the ice bath was removed, followed by stirring at room temperature for 5.5 hours. After the reaction was completed, the solution was diluted with AcOEt, and then washed with $H_2O$, $NaHCO_3$ saturated, $H_2O$, and brine, successively. The resulting organic layer was dried with $Na_2SO_4$, and then the organic layer was separated from the solid by filtration. The filtrate and the

wash solution were combined, and then concentrated under reduced pressure. The resulting syrup was purified by silica gel chromatography ($CHCl_3$:MeOH=25:1) to yield Compound 36A.

[Chem. 145]

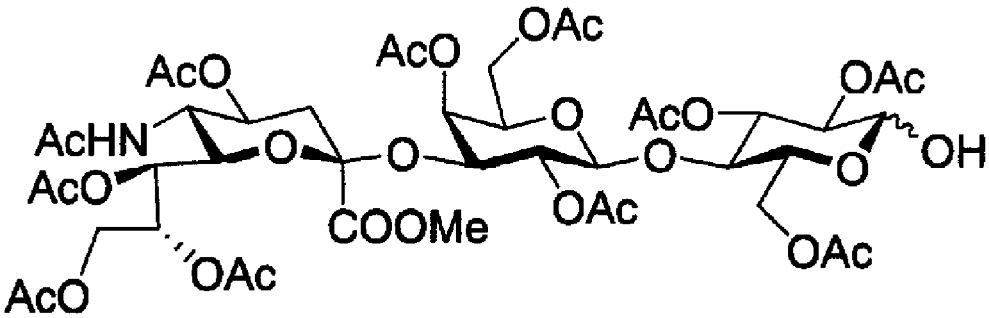

The resulting Compound 36A was dissolved in $CH_2Cl_2$ (2 mL), and cooled in an ice bath, and then $CCl_3CN$ (452 µl, 4.516 mmol) and DBU (3.4 µl, 0.021 mmol) were added. After the addition, the ice bath was removed, followed by stirring at room temperature for 2 hours. After the reaction was completed, the solution was concentrated under reduced pressure. The resulting syrup was purified by silica gel chromatography (toluene:acetone=5:1) to yield Compound 37A (44 mg, 81% for 2 steps).

[Chem. 146]

$[\alpha]D^{?}$ -7.89($c$ = 0.2 $CHCl_3$);

$^1$H-NMR(600MHz,$CDCl_3$):δ(ppm) 1.67 (t, 1H, $J_{gem}$=12.3Hz, H-3ax$^{Neu}$), 1.85, 2.00, 2.05, 2.07, 2.07, 2.08, 2.09, 2.16, 2.18, 2.24 (10s, 33H, 10AcO, AcN), 2.58 (dd, 1H, $J_{3eq,4}$=4.8Hz ,H-3eq$^{Neu}$) ,3.64 (dd, 1H, $J_{6,7}$=2.1Hz, H-6$^{Neu}$), 3.84(s, 3H, COOMe), 3.84-3.87 (m, 1H, H-6$^{Gal}$), 3.93-4.05 (m, 5H, H-4$^{Glc}$, H-5$^{Gal}$, H-6'$^{Gal}$, H-5$^{Neu}$, H-9$^{Neu}$), 4.11-4.13 (m, $J_{3,4}$=2.7Hz, H-3$^{Gal}$), 4.67 (d, 1H, $J_{1,2}$=7.5Hz, H-1$^{Gal}$), 4.87-4.92 (m, 2H, H-4$^{Gal}$, H-4$^{Neu}$), 4.95 (t, 1H, $J_{2,3}$=9.6Hz, H-2$^{Gal}$), 5.07 (t, 1H, $J_{2,3}$=3.4Hz, H-2$^{Glc}$), 5.31-5.32 (m, 1H, NH), 5.41 (dd, 1H, $J_{7,8}$=9.6Hz, H-7$^{Neu}$), 5.49-5.50 (m, 1H, H-8$^{Neu}$), 5.54 (d, 1H, $J_{2,3}$=8.1Hz, H-3$^{Glc}$), 6.48 (d, 1H, $J_{2,3}$=2.7Hz, H-1$^{Glc}$), 8.67 (s, 1H, $CNHCCl_3$);

$^{13}$C-NMR(150MHz,$CDCl_3$):δ(ppm)20.3, 20.5, 20.5, 20.6, 20.6, 20.7, 20.9, 21.4, 23.0, 29.1, 29.5, 37.2, 49.0, 53.0, 61.4, 61.6, 61.9, 66.6, 67.1, 67.7, 69.2, 69.8, 69.9, 70.4, 70.9, 71.4, 71.9, 75.6, 90.6, 92.9, 96.6, 101, 160, 167, 169, 169, 169, 170, 170, 170, 170, 170, 170, 170,

MS(Positive ion MALDI-TOF MS.):$C_{51}H_{67}NO_{30}$ : $m/z$ calcd. for $[M+Na]^+$: 1233.23, found :1233.48.

(Preparation of Compound 38A)

[Chem. 147]

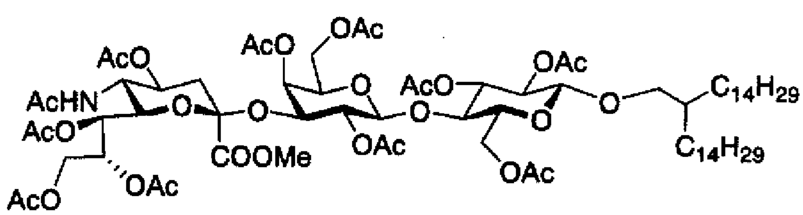

Methyl 5-acetamide-4,7,8,9-tetra-O-acetyl-3,5-dideoxy-D-glycer o-α-D-galacto-2-nonulopyranosylonate]-(2→3)-(2,4,6-O-ac etyl-β-D-galactopyranosyl)-(1→4)-2,3,6-O-acetyl-β-D-glu copyranosyl-(1→1)-2-(tetradecyl)-hexadecanol (38A)

Compound 8A:

[Chem. 148]

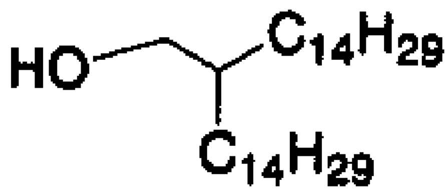

8A

(91 mg, 0.207 mmol) and Compound 37A (50 mg, 0.0414 mmol) were dissolved in $CH_2Cl_2$ (2.5 mL), and then stirred in the presence of AW300 (150 mg) at room temperature for 1 hour. After cooling to 0 degree Celsius, TMSOTf (1.5 μl, 0.0082 mmol) was added, followed by stirring at 0 degree Celsius for 10 hours. TEAwas added to neutralize the reactionmixture, the solid was filtrated through a Celite®, and then washed with chloroform. The filtrate and the wash solution were combined, and then washed with $NaHCO_3$ saturated, $H_2O$, sat., and brine, successively. The resulting organic layer was dried with $Na_2SO_4$, and then the organic layer was separated from the solid by filtration. The filtrate and the wash solution were combined, and then concentrated under reduced pressure. The resulting syrup was purified by silica gel chromatography (toluene:acetone=2:1) to yield Compound 38A (22 mg, 36%).

[Chem. 149]

[a]D-9.25° (c = 0.05 $CHCl_3$);

$^1$H-NMR(600MHz,$CDCl_3$):δ(ppm)0.87 (t, 6H, J=6.9Hz, $2CH_3^{alkylpart}$), 1.22-1.30 (m, 52H, $26CH_2^{alkylpart}$), 1.63 (m, 1H, $CH^{alkylpart}$), 1.85, 2.00, 2.01, 2.03, 2.06, 2.07, 2.08, 2.09, 2.15, 2.24 (10s, 33H, 10AcO, AcN), 2.22 (dd, 1H, $J_{3aq,3eq}$=12.0Hz, H-$3ag^{Neu}$), 2.57 (dd, 1H, $J_{3eq,4}$=4.1Hz, H-$3eq^{Neu}$), 3.26 (dd, 1H, $J_{gem}$=9.6Hz, H-$6^{Gal}$), 3.57-3.60 (m, 1H, H-$5^{Glc}$), 3.63 (dd, 1H, $J_{6,7}$=2.76Hz, H-$6^{Neu}$), 3.77 (dd, 1H, $J_{gem}$=9.6Hz, H-$6'^{Gal}$), 3.84 (s, 3H, COOMe), 3.83-3.89 (m, 2H, H-$4^{Glc}$, H-$5^{Gal}$), 3.97-4.04 (m, 4H, H-$5^{Neu}$, H-$9^{Neu}$, $OCH_2C^{alkylpart}$), 4.18 (dd, 1H, $J_{gem}$=11.7Hz, H-$6^{Glc}$), 4.40-4.44 (m, 2H, , H-$6'^{Glc}$, H-$9'^{Neu}$), 4.41(d, 1H, $J_{1,2}$=7.56Hz, H-$1^{Glc}$), 4.51 (dd, 1H, $J_{3,4}$=3.4Hz, H-$3^{Gal}$), 4.66 (d, 1H, $J_{1,2}$=8.2Hz, H-$1^{Gal}$), 4.86-4.94 (m, 4H, H-$2^{Glc}$, H-$2^{Gal}$, H-$4^{Gal}$, H-$4^{Neu}$), 5.09 (d, 1H, NH), 5.18 (t, 1H, $J_{3,4}$=8.9, H-$3^{Glc}$), 5.39 (dd, 1H, H-$7^{Neu}$), 5.52-5.55 (m, 1H, H-$8^{Neu}$);

$^{13}$C-NMR(150MHz,$CDCl_3$):δ(ppm)14.0, 20.5, 20.6, 20.6, 20.7, 20.7, 20.8, 20.9, 21.4, 22.6, 23.1, 26.5, 26.8, 29.3, 29.6, 30.0, 30.0, 30.8, 31.0, 31.8, 37.3, 37.9, 49.0, 53.1, 61.5, 62.1, 62.3, 66.8, 67.3, 67.7, 69.4, 69.9, 70.3, 71.3, 71.8, 72.0, 72.5, 73.0, 73.3, 76.3, 96.7, 100, 100, 167, 169, 169, 169, 170, 170, 170, 170, 170, 170;

MS(Positive ion MALDI-TOF MS.):$C_{74}H_{121}NO_{29}$ : m/z calcd. for $[M+Na]^+$: 1510.79, found :1510.62.

(Preparation of Compound 3A)

[Chem. 150]

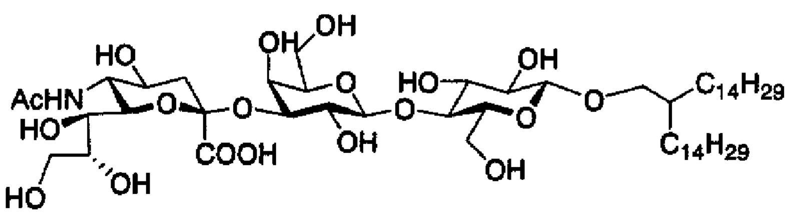

5-acetamide-3,5-dideoxy-D-glycero-α-D-galacto-2-non ulopyranosylonate-(2→3)-β-D-galactopyranosyl-(1→4)-β-D-glucopyranosyl-(1→1)-2-(tetradecyl)hexadecanol (3A)

Compound 38A (22 mg, 0.0148 mmol) was suspended in MeOH (1.0 mL), and then NaOMe (0.040 mg, 0.00074 mmol) was added, followed by stirring at room temperature for 21 hours. By MALDI-TOF MS, all of the acetyl groups of the compound were confirmed to be removed by deprotection, and then $H_2O$ was added. After stirring for 3.5 hours, by MALDI-TOF MS, the production of a carboxylic acid was confirmed. The solution was neutralized with Dowex($H^+$) to pH 7, and then separated from Dowex ($H^+$) by filtration. The resulting solution was concentrated under reduced pressure, and then the resulting syrup was purified by column chromatography (Sephadex LH-20, MeOH) to yield Compound 3A (11 mg, 69%).

[Chem. 151]

[a]D-74.4° ($c$ = 0.05 $CHCl_3$); $^1$H-NMR(600MHz, $CDCl_3$):δ(ppm)0.885-0.908 (m, 6H, $2CH_3^{alkylpart}$), 1.28-1.40 (m, 54H, $26CH_2^{alkylpart}$, $OCH_2CH$), 1.60 (m, 1H, $CH^{alkylpart}$), 1.72 (dd, 1H, $J_{3aq,3eq}$=11.7Hz, H-$3ag^{Neu}$), 2.00 (s, 3H, AcN), 2.85 (dd, 1H, $J_{3eq,4}$=4.1Hz, H-$3eg^{Neu}$), 3.20 (t, 1H, $J_{2,3}$=8.9Hz, H-$2^{Glc}$), 3.37-3.91 (m, 18H, $OCH_2C^{alkylpart}$, H-$4^{Glc}$, H-$5^{Glc}$, H-$6^{Glc}$, H-$6'^{Glc}$, H-$2^{Gal}$, H-$4^{Gal}$, H-$5^{Gal}$, H-$6^{Gal}$, H-$6'^{Gal}$, H-$4^{Neu}$, $NH^{Neu}$, H-$5^{Neu}$, H-$6^{Neu}$, H-$7^{Neu}$, H-$8^{Neu}$, H-$9^{Neu}$, H-$9'^{Neu}$), 3.51 (t, 1H, $J_{2,3}$=8.9Hz, H-$3^{Glc}$), 4.04 (dd, 1H, $J_{3,4}$=2.7Hz, H-$3^{Gal}$), 4.25 (d, 1H, $J_{1,2}$=7.5Hz, H-$1^{Glc}$), 4.43 (d, 1H, $J_{1,2}$=7.5Hz, H-$1^{Gal}$);

$^{13}$C-NMR(150MHz, $CDCl_3$):δ(ppm)14.6, 22.7, 23.9, 27.9, 30.6, 30.9, 30.9, 31.2, 32.2, 33.2, 39.6, 42.2, 54.1, 62.1, 62.3, 62.9, 64.7, 69.1, 69.5, 70.2, 71.0, 73.1, 74.3, 74.9, 75.1, 76.5, 76.6, 77.2, 77.8, 81.4, 101, 104, 105, 175, 175

MS(Positive ion MALDI-TOF MS.):$C_{74}H_{121}NO_{29}$ : m/z calcd. for $[M+Na]^+$: 1076.67, found :1076.68

(Preparation of Compound 39A)

[Chem. 152]

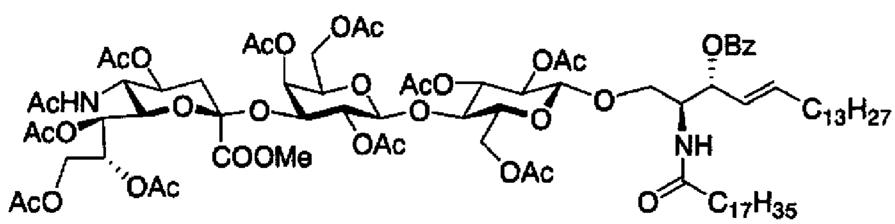

Methyl 5-acetamide-4,7,8,9-tetra-O-acetyl-3,5-dideoxy-D-glycer o-α-D-galacto-2-nonulopyranosylonate]-(2→3)-(2,4,6-O-ac etyl-β-D-galactopyranosyl)-(1→4)-2,3,6-O-acetyl-β-D-glu copyranosyl-(1→1)-(2S,3R,4E)-3,4-O-dibenzoyl-2-octadeca namide-4-octadecen-1,3-diol(39A)

Compound 6A:

[Chem. 153]

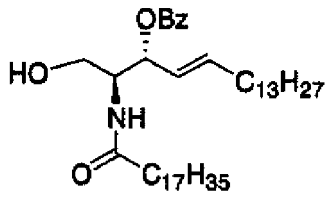

6A

(55 mg, 0.0826 mmol) and Compound 37A (50 mg, 0.0413 mmol) were dissolved in $CH_2Cl_2$ (1 mL), and then stirred in the presence of AW300 (150 mg) at room temperature for 1 hour. After cooling to 0 degree Celsius, TMSOTf (1.5 μl, 0.00818 mmol) was added, followed by stirring at 0 degree Celsius for 22 hours. After TEA was added to neutralize the reaction mixture, the solid was filtrated through a Celite®, and then washed with chloroform. The filtrate and the wash solution were combined, and then washed with $NaHCO_3$ saturated, $H_2O$, and brine, successively. The resulting organic layer was dried with $Na_2SO_4$, filtrated, and then concentrated under reduced pressure. The resulting syrup was purified by silica gel chromatography (toluene:acetone=2:1) to yield Compound 39A (13 mg, 18%).

[Chem. 154]

[a]D-75.7° (c = 0.03 $CHCl_3$);

$^1$H-NMR(600MHz,$CDCl_3$):δ(ppm) 0.87 (t, J=6.8Hz, 2$CH_3^{Cer}$), 1.22-1.32 (m, 48H, 24$CH_2$), 1.85, 2.00, 2.01, 2.03, 2.06, 2.07, 2.07, 2.08, 2.16 ,2.21 (10s, 33H, 10AcO, AcN), 2.57 (dd, 1H, $J_{3eq,4}$=4.8Hz, H-3eq$^{Neu}$), 3.54-3.56 (m, 1H, H-5$^{Glc}$), 3.62 (dd, 1H, $J_{6,7}$=2.7Hz, H-6$^{Neu}$), 3.84 (s, 3H, COOMe), 3.82-3.86 (m, 3H, H-4$^{Glc}$, H-9$^{Neu}$), 3.96-4.06 (m, 5H, H-1$^{Cer}$, H-6$^{Glc}$, H-6'$^{Gal}$, H-5$^{Neu}$), 4.34 (dd, 1H, $J_{gem}$=12.3Hx, H-6'$^{Glc}$), 4.40 (dd, 1H, $J_{gem}$=12.3Hz, H-9'$^{Neu}$), 4.44 (d, 1H, $J_{1,2}$=8.2Hz, H-1$^{Glc}$), 4.45-4.48 (m, 1H, H-2$^{Cer}$), 4.51 (dd, 1H, $J_{3,4}$=3.4Hz, H-3$^{Gal}$), 4.63 (d, 1H, $J_{1,2}$=7.5Hz, H-1$^{Gal}$), 4.86-4.92 (m, 4H, H-2$^{Glc}$, H-2$^{Gal}$, H-4$^{Gal}$, H-4$^{Neu}$), 5.05 (d, 1H, $J_{5,NH}$=10.3, NH$^{Neu}$), 5.16 (t, 1H, $J_{3,4}$=9.60, H-3$^{Glc}$), 5.39 (dd, 1H, $J_{7,8}$=8.9Hz, H-7$^{Neu}$), 5.44-5.54 (m, 3H, H-3$^{Cer}$, H-4$^{Cer}$, H-8$^{Neu}$), 5.75 (d, 1H, $J_{2,NH}$=9.60, NH$^{Cer}$), 5.83-5.88 (m, 1H, H-5$^{Cer}$), 7.42-8.00 (m, 5H, $COC_6H_5$);

$^{13}$C-NMR(150MHz,$CDCl_3$):δ(ppm)10.8, 14.0, 20.5, 20.6, 20.6, 20.7, 20.8, 20.9, 21.5, 22.6, 23.1, 25.7, 28.9, 29.2, 29.3, 29.4, 29.4, 29.5, 29.6, 31.9, 32.3, 36.8, 37.3, 49.1, 50.7, 53.1, 61.5, 62.0, 62.1, 66.8, 67.3, 67.4, 67.7, 68.0, 69.2, 69.8, 70.4, 71.3, 71.8, 72.0, 72.8, 73.0, 74.1, 96.7, 100, 100, 100, 124, 128, 129, 130, 133, 137, 165, 167, 169, 169, 169, 169, 170, 170, 170, 170, 170, 170, 172;

MS(Positive ion MALDI-TOF MS.): $C_{53}H_{67}NO_{38}$ : m/z calcd. for $[M+Na]^+$: 1741.88, found :1741.86

(Example 3. Preparation of GM4)

(Preparation of Compound 45A)

[Chem. 155]

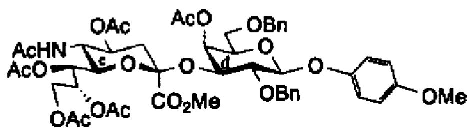

4-Methoxyphenyl(methyl5-acetamide-4,7,8,9-tetra-O-a cetyl3,5-dideoxyD-glycero-a-D-galacto-2-nonulopyranosyl onate)-(2→3)-4-O-acetyl-2,6-di-O-benzyl-β-D-galactopyra noside (45A)

To a solution of Compound 43A:

[Chem. 156]

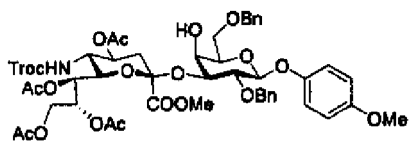

43A

(2.0g, 1.87 mmol: prepared according to Fuse, T.; Ando, H.; Imamura, A.; Sawada, N.; Ishida, H.; Kiso, M.; Ando, T.; Li, S. C.; and Li li Y.-T. Glycoconj. J. 2006, 23, 329-343) in 1, 2-dichloroethane (15.0 mL) was added acetic acid (45.0 mL) and Zn-Cu(10.0g) under argon atmosphere at 0 degree Celsius. This mixture was stirred at 40 degrees Celsius for 1.5 hours, while the progress of the reaction was monitored by TLC ($CHCl_3$:MeOH=15:1). This reaction mixture was filtrated through a Celite®. The filtrate and the wash solution were combined, and then extracted with $CHCl_3$. The organic layer was washed with $H_2O$, saturated $Na_2CO_3$, and brine, dried with $Na_2SO_4$, and then concentrated. To the solution of the residue in pyridine (9.0 mL) was added acetic anhydride (614 μL) under argon atmosphere at 0 degree Celsius. This mixture was stirred for 13 hours at ambient temperature, while the progress of the reaction was monitored by TLC ($CHCl_3$:MeOH=15:1). This reaction mixture was co-evaporated with toluene, and then extracted with $CHCl_3$. The organic phase was washed with 2M HCl, $H_2O$, saturated $NaHCO_3$, and brine, dried with $Na_2SO_4$, and then concentrated. The residue was purified by silica gel column chromatography (EtOAc:hexane=3:1) to yield 45A (1.69 g, 92%).

[Chem. 157]

$[\alpha]_D$ = -15.4° (c 0.9, $CHCl_3$);

$^1$H-NMR (600 MHz, $CDCl_3$): δ 7.45-6.77 (m, 14 H, 3 Ph), 5.53 (m, 1 H, H-8e), 5.33 (dd, 1 H, H-7e), 5.24 (d, 1 H, $J_{5,NH}$ = 8.9 Hz, NH), 5.07 (m, 2 H, H-1d, 4d), 4.96-4.88 (m, 3 H, H-4e, 2 $OCH_2Ph$), 4.63 (dd, 1 H, H-3d), 4.53 (d, 1 H, $OCH_2Ph$), 4.46 (d, 1 H, $OCH_2Ph$) 4.36 (dd, 1 H, H-9'e), 4.13 (q, 1 H, $J_{5,NH}$ = 8.9 Hz, H-5e), 3.96-3.94 (m, 2 H, H-6'd, 9e), 3.85 (s, 3 H, OMe), 3.76-3.73 (m, 5 H, H-2d, 6e, OMe), 3.56-3.52 (m, 2 H, H-5d, 6d), 2.63 (dd, 1 H, H-$3e_{eq}$), 2.12-1.83 (m, 19 H, 6 Ac, H-$3e_{ax}$);

$^{13}$C-NMR (100 MHz, $CDCl_3$) δ 170.9, 170.6, 170.3, 170.2, 170.0, 168.1, 155.1, 151.7, 139.4, 138.0, 128.3, 128.1, 127.7, 127.6, 127.1, 118.2, 114.4, 102.4, 97.1, 78.1, 74.8, 73.5, 73.1, 72.3, 72.2, 69.5, 68.9, 68.7, 68.6, 67.2, 62.2, 55.6, 53.1, 49.2, 37.6, 23.2, 21.3, 20.8, 20.8, 20.5;

MALDI MS: m/z calcd. for $C_{45}H_{59}O_{20}NNa$ : 1004.35;

found: 1004.35 $[M+Na]^+$.

(Preparation of Compound 47A)

4-Methoxyphenyl(methyl5-acetamide-4,7,8,9-tetraO-ac etyl3,5-dideoxyD-glycero-a-D-galacto-2-nonulopyranosylo nate)-(2→3)-4-O-acetyl-2,6-di-O-benzoyl-β-D-galactopyra noside (47A)

[Chem. 158]

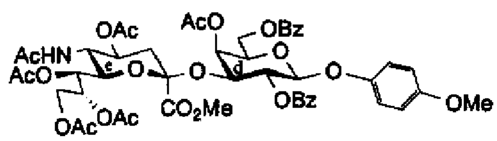

To a solution of Compound 45A (385 mg, 392 μmol) in EtOH (30 mL) was added palladium hydroxide [Pd $(OH)_2$] (20 wt.% Pd supported on Carbon) (400 mg) at ambient temperature under argon atmosphere. This mixture was vigorously stirred for 4 hours under hydrogen atmosphere at ambient temperature, while the progress of the reaction was monitored by TLC ($CHCl_3$:MeOH=15:1). This reaction mixture was then filtrated through a Celite®. The filtration and the washing solution were combined, and then concentrated. To the solution of the residue inpyridine (5. 0 mL) was addedbenzoic anhydride (354 mg, 1.57 mmol) under argon atmosphere at 0 degree Celsius. This mixture was stirred for 16 hours at ambient temperature, while the progress of the reaction was monitored by TLC ($CHCl_3$:MeOH=15:1). This reaction mixture was co-evaporated with toluene, and then extracted with $CHCl_3$. The organic phase was washed with 2M HCl, $H_2O$, saturated $NaHCO_3$, and brine, and then concentrated. The residue was purified by silica gel column chromatography (EtOAc:hexane=3:1) to yield 47A (380 mg, 95%).

[Chem. 159]

$[\alpha]_D$ = +27.9° (c 4.2, $CHCl_3$);

$^1$H-NMR (600 MHz, $CDCl_3$): δ 8.17-6.67 (m, 14 H, 3 Ph), 5.59 (m, 1 H, H-8e), 5.55 (near t, 1 H, $J_{1,2}$ = 8.3 Hz, $J_{2,3}$ = 10.3 Hz, H-2d), 5.26 (d, 1 H, $J_{1,2}$ = 8.3 Hz, H-1d), 5.20 (dd, 1 H, $J_{6,7}$ = 2.8 Hz, H-7e), 5.16 (d, 1 H, $J_{3,4}$ = 3.4 Hz, H-4d), 5.14 (d, 1 H, NH), 4.87 (dd, 1 H, $J_{2,3}$ = 10.3 Hz, $J_{3,4}$ = 3.4 Hz, H-3d), 4.85 (m, 1 H, H-4e), 4.46 (near t, 1 H, H-6'd), 4.35 (dd, 1 H, H-6d), 4.27 (dd, 1 H, H-9'e), 4.19 (t, 1 H, H-5e), 3.91 (dd, 1 H, H-9e), 3.86-3.79 (m, 4 H, H-5e, OMe) 3.71 (s, 3 H, OMe), 3.61 (dd, 1 H, $J_{6,7}$ = 2.8 Hz, H-6e), 2.59 (dd, 1 H, H-$3e_{eq}$), 2.19-1.44 (m, 19 H, 6 Ac, H-$3e_{ax}$);

$^{13}$C-NMR (100 MHz, $CDCl_3$) δ 170.7, 170.6, 170.3, 170.2, 170.0, 168.0, 165.7, 165.3, 155.4, 151.3, 133.2, 133.0, 130.1, 130.0, 129.7, 128.3, 128.3, 118.9, 114.2, 101.1, 96.7, 71.6, 71.1, 70.8, 69.3, 67.6, 67.4, 66.4, 62.3, 62.0, 55.4, 53.0, 48.7, 37.2, 23.2, 21.3, 20.7, 20.1;

MALDI MS: m/z calcd. for $C_{49}H_{55}O_{22}NNa$ : 1032.31;
found: 1032.38 $[M+Na]^+$.

With regard to Compound 47A, the chemical formula is $C_{49}H_{55}NO_{22}$, the exact mass is 1009.3216, and the molecular weight is 1009.9545. With regard to the Na salt thereof, the chemical formula is $C_{49}H_{55}NNaO_{22}$, the exact mass is 1032. 3113, and the molecular weight is 1032.9443. With regard to the K thereof, the chemical formula is $C_{49}H_{55}NKO_{22}$, the exact mass is 1048.2853, and the molecular weight is 1049.0528.

(Preparation of Compound 49A)

[Chem. 160]

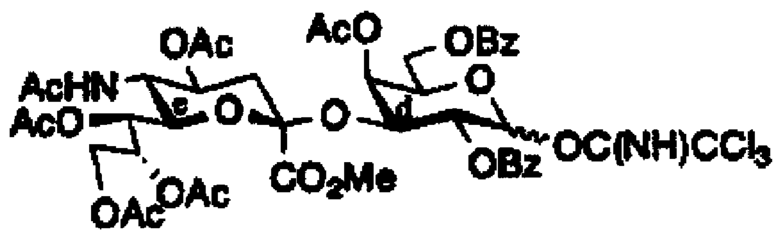

Methyl 5-acetamide-4,7,8,9-tetra-O-acetyl-3,5-dideoxy-D-glycer o-α-D-galacto-2-nonulopyranosylonate-(2→3)-4-O-acetyl-2 ,6-di-O-benzoyl-β-D-galactopyranosyltrichloroacetimidat e (49A)

To a solution of Compound 47A (164 mg, 162μmol) in mixed solvent (MeCN-PhMe-$H_2O$=3.5 mL:2.9 mL:1.7 mL) was added cerium(IV) diammoniumnitrate (CAN) (445mg, 812 μmol). This mixture was stirred for 5 hours at ambient temperature, while the progress of the reaction was monitored by TLC ($CHCl_3$:MeOH=20:1). This reactionmixture was then extracted with $CHCl_3$. The organic layer was washed with $H_2O$, saturated $NaHCO_3$, and brine, dried with $Na_2SO_4$, and then concentrated. The residue was purified by silica gel column chromatography ($CHCl_3$:MeOH=65:1) to yield the target compound (147 mg). To a solution of the compound in $CH_2Cl_2$ (5.0 mL) was added trichloroacetonitrile (410 μL, 407 μmol) and 1,8-diazabicyclo[5,4,0]-7-undecene (DBU) (4.9 μL, 33.0 μmol). This mixture was stirred for 2 hours at 0 degree Celsius, while the progress of the reaction was monitored by TLC ($CHCl_3$:MeOH=20:1). The reaction mixture was concentrated, and then the residue was purified by silica gel column chromatography ($CHCl_3$:MeOH=75:1) to yield 49A (132 mg, 78%).

[Chem. 161]

$[\alpha]_D$ = +18.6° (c 0.8, $CHCl_3$);
$^1$H-NMR (600 MHz, $CDCl_3$): δ 8.67 (s, 1 H, $NH^{imidate}$), 8.10-7.41 (m, 10 H, 2 Ph), 6.20 (d, 1 H, $J_{1,2}$ = 8.3 Hz, H-1d), 5.60-5.56 (m, 2 H, H-2d, H-8e), 5.22-5.20 (m, 2 H, H-4d, H-7e), 4.98 (d, 1 H, $J_{5,NH}$ = 10.3 Hz, NH-e), 4.93 (dd, 1 H, H-3d), 4.87 (m, 1 H, H-4e), 4.49 (q, 1 H, H-6'd), 4.34-4.29 (m, 3 H, H-5d, 6d, 9'e), 3.93 (dd, 1 H, H-9e), 3.85-3.77 (m, 4 H, H-5e, OMe), 3.60 (dd, 1 H, H-6e), 2.58 (dd, 1 H, H-$3e_{eq}$), 2.19-1.43 (m, 19 H, 6 Ac, H-$e_{ax}$);
$^{13}$C-NMR (100 MHz, $CDCl_3$) δ 170.8, 170.7, 170.6, 170.2, 170.2, 170.0, 168.0, 165.7, 165.1, 161.1, 133.2, 130.1, 129.9, 129.7, 129.7, 128.3, 128.3, 96.8, 96.4, 90.3, 77.2, 71.8, 71.5, 71.1, 70.0, 69.4, 67.6, 67.4, 66.5, 62.4, 61.5, 53.1, 48.8, 37.3, 29.7, 23.1, 21.4, 20.8, 20.7, 20.2;
MALDI MS: m/z calcd. for $C_{44}H_{49}O_{23}N_2Cl_3Na$ : 1069.18;
found : 1069.41 $[M+Na]^+$.

(Preparation of Compound 50A)
[Chem. 162]

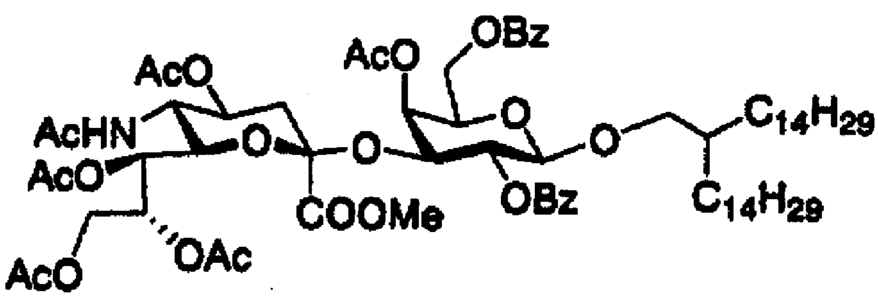

Methyl 5-acetamide-4,7,8,9-tetra-O-acetyl-3,5-dideoxy-D-glycer o-α-D-galacto-2-nonulopyranosylonate-(2→3)-4-O-acetyl-2 ,6-O-dibenzoylβ-D-galactopyranosyl)-(1→1)-2-(tetradecyl )-hexadecanol (50A)

Compound 8A:
[Chem. 163]

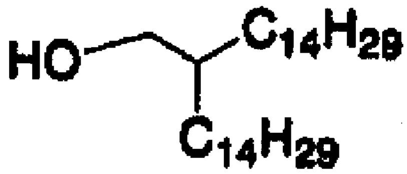

8A

(136 mg, 0.310 mmol) and Compound 49A (65 mg, 0.0621 mmol) was dissolved in $CH_2Cl_2$ (3 mL), and then stirred in the presence of AW300 (200 mg) at room temperature for 1 hour. After cooling to 0 degree Celsius, TMSOTf (2.2 μl, 0.0124 mmol) was added, followed by stirring at 0 degree Celsius for 20 hours. After TEA was added to neutralize the reaction mixture, the solid was filtrated through a Celite®, and then washed with chloroform. The filtrate and the wash solution were combined, saturated $NaHCO_3$, $H_2O$, and brine, successively. The resulting organic layer was dried with $Na_2SO_4$, and then the organic layer was separated from the solid by filtration. The filtrate and the wash solution were combined, and then concentrated under reduced pressure. The resulting syrup was purified by silica gel chromatography (toluene:acetone=2:1) to yield Compound 50A (62 mg, 76%).

[Chem. 164]

[a]D+7.69° (c = 0.26 $CHCl_3$);

$^1$H-NMR(600MHz,$CDCl_3$):δ(ppm)0.87-0.89(m,6H,2$CH_3^{alkylpart}$),
0.97-1.29(m,52H,26$CH_2^{alkylpart}$), 1.43(s,3H,AcN), 1.46(m,1H,$CH^{alkylpart}$),
1.78,1.96,2.06,2.14,2.15(5s,15H,5AcO), 1.73(t,1H,$J_{gem}$=13.0Hz,H-3$ax^{Neu}$),
2.54(dd,1H,$J_{3eq,4}$=4.8Hz,H-3$eq^{Neu}$), 3.29(dd,1H,$OCH_2CH^{alkylpart}$),
3.57(dd,1H,$J_{6,7}$=2.7Hz,H-$6^{Neu}$), 3.76(s,3H,COOMe),
3.77-3.84(m,2H,$OCH_2CH^{alkylpart}$,H-$5^{Neu}$), 3.96(dd,1H,$J_{gem}$=12.3Hz,H-$9^{Neu}$),
4.04(t,1H,$J_{5,6}$=6.8Hz,H-$5^{Gal}$), 4.25(dd,1H,$J_{gem}$=11.6Hz,H-$6^{Gal}$),
4.30(m,1H,$J_{gem}$=12.3Hz,H-$9'^{Neu}$), 4.46(dd,1H,$J_{gem}$=11.0Hz,H-$6'^{Gal}$),
4.72-4.74(m,1H,H-$3^{Gal}$), 4.73(d,1H,$J_{1,2}$=7.5Hz,H-$1^{Gal}$),
4.83-4.87(m,1H,H-$4^{Neu}$), 4.98(d,1H,$J_{5,NH}$=10.3Hz,NH),
5.13(d,1H,$J_{3,4}$=3.4Hz,H-$4^{Gal}$), 5.20(dd,1H,$J_{7,8}$=9.6Hz,H-$7^{Neu}$),
5.30(dd,1H,$J_{3,4}$=10.3Hz,H-$2^{Gal}$), 5.58-5.61(m,1H,H-$8^{Neu}$),
7.42-8.14(m,10H,2OCOPh);

$^{13}$C-NMR(150MHz,$CDCl_3$):δ(ppm)14.0, 20.1, 20.7, 20.7, 20.7, 21.3, 22.6, 23.1, 26.5, 26.7, 29.2, 29.3, 29.5, 29.6, 29.6, 29.7, 29.8, 30.7, 31.0, 31.9, 37.3, 37.9, 48.8, 52.9, 61.8, 62.2, 66.4, 67.4, 67.7, 69.3, 70.3, 71.1, 71.3, 71.6, 73.6, 96.7, 101, 128, 129, 129, 130, 130, 132, 133, 165, 165, 168, 170, 170, 170, 170, 170, 170;

MS(Positive ion MALDI-TOF MS.): $C_{72}H_{109}NO_{21}$ : m/z calcd. for $[M+Na]^+$: 1346.73, found :1346.94

(Preparation of Compound 51A)

[Chem. 165]

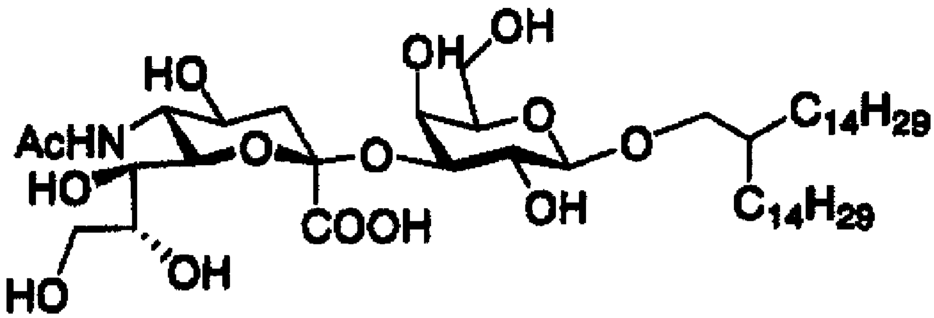

5-Acetamide-3,5-dideoxy-D-glycero-α-D-galacto-2-non ulopyranosylonate-(2→3)-β-D-galactopyranosyl-(1→1)-2-(t etradecyl)-hexadecanol (51A)

Compound 50A (28 mg, 0.0212 mmol) was suspended in MeOH (5.0 mL), and then a catalytic amount of NaOMe was added, followed by stirring at room temperature for 21 hours. After heating to 55 degrees Celsius, the reaction mixture was stirred for 48 hours. By MALDI-TOF MS, an Ac or Bz group of each hydroxyl group was confirmed to be removed by deprotection, and then $H_2O$ was added. After stirring for 3.5 hours, by MALDI-TOF MS, the production of a carboxylic acid was confirmed. The solution was neutralized with Dowex ($H^+$) to pH 7, and then separated from Dowex ($H^+$) by filtration. The resulting solution was concentrated under reduced pressure, and then the resulting syrup was purified by column chromatography (Sephadex LH-20, MeOH) to yield Compound 51A (15 mg, 79%).

[Chem. 166]

[a]D+54.1° ($c$ =0.60 0. MeOH); $^1$H-NMR(600MHz, $CD_3OD$):δ (ppm)0.88-0.90(m,6H,$2CH_3^{alkylpart}$), 1.28-1.39(m,52H,$26CH_2^{alkylpart}$), 1.63(m,1H,$CH_2CH^{alkylpart}$), 1.76(t,1H,$J_{gem}$=10.3Hz,H-$3ax^{Neu}$), 2.03(s,3H,AcN), 2.83(dd,1H,$J_{3eq,4}$=4.1Hz,H-$3eq^{Neu}$), 3.42(dd,1H,$J_{1,2}$=6.2Hz,$OCH_2CH^{alkylpart}$), 3.49-3.53(m,2H,H-$5^{Gal}$,H-$5^{Neu}$), 3.56-3.59(m,1H,H-$2^{Gal}$,H-$8^{Neu}$), 3.63-3.66(m,1H,H-$6^{Gal}$), 3.70-3.74(m,4H,H-$4^{Neu}$,H-$6^{Neu}$,H-$7^{Neu}$,H-$9^{Neu}$,$NH^{Neu}$), 3.78(m,1H,$OCH_2CH^{alkylpart}$), 3.84-3.89(m,2H,H-$6'^{Gal}$,H-$9'^{Neu}$), 3.96(d,1H,H-$4^{Gal}$), 4.02(dd,1H,$J_{3,4}$=2.9Hz,H-$3^{Gal}$), 4.27(d,1H,$J_{1,2}$=7.5Hz,H-$1^{Gal}$)

$^{13}$C-NMR(150MHz,$CD_3OD$):δ(ppm)13.4, 21.6, 22.4, 26.2, 26.4, 29.1, 29.3, 29.4, 29.4, 29.4, 29.7, 30.6, 31.7, 38.0, 40.5, 52.5, 61.0, 63.1, 67.3, 68.0, 68.6, 69.4, 71.5, 73.0, 73.4, 74.7, 76.5, 99.7, 103. 173, 174

MS(Positive ion MALDI-TOF MS.):$C_{47}H_{89}NO_{14}$ : $m/z$ calcd. for $[M+Na]^+$: 914.61, found :914.77

(Preparation of Compound 52A)

[Chem. 167]

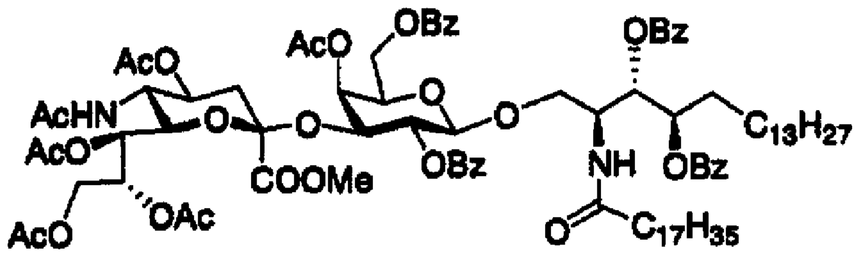

Methyl 5-acetamide-4,7,8,9-tetra-O-acetyl-3,5-dideoxy-D-glycer o-α-D-galacto-2-nonulopyranosylonate]-(2→3)-(2,4,6-O-ac etyl-β-D-galactopyranosyl)-(1→1)-(2S,3R,4E)-3,4-O-benzo yl-2-octadecanamide-4-octadeca-1,3,4-triol (52A)

Compound 49A (76 mg, 0.956 mmol) and Compound 7A (50 mg, 0.0478 mmol) were dissolved in $CH_2Cl_2$ (1.5 mL), and then stirred in the presence of AW300 (150 mg) at room temperature for 1 hour. After cooling to 0 degree Celsius, TMSOTf (1.8 μl, mmol) was added, followed by stirring at 0 degree Celsius for 24 hours.

[Chem. 168]

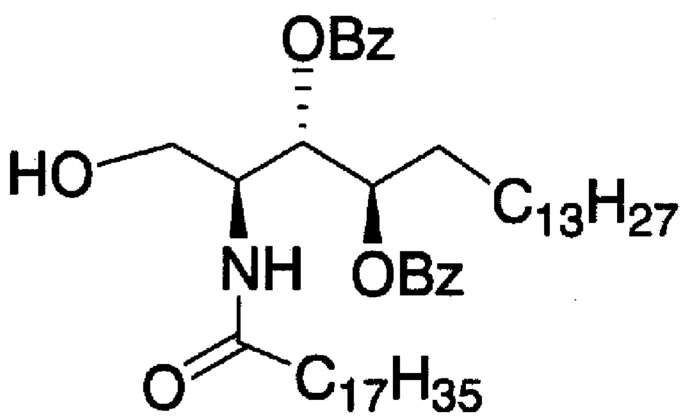

After TEA was added to neutralize the reaction mixture, the solid was filtrated through a Celite®, and then washed with chloroform. The filtrate and the wash solution were combined, and then washed with saturated $NaHCO_3$, $H_2O$, and brine, successively. The resulting organic layer was dried with $Na_2SO_4$, and then the organic layer was separated from the solid by filtration. The filtrate and the wash solution were combined, and then concentrated under reduced pressure. The resulting syrup was purified by silica gel chromatography (toluene:acetone=2:1) to yield Compound 52A (56 mg, 70%).

[Chem. 169]

[a]D+10.0° (c = 0.25 $CHCl_3$);

$^1$H-NMR(600MHz, $CDCl_3$):δ(ppm)0.85-0.89(m,6H,$2CH_3^{alkylpart}$),
1.18-1.29(m,52H,$26CH_2^{alkylpart}$),
1.39-1.43(m,1H,$CH^{alkylpart}$) ,1.46(s,3H,AcN),
1.77,1.95,2.03,2.09,2.11(5s,15H,5AcO), 1.70(t,1H,$J_{gem}$=13.0Hz,H-$3ax^{Neu}$),
2.51(dd,1H,$J_{3eq,4}$=4.8Hz,H-$3eq^{Neu}$), 3.54(dd,1H,$J_{6,7}$=2.7Hz,H-$6^{Neu}$),
3.63(dd,1H,$J_{gem}$=9.6Hz,H-$6^{Gal}$), 3.71(s,3H,COOMe),
3.80(dd,1H,$J_{5,6}$=10.3Hz,H-$5^{Neu}$), 3.83-3.89(m,2H,H-$1^{Cer}$,H-$5^{Gal}$),
3.93(dd,1H,$J_{gem}$=12.3Hz,H-$9^{Neu}$), 4.10(dd,1H,$J_{gem}$=11.0Hz,H-$6'^{Gal}$),
4.25(dd,1H,$J_{gem}$=12.3Hz,H-$9'^{Neu}$), 4.52-4.57(m,1H,H-$2^{Cer}$),
4.67(d,1H,$J_{1,2}$=7.5Hz,H-$1^{Gal}$),
4.68-4.71(m,1H,H-$3^{Gal}$) ,4.80-4.85(m,1H,H-$4^{Neu}$),
4.96(d,1H,$J_{5,NH}$=10.3Hz,NH), 5.01(d,1H,$J_{3,4}$=3.4Hz,H-$4^{Gal}$),
5.18(dd,1H,$J_{7,8}$=9.6Hz,H-$7^{Neu}$), 5.21(dd,1H,$J_{3,4}$=10.3Hz,H-$2^{Gal}$),
5.28-5.31(m,1H,H-$4^{Cer}$), 5.52-5.56(m,2H,H-$3^{Cer}$,H-$8^{Neu}$), 6.07(d,1H,$NH^{Cer}$),
7.37-8.11(m,20H,4OCOPh);

$^{13}$C-NMR(150MHz, $CDCl_3$):δ(ppm)14.1, 20.3, 20.8, 20.8, 21.4, 22.7, 23.2, 25.4, 25.7, 29.4, 29.4, 29.6, 29.6, 29.6, 29.7, 29.8, 29.8, 32.0, 36.2, 37.3, 47.9, 48.9, 53.0, 61.4, 62.4, 66.5, 67.2, 67.5, 69.4, 70.4, 70.9, 71.1, 71.8, 72.4, 74.0, 96.8, 100, 128, 128, 128, 129, 129, 129, 130, 130, 132, 133, 133, 133, 164, 165, 165, 166, 168, 170, 170, 170, 170, 170, 170, 173;
MS(Positive ion MALDI-TOF MS.): $C_{92}H_{128}N_2O_{26}$ : m/z calcd. for $[M+Na]^+$: 1699.86, found :1700.14

(Preparation of Compound 53A)
[Chem. 170]

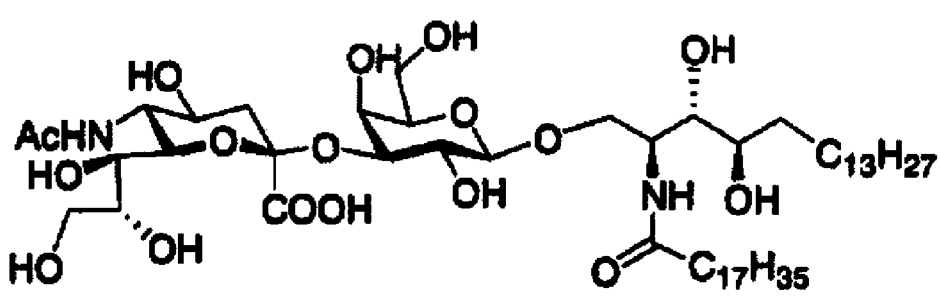

5-Acetamide-3,5-dideoxy-D-glycero-α-D-galacto-2-non ulopyranosylonate-(2→3)-β-D-galactopyranosyl-(1→1)-(2S, 3R,4E)-3,4-O-benzoyl-2-octadecanamide-4-octadeca-1,3,4-triol (53A)

Compound 52A (35 mg, 0.0208 mmol) was suspended in MeOH (5.0 mL), a catalytic amount of NaOMe was added, followed by stirring at 55 degrees Celsius for 60 hours. By MALDI-TOF MS, an Ac or Bz group of each hydroxyl group of the compound was confirmed to be removed by deprotection, and then $H_2O$ was added. After stirring for 3.5 hours, by MALDI-TOF MS, the production of a carboxylic acid was confirmed. The solution was neutralized with Dowex ($H^+$) to pH 7, and then separated from Dowex ($H^+$) by filtration. The resulting solution was concentrated under reduced pressure, and then the resulting syrup was purified by column chromatography (Sephadex LH-20, MeOH) to yield Compound 53 (21 mg, quant.).

[Chem. 171]

[a]D-25.0° (c=0.02 MeOH);

$^1$H-NMR(600MHz, $CD_3OD$): δ(ppm) 0.88-0.90(m, 6H, $2CH_3^{Cer}$), 1.28-1.31(m, 52H, $26CH_2^{Cer}$), 1.39-1.42(m, 2H, H-$5^{Cer}$), 1.72(t, 1H, $J_{gem}$=13.0Hz, H-$3ax^{Neu}$), 2.00(s, 3H, AcN), 2.86(dd, 1H, $J_{3eq,4}$=4.1Hz, H-$3eq^{Neu}$), 3.49-3.51(m, 2H, H-$1^{Cer}$, H-$2^{Cer}$), 3.53-3.62(m, 5H, H-$3^{Cer}$, $NH^{Cer}$, H-$8^{Neu}$, H-$9^{Neu}$), 3.58(dd, 1H, $J_{gem}$=9.6Hz, H-$2^{Gal}$), 3.66-3.76(m, 5H, H-$5^{Gal}$, H-$6^{Gal}$, H-$4^{Neu}$, $NH^{Neu}$, H-$9'^{Neu}$), 3.81-3.82(m, 1H, H-$7^{Neu}$), 3.82-3.85(m, 1H, H-$4^{Cer}$), 3.92(d, 1H, H-$4^{Gal}$), 4.03(dd, 1H, $J_{3,4}$=3.4Hz, H-$3^{Gal}$), 4.10-4.12(m, 1H, H-$5^{Neu}$), 4.19(dd, 1H, $J_{6,7}$=3.4Hz, H-$6^{Neu}$), 4.29(d, 1H, $J_{1,2}$=8.2Hz, H-$1^{Gal}$);

$^{13}$C-NMR(150MHz, $CD_3OD$): δ(ppm) 14.6, 22.7, 23.9, 27.3, 27.3, 30.6, 30.6, 30.8, 30.9, 30.9, 31.0, 31.0, 31.0, 31.1, 32.3, 33.2, 37.4, 42.4, 51.9, 54.1, 62.9, 64.6, 69.0, 69.5, 70.0, 70.1, 71.2, 73.0, 73.2, 75.1, 76.9, 77.8, 101, 105, 175, 175, 176

MS(Positive ion MALDI-TOF MS.): $C_{92}H_{126}N_2O_{26}$ : m/z calcd. for $[M+Na]^+$: 1059.69, found :1059.75

(Example 4. Preparation using synthetic GM3 and synthetic GM4)

Aliposome is prepared by an improved cholate dialysis method.

In the present example, the gangliosides (GM3 and GM4 (of which the structures of ceramides are natural type, plant type, or an analog type (Fig. 4)) prepared in Examples 1 and 2 are used.

As lipids composing the liposome, dipalmitoylphosphatidylcholine(DPPC), cholesterol, dicetylphosphate(DCP), and ganglioside, dipalmitoylphosphatidylethanolamine(DPPE) are mixed at a molar ratio of 35:40:5:15:5 (total lipid amount: 45.6 mg) .

Then, to this mixed lipids composing the liposome is added sodium cholate 46.9 mg, and then dissolved in 3 mL of chloroform/methanol (1:1) solution. This solution is evaporated, and then the residue is dried *in vacuo* to yield the lipid membrane. The resulting lipid membrane is resuspended in 3 mL of N-tris(hydroxymethyl)-3-aminopropane sulfonate buffer solution (pH 8.4), and then stirred. For this solution, displacement with nitrogen is carried out, and then the solution is sonicated to yield a clear micelle suspension. Furthermore, the micelle suspension is ultrafiltered (molecularcutoff: 10, 000) using PM10 membrane (Amicon Co., USA) and N-tris(hydroxymethyl)-3-aminopropane sulfonate buffer solution (pH 8.4) to prepare a homogenous liposome.

10 mL of the liposome solution prepared in the present Example is ultrafiltered (molecular cutoff: 300,000)

using a XM300 membrane (Amicon Co., USA) and carbonate buffer solution (pH 8.5) to adjust the pH of the solution to 8.5. Then 10 mg of bis(sulfosuccinimidyl)suberate ($BS^3$; Pierce Co., USA) as a crosslinking agent is added thereto, followed by stirring at room temperature for 2 hours. By further stirring under refrigerated condition overnight, the reaction to chemically bond $BS^3$ with dipalmitoylphosphatidylethanolamine, which is the lipid on the liposome membrane, is completed. Then, this liposome solution is ultrafiltered (molecular cutoff: 300, 000) with the XM300 membrane and carbonate buffer solution (pH 8.5). Then, 40 mg of tris(hydroxymethyl)aminomethane dissolved in 1 mL of carbonate buffer solution (pH 8.5) is added to 10 mL of the liposome solution. This solution is stirred at room temperature for 2 hours followed by further stirring under refrigerated condition overnight, and then ultrafiltered (molecular cutoff: 300,000) so that: free tris(hydroxymethyl)aminomethane is removed; the carbonate buffer solution is exchanged to N-tris(hydroxymethyl)-3-aminopropane sulfonate buffer solution (pH 8.4); and the reaction to chemically bond tris (hydroxymethyl) aminomethane with $BS^3$ bound to the lipid on the liposome membrane is completed. As a result, hydroxyl groups of tris (hydroxymethyl)aminomethane coordinate onto dipalmitoylphosphatidylethanolamine, which is the lipid of the liposome membrane, and the liposome is hydrated-hydrophilized.

(Binding of human serum albumin (HSA) onto the liposome membrane surface)

To form the binding of human serum albumin (HSA) onto the liposomemembrane surface, inapublishedmethod (Yamazaki, N., Kodama, M. and Gabius, H.-J. (1994) Methods Enzymol. 242, 56-65), a coupling reaction method is used. That is to say, this reaction is performed as a 2 step chemical reaction. First, to ganglioside existing on the liposome membrane surface in 10 mL of the liposome membrane surface obtained by the present Example is added 43 mg of sodium metaperiodate dissolved in 1 mL of N-tris(hydroxymethyl)-3-aminopropane sulfonate buffer solution (pH 8.4), and then stirred under refrigerated condition overnight to oxidize the ganglioside with periodate. By ultrafiltration (molecular cutoff: 300,000) using the XM300 membrane and PBS buffer solution (pH 8.0), free sodium periodate is removed, N-tris(hydroxymethyl)-3-aminopropane sulfonate buffer solution is exchanged to PBS buffer solution (pH 8.0), and 10 mL of the oxidized liposome is yielded. To this liposome solution is added 20 mg of human serum albumin (HSA)/PBS buffer solution (pH 8.0), and then reacts at room temperature for 2 hours. Then, 100 $\mu$l of 2M $NaBH_3CN$/PBS buffer solution (pH 8.0) is added thereto, stirred at room temperature for 2hours, and then further stirred under refrigerated condition overnight to bond a ganglioside on the liposome with HSA by a coupling reaction with HSA. Then, ultrafiltration (molecular cutoff: 300,000) is per formed to remove free sodium cyanoboronate and human serum albumin is removed, and exchange the buffer solution of this solution to carbonate buffer solution (pH 8.5), and consequently 10 mL of a HSA bound liposome solution (liposome intermediate HSA) is yielded.

(Comparative Example 1: Preparation of liposome using naturally-derived ganglioside)

Except using Porcine Brain Total Gangliosides produced by Avanti (Catalog No. 100232) as gangliosides, by a similar method to that in Example 4, 10 mL of a liposome solution (liposome intermediate HSA) is yielded.

(Determination of lipid·protein)

With regard to the lipid amount of the liposome intermediate HSA obtained by Example 4 and Comparative Example 1, the total cholesterol amount is measured in the presence of 0.5% Titon X-100 using the Determiner TC555 (Kyowa Medex), and then, by calculating the total lipid amount from a molar ratio of each lipid, the lipid amount of the liposome intermediate HSA can be determined.

The protein amount can be measured in the presence of 1% SDS using the Micro BCA™ Protein Assay Kit (PIERCE).

(Measurement of particle size·zeta potential)

With regard to a particle size and zeta potential, after a liposome solution is diluted 50 times with purified water, they can be measured by using the Zetasizer Nano-S90 (SYSMEX) at 25 degrees Celsius.

(Example 5. Preparation of Cy5.5-encapsulating liposome using synthetic GM3 and synthetic GM4)

A liposome was prepared by an improved cholate dialysis method.

In the present example, gangliosides (GM3 and GM4 (of which the structure of a ceramide is natutal-, plant-, or pseude-type (Fig. 4)) prepared in Examples 1 and 2 were used.

As lipids composing the liposome, dipalmitoylphosphatidylcholine (DPPC), cholesterol, dicetylphosphate (DCP), ganglioside, dipalmitoylphosphatidylethanolamine (DPPE) was mixed at a molar ratio of 35:40:5:15:5 (total lipid

amount: 45.6 mg) .

Then, to these mixed lipids composing the liposome was added 46.9 mg of sodium cholate, dissolved in 3 mL of dichloroform/methanol (1:1, v/v), evaporated with an evaporator, and then dried *in vacuo* to yield lipid membrane. This lipid membrane is resuspended in TAPS buffer solution (pH 8.4), and then sonicated to yield a micelle suspension.

Meanwhile, 20 mg of human serum albumin (HSA) was dissolved in 3 mL of tris(hydroxymethyl)methylaminopropane sulfonate buffer solution (TAPS pH 8.4), 2 mg of Cy5.5-NHS ester (GE Healthcare Bioscience) was mixed therewith, and then reacted at 37 degrees Celsius for 3 hours. By ultrafiltration (molecular cutoff: 10,000) using a PM10 membrane (Amicon Co., USA) and N-tris(hydroxymethyl)-3-aminopropane sulfonate buffer solution (pH 8.4), free Cy5.5-NHS ester was removed to yield 3 mL of a Cy5.5-bound HSA solution. The above micelle suspension was mixed Cy5.5-bound HSA solution, and then ultrafiltration (molecular cutoff: 10,000) using the PM10 membrane (Amicon Co., USA) and N-tris(hydroxymethyl)-3-aminopropane sulfonate buffer solution (pH 8.4) was performed. Additionally, ultrafiltration (molecular cutoff: 300, 000) using the PM10 membrane (Amicon Co., USA) and carbonate buffer solution (pH9.0) was performed to yield 10 mL of a liposome solution (Liposome-Cy5.5). To 10 mL of this liposome solution (Liposome-Cy5.5) was added 10 mg of bis (sulfosuccinimidyl) suberate ($BS_3$; PIERCE) as a crosslinking agent, stirred at room temperature for 2 hours, and then further stirred at 4 degrees Celsius overnight to chemically bond $BS_3$ to the liposome membrane. By ultrafiltration (molecular cutoff: 300,000) using the PM10 membrane (Amicon Co., USA) and carbonate buffer solution (pH9.0), free $BS_3$ was removed. Then, 40 mg of tris(hydroxymethyl)aminomethane (Tris) was added, stirred at room temperature for 2 hours, and then further stirred at 4 degrees Celsius overnight. By ultrafiltration (molecular cutoff: 300, 000) using the PM10 membrane (Amicon Co., USA) and N-tris(hydroxymethyl)-3-aminopropane sulfonate buffer solution (pH 8.4), free Tris was removed. Then, 10.8 mg of sodium metaperiodate was added, followed by stirring at 4 degrees Celsius overnight.

By ultrafiltration (molecular cutoff: 300,000) using the PM10 membrane (Amicon Co., USA) and phosphate buffer (pH 7.2), periodate that had still not reacted was removed. Subsequently, 20 mg of human serum albumin (HSA) was added, and then reacted at room temperature for 2 hours. 1.25 mg of sodium cyanoborohydride was added, stirred at room temperature for 2 hours, and then further stirred at 4 degrees Celsius overnight. By ultrafiltration (molecular cutoff: 300,000) using the PM10 membrane (Amicon Co., USA) and carbonate buffer solution (pH9.0), sodium cyanoborohydride that had not still reacted was removed to yield 10 mL of a synthetic GM3 liposome solution (synthetic GM3 liposome intermediate HSA (in a state in which human serum albumin binds to a synthetic GM3 liposome).

(Comparative Example 2: Preparation of liposome using naturally-derived ganglioside)

Except using Porcine Brain Total Gangliosides produced by Avanti (Catalog No. 100232) as gangliosides, by a similar method to that in Example 5, 10 mL of a liposome solution (liposome intermediate HSA) is yielded.

(Determination of lipid·protein)

With regard to the lipid amount of the liposome intermediate HSA obtained by Example 5 and Comparative Example 2, the total cholesterol amount is measured in the presence of 0.5% Titon X-100 using the Determiner TC555 (Kyowa Medex), and then, by calculating the total lipid amount from a molar ratio of each lipid, the lipid amount of the liposome intermediate HSA can be determined.

The protein amount can be measured in the presence of 1% SDS using the Micro BCA™ Protein Assay Kit (PIERCE).

(Measurement of particle size·zeta potential)

With regard to a particle size and zeta potential, after a liposome solution is diluted 50 times with purified water, they can be measured by using the Zetasizer Nano-S90 (SYSMEX) at 25 degrees Celsius.

(Example 6A. Preparation of a sugar-chain-modified liposome using synthetic ganglioside)

(Addition of a sugar-chain to liposome)

In the present example, synthetic GM3 and synthetic GM4 liposome solutions prepared in Example 4 are used.

To 10 mL of each liposome solution (liposome intermediate HSA) is added 10 mg of 3,3-dithiobis(sulfosuccinimidylpropionate) (DTSSP; PIERCE) as a crosslinking agent. After stirring, free DTSSP is removed by ultrafiltration.

To sialyl Lewis X (Lewis X (SLX: Calbiochem) dissolved in purified water is added ammonium hydrogen carbonate, and then stirred to yield an aminated SLX solution. To the above liposome solution, to which DTSSP was added, is added aminated SLX, and then reacted. Subsequently, a Tris solution is added, and then stirred. By ultrafiltration,

free SLX and Tris are removed.

(Example 6B. Preparation of an antibody-modified liposome using synthetic ganglioside)

In the present example, using the synthetic GM3 and synthetic GM4 liposome solution prepared in Example 4, an antibody-modified liposome is prepared.

To 10 mL of liposome solution (liposome intermediate HSA) is added 10 mg of DTSSP (PIERCE) as a crosslinking agent, and then stirred at room temperature for 2 hours and further at 4 degrees Celsius overnight. Subsequently, free DTSSP is removed by ultrafiltration (molecular cutoff: 300,000) using the PM10 membrane (Amicon Co., USA) and carbonate buffer solution (pH 9.0).

To this liposome solution is added an antibody, and then reacted at room temperature for 2 hours. Subsequently, a 132 mg/mL Tris solution is added, and then stirred at room temperature for 2 hours and further at 4 degrees Celsius overnight. By ultrafiltration (molecular cutoff: 300,000) using the PM10 membrane (Amicon Co., USA) and HEPES buffer solution (pH 7.2), a free antibody and Tris are removed to yield an antibody-bound liposome.

(Comparative Example 3. Preparation of liposome not bound with the recognition probe)

In the present Comparative Example, liposomes that do not bind with the recognition probe, and use synthetic GM3 liposome, synthetic GM4 liposome and a naturally-derived ganglioside are prepared by a similar method to that in Example 4 and Comparative Example 1.

(Evaluation of the physical properties of the liposome prepared in Examples 6A and 6B and Comparative Example 3)

(Evaluation of the recognition probe bound to each liposome surface)

(1. Evaluation of the amount of sugar chain bound (Evaluation of FITC binding))

To the liposome solution (liposome intermediate HSA) is added DTSSP (PIERCE) as a crosslinking agent. After stirring, free DTSSP was removed by ultrafiltration.

To FITC dissolved in purified water is added ammonium hydrogen carbonate, and then stirred to yield an aminated FITC solution. To the above liposome solution, to which DTSSP was added, is added aminated FITC, and then reacted. Subsequently, a Tris solution is added, and then stirred. By ultrafiltration, free FITC and Tris are removed to yield a FITC-bound liposome.

The amount of FITC bound to liposome can be determined by measuring the amount of fluorescence (excitation wavelength: 495nm, fluorescence wavelength: 520nm).

(2. Evaluation of the amount of antibody bound)

To the liposome solution (liposome intermediate HSA) is added DTSSP (PIERCE) as a crosslinking agent. After stirring, free DTSSP is removed by ultrafiltration.

To this liposome solution is added an antibody (for example, anti-E-selectin antibody), and then reacted. Subsequently, a Tris solution is added, and then stirred. By ultrafiltration, a free antibody and Tris are removed to yield an antibody-bound liposome.

The amount of an antibody bound onto the liposome surface can be measured by an Enzymed immuno assay (EIA) method. 50 μl of a PBS solution of a protein (for example, E-selectin (R&D Systems)), which is an antigen, is added to each well of a 96-well microplate, and then solid-phased. After an antigen solution in the plate is discarded, 300 μl of 2% BSA/PBS is added to each well, and then stood. The BSA solution is discarded, and then washed 3 times with PBS . 100 μl of an antibody standard solution and 100μl of an antibody-bound liposome sample are added, followed by standing for 1 hour. The solution in the wells is discarded, and then washed with PBS. HRP-labeled anti-mice IgG antibody is diluted with 10% Tween 20/9% EDTA/PBS, 100μl of this solution is added to each well, subsequently standing for 1 hour. The solution in the wells is discarded, and then washed with PBS. 1-Step™ TMB-Blotting (PIERCE) is added, and reacted, and then 100 μl of a quenching solution (2M sulfuric acid) is added to each well. The absorbance at 450nm is measured, and thereby the amount of an antibody-bound onto the liposome surface can be determined on the basis of the antibody standard solution.

(Determination of lipid·protein)

With regard to the lipid amount of each resulting liposome, the total cholesterol amount is measured in the

presence of 0.5% Titon X-100 using the Determiner TC555 (Kyowa Medex), and then the total lipid amount can be calculated from the molar ratio of each lipid.

The protein amount can be measured in the presence of 1% SDS using the Micro BCA™ Protein Assay Kit (PIERCE).

(Measurement of particle size·zeta potential)

With regard to a particle size and zeta potential, after each liposome solution is diluted 50 times with purified water, they can be measured using the Zetasizer Nano-S90 (SYSMEX) at 25 degrees Celsius.

(Example 7A. Preparation of SLX-modified Cy5.5-encapsulating liposome using a synthetic ganglioside)

In the present example, SLX was used as a target site recognition probe, and synthetic GM3 and GM4 were used as synthetic gangliosides.

(Addition of a sugar chain to the liposome)

In the present example, Cy5.5-encapsulating liposome prepared in Example 5 was used. To 10 mL of each liposome solution (liposome intermediate HSA) was added 10 mg of 3,3-dithiobis(sulfosuccinimidylpropionate) (DTSSP; PIERCE) as a crosslinking agent, and then stirred at room temperature for 2 hours and further at 4 degrees Celsius overnight. Then, free DTSSPwasremoved by ultrafiltration.

To 2 mg of sialyl Lewis X (Lewis X (SLX: Calbiochem) dissolved in 0.5 mL of purified water was added 0.25g of ammonium hydrogen carbonate, and then stirred at 37 degrees Celsius for 3 days to yield an aminated SLX solution. To the above liposome solution to which DTSSP was added the aminated SLX such that the final concentration of sugar chain was 15 $\mu$/mL. After reacting at room temperature for 2 hours, 132 mg/mL Tris solution was added, and then stirred at room temperature for 2 hours and further at 4 degrees Celsius overnight. By ultrafiltration (molecular cutoff: 300,000) using the PM10 membrane (Amicon Co., USA) and HEPES buffer solution (pH 7.2), free SLX and Tris was removed.

(Example 7B. Preparation of antibody-modified Cy5.5 encapsulating liposome using synthetic ganglioside)

In the present example, using Cy5.5 encapsulating liposome prepared in Example 5, an antibody-modified liposome was prepared.

(Preparation of anti-E-selectin monoclonal antibody)

Ahybridoma (Line Name: CL-3, ATCC Number: CRL-2515) producing an anti-human E-selectin monoclonal antibody was purchased and then the monoclonal antibody was purified. Balb/c, 6-week old, and female mice were purchased from Japan SLC Inc. 500 $\mu$l of Pristane was intraperitoneally administered twice (5 day interval) to Balb/c mice. $1\times10^7$ cells were intraperitoneally administered. After 20 days, the ascites were collected. The ascites were centrifuged (12,000 gX 30 minutes), and then the supernatant was collected. Ammonium sulfate (0.6 fold of saturation) was added to the supernatant, and then stood at 4 degrees Celsius for several hours. After the precipitate was resuspended in PBS, the external solution PBS was dialyzed. Then, affinity purification by Hi Trap Protein G column (GE healthcare) yielded an anti-E-selectin antibody.

(Addition of antibody to liposome)

To 10 mL of the liposome solution (liposome intermediate HSA) added 10 mg of DTSSP (PIERCE) as a crosslinking agent, and then stirred at room temperature for 2 hours and further at 4 degrees Celsius overnight. Free DTSSP was removed by ultrafiltration (molecular cutoff: 300,000) using a membrane (Amicon Co., USA) and carbonate buffer solution (pH 9.0).

To this liposome solution was added an anti-E-selectin monoclonal antibody, followed by reacting at room temperature for 2 hours. Subsequently, 132 mg/mL Tris solution was added, and then stirred at room temperature for 2 hours and further at 4 degrees Celsius overnight. By ultrafiltration (molecular cutoff: 300, 000) using the PM10 membrane (Amicon Co., USA) and HEPES buffer solution (pH 7.2), free antibody and Tris were removed to yield an antibody-bound liposome.

(Comparative Example 4. Preparation of Cy5.5 encapsulating liposome that does not bind to the recognition probe)

In the present Comparative Example, liposome solution prepared in Example 5 and Comparative Example 2 was used. Except not binding a sugar chain, a Cy5.5 encapsulating liposome (Lipo-Cy5.5) not bound with SLX was prepared by a similar method to the case of SLX-bound Cy5.5 encapsulating liposome until the last step.

(Evaluation of the physical properties of the liposomes prepared in Examples 7A and 7B and Comparative Example 4)

(Evaluation of the recognition probe bound onto the liposome surface)

(1. Evaluation of the amount of sugar chain bound (Evaluation of FITC binding))

To 10 mL of liposome solution (liposome intermediate HSA) was added 10 mg of DTSSP (PIERCE) as a crosslinking agent, and then stirred at room temperature for 2 hours and further at 4 degrees Celsius overnight. Free DTSSP was removed by ultrafiltration (molecular cutoff: 300,000) using the PM10 membrane (Amicon Co., USA) and carbonate buffer solution (pH9.0).

To 9.5 mg of FITC dissolved in 5 mL of purified water was added 1.0 g of ammonium hydrogen carbonate, and then stirred at 37 degrees Celsius for 3 days to yield an aminated FITC solution. To the above liposome solution to which DTSSP had been added, was added the aminated FITC. After reacting at room temperature for 2 hours, 132 mg/mL Tris solution was added, and then stirred at room temperature for 2 hours and further at 4 degrees Celsius overnight. By ultrafiltration (molecular cutoff: 300, 000) using the PM10 membrane (Amicon Co., USA) and HEPES buffer solution (pH 7.2), free FITC and Tris were removed to yield a FITC-bound liposome.

The amount of FITC bound to liposome is determined by measuring the amount of fluorescence (excitation wavelength: 495nm, fluorescence wavelength: 520nm).

(2. Evaluation of the amount of antibody bound)

The amount of anti-E-selectin monoclonal antibody bound onto the liposome surface was measured by Enzyme immuno assay (EIA) method. 50μl of an E-selectin (R&D Systems) 2 μg/mL PBS solution was added to each well of a 96-well microplate, and then stood at room temperature for 1 hour to be in the solid-phase. After an antigen solution in the plate was discarded, 300 μl of 2% BSA/PBS was added to each well, and then stood at room temperature for 2 hours. The BSA solution was discarded, and then washed 3 times with PBS. 100 μl of an anti-E-selectin antibody standard solution and 100μl of an antibody-bound liposome sample were added, followed by standing at room temperature for 1 hour. The solution in the wells was discarded, and then washed 3 times withPBS. HRP-labeled antimice IgG antibody (SIGMA) diluted with 10% Tween 20/9% EDTA/PBS, 100 μl of this solution was added to each well, subsequently standing at room temperature for 1 hour. The solution in the wells was discarded, and then washed 3 times with PBS. 100 μl of 1-Step™ TMB-Blotting (PIERCE) was added to each well, and reacted at room temperature for 20 minutes, and then 100 μl of a quenching solution (2M sulfuric acid) was added to each well. The absorbance at 450nm was measured, and thereby the amount of the antibody bound onto the liposome surface was determined on the basis of the anti-E-selectin antibody standard solution.

(Determination of lipid·protein)

With regard to the lipid amount of SLX-Lipo-Cy5.5 and Lipo-Cy5.5 obtained by the present Example, the total cholesterol amount was measured in the presence of 0.5% Triton X-100 using Determiner TC555 (Kyowa Medex), and then the total lipid amount was calculated from the molar ratio of each lipid.

With regard to the protein amount, in the presence of 1% SDS, Micro BCA™ Protein Assay Kit (PIERCE) was used.

(Measurement of particle size·zeta potential)

With regard to a particle size and zeta potential, after the liposome solution was diluted 50 times with purified water, they were measured by using Zetasizer Nano-S90 (SYSMEX) at 25 degrees Celsius.

Results regarding SLX-Lipo-Cy5.5 are shown in Table 5 below.

[Table 6]

(Table 5)

| Lipid amount • Particle size • Z potential | | | |
|---|---|---|---|
| | Limpid tamount **(mg/ml)** | Particle size **(nm)** | Z potential **(mV)** |
| Control | **2.6** | **119** | **-78** |
| Synthetic GM3 plant ceramide | **3.2** | **112** | **-73** |
| Synthetic GM3 natural ceramide | **2.0** | **73** | **-71** |
| Synthetic GM3 pseudo-ceramide | **2.3** | **92** | **-70** |
| Synthetic GM4 plant ceramide | **2.0** | **118** | **-99** |
| Synthetic GM4 pseudo-ceramide | **2.3** | **132** | **-95** |

* Control: a liposome prepared using a naturally-derived ganglioside

(Evaluation using tumor-bearing mice)

An accumulation property to a tumor site was checked using tumor-bearing mice. The tumor-bearing mice were used for an experiment 10 days after Ehrlich ascites tumor (EAT) cells (purchased from ATCC; ATCC Number: CCL-77; about $5\times10^6$) were transplanted subcutaneously into the right femoral region of female, Balb/c mice (6 week old; purchased from Japan SLC). Isoflurane was used for anesthesia. Then, the liposome (100 $\mu$l/mouse) was administered to the caudal vein. Immediately, 24, and 48 hours after administering, using explore Optix (GE Healthcare Bioscience), the tumor site (right femoral region) of the same individual was observed. A fluorescent substance Cy5.5 was detected (excitation wavelength: 680nm, fluorescence wavelength: 700nm).

(Result)

With regard to liposomes using a variety of synthetic gangliosides (synthetic GM3 and synthetic GM4), the physical properties, binding capacity of a recognition probe, and the accumulation property to a tumor site were compared and evaluated. As a control, Porcine Brain Total Gangliosides (produced by Avanti, Catalog No. 100232) of a natural ganglioside were used.

As a result, the particle size of both liposomes using a synthetic ganglioside and liposomes using a natural ganglioside was about 100nm (Fig 3, Fig 5a-d, Table 5).

Evaluation of the binding capacity of a recognition probe to the synthetic GM3 (plant ceramide, natural ceramide, pseudo-ceramide) liposome surface was performed using evaluation of FITC binding. Regarding a synthetic GM3 liposome of a natural ceramide and a synthetic GM3 liposome pseudo-ceramide, an approximately equivalent amount to that of control (a liposome using a natural ganglioside) was bound. Regarding synthetic GM3 of a plant ceramide, the amount of the antibody bound was about 65% relative to control (Fig. 6a and 6b).

Furthermore, evaluation of the binding capacity of the recognition probe to the synthetic GM4 (plant ceramide or pseudo-ceramide) liposome was performed by checking the binding property of an antibody. An anti-E-selectin antibody is added to the liposome surface, and then the amount of the antibody added was measured by the EIA method. Regarding both plant ceramide and pseudo-ceramide, the amount of the antibody added was approximately equivalent to that of control (Fig. 6c).

With regard to the Cy5.5-encapsulating SLX liposome prepared using synthetic GM3 (plant ceramide), an accumulation property to born tumor was examined. The SLX liposome accumulated significantly in comparison with control (a liposome to which SLX is added) (Fig. 7A).

With regard to the anti-E-selectin antibody liposome prepared using the synthetic GM4 (plant ceramide or pseudo-ceramide), an accumulation property to a born tumor site was examined. The liposome of a plant ceramide accumulated significantly in comparison with control (a liposome to which an antibody is not added) (Fig. 7B). The antibody liposome prepared using a pseudo-ceramide is not found to be different in accumulation property from a liposome to which an antibody is not added (Fig. 7C).

(Example 8. Relationship between ganglioside and accumulation property)

(Method)

(Preparation of a tumor-bearing mouse)

Ehrlich ascites tumor cells (EAT cells, $5 \times 10^6$) were transplanted subcutaneously into the right femoral region of a Balb/c mouse (female, 6-weeks old). 10 days after the transplantation, 100 μl of the liposome was administered to the caudal vein, and thereby an accumulation property to the tumor site was checked.

(Preparation of liposome)

A liposome encapsulating Cy5.5-labeled human serum albumin was prepared by a similar method to that in Example 7 using the total ganglioside (Lot. TGANG 13, TGANG 14, and TGANG 16) and synthetic GM3 plant type, as gangliosides, in the lipid component of the liposome. Comparison and examination regarding an accumulation property to a tumor site of a tumor-bearing mouse were performed.

(Evaluation method)

300 μl of 1/10 Nembutal solution was intraperitoneally administered to a tumor-bearing mouse to anesthetize it. By the fluorescence imaging system explore Optix (GE Healthcare), image data prior to the administration was taken. The cy5.5-encapsulating sugar-chain modified liposome (K1) (200μl: corresponding to 750 μg of lipid amount) was administered to the caudal vein. Image data were taken 24 and 48 hours after the administration.
As a control, a liposome not bound with a sugar chain was administered in the same amount. Image data were chronologically taken. All of the image data were taken from the ventral side.

(Result)

In the case that the total ganglioside (Lot. No. TGANG13: Avanti) was used as a raw material of a sugar-chain-bound liposome, when a density of sugar chain was 50 μg/mL, 24 and 48 hours after the administration, in comparison with a liposome that does not bind with a sugar chain, a significant difference of accumulation property was found (Figs. 8C and 8D).
However, in the case of a sugar-chain-bound liposome using a ganglioside in a different lot (Lot. No. TGANG14: Avanti), when a density of sugar chain was the same, a difference of accumulation property was not found in comparison with a liposome without a sugar chain (Figs. 8A and 8B). Regarding these liposomes, the density of sugar chain was the same, but the difference of the accumulation property between the lots was found.
With regard to the relationship between the density of sugar chain and accumulation property, it is apparent from the previously-reported experiments in which a rheumatism model mouse was used (International Publication No. WO. 2007/091661 pamphlet) that, when there is an excess amount of a sugar chain on the liposomal membrane surface, the accumulation property lowers (Fig. 9).
Based on that, in the sugar-chain modified liposome prepared using the total ganglioside (Lot. No. TGANG14), it was expected to be possible that the density of sugar chain of the liposomal membrane surface was out of the optimal density thereof.
Then, after a sugar-chain-bound liposome was prepared using the total ganglioside (Lot. No. TGANG14 and Lot. No. TGANG16) as gangliosides, the density of sugar chain and the accumulation property thereof were evaluated.
As a result, with regard to all liposomes prepared using gangliosides of TGANG14 and TGANG16, when the density of sugar chain was 50 μg/mL, there was no difference in accumulation property in comparison with the liposome without a sugar chain. On the other hand, when the density of sugar chain is 10 μg/mL, 15 μg/mL, or 20 μg/mL, the sugar-chain-modified liposome exhibited a higher accumulation property than the liposome without a sugar chain.
For the above reason, it is thought that, because of the difference between the lots of a ganglioside, even if the same amount of a sugar chain is used, the density of sugar chain bound onto the liposomal membrane surface will vary, and consequently, in the case of TGANG14, the density of sugar chain can get outside the optimal density of sugar chain (Figs. 10 and 11).
After a sugar-chain-bound liposome was prepared using synthetic GM3 (plant type), an accumulation property to aborn tumor site was checked. As a result, when the density of sugar chain was 50 μg/mL, a higher accumulation property was exhibited than the liposome without a sugar chain (Fig. 12A-C). Based on that, it was understood that the synthetic GM3 can be used as a raw material of a sugar-chain-bound liposome suitable for molecular imaging, instead of the total ganglioside, which is commercially available and affects accumulation property depending on a lot.

The synthetic GM3 is a synthetic product, and is constituted of a single substance. It is thus expected that, as a raw material, there is little difference between lots. Accordingly, it is thought that the liposome prepared using the synthetic GM3 can make a certain amount of a sugar chain always bind onto the liposomal membrane surface. It is thus thought that the synthetic GM3 is optimal as a raw material of a sugar-chain liposome.

(Example 9. Preparation of sugar-chain-modified Cy5.5-encapsulating liposome particles)

(Cy5.5-label of human serum albumin (HSA))

To a solution of HSA/tris(hydroxymethyl)methylaminopropanesulfonate (TAPS) buffer solution (pH 8.4) was mixed with a solution of Cy5.5-NHS ester (Amersham Bioscience)/TAPS buffer solution (pH 8.4), and then stirred at 37 degrees Celsius for 3 hours. To remove free Cy5.5, ultrafiltration (NMWL: 10,000) was performed.

(Preparation of liposome)

(Liposome formation and encapsulation of Cy5.5-labeled HSA (step A))

16.8 mg of dipalmitoylphosphatidylcholine (DPPC), 10.1 mg of cholesterol (Chol), 14.6 mg of ganglioside (synthetic GM3), 1.8 mg of dicetylphosphate (DCP), 2.3 mg of dipalmitoylphosphatidylethanolamine (DPPE), and 46.9 mg of sodium cholate were each weighed, suspended in 3 mL of a methanol · chloroform solution (1:1), and then stirred at 37 degrees Celsius for 1 hour.

Chloroform·methanol was evaporated by a rotary evaporator, dried *in vacuo,* and then resuspended in 3 mL of TAPS buffer solution (pH8.4). After stirring at 37 degrees Celsius for 1 hour, nitrogen displacement was performed, followed by sonication. This sonicated solution and Cy5.5-labeled HSA solution were mixed, and then ultrafiltered (NMWL: 10,000). As a result, Cy5.5-labeled HSA-encapsulating liposome particles were yielded.

(Hydrophilization of the liposome (step B))

To exchange the buffer for CBS buffer solution (pH 8.5), ultrafiltration (NMWL: 300,000) was performed. 10 mg of $BS^3$ (PIERCE) as a crosslinking agent was added, and then stirred at room temperature for 2 hours and further under refrigerated condition overnight. To remove free $BS^3$, ultrafiltration (NMWL: 300,000) was then performed. 40 mg of Tris was added, and then stirred at room temperature for 2 hours and further under refrigerated condition overnight. After free Tris was removed, to exchange the buffer for TAPS buffer solution (pH 8.4), ultrafiltration (NMWL: 300,000) was performed.

(Binding of the liposome and HSA (step C))

10.8 mg of sodium metaperiodate was added, and then stirred under refrigerated condition overnight to oxidize the liposome particle surface. After free sodium metaperiodate was removed, to exchange the buffer for PBS buffer solution (pH 8.0), ultrafiltration (NMWL: 300,000) was performed. 20 mg of HSA was added, and then reacted at room temperature for 2 hours. To the resulting solution was added 3.13 mg of sodium cyanoboronate, and then stirred at room temperature for 2 hours and further under refrigerated condition overnight. After free sodium cyanoboronate and HSA were removed, to exchange the buffer for CBS buffer solution (pH 8.5), ultrafiltration (NMWL: 300,000) was performed.

(Binding of sugar chain to the liposome (step D))

2 mg of a sugar chain was dissolved in 0.5 mL of purified water, and then reacted under condition of saturation of ammonium hydrogen carbonate at 37 degrees Celsius for 3 days (aminated sugar chain solution). To 10 mL of the liposome solution was added 10 mg of DTSSP (PIERCE) as a crosslinking agent, and then stirred at room temperature for 2 hours and further under refrigerated condition overnight. To remove free DTSSP, ultrafiltration (NMWL: 300,000) was then performed. 3.74 μl of the aminated sugar chain solution per 1 mL of the liposome solution (DTSSP-bound) was added, and then reacted at room temperature for 2 hours. 132 mg of Tris was added thereto, and then stirred under refrigerated condition overnight. After free sugar chain and Tris were removed, to exchange the buffer for HEPES buffer solution (pH 7.2), ultrafiltration (NMWL: 300,000) was performed.

(Binding of FITC to the liposome (step D'))

9.5 mg of FITC was dissolved in 5.00 mL of CBS buffer solution (pH 8.5), and then reacted under condition of

saturation of ammonium hydrogen carbonate at 37 degrees Celsius for 1 day (aminated FITC). To 1 mL of liposome solution was added 1 mg of DTSSP (PIERCE) as a crosslinking agent, and then stirred at room temperature for 2 hours and further under refrigerated condition overnight. To remove DTSSP, ultrafiltration (NMWL: 300,000) was performed. Aminated FITC was then added thereto. The amount of aminated FITC added that corresponds to each FITC concentration is shown in the table below. These are the values for 0.5 mL of liposome solution.

[Table 7]

(**Table 6A**)

| No. | FITC **Concentration** (μg/ml) | FITC **Concentration** (μM) | **Liposome amount** (ml) | **Amount of** FITC **added** (μl) | **Amount of** 132mg/ml Tris **added** (μl) |
|---|---|---|---|---|---|
| 0 | 0 | 0 | 0.5 | 0 | 10 |
| ① | 50 | 128 | 0.5 | 13 | 1 |
| ② | 100 | 257 | 0.5 | 26 | 10 |
| ③ | 500 | 1294 | 0.5 | 132 | 10 |
| ④ | 1000 | 2568 | 0.5 | 263 | 10 |

Then, after reacting at room temperature for 2 hours, 10 μl of a Tris/CBS buffer solution (pH 8.5) was added per 0.5 mL of liposome solution, and then stirred under refrigerated condition overnight. After free FITC and Tris were removed, to exchange the buffer for HEPES buffer solution (pH 7.2), ultrafiltration (NMWL: 300,000) was performed.
Then, filtration through a 0.45 μm filter was performed.

(Evaluation of the synthetic GM3 liposome (comparison to the control liposome))

In the present example, the liposome prepared using the synthetic GM3, and the liposome (control liposome) prepared using a ganglioside (an extract from porcine), which had been conventionally used, were compared.

(Measurement of particle size·Z potential)

After the above step A was finished, 20 μl of the liposome solution was mixed with 980 μl of Milli-Q water, and then the particle size distribution and the Z potential were measured using Zetasizer nano (SYSMEX).

(Encapsulating efficiency)

With regard to each liposome solution prepared in the present Example, the absorbance of Cy5.5 (680nm) was measured and compared. Using micro BCA method (BCA was used as a standard substance), the protein (HSA) concentration was measured and compared.

(Binding of sugar chain)

With regard to each liposome solution prepared in the present Example, FITC instead of a sugar chain was bound to the liposome (step D'), and then, by measuring fluorescence, the amount of FITC binding to the liposome was measured and compared (Fig. 13A).
As a fluorophotometer, the Shimadzu RF5300PC was used.
As a standard substance, 1.96 mg/mL aminated FITC was diluted 1600 times with purified water (the final concentration: 1.225 μg/mL, 3.15 μM).
Additionally, this aminated FITC solution was diluted with purified water to prepare a 0/10 solution, 1/10 solution, 2/10 solution, 3/10 solution, 4/10 solution, 5/10 solution, 6/10 solution, 7/10 solution, 8/10 solution, 9/10 solution, and 10/10 solution.
Furthermore, 10 μL of this standard substance was added to 3 mL of purified water, and then transferred into

a four-side-transmission cell. The fluorescence intensity was then measured.

As a result, FITC as shown in the table below was used to make a calibration curve (excitation wavelength: 495nm, fluorescence wavelength: 516nm) (Figs. 13B and 6B).

[Table 8]

(**Table 6 B**)

| Dilution rate (ratio) | conc. (ng/ml) | FITC Conc. (nM) | Intensity |
|---|---|---|---|
| 0 | 0.0 | 0.00 | 0.133 |
| 1 | 122.5 | 314.59 | 3.652667 |
| 2 | 245.0 | 629.19 | 6.194667 |
| 3 | 367.5 | 943.78 | 12.933 |
| 4 | 490.0 | 1258.38 | 19.00267 |
| 5 | 612.5 | 1572.97 | 23.849 |
| 6 | 735.0 | 1887.57 | 26.811 |
| 7 | 857.5 | 2202.16 | 35.98233 |
| 8 | 980.0 | 2516.76 | 41.2855 |
| 9 | 1102.5 | 2831.35 | 50.67767 |
| 10 | 1225.0 | 3145.95 | 59.486 |

The liposome solution reacted with aminated FITC, 0 μM, 128 μM, 257 μM, 1284 μM, or 2568 μM was used as a sample, and thus the amount of FITC binding to the surface was determined.

With regard to aminated FITC 0 μM, 128 μM, and 257 μM, to 3 mL of purified water was added 30 μL of the liposome solution, and transferred into a four-side-transition cell, and then the fluorescence intensity was measured. With regard to 1284 μM and 2568 μM, to 3 mL of purified water was added 10μL of the liposome solution, transferred into a four-side-transmission cell, and then the fluorescence intensity was measured. These are transferred into a four-side-transition cell, and then the fluorescence intensity was measured (excitation wavelength: 495nm, fluorescence wavelength: 516nm).

(Result)

The liposome including the synthetic GM3 exhibited approximately equivalent particle size distribution to that of the liposome using a ganglioside extracted from the porcine brain (Fig. 3).

Comparing the liposome prepared using a naturally-derived ganglioside with the synthetic GM3 liposome, the values of all of the particle size distribution, the Z potential, the lipid amount, and the amount of Cy5.5-labeled HSA encapsulated were approximately equivalent. See Table 7 below.

[Table 9]

(**Table 7**)

| Measurement Result | | | | | |
|---|---|---|---|---|---|
| | Abs680 | Lipid (mg/mL) | HSA mg/mL) | **Mean particle size** (d.nm) | Z **potential** (mV) |
| **Conventional lipo** | 0.835 | 2.60 | 0.58 | 119 | -77.7 |
| **Synthetic GM3 lipo** | 0.845 | 3.22 | 0.53 | 112 | -73.0 |

Conventional lipo: A liposome prepared using a naturally-derived ganglioside

Synthetic GM3 lipo: A liposome prepared using the synthetic GM3

Abs680: Absorbance at 680 nm is indicated.
Lipid: A lipid amount included in a liposome is indicated. HSA: An amount of Cy-5.5 labeled HSA included in a liposome is indicated.

The amount of FITC bound to the synthetic GM3 liposome surface was about 80-90% of the liposome using a ganglioside extracted from porcine (Figs. 14-16, Table 8).

[Table 10]

**(Table 8)**

| FITC Reaction Concentration (μM) | FITC added to lipo (nM) | |
|---|---|---|
| | Conventional lipo | Synthetic GM3 lipo |
| 0 | 0.0030 | 0.0015 |
| 128 | 0.0169 | 0.0113 |
| 257 | 0.0429 | 0.0354 |
| 1284 | No data | 0.3301 |
| 2568 | 0.3089 | 0.2888 |

Conventional lipo: A liposome prepared using a naturally-derived ganglioside

Synthetic GM3 Lipo: A liposome prepared using the synthetic GM3

FITC added to lipo: An amount of FITC added to a liposome FITC reaction concentration: a concentration of FITC reacted with a liposome

Based on the above results, it was confirmed that the liposome prepared using the synthetic GM3 was obtained as a liposome approximately equivalent to the liposome prepared using a ganglioside extracted from porcine.

(Example 10. Simple evaluation of the amount of a sugar chain added onto a liposomal membrane surface)

(FITC binding evaluation)

With regard to the addition of a sugar chain to liposomal membrane surface, a crosslinking agent, such as DTSSP and the like, bound on to the liposome membrane surface is bound with an aminated sugar chain. In the past, to evaluate the amount of a sugar chain bound, a lot of time and a lot of effort have been required, as in a HPLC post-fluorescence labeling method and the like, for example, a sample was pretreated and then HPLC analysis is performed. In the present FITC binding evaluation method, a primary amino group is bound to a fluorescent substance in a molecular ratio of 1:1, a similar reaction to the reaction to bind a sugar chain onto the liposomal membrane surface was performed, and then the amount of the fluorescent substance bound onto the liposomal membrane surface, and consequently the amount of a sugar chain that can be bound can be evaluated.
(Reaction) The addition of sialyl Lewis X (SLX M.W. 820.83) to the liposomal membrane is performed as follows: sialyl Lewis X is added to the liposome solution such that a sugar chain has the concentration 0 μg/mL, 5 μg/mL, 15 μg/mL, 50 μg/mL, 100 μg/mL, 200 μg/mL, 500 μg/mL, or 1200 μg/mL, and then reacted. In this case, a molarity of SLX is 0 μM, 18.2 μM, 60.6 μM, 121.3 μM, 242.7 μM, 606.7 μM, 1213.3 μM, or 1743 μM, respectively.
In the present example, as a fluorescent substance, Fluorescein isothiocyanate isomer-I (FITC) (M.W. 389.38, excitation wavelength: 495nm, fluorescence wavelength: 520nm) was used. The excitation wavelength of FITC is 495nm,

and the fluorescence wavelength is 520nm.

To aminate FITC, 18.8 mg of FITC was dissolved in 10 mL of purified water, 5g of ammonium hydrogen carbonate was added, reacted at 37 degrees Celsius for 2 hours, and then cooled on ice, followed by retrieving the solution. To 2 mL of a solution (lipid amount: 4 mg/mL) of Cy5.5-encapsulating liposome of which the surface was bound with DTSSP was added the FITC solution such that the final molarity thereof is 0 μM, 18.2 μM, 60.6 μM, 121.3 μM, 242.7 μM, 606.7 μM, 1213.3 μM, or 1747 μM, and then reacted for 2 hours at room temperature. 40 μL of a 132 mg Tris/CBS buffer solution was added respectively, and then reacted at 2-8 degrees Celsius for 16 hours. By ultrafiltration using an ultrafilter membrane (molecular cutoff: 300K), free FITC and Tris were removed.

(Determination)

As a fluorophotometer, the Shimadzu RF5300PC was used.

As a standard substance, 1.88 mg/mL aminated FITC was diluted 1600 times with purified water (the final concentration: 1.18 μg/mL, 4.5 μM).

Additionally, this aminated FITC solution was diluted with purified water to prepare a 0/10 solution, 1/10 solution, 2/10 solution, 3/10 solution, 4/10 solution, 5/10 solution, 6/10 solution, 7/10 solution, 8/10 solution, 9/10 solution, and 10/10 solution.

Furthermore, 10 μL of this standard substance was added to 3 mL of purified water, and then transferred into a four-side-transmission cell. The fluorescence intensity was then measured.

As a result, FITCs of 10.13 pM, 9.12 pM, 8.11 pM, 7.09 pM, 6.08 pM, 5.07 pM, 4.05 pM, 3.04 pM, 2.03 pM, 1.01 pM, 0 pM were used to make a calibration curve (excitation wavelength: 495nm, fluorescence wavelength: 516nm) (Table 9).

[Table 11]

(Table 9)

| FITC concentration (pM) | Intensity |
|---|---|
| 10.13 | 117.682 |
| 9.12 | 112.375 |
| 8.11 | 91.322 |
| 7.09 | 76.396 |
| 6.08 | 58.113 |
| 5.07 | 43.55 |
| 4.05 | 33.427 |
| 3.04 | 23.722 |
| 2.03 | 13.409 |
| 1.01 | 5.773 |
| 0.00 | 0.11 |

The liposome solution (the molarity thereof is the same as the molarity in the reaction of the sugar chain) reacted with aminated FITC 0μM, 18.2μM, 60.6μM, 121.3μM, 242.7μM, 606.7μM, 1213.3μM, or 1747μM was used as a sample to determine the amount of FITC binding to the surface.

With regard to 0 μM, 18.2 μM, 60.6 μM, and 121.3 μM of aminated FITC, 30 μL of each was added to 3 mL of purified water. With regard to 242.7 μM and 606.7 μM of aminated FITC, 10μL of each was added to 3 mL of purified water. With regard to 1213.3μM and 1743μMof aminated FITC, 5μL of each was added to 3 mL of purified water. These were each transferred into a four-side-transmission cell to measure the fluorescence intensity (excitation wavelength: 495nm, fluorescence wavelength: 516nm).

(Result)

Aminated FITC was added onto the liposomal membrane surface in a FITC concentration corresponding to SLX concentration in the reaction to give the results as shown in Table 10 below. It was understood that, by measuring the amount of aminated FITC bound onto the liposomal membrane surface, the amount of a sugar chain bound can be evaluated.

[Table 12]

(**Table 10**)

| SLX concentration in the reaction (ug/ml) | FITC added to liposome (nM) |
|---|---|
| 0 | 0.02 |
| 5 | 0.21 |
| 15 | 0.29 |
| 50 | 0.40 |
| 100 | 0.76 |
| 200 | 1.24 |
| 500 | 2.02 |
| 1200 | 2.56 |

(Example 11. Liposome prepared using a glycolipid other than GM3 and GM4)

(1. Preparation of liposome using synthetic glycolipid)

In the present example, using a similar method to Examples 1-3, gangliosides (GM1, GM2, GD3, GD2, GD1a, GD1b, GT3, GT2, GT1a, GT1b, GT1c, GQ1b, GQ1c, and GP1c) are prepared.
By a similar method to Example 4, a liposome was prepared using an improved cholate dialysis method.
Lipids composing the liposome were mixed.
Then, to these mixed lipids composing the liposome is added sodium cholate, and then dissolved in chloroform/methanol (1:1) solution. This solution is evaporated, and then the precipitate is dried *in vacuo* to yield lipid membrane. The resulting lipid membrane is resuspended in N-tris(hydroxymethyl)-3-aminopropane sulfonate buffer solution, and then stirred. Then, for this solution, displacement with nitrogen is carried out, and then sonicated to yield a clear micelle suspension. Moreover, the micelle suspension is ultrafiltered to prepare a homogenous liposome.
10 mL of this liposome solution is ultrafiltered to adjust the pH of the solution. Then, a crosslinking agent is added, and then stirred. This liposome solution is then ultrafiltered. Tris (hydroxymethyl) aminomethane dissolved in a buffer solution is then added to the liposome solution. This solution is then stirred, followed by ultrafiltration.

(Binding of human serum albumin (HSA) onto the liposomal membrane surface)

According to a similar method to Example 4, to form the binding of human serum albumin (HSA) onto the liposomal membrane surface, in apublishedmethod (Yamazaki, N., Kodama, M. and Gabius, H.-J. (1994) Methods Enzymol. 242, 56-65), a coupling reaction method is used.

(Determination of lipid·protein)

Using a similar method to Example 4, the total cholesterol amount is measured, and then, by calculating the total lipid amount from a molar ratio of each lipid, the lipid amount of the liposome can be determined.
The protein amount can be measured in the presence of 1% SDS using the Micro BCA™ Protein Assay Kit (PIERCE).

(Measurement of particle size·zeta potential)

With regard to a particle size and zeta potential, after a liposome solution is diluted with purified water, they can be measured by using the Zetasizer Nano-S90 (SYSMEX) at 25 degrees Celsius.

(Preparation of Cy5.5-encapsulating liposome using a synthetic ganglioside)

In the present example, by a similar method to Example 5, a micelle suspension is prepared.

Meanwhile, human serum albumin (HSA) is dissolved in a buffer solution, Cy5.5-NHS ester (GE Healthcare Bioscience) is mixed therewith, and then reacted. By ultrafiltration, free Cy5.5-NHS ester is removed to yield a Cy5.5-bound HSA solution.

The above micelle suspension was mixed with the Cy5.5-bound HSA solution, and then ultrafiltration yields a solution of Cy5.5-encapsulatingliposome (Liposome-Cy5.5). Hereinafter, a similar treatment to Example 5 is performed.

(Determination of lipid·protein)

Using a similar method to Example 5, the total cholesterol amount is measured, and then, by calculating the total lipid amount from a molar ratio of each lipid, the lipid amount of the liposome can be determined.

The protein amount can be measured in the presence of 1% SDS using the Micro BCA™ Protein Assay Kit (PIERCE).

(Measurement of particle size·zeta potential)

With regard to a particle size and zeta potential, after a liposome solution is diluted with purified water, they can be measured by using the Zetasizer Nano-S90 (SYSMEX) at 25 degrees Celsius.

(2. Preparation of a sugar-chain-modified liposome using a synthetic glycolipid)

(Addition of a sugar-chain to liposome)

A synthetic glycolipid liposome solution prepared in the present Example is used.

To 10 mL of each liposome solution (liposome intermediate HSA) is added 10 mg of 3,3-dithiobis(sulfosuccinimidylpropionate) (DTSSP; PIERCE) as a crosslinking agent. After stirring, free DTSSP is removed by ultrafiltration.

To sialyl Lewis X (SLX: Calbiochem) dissolved in purified water is added ammonium hydrogen carbonate, and then stirred to yield an aminated SLX solution. To the above liposome solution, to which DTSSP has been added, is added aminated SLX, and then reacted. Subsequently, a Tris solution is added, and then stirred. By ultrafiltration, free SLX and Tris are removed.

(Evaluation of the binding of the recognition probe to the liposome surface)

(Evaluation of FITC binding)

To the liposome solution (liposome intermediate HSA) is added DTSSP (PIERCE) as a crosslinking agent. After stirring, free DTSSP is removed by ultrafiltration.

To FITC dissolved in purified water is added ammonium hydrogen carbonate, and then stirred to yield an aminated FITC solution. To the above liposome solution, to which DTSSP has been added, is added the aminated FITC. After reacting, Tris solution is added, and then stirred. Byultrafiltration, free FITC and Tris are removed to yield a FITC-bound liposome.

The amount of FITC bound to liposome can be determined by measuring the amount of fluorescence (excitation wavelength: 495nm, fluorescence wavelength: 520nm).

(Evaluation of an amount of antibody bound)

To the liposome solution (liposome intermediate HSA) is added DTSSP (PIERCE) as a crosslinking agent. After stirring, free DTSSP is removed by ultrafiltration.

To this liposome solution is added an antibody (for example, anti-E-selectin monoclonal antibody), followed by reacting. Subsequently, a Tris solution is added, and then stirred. By ultrafiltration, free antibody and Tris are removed to yield an antibody-bound liposome.

The amount of an antibody bound to the liposome surface can be measured by an Enzyme immuno assay (EIA) method. 50 μl of a PBS solution of a protein (for example, E-selectin (R&D Systems)), which is an antigen, is added to each well of a 96-well microplate, and then solid-phased. After an antigen solution in the plate is discarded, 300 μl of 2% BSA/PBS is added to each well, and then left to stand. The BSA solution is discarded, and then washed 3 times with PBS. 100 μl of an antibody standard solution and 100μl of an antibody-bound liposome sample are added, followed by standing for 1 hour. The solution in the wells is discarded, and then washed with PBS. HRP-labeled anti-mice IgG antibody is diluted with 10% Tween 20/9% EDTA/PBS, 100μl of this solution is added to each well, subsequently standing for 1 hour. The solution in the wells is discarded, and then washed with PBS. 1-Step™ TMB-Blotting (PIERCE) is added, and reacted, and then 100 μl of a quenching solution (2M sulfuric acid) is added to each well. The absorbance at 450nm is measured, and thereby the amount of an antibody bound to the liposome surface can be determined on the basis of the antibody standard solution.

(Determination of lipid·protein)

With regard to the lipid amount of the liposome obtained, the total cholesterol amount is measured in the presence of 0.5% Triton X-100 using the Determiner TC555 (Kyowa Medex), and then the total lipid amount can be calculated from the molar ratio of each lipid.
The protein amount can be measured in the presence of 1% SDS using the Micro BCA™ Protein Assay Kit (PIERCE).

(Measurement of particle size·zeta potential)

With regard to a particle size and zeta potential, after the liposome solution is diluted 50 times with purified water, they can be measured using the Zetasizer Nano-S90 (SYSMEX) at 25 degrees Celsius.

(3. Preparation of Cy5.5-encapsulating sugar-chain-modified liposome using a synthetic glycolipid)

(Addition of a sugar chain to the liposome)

A solution of Cy5.5-encapsulatingliposome prepared in the present Example is used.
By a similar method to Example 7, a target site recognition probe was added to a Cy5.5-encapsulating liposome.

(Evaluation of the recognition probe bound to the liposome surface)

(Evaluation of FITC binding)

By a similar method to Example 7, the amount of FITC bound to the liposome can be determined.

(Evaluation of the amount of bound antibody)

By a similar method to Example 7, the amount of antibody bound to the liposome can be determined.

(Determination of lipid·protein)

By a similar method to Example 7, the lipid amount and the protein amount of the liposome can be determined.

(Measurement of particle size·zeta potential)

By a similar method to Example 7, the particle size and the zeta potential of the liposome can be determined.

(Evaluation using a tumor-bearing mouse)

Using a tumor-bearing mouse, accumulation property to a tumor site is checked by a similar method to Example 7.

(Example 12. The relationship between accumulation property and a ganglioside (GM3) in a sugar-chain-modified liposome)

In the present example, with regard to liposomes including a variety of gangliosides (GM3), accumulation property to a born tumor site was compared and evaluated.

(Preparation of a tumor-bearing mouse)

Ehrlich ascites tumor cells (EAT cells, $5\times10^6$) were transplanted subcutaneously into the right femoral region of Balb/cmice (12 mice) (female, 6-weeks old). 10 days after the transplantation, the mice were used in following experiments.

(Preparation of liposome)

A SLX-modified liposome (K1) encapsulating Cy5.5-labeled human serum albumin was prepared by a similar method to that in Example 7A using the total ganglioside (porcine-derived one, TGANG 14, and AVANTI), synthetic GM3 plant ceramide, synthetic GM3 pseudo-ceramide, and synthetic GM3 natural ceramide, as gangliosides, in the lipid composition component of the liposome. 50 μg/mL of density of a sugar chain was used.

With regard to each liposome, by a similar method to Example 7, physical properties (lipid amount, particle size and z potential) were checked. With regard to the amount of lipid included in each liposome, the total cholesterol amount is measured in the presence of 0.5% Triton X-100 using Determiner TC555 (Kyowa Medex), and then the total lipid amount was calculated from the molar ratio of each lipid. The amount of protein included in each liposome was calculated using the Micro BCA™ Protein Assay Kit (PIERCE) in the presence of 1% SDS. With regard to a particle size and zeta potential, after the liposome solution was diluted 50 times with purified water, they were measured by using the Zetasizer Nano-S90 (SYSMEX) at 25 degrees Celsius.

As a result, the liposome prepared by the present Example exhibited physical properties (lipid amount, particle size, and z potential) equivalent to those shown in Table 5.

(Evaluation of the administration of liposome to a tumor-bearing mouse and the accumulation property)

300 μl of 1/10 Nembutal solution was intraperitoneally administered to each tumor-bearing mouse to anesthetize it. The Cy5.5-encapsulating sugar-chain-modified liposome (K1) was then transplanted from the caudal vein. 10 days after the transplantation, 100 μl of the liposome (corresponding to 375μg of lipid amount) was administered. After 48 hours, by the fluorescence imaging system explore Optix (GE Healthcare), image data was taken.

(Result)

As shown in Figs. 17A and 17B, the sugar-chain-modified liposome prepared using the plant type GM3 exhibited the highest accumulation property to a born tumor site. In the natural type GM3 shown in (A), on the contrary to the original prediction, accumulation of a born tumor site was low. Furthermore, it was confirmed the sugar-chain-modified liposome prepared using the plant type GM3 exhibited about 1.4 times higher accumulation to a born tumor site than the sugar-chain-modified liposome prepared using the total ganglioside. Based on that, it was found that, when a liposome targeting a born tumor site is prepared, the plant type GM3 is most useful among synthetic gangliosides and the total ganglioside evaluated.

(Example 13. Relationship between the ganglioside (GM4) in a sugar-chain-modified liposome and the accumulation property)

(Preparation of a tumor-bearing mouse)

Ehrlich ascites tumor cells (EAT cells, $5\times10^6$) are transplanted subcutaneously into the right femoral region of Balb/cmice (12 mice) (female, 6-weeks old). 10 days after the transplantation, the mice are used in following experiments.

(Preparation of liposome)

A SLX-modified liposome (K1) encapsulating Cy5.5-labeled human serum albumin was prepared by a similar method to that in Example 7A using the total ganglioside (porcine-derived one, TGANG 14, and AVANTI), synthetic

GM4 plant ceramide, synthetic GM4 pseudo-ceramide, synthetic GM4 natural ceramide, as gangliosides, in the lipid composition component of the liposome. 50 μg/mL of density of a sugar chain was used.

With regard to a liposome prepared by the present Example, by a similar method to Example 7, physical properties thereof (lipid amount, particle size, and z potential) can be checked. With regard to the amount of lipid included in the liposome, the total cholesterol amount is measured in the presence of 0.5% Titon X-100 using Determiner TC555 (Kyowa Medex), and then the total lipid amount can be calculated from the molar ratio of each lipid. The protein amount can be calculated using the Micro BCA™ Protein Assay Kit (PIERCE) in the presence of 1% SDS. With regard to a particle size and zeta potential, after the liposome solution is diluted 50 times with purified water, they can be measured by using the Zetasizer Nano-S90 (SYSMEX) at 25 degrees Celsius.

(Evaluation of the administration of liposome to a tumor-bearing mouse and the accumulation property)

300 μl of 1/10 Nembutal solution is intraperitoneally administered to each tumor-bearing mouse to anesthetize it. 100 μl of the cy5.5-encapsulating sugar-chain-modified liposome (K1) (corresponding to 375μg of lipid amount) is administered. After 48 hours, by taking image data using the fluorescence imaging system eXplore Optix (GE Healthcare), with regard to each liposome, accumulation property to tumor can be evaluated.

(Example 14. Relationship between gangliosides (GM3 and 4) and the accumulation property in the antibody-modified liposomes)

(Preparation of a tumor-bearing mouse)

Ehrlich ascites tumor cells (EAT cells, $5\times10^6$) were transplanted subcutaneously into the right femoral region of Balb/cmice (21 mice) (female, 6-weeks old). 10 days after the transplantation, the mice were used in following experiments.

(Preparation of liposome)

A anti-E-selectin antibody-modified liposome encapsulating Cy5.5-labeled human serum albumin was prepared by a similar method to that in Example 7B using the total ganglioside (porcine-derived one, TGANG 14, and AVANTI), synthetic GM3 plant ceramide, synthetic GM3 pseudo-ceramide, synthetic GM3 natural ceramide, synthetic GM4 plant ceramide, synthetic GM4 pseudo-ceramide, and synthetic GM4 natural ceramide, as gangliosides, in the lipid composition component of the liposome. An amount of an antibody used is 50-500 μg/mL.

With regard to a liposome prepared by the present Example, by a similar method to Example 7, physical properties thereof (lipid amount, particle size, z potential, and the like) can be checked. With regard to the amount of lipid included in the liposome, the total cholesterol amount is measured in the presence of 0.5% Triton X-100 using Determiner TC555 (Kyowa Medex), and then the total lipid amount can be calculated from the molar ratio of each lipid. The protein amount can be calculated using the Micro BCA™ Protein Assay Kit (PIERCE) in the presence of 1% SDS. With regard to a particle size and zeta potential, after the liposome solution is diluted 50 times with purified water, they can be measured by using the Zetasizer Nano-S90 (SYSMEX) at 25 degrees Celsius.

(Evaluation of the administration of liposome to a tumor-bearing mouse and the accumulation property)

300 μl of 1/10 Nembutal solution is intraperitoneally administered to each tumor-bearing mouse to anesthetize it. 100 μl of the cy5. 5-encapsulating antibody-modifiedliposome (corresponding to 375μg of lipid amount) is administered from the caudal vein. After 48 hours, by taking image data using the fluorescence imaging system eXplore Optix (GE Healthcare), with regard to each liposome, accumulation property to tumor can be evaluated.

The present invention has been exemplified so far with reference to preferable embodiments of the present invention, but it should not be construed that the present invention is restricted by the embodiments of the present invention. It should be understood that the scope of the present invention should be construed only by the claims. It would be understood that those skilled in the art can perform an invention practically equivalent to the present invention, based on the description of the present specification and technical common sense from the description of typical preferable embodiments of the present invention. It would be understood that the patents, patent applications and literatures cited herein are incorporated herein by reference to the present specification, similarly to the case where the description is described specifically herein.

## Claims

1. A glycolipid-containing liposome, wherein the glycolipid comprises a plant ceramide portion and sugar chain portion.

2. The liposome according to claim 1, wherein the plant ceramide portion is

[Chem. 1]

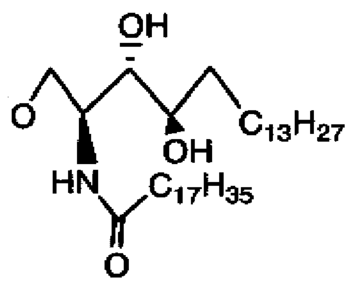

3. The liposome according to claim 1, wherein the sugar chain portion is

[Chem. 2]

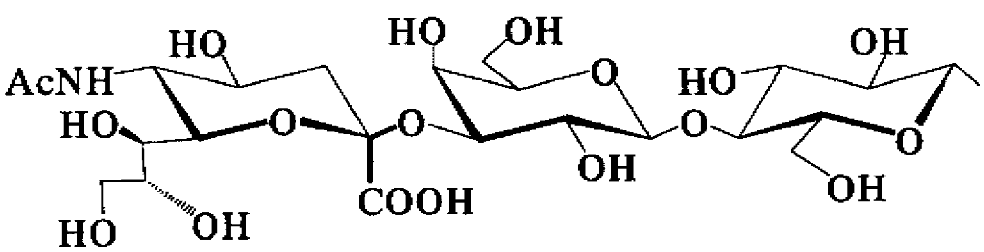

.

4. The glycolipid-containingliposome according to claim 1, wherein the glycolipid is

[Chem. 3]

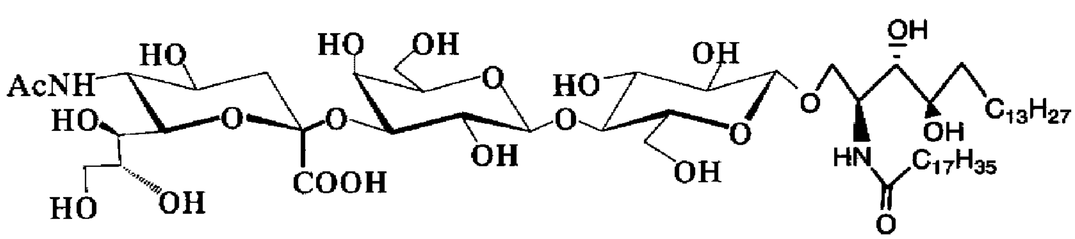

.

5. The liposome according to claim 1, wherein the glycolipid has

[Chem. 4]

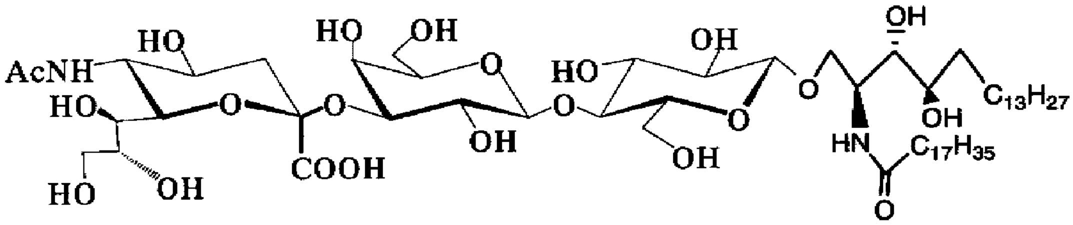

, and the glycolipid-containingliposome contains, aslipids for forming the liposome, dipalmitoylphosphatidylcholine (DPPC), cholesterol, dicetylphosphate(DCP), the glycolipid, and dipalmitoylphosphatidylethanolamine(DPPE) at a molar ratio of 35:40:5:15:5.

**6.** The liposome according to claim 1, wherein the liposome has absorbance of 0.5 to 3.0 at 680nm.

**7.** The liposome according to claim 1, wherein the liposome has a lipid amount of 0.5 to 5 mg/mL.

**8.** The liposome according to claim 1, wherein the liposome contains human serum albumin (HSA), an amount of which is 0.1 to 1 mg/mL.

**9.** The liposome according to claim 1, wherein the liposome has a mean particle size of 50 to 300 nm.

**10.** The liposome according to claim 1, wherein the liposome has a Z potential of -30mV to -120mV.

**11.** The liposome according to the claim 1, wherein the liposome encapsulates a desired substance.

**12.** The liposome according to claim 11, wherein the desired substance is selected from the group consisting of cy5.5, cy5, cy7, cy3B, cy3.5, Alexa Fluor350, Alexa Fluor488, Alexa Fluor532, Alexa Fluor546, Alexa Fluor555, Alexa Fluor568, Alexa Fluor594, Alexa Fluor633, Alexa Fluor647, Alexa Fluor680, Alexa Fluor700, Alexa Fluor750, fluorescein-4-isothiocyanate (FITC), europium-containing label, GFP, CFP, YFP, luciferases, antibody, tPA, β-galactosidase, albumin, botulinus toxin, diphtherotoxin, methylprednisolone, prednisolone phosphate, peptide, gold colloid, Gd complex, Fe complex, cisplatin, pravastatin, heparin, fasudil hydrochloride, clodronic acid, water-soluble iodine, chitin, chitosan, plasmid DNA and RNAi.

**13.** The liposome according to claim 1, wherein the liposome comprises a target-recognizing probe on a surface thereof.

**14.** The liposome according to claim 13, wherein the target-recognizing probe is selected from the group consisting of sugar chain, antibody, antigen, peptide, nucleic acid and hyaluronic acid.

**15.** The liposome according to claim 14, in which an amount of the sugar chain added is such that a bond density of sugar chain is 0.5 to 500μg/mL.

**16.** The liposome according to claim 14, in which an amount of the antibody added is 0.1 to 50μg/mL.

**17.** The liposome according to claim 1, wherein the glycolipid does not contain a component accompanying a naturally-derived glycolipid.

**18.** A method of producing a glycolipid-containing liposome, comprising the following steps of:

A) providing a glycolipid, wherein the glycolipid comprising a plant ceramide portion and a sugar chain portion; and

B) mixing the provided glycolipid with a liposome raw material and subjecting the mixture to conditions in which a liposome is formed.

**19.** The method according to claim 18, wherein the step A) includes the following steps of:

(a) reacting a protected sugar with a protected lipid amide under conditions in which the protected sugar binds with the protected lipid amide, so as to produce a sugar-lipid amide acceptor precursor;
(b) allowing the sugar-lipid amide acceptor precursor to react under conditions in which an intramolecular condensation reaction in the sugar-lipid amide acceptor precursor proceeds, so as to produce a sugar-lipid amide acceptor;
(c) reacting the sugar-lipid amide acceptor with a protected sugar chain donor under conditions in which the sugar-lipid amide acceptor binds with the protected sugar chain donor, so as to produce a protected glycolipid; and
(d) performing a deprotection reaction of the protected glycolipid under conditions in which the protected sugar chain donor is deprotected, so as to produce a glycolipid, wherein the protected lipid amide is

[Chem. 5]

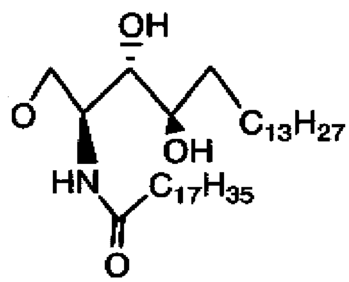

.

Fig.1

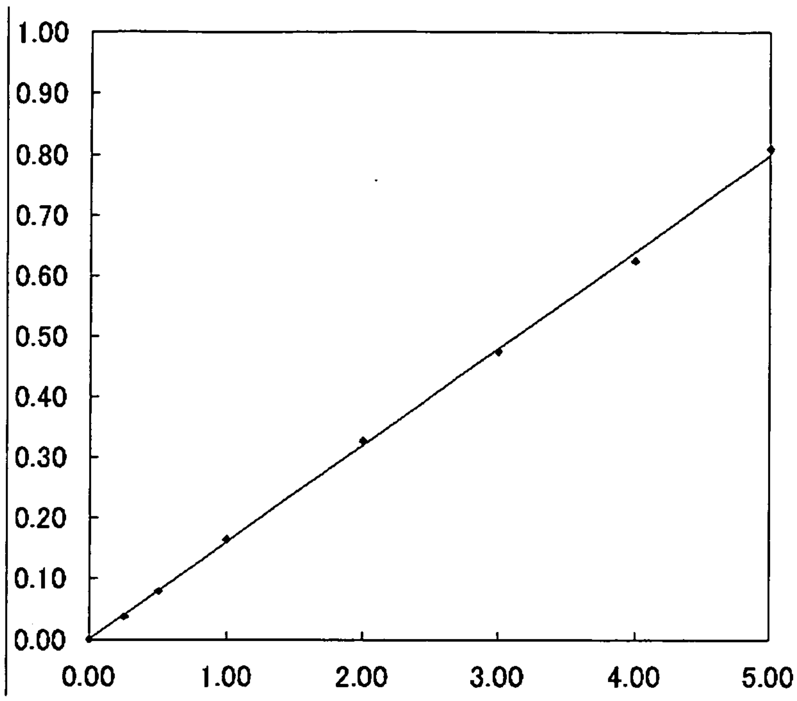
1.00
0.90
0.80
0.70
0.60
0.50
0.40
0.30
0.20
0.10
0.00
0.00
1.00
2.00
3.00
4.00
5.00

**Fig.2**

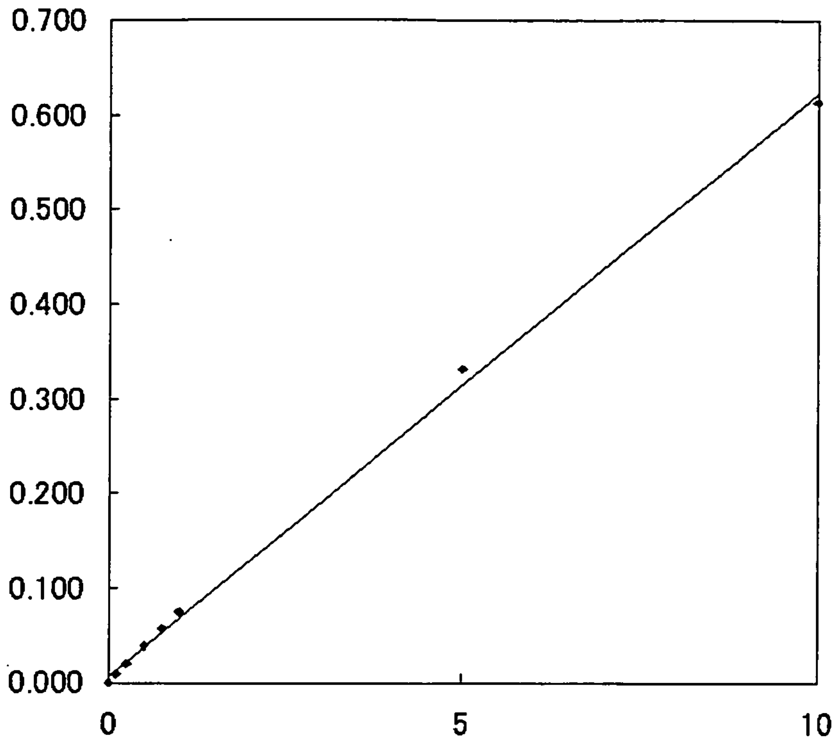

Fig.3

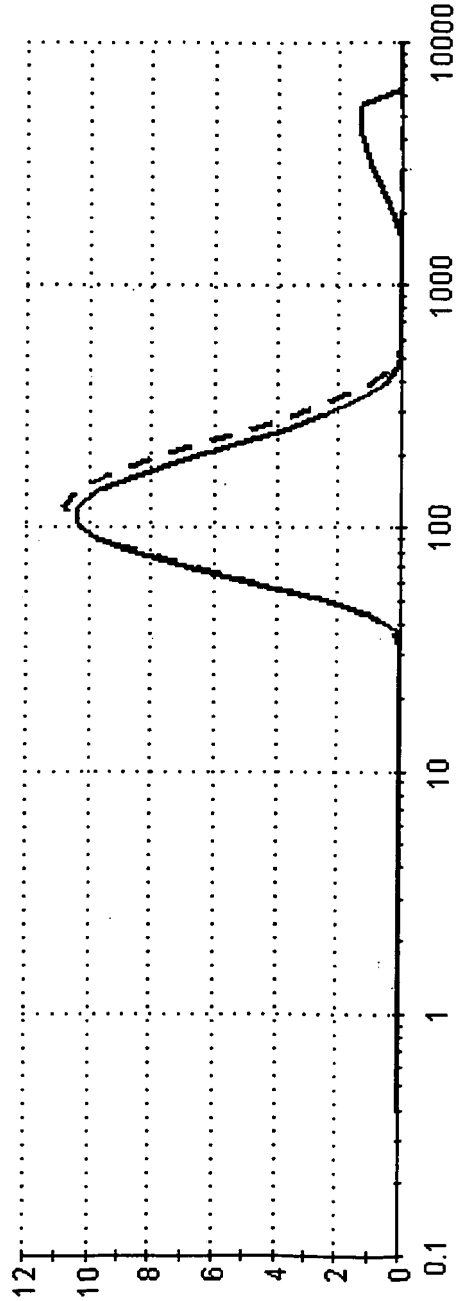

Fig.4

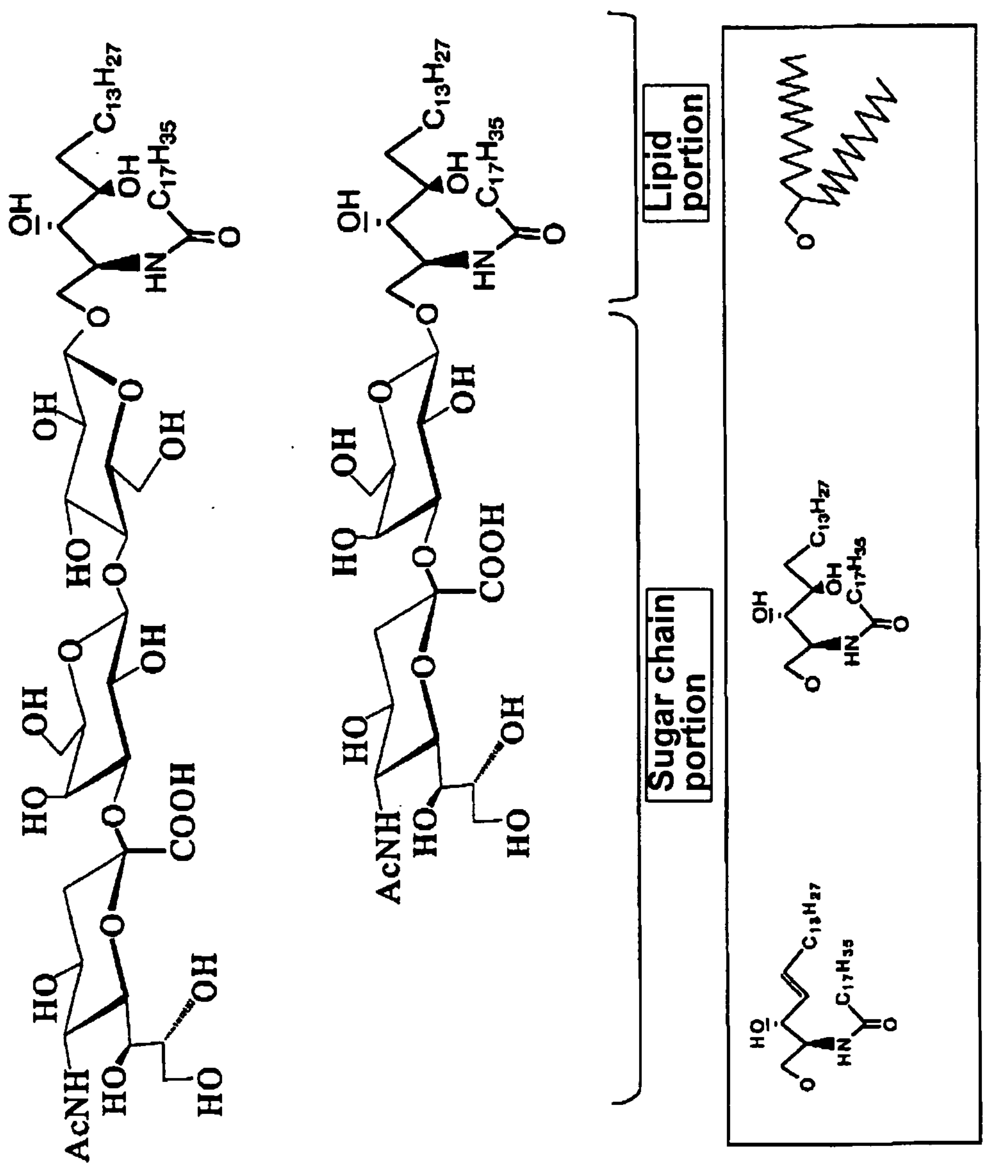

Fig.5a

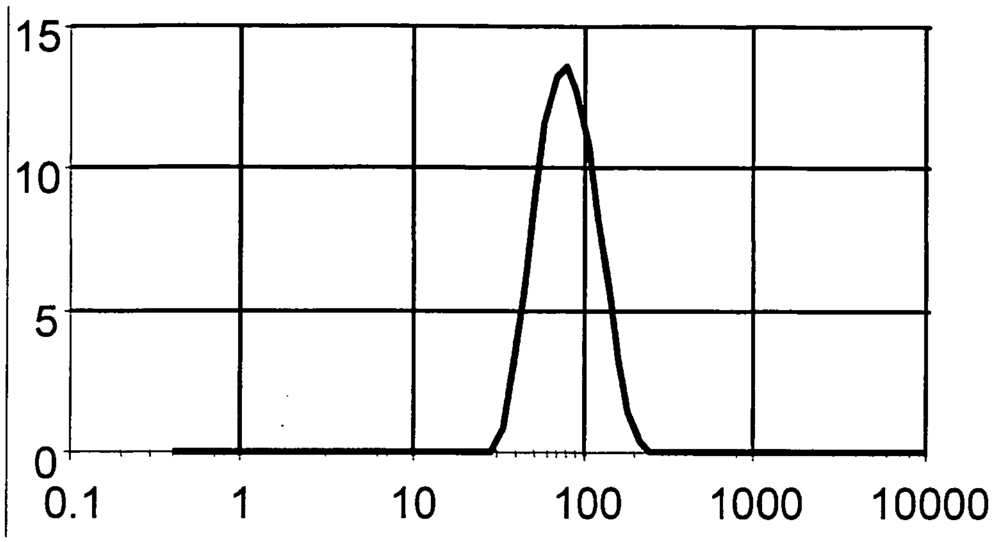


Fig.5b

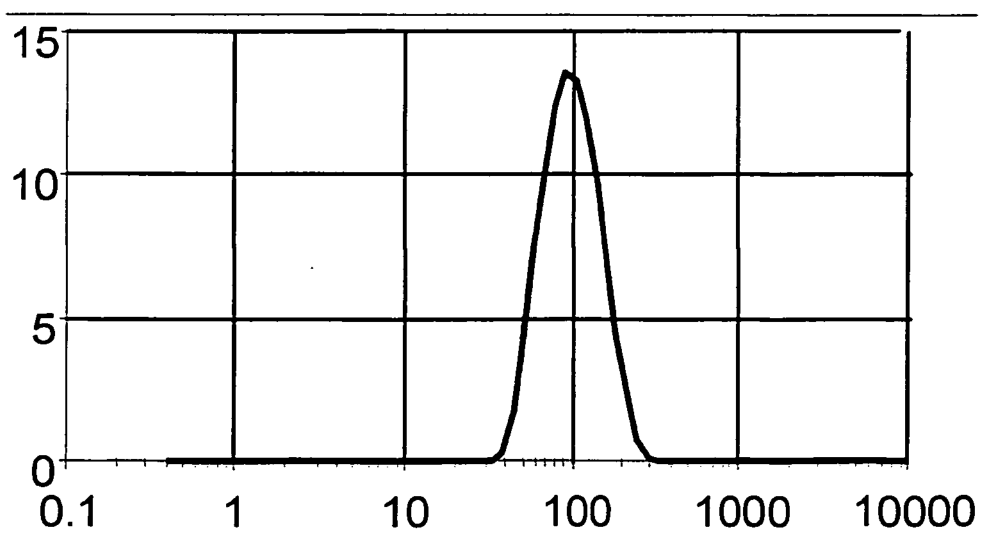

Fig.5c

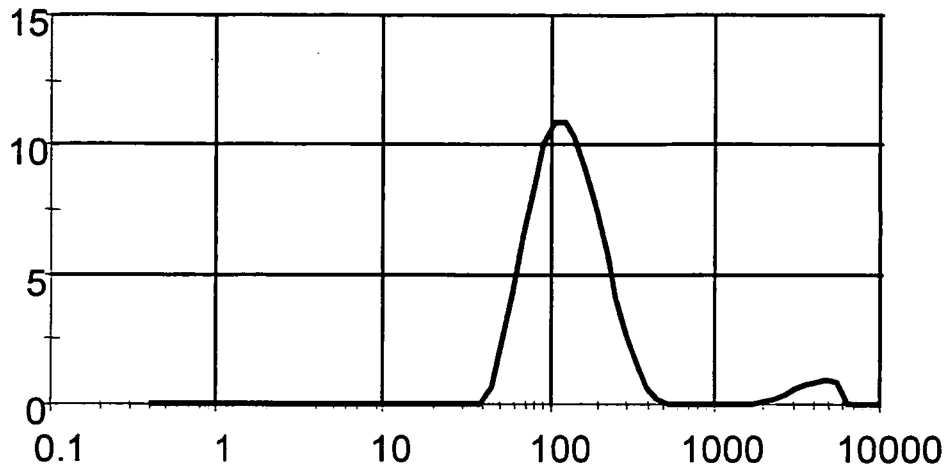


Fig.5d

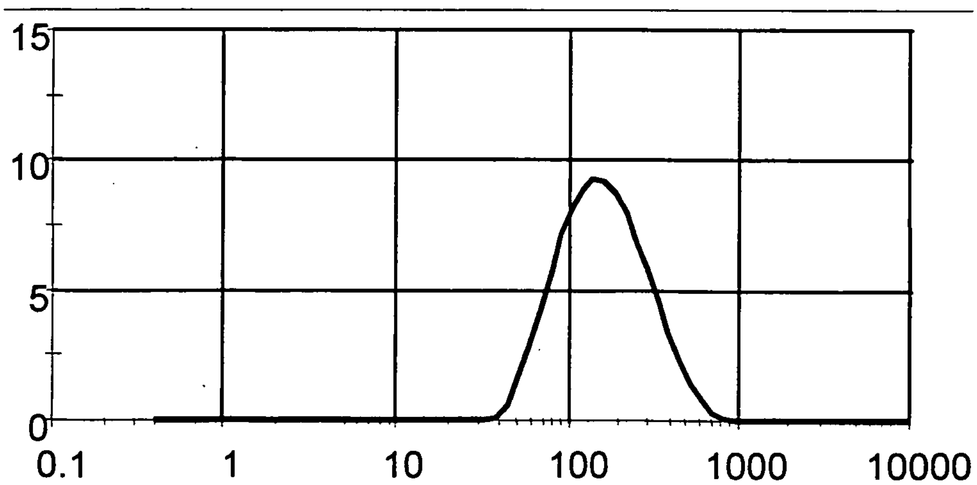

Fig.6a

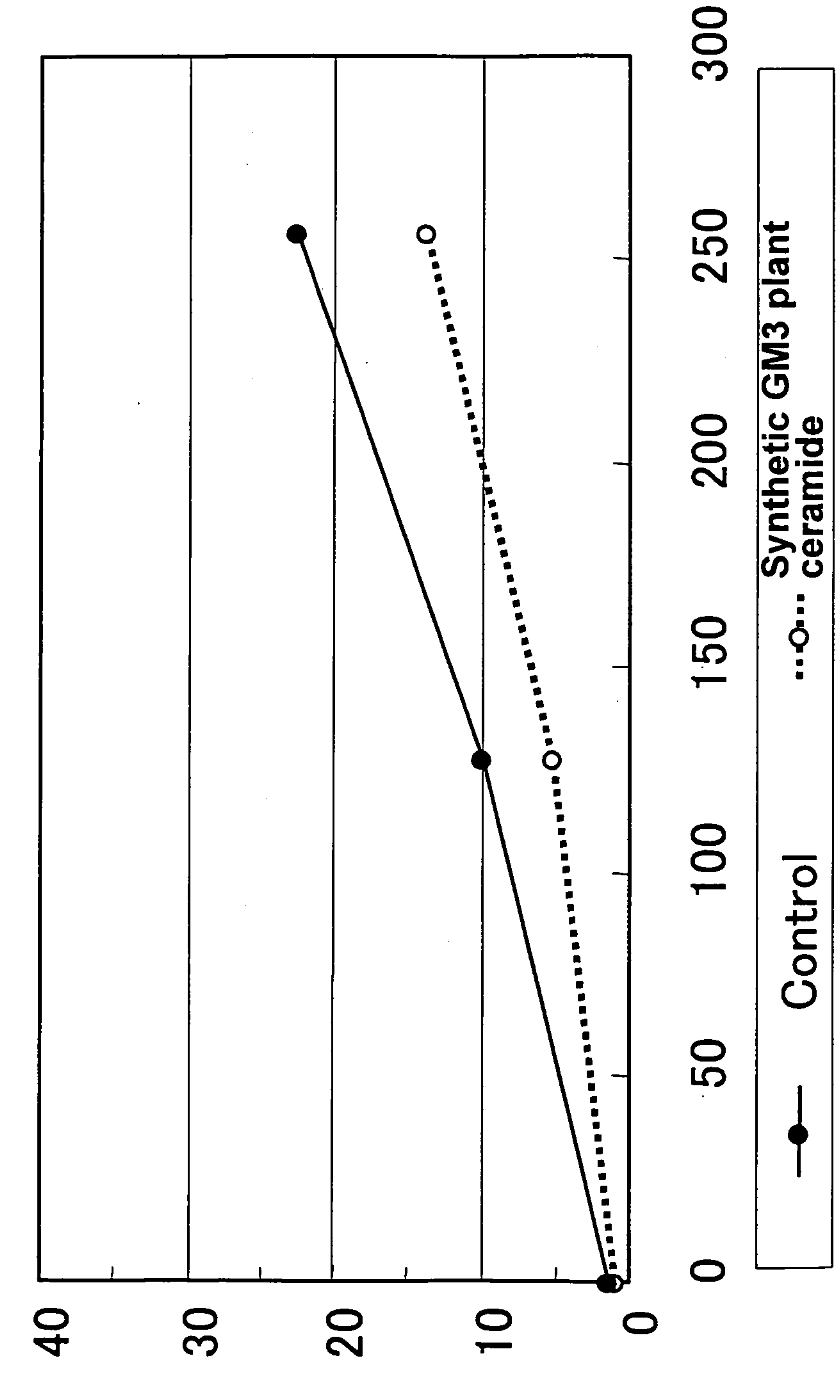

0
10
20
30
40
0
50
100
150
200
250
300
Control
Synthetic GM3 plant ceramide

Fig.6b

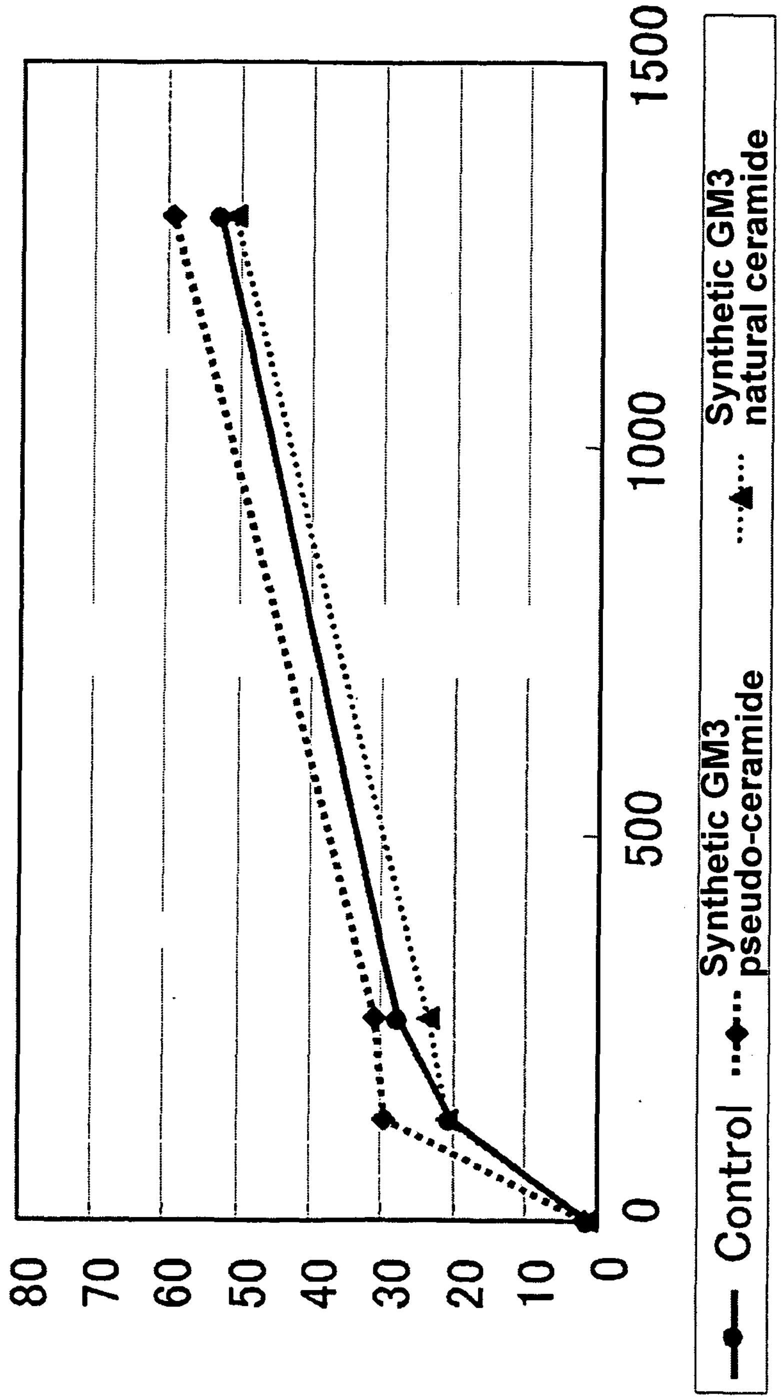
80
70
60
50
40
30
20
10
0
0
500
1000
1500
Control
Synthetic GM3 pseudo-ceramide
Synthetic GM3 natural ceramide

Fig.6c

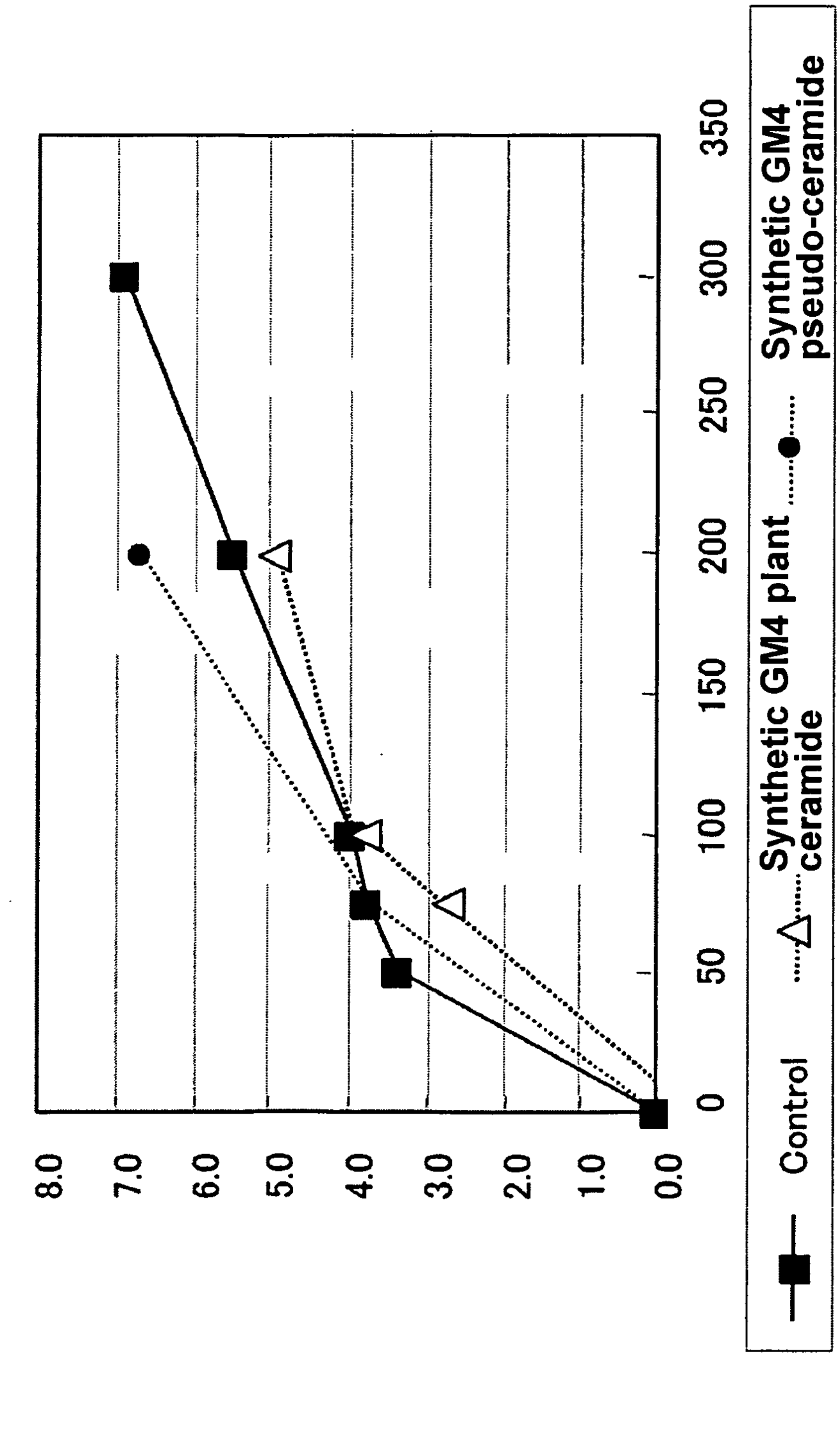
8.0
7.0
6.0
5.0
4.0
3.0
2.0
1.0
0.0
0
50
100
150
200
250
300
350
Control
Synthetic GM4 plant ceramide
Synthetic GM4 pseudo-ceramide

# Fig.7A

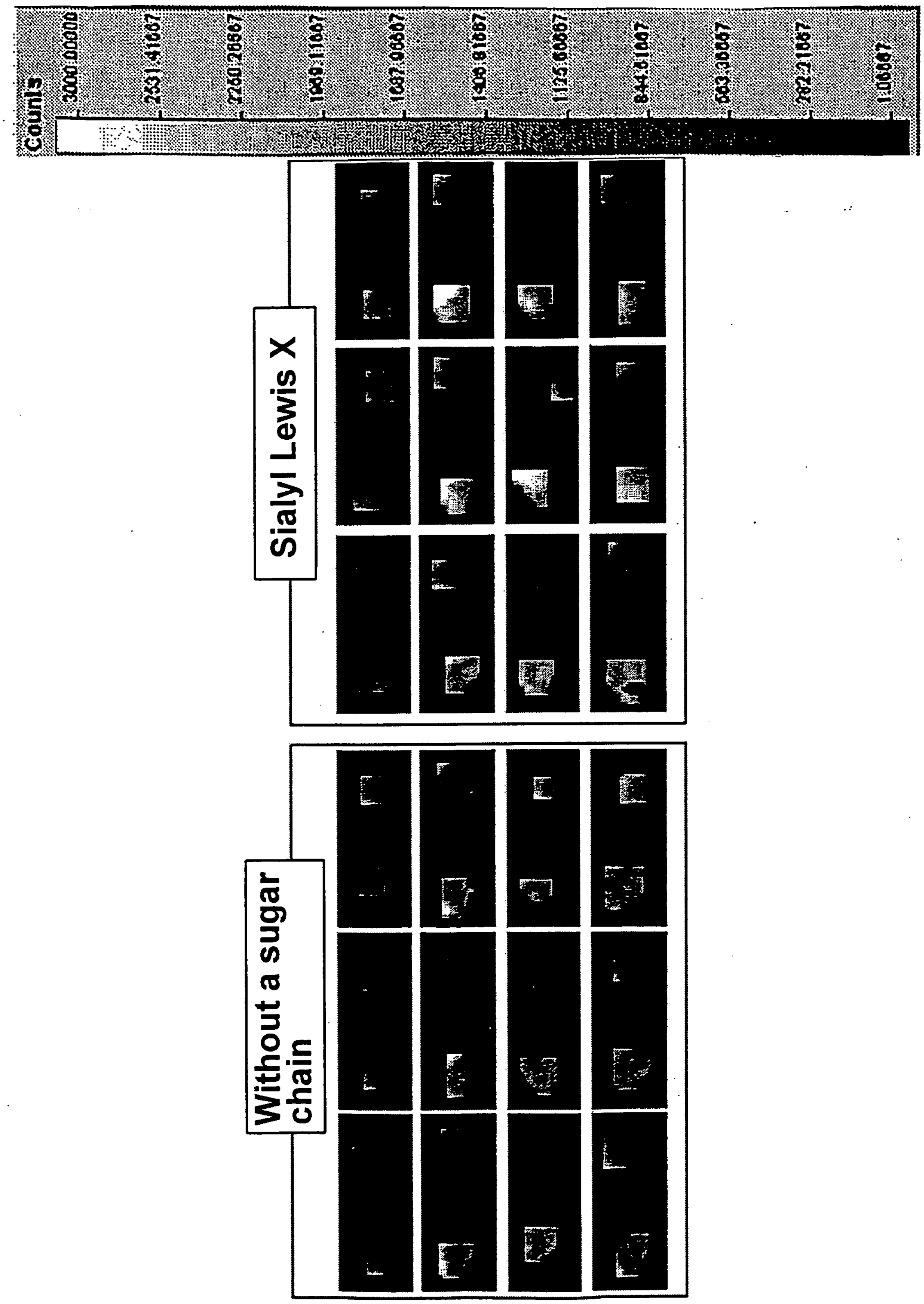
Counts
3000.00000
2531.41667
2250.26667
1969.11667
1687.96667
1406.81667
1125.66667
844.51667
563.36667
282.21667
1.06667
Sialyl Lewis X
Without a sugar chain

# Fig.7B

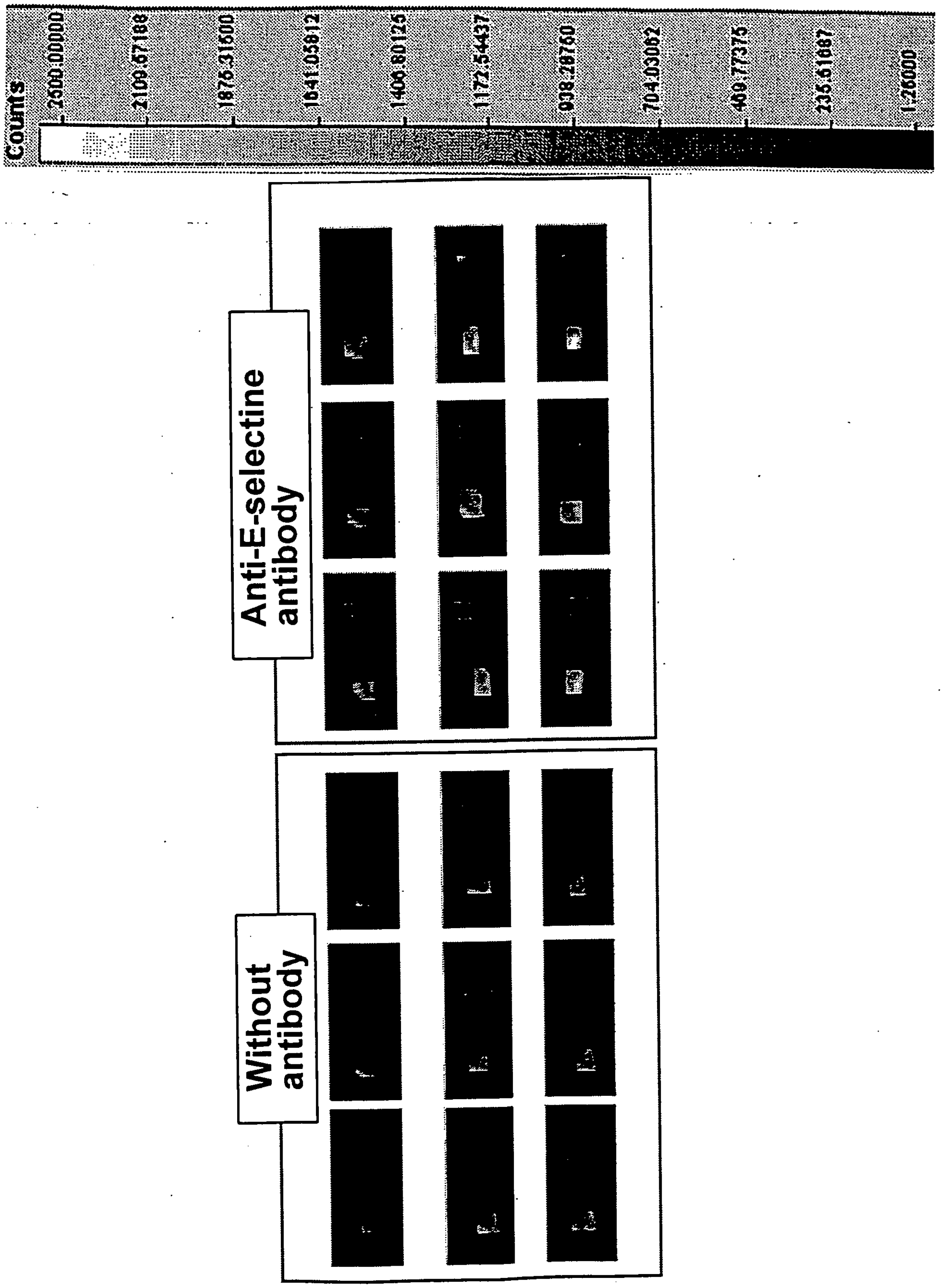
Counts
2500.00000
2109.57188
1875.31500
1641.05812
1406.80125
1172.54437
938.28750
704.03062
469.77375
235.51687
1.26000
Anti-E-selectine antibody
Without antibody

# Fig.7C

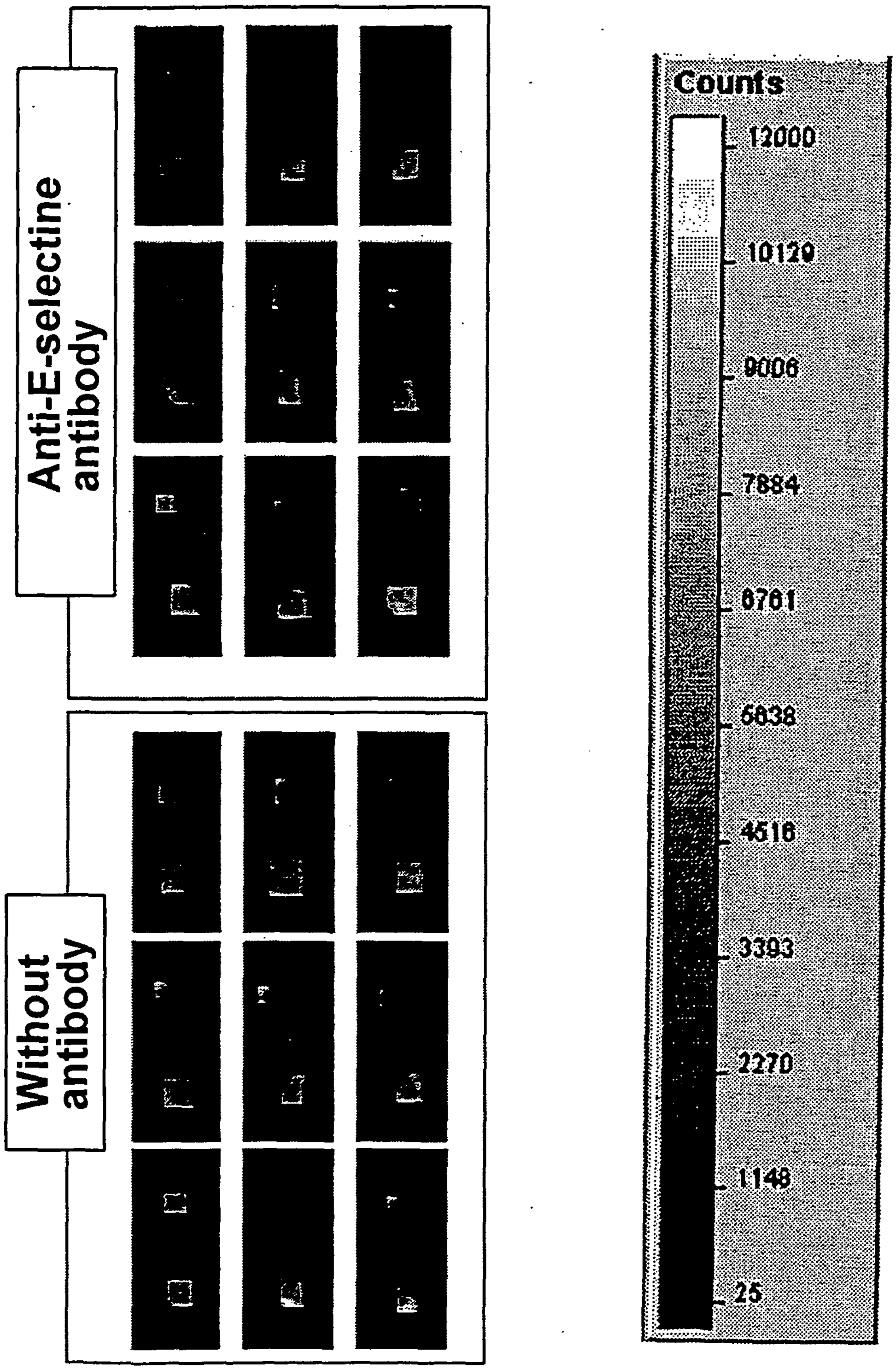
Anti-E-selectine antibody
Without antibody
Counts
12000
10129
9006
7884
6761
5638
4516
3393
2270
1148
25

Fig.8A

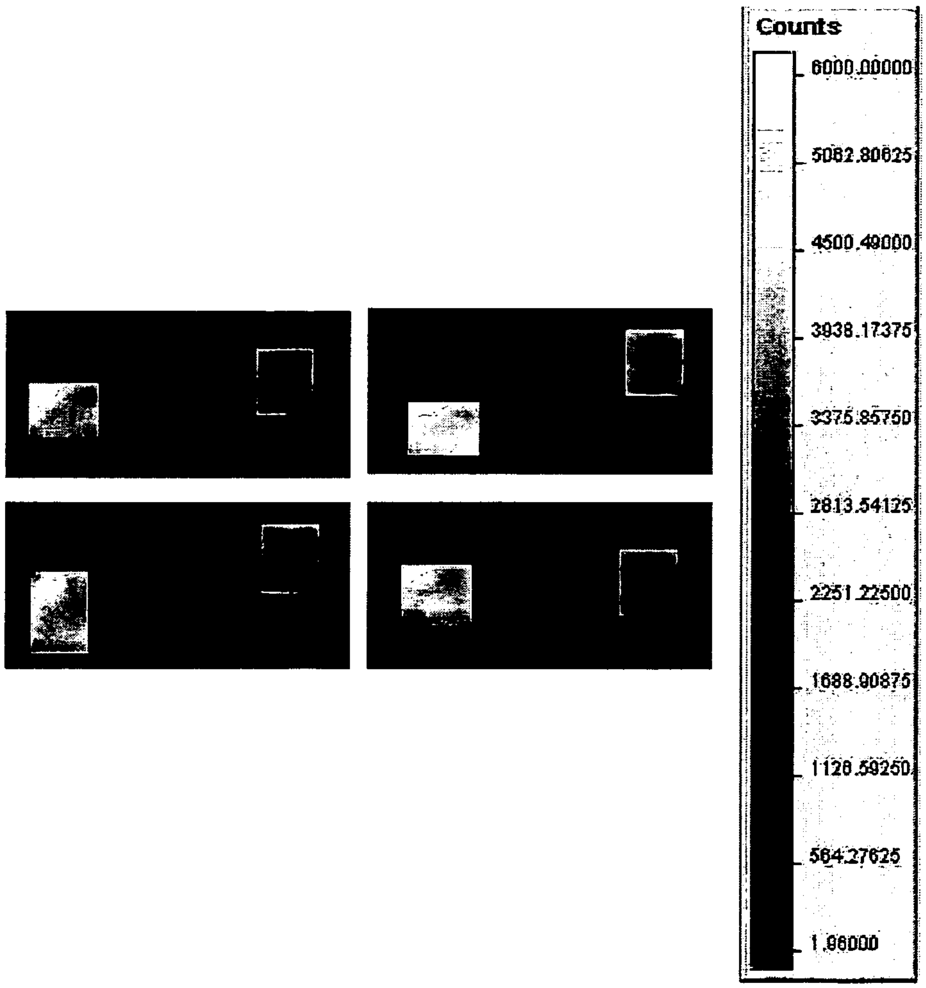
Counts
6000.00000
5062.80625
4500.49000
3938.17375
3375.85750
2813.54125
2251.22500
1688.90875
1126.59250
564.27625
1.96000

Fig.8B

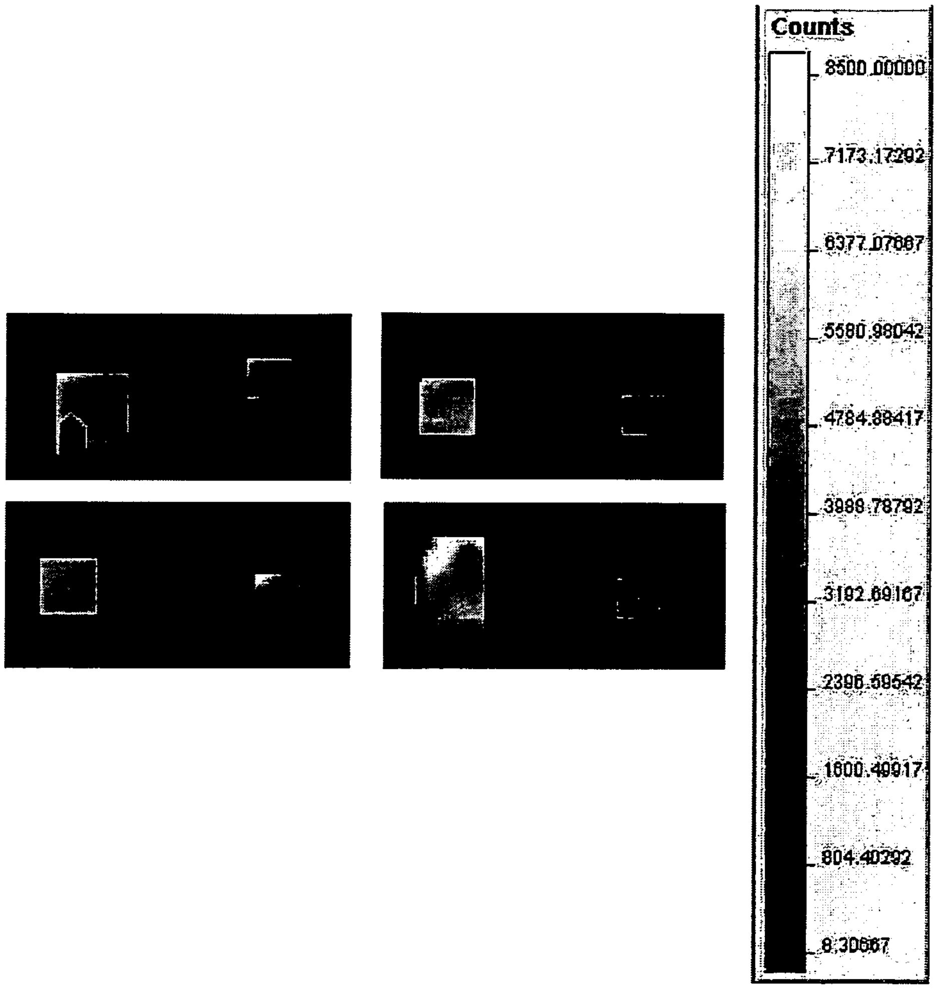
Counts
8500.00000
7173.17292
6377.07667
5580.98042
4784.88417
3988.78792
3192.69167
2396.59542
1600.49917
804.40292
8.30667

**Fig.8C**

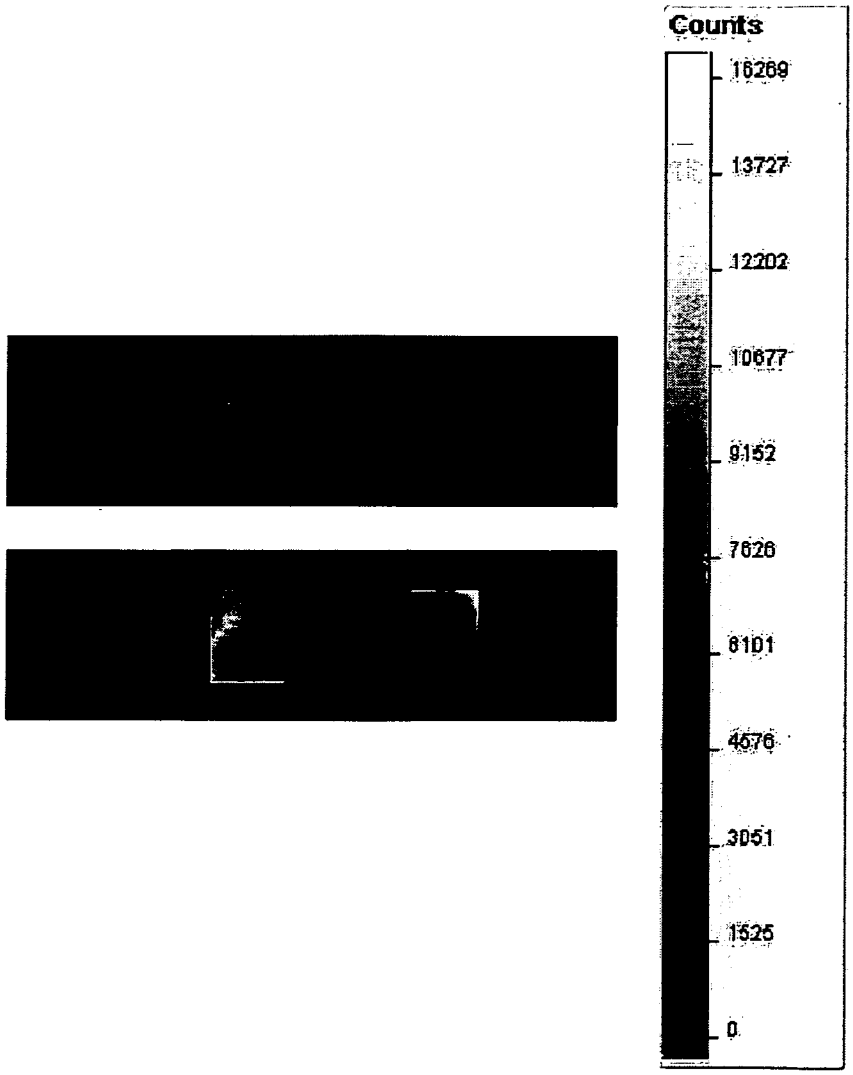
Counts
16269
13727
12202
10677
9152
7626
6101
4576
3051
1525
0

Fig.8D

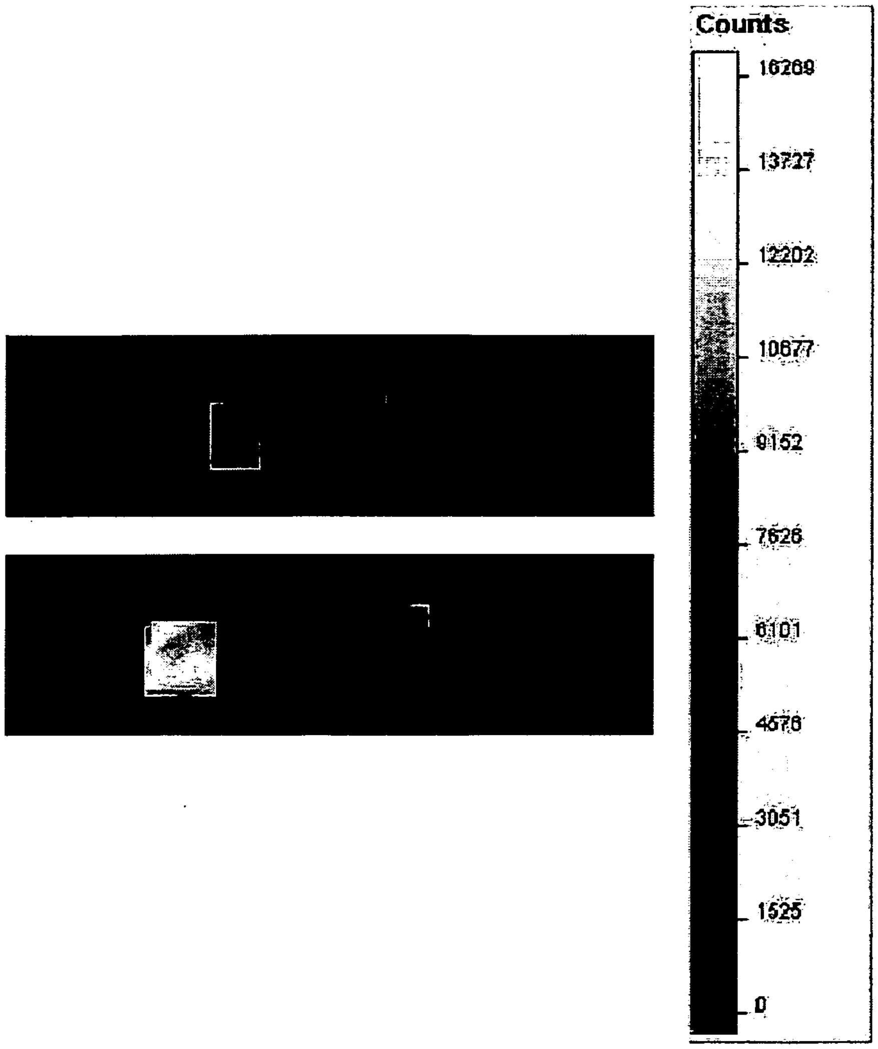
Counts
16269
13727
12202
10677
9152
7626
6101
4576
3051
1525
0

Fig.9

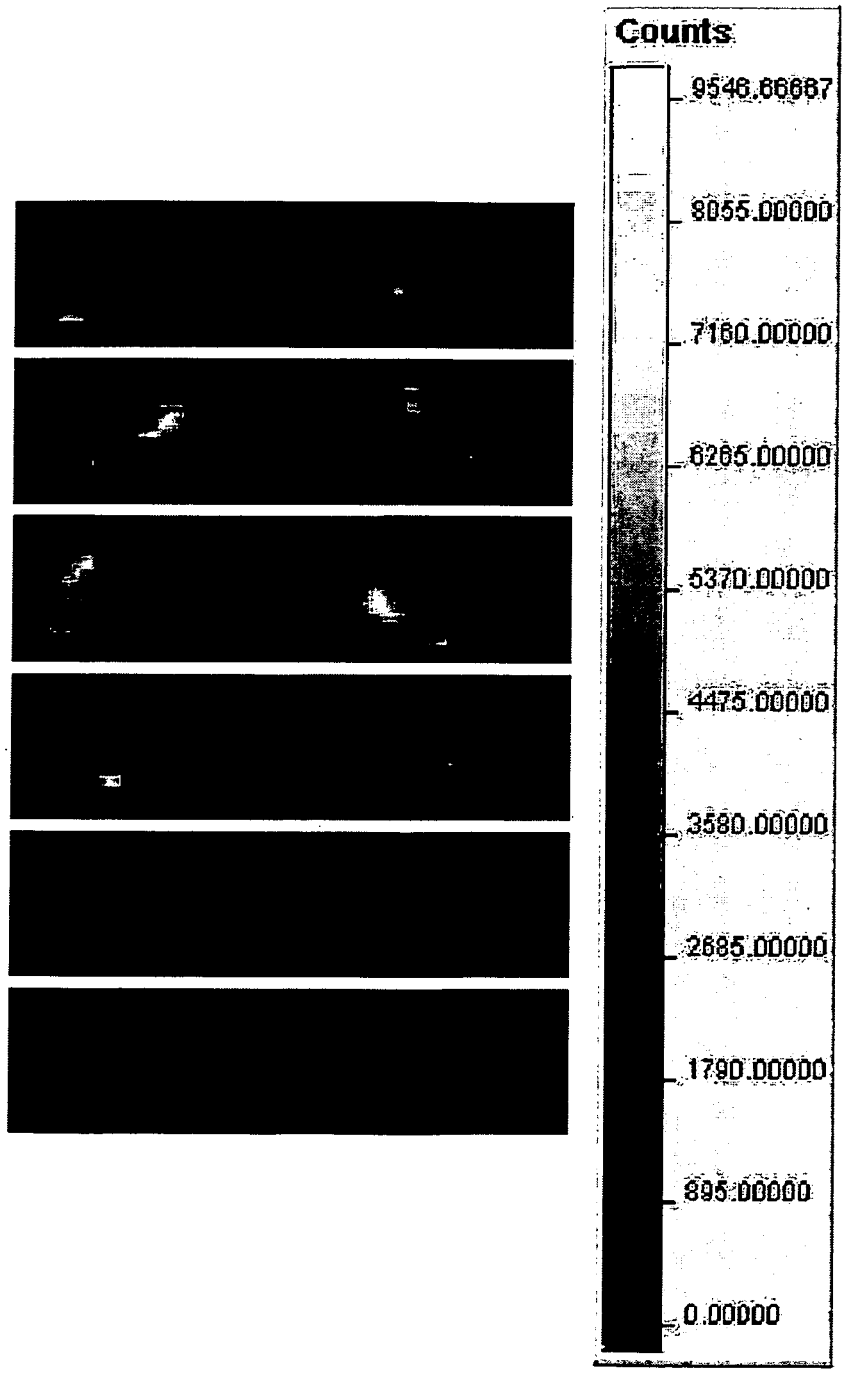
Counts
9546.66667
8055.00000
7160.00000
6265.00000
5370.00000
4475.00000
3580.00000
2685.00000
1790.00000
895.00000
0.00000

**Fig.10**

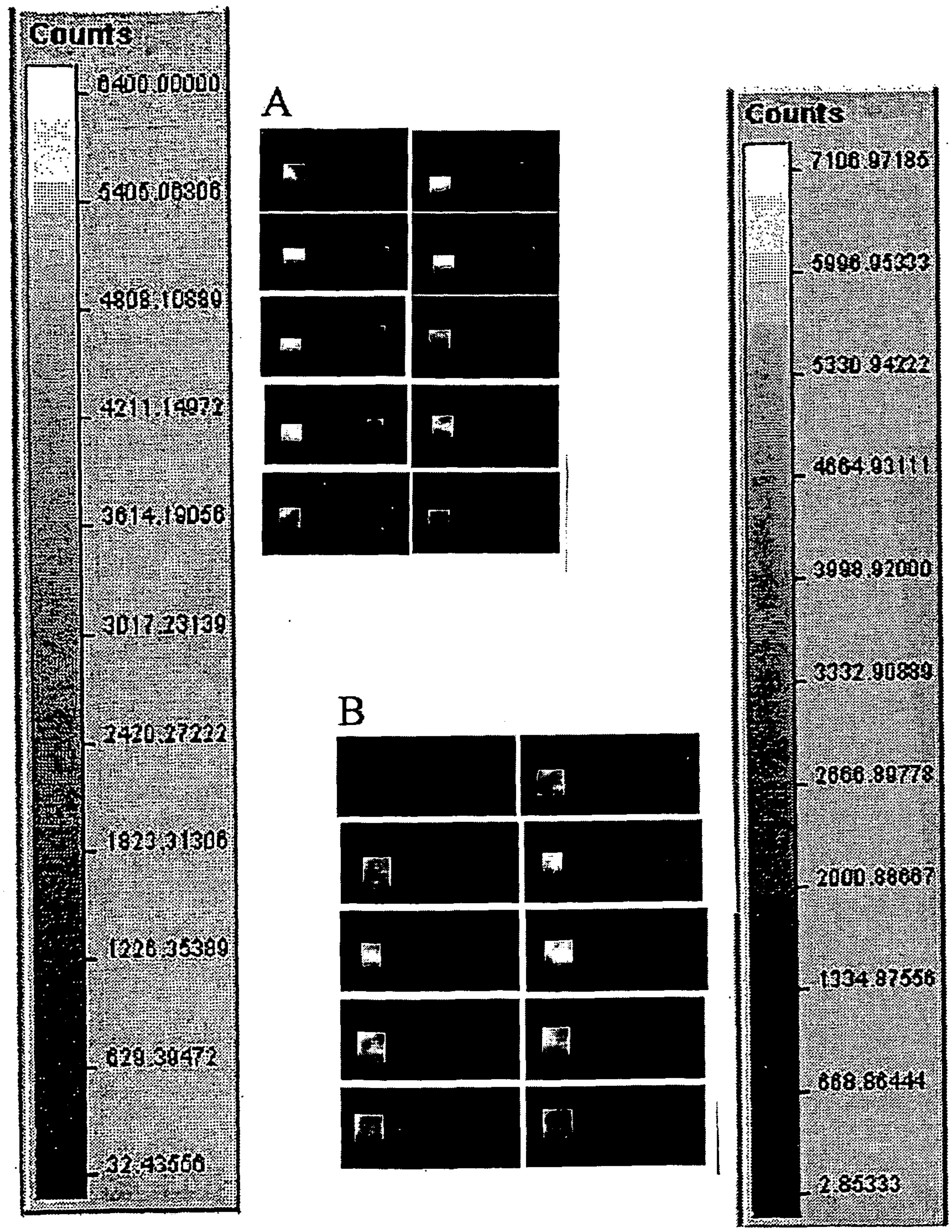
Counts
6400.00000
5405.06306
4808.10889
4211.14972
3614.19056
3017.23139
2420.27222
1823.31306
1226.35389
629.39472
32.43556
A
B
Counts
7106.97185
5996.95333
5330.94222
4664.93111
3998.92000
3332.90889
2666.89778
2000.88667
1334.87556
668.86444
2.85333

Fig.11

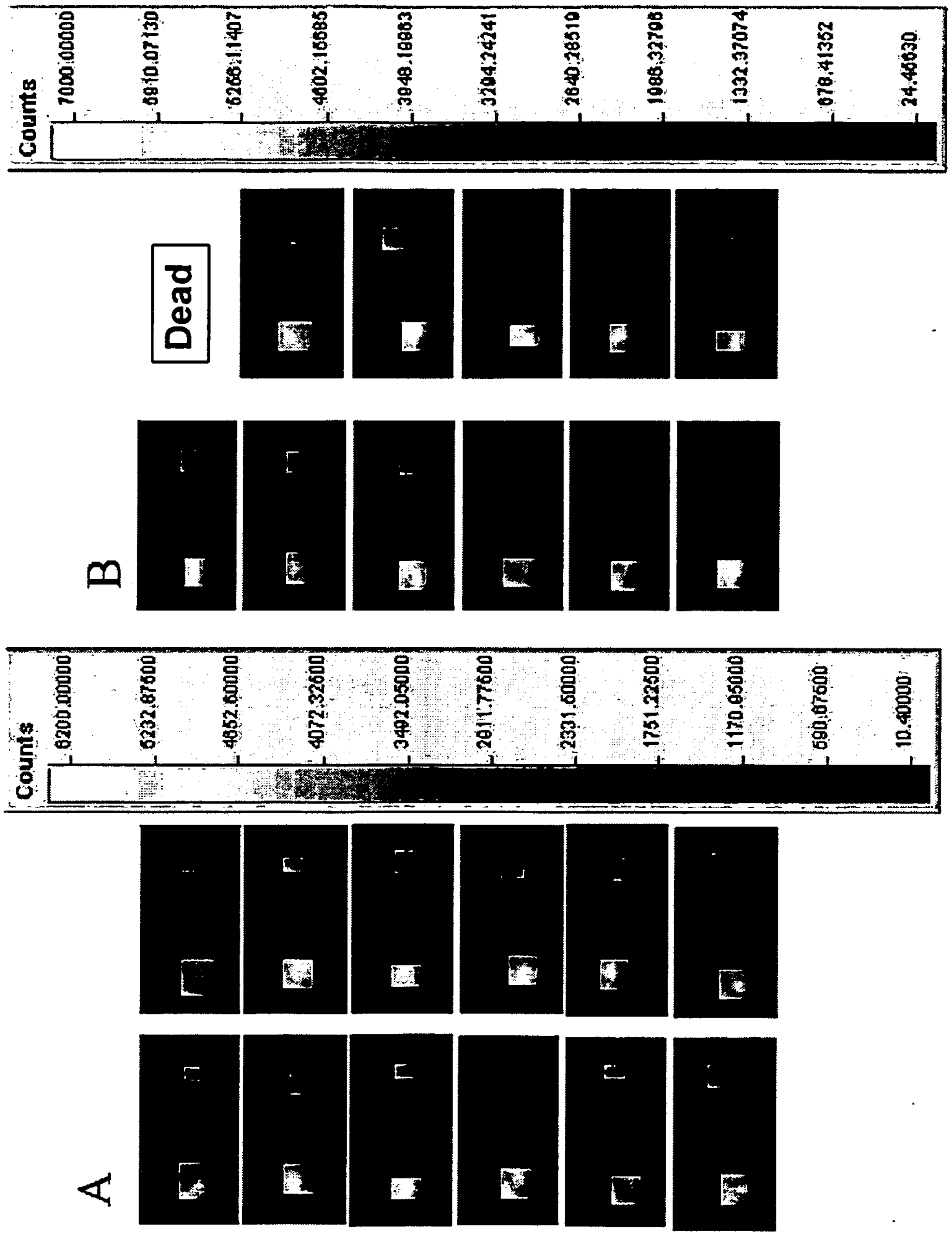

**Fig.12A**

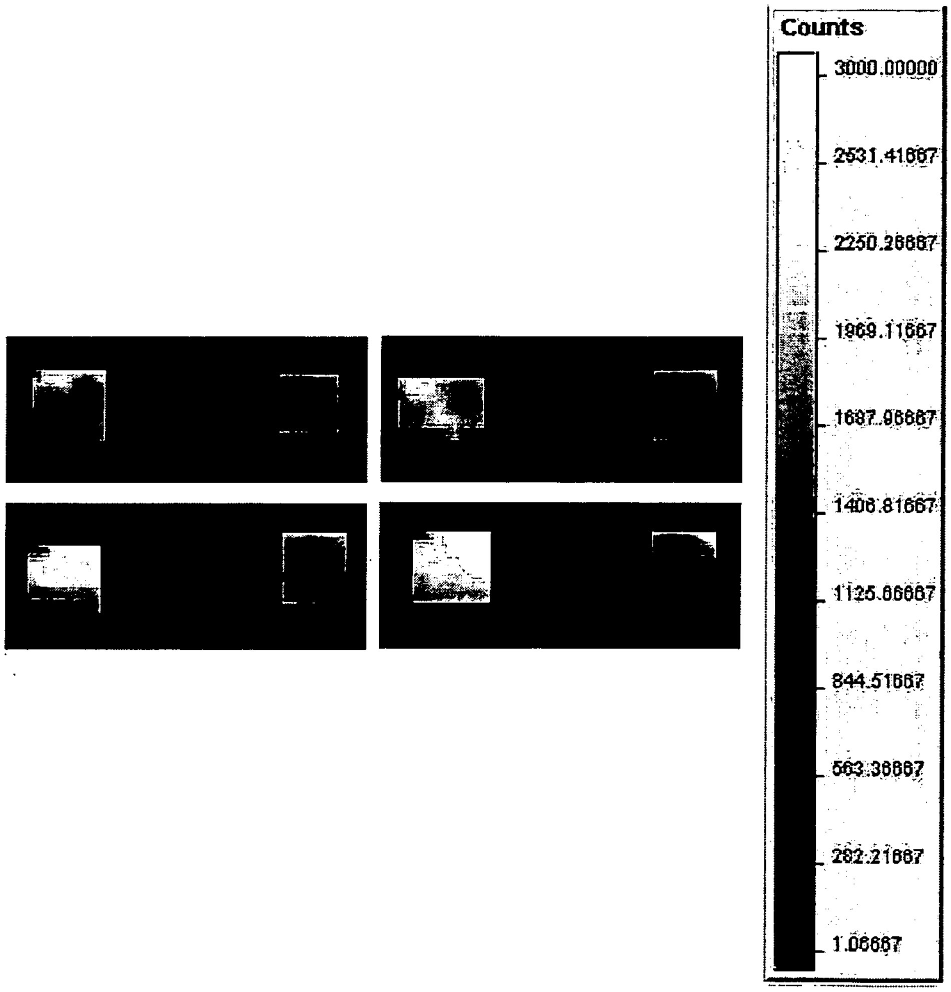
Counts
3000.00000
2531.41667
2250.26667
1969.11667
1687.96667
1406.81667
1125.66667
844.51667
563.36667
282.21667
1.06667

Fig.12B

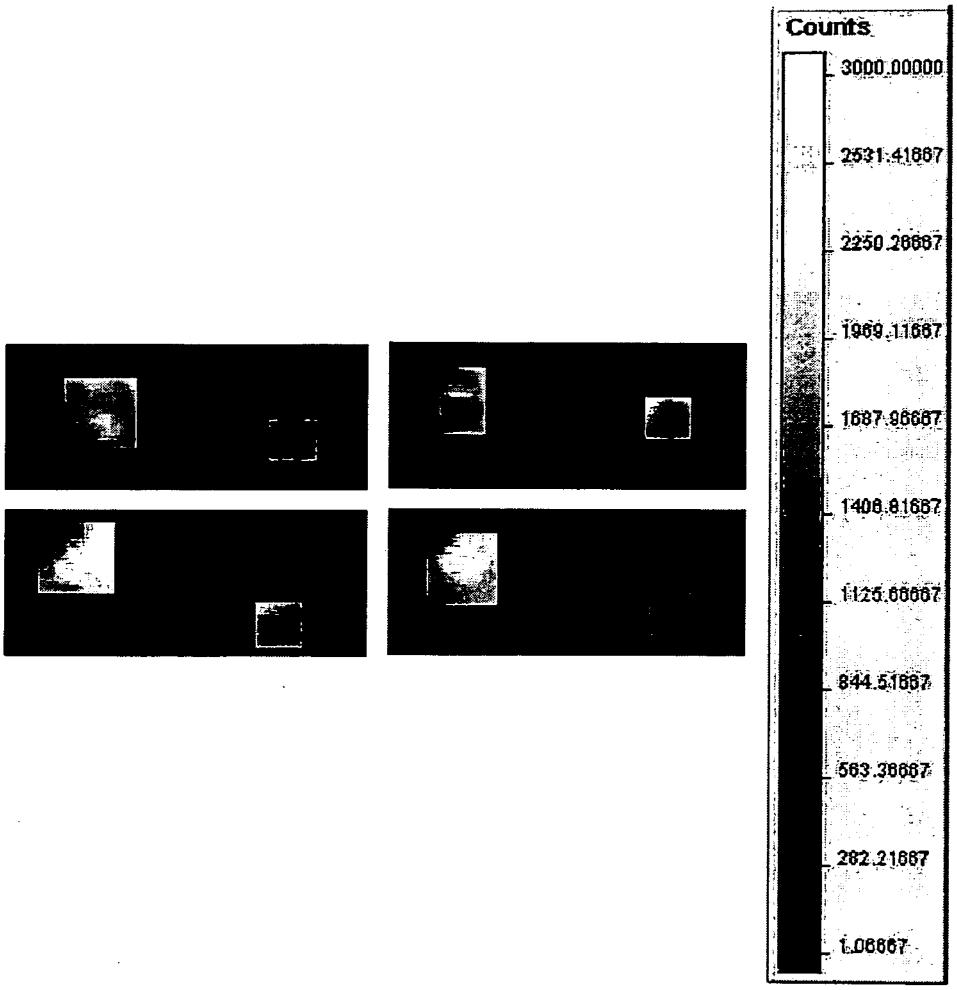
Counts
3000.00000
2531.41667
2250.26667
1969.11667
1687.96667
1406.81667
1125.66667
844.51667
563.36667
282.21667
1.06667

Fig.12C

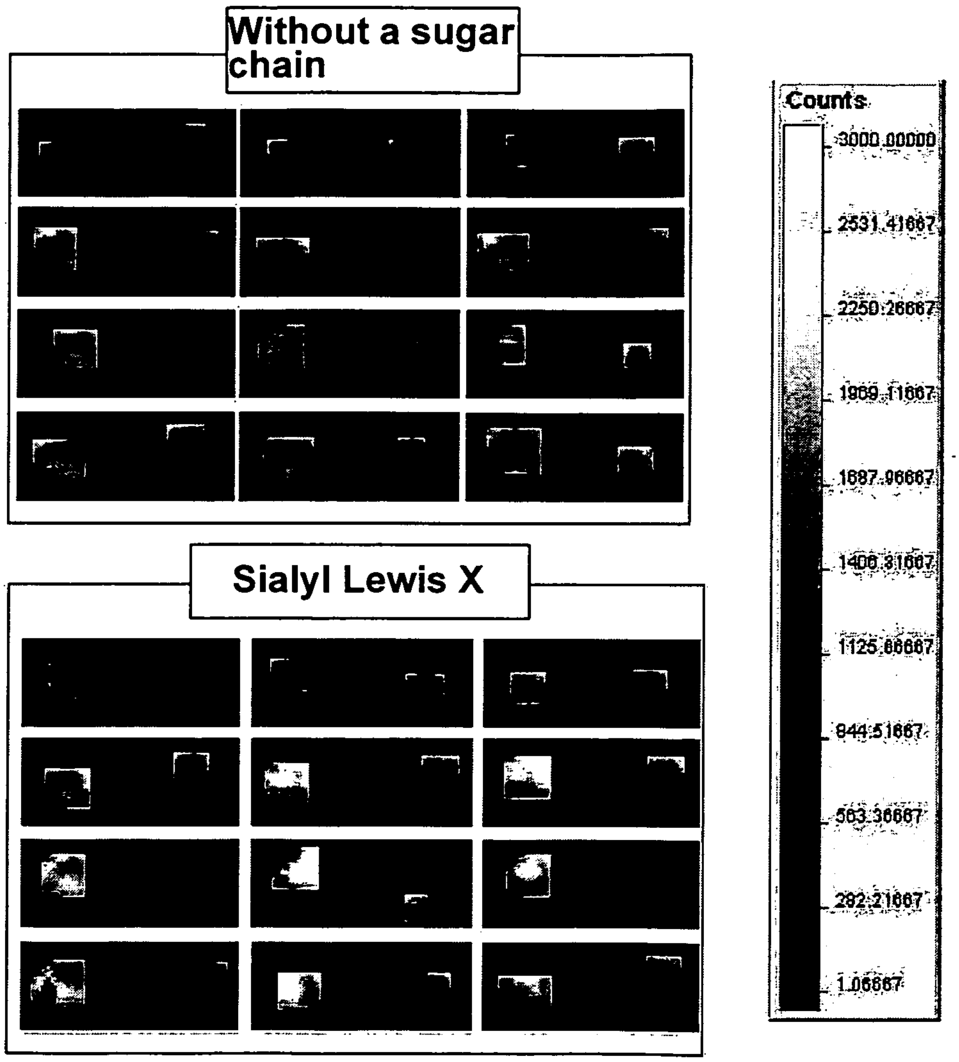
Without a sugar chain
Sialyl Lewis X
Counts
3000.00000
2531.41667
2250.26667
1969.11667
1687.96667
1406.81667
1125.66667
844.51667
563.36667
282.21667
1.06667

Fig.13A

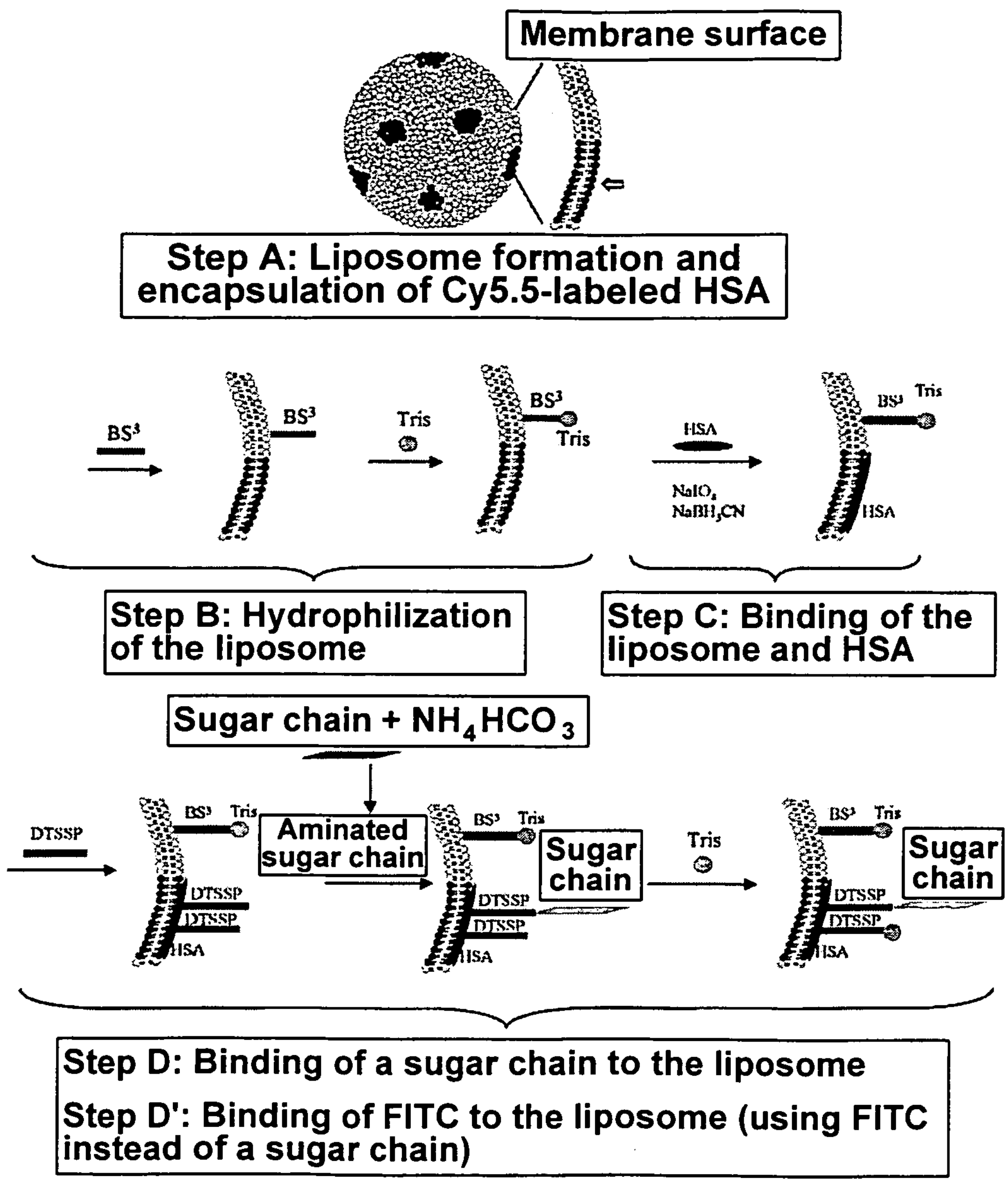
Membrane surface
Step A: Liposome formation and encapsulation of Cy5.5-labeled HSA
BS3
BS3
Tris
BS3
Tris
HSA
NaIO4
NaBH3CN
BS3
Tris
HSA
Step B: Hydrophilization of the liposome
Step C: Binding of the liposome and HSA
Sugar chain + NH4HCO3
DTSSP
BS3
Tris
DTSSP
DTSSP
HSA
Aminated sugar chain
BS3
Tris
Sugar chain
DTSSP
DTSSP
HSA
Tris
BS3
Tris
Sugar chain
DTSSP
DTSSP
HSA
Step D: Binding of a sugar chain to the liposome
Step D': Binding of FITC to the liposome (using FITC instead of a sugar chain)

Fig.13B

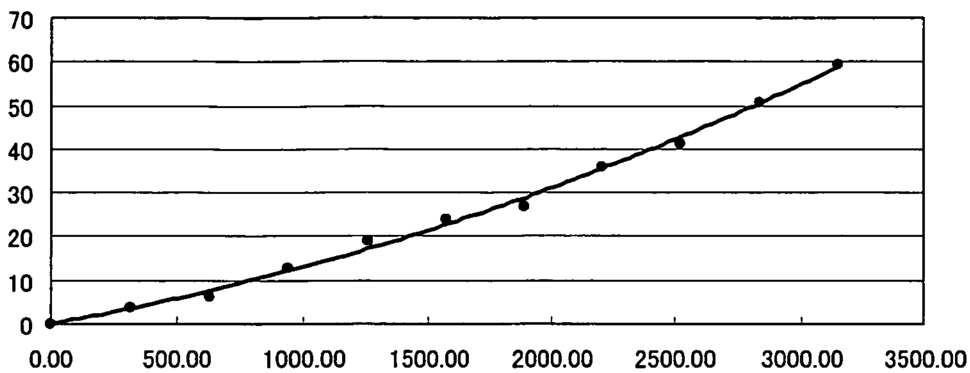
70
60
50
40
30
20
10
0
0.00
500.00
1000.00
1500.00
2000.00
2500.00
3000.00
3500.00

Fig.14

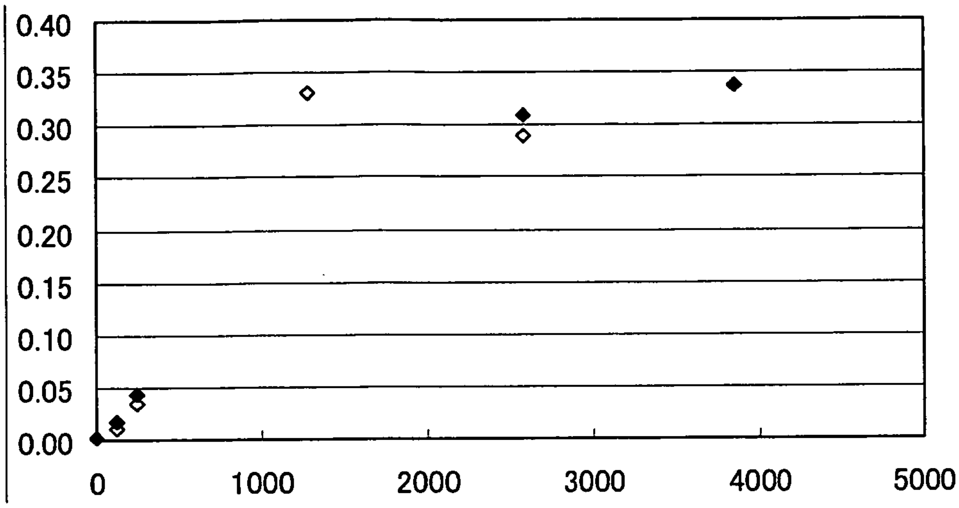
0.40
0.35
0.30
0.25
0.20
0.15
0.10
0.05
0.00
0
1000
2000
3000
4000
5000

Fig.15

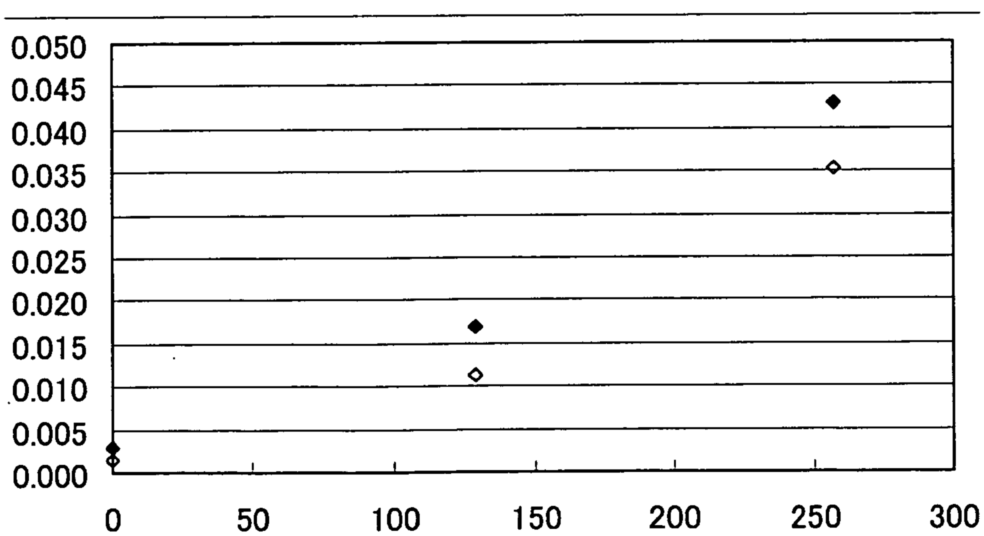
0.050
0.045
0.040
0.035
0.030
0.025
0.020
0.015
0.010
0.005
0.000
0
50
100
150
200
250
300

**Fig.16**

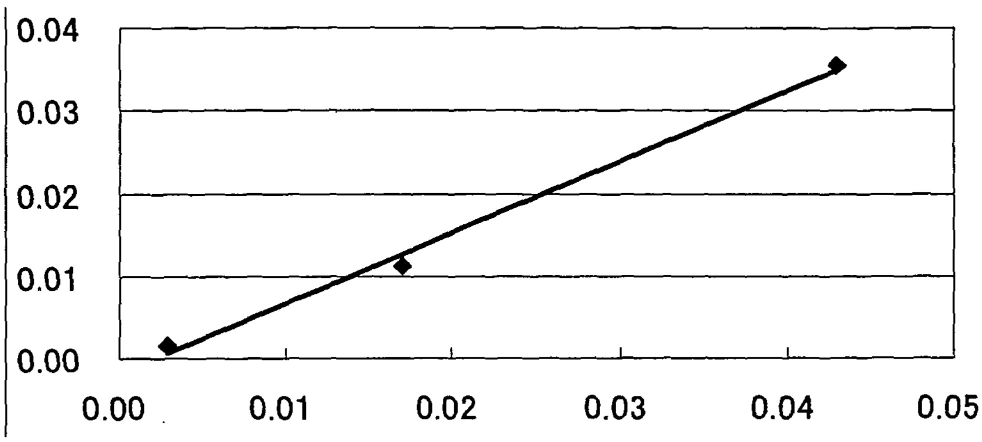

**Fig.17A**

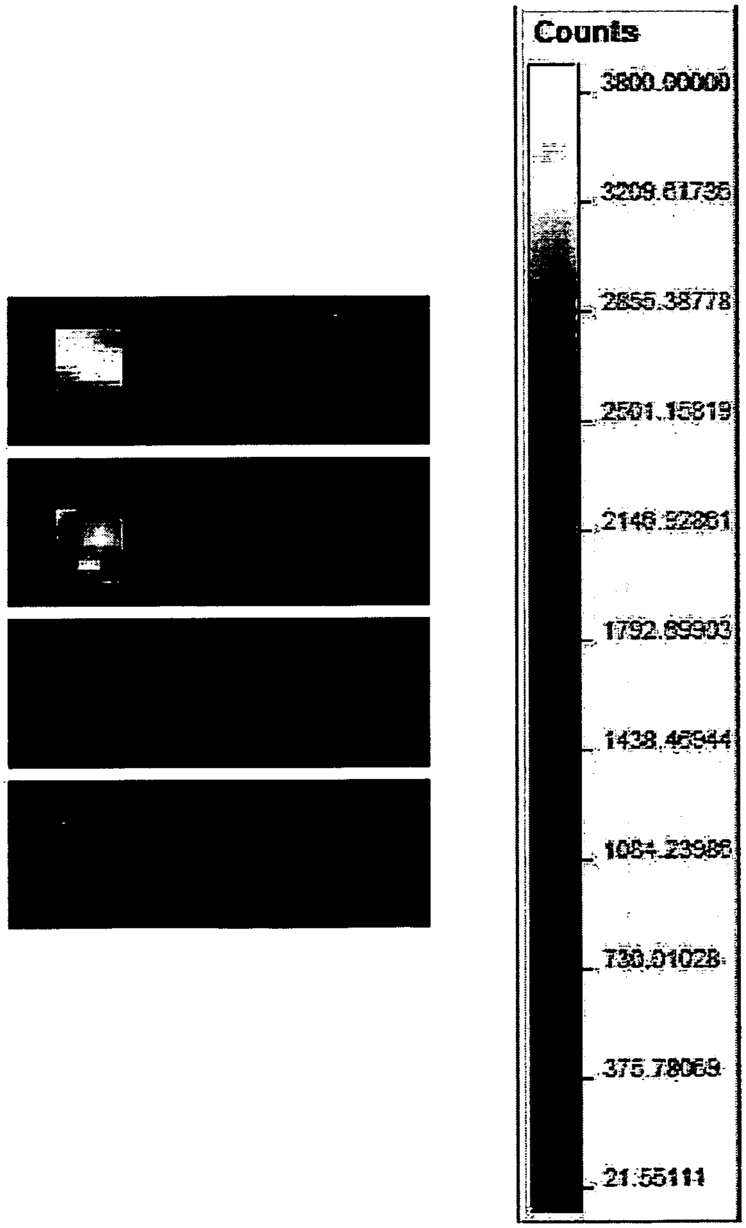
Counts
3800.00000
3209.61736
2855.38778
2501.15819
2146.92861
1792.69903
1438.46944
1084.23986
730.01028
375.78069
21.55111

Fig.17B

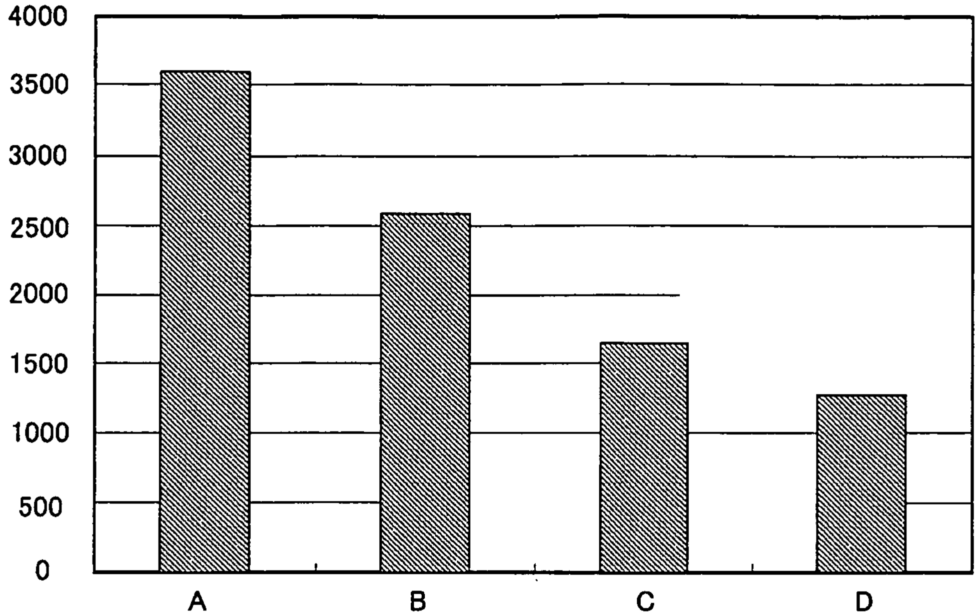

## INTERNATIONAL SEARCH REPORT

International application No.
PCT/JP2009/052814

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
A61K9/127, A61K31/22, A61K31/551, A61K31/573, A61K31/663, A61K31/722, A61K31/727, A61K33/18, A61K33/24, A61K38/00, A61K38/46, A61K47/24, A61K47/26, A61K47/42, A61K48/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
JSTPlus/JMEDPlus/JST7580(JDreamII), CAplus(STN), REGISTRY(STN), Igaku·Yakugaku Yokoshu Zenbun Database

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 10-511944 A (Anstitu Du Rusherushe Furakutaru Soshiete A Resuponsabirite Rimite), 17 November, 1998 (17.11.98), Claims 1 to 4; page 5, line 16 to page 6, line 7; examples 2, 3 & US 6045809 A & EP 804225 A & WO 1996/021462 A1 | 1,2,18<br>3,6-17,19<br>4,5 |
| X<br>Y<br>A | Chihiro KAISE et al., "Shokubutsu Yurai no Sphingo To Shishitsu Narabini Steryl Glycoside o Gan'yu shita Liposome Seizai no Anteika to sono Oyo", Journal of SCCJ, 20 December, 2004 (20.12.04), Vol.38, No.4, pages 299 to 303, particularly, Abstract, 2. Jikken | 1,18<br>3,6-16<br>4,5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 May, 2009 (14.05.09) | 26 May, 2009 (26.05.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2009/052814

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 63-275522 A (Terumo Corp.),<br>14 November, 1988 (14.11.88),<br>Claims 1, 2; page 2, upper left column, lines 10 to 14<br>(Family: none) | 3,6-16<br>4,5 |
| Y<br>A | Hiroki FUJIKAWA et al., "Bunshi Nai Glucosyl-kao Riyo shita Phytosphingosine Gan'yu GM3 no Gosei", Dai 27 Kai The Japanese Society of Carbohydrate Research Nenkai Yoshishu, 10 July, 2007 (10.07.07), page 128, P1-83 | 3,6-17,19<br>4,5 |
| Y<br>A | WO 2007/018272 A1 (National Institute of Advanced Industrial Science and Technology),<br>15 February, 2007 (15.02.07),<br>Par. Nos. [0119], [0173], [0179], [0188], [0190] to [0193]<br>(Family: none) | 6-16<br>5 |
| Y<br>A | WO 2007/091661 A1 (National Institute of Advanced Industrial Science and Technology),<br>16 August, 2007 (16.08.07),<br>Par. Nos. [0057], [0075], [0083], [0186], [0198], [0199]; examples 7, 19<br>(Family: none) | 6-16<br>5 |

Form PCT/ISA/210 (continuation of second sheet) (April 2007)

**INTERNATIONAL SEARCH REPORT**

International application No.
PCT/JP2009/052814

Continuation of A. CLASSIFICATION OF SUBJECT MATTER
(International Patent Classification (IPC))

*A61K9/127*(2006.01)i, *A61K31/22*(2006.01)i, *A61K31/551*(2006.01)i, *A61K31/573*(2006.01)i, *A61K31/663*(2006.01)i, *A61K31/722*(2006.01)i, *A61K31/727*(2006.01)i, *A61K33/18*(2006.01)i, *A61K33/24*(2006.01)i, *A61K38/00*(2006.01)i, *A61K38/46*(2006.01)i, *A61K47/24*(2006.01)i, *A61K47/26*(2006.01)i, *A61K47/42*(2006.01)i, *A61K48/00*(2006.01)i

(According to International Patent Classification (IPC) or to both national classification and IPC)

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007091661 A
- JP 3924606 B
- JP 3882034 B
- WO 2007018272 A
- WO 2002081723 A
- JP 9031095 A
- JP 11042096 A
- JP 2004180676 A
- JP 2000302685 A


### Non-patent literature cited in the description


- **Hirai, M. ; Minematsu, H. ; Kondo, N. ; Oie, K. ; Igarashi, K. ; Yamazaki, N.** Accumulation of liposome with sialyl Lewis X to inflammation and tumor region: Application to in vivo bio-imaging. *Biochem. Biophys. Res. Commum.,* 2007, vol. 353, 553-558
- JIKKENKAGAKUKOZA. Maruzen, 1992
- YUKIKAGAKU JIKKEN NO TEBIKI. 1-5
- KAGAKU DOJIN, TORIATSUKAI CHUI SHIYAKU LABO GUIDE
- SEIRIKASSEI TOSA KENKYUHO SEIBUTSUKAGAKU JIKKENHO. Kodansha Scientific Ltd, 42
- SEIMITSU YUKIGOSEI JIKKENMANUAL. Japan Scientific Societies Press
- **Kameyama, A. ; Ishida, H. ; Kiso, M. ; Hasegawa, A.** *Cabohydr. Res.,* 1990, vol. 200, 269-285
- **Hasegawa, A. ; Nagahama, T. ; Ohki, H. ; Kiso, M.** *J. Carbohydr. Chem.,* 1992, vol. 11, 699-714
- **Ando, H. ; Ishida, H. ; Kiso, M. ; Hasegawa, A.** *Carbohydr. Res.,* 1997, vol. 300, 207-217
- **Ishida, H.-K. ; Ishida, H. ; Kiso, M. ; Hasegawa, A.** *Carbohydr. Res.,* 1994, vol. 260, C1-C6
- **Kenichi Hatanaka ; Shinichiro Nishimura ; Tatsuro Ouchi ; Kazukiyo Kobayashi.** TOSHITSU NO KAGAKU TO KOGYO. Kodansha Ltd, 1997
- LIPOSOME OYO NO SHINTENKAI - JINKO SAIBO NOKAIHATSUNIMUKETE. NTS, 2005, 33-45
- **Shoshichi Nojima.** Liposomes. Nankodo, 1988, 21-40
- **H. Kikuchi ; N. Suzuki et al.** *Biopharm. Drug Dispos.,* 1999, vol. 17, 589-605
- **Bangham et al.** *J.Mol.Bio1.,* 1964, vol. 8, 660-668
- New development in liposome Application-For development of an artificial cell. NTS, 2005, 33-45
- **Shoshichi NOJIMA.** Liposomes. Nankodo, 1988, 21-40
- **Paulsen, H.** *Angew. Chem. Int. Ed. Engl.,* 1982, vol. 21, 155
- **Wulff, G. ; Rohl, G.** *Angew. Chem. Int. Ed.,* 1974, vol. 13, 157
- **Nicolaou, K. C. ; Daines, R. A. ; Ogawa, Y. ; Chakraboty, T. K.** *J. Am. Chem. Soc.,* 1988, vol. 110, 4696
- **Jung, K. H. ; Muller, M. ; Schmidt, R. R.** *Chem. Rev.,* 2000, vol. 100, 4423-4442
- **Ito, Y. ; Ogawa, T.** *J. Am. Chem. Soc.,* 1997, vol. 119, 5562
- **Berg, R. V. ; Korevaar, C. ; Overkleeft, H. ; Marel, G. V. ; BoomJ. V.** *J. Org. Chem.,* 2004, vol. 69, 5699-5704
- **Alexander, M. ; Richard, J.K. T. ; Robert, J. W. ; Norman, Lewis.** *Synthesis,* 1994, 31-33
- **Murakami, T. ; Furusawa. K.** *Tetrahedron,* 2002, vol. 58, 9257-9263
- **Hiromune Ando ; Yusuke Koike ; Hideharu Ishida ; Makoto Kiso et al.** *Tetrahedron Letters,* 2003, vol. 44, 6883-6886
- **Numata, M. ; Sugimoto, M. ; Koike, K. ; Ogawa, T.** *Carbohydr. Res.,* 1990, vol. 203, 205-217
- **Borbas, A. ; Csavas, M. ; Szilagyi, L. ; Majer, G. ; Liptak, A.** *J. Carbohydr. Chem.,* 2004, vol. 23, 133-146
- **Ando, H. ; Koike, Y. ; Ishida, H. ; Kiso, M.** *Tetrahedron Lett.,* 2003, vol. 44, 6883-6886
- **Fuse, T. ; Ando, H. ; Imamura, A. ; Sawada, N. ; Ishida, H. ; Kiso, M. ; Ando, T. ; Li, S. C. ; Li Ii Y.-T.** *Glycoconj. J.,* 2006, vol. 23, 329-343
- **Yamazaki, N. ; Kodama, M. ; Gabius, H.-J.** *Methods Enzymol.,* 1994, vol. 242, 56-65